(19) Europäisches Patentamt
European Patent Office
Office européen des brevets

(11) EP 2 515 899 B1

(12) **EUROPEAN PATENT SPECIFICATION**

(45) Date of publication and mention of the grant of the patent:
**25.05.2016 Bulletin 2016/21**

(21) Application number: **10840169.6**

(22) Date of filing: **23.12.2010**

(51) Int Cl.:
***C12Q 1/68*** *(2006.01)* ***A61K 31/34*** *(2006.01)*
***A61P 9/00*** *(2006.01)*

(86) International application number:
**PCT/US2010/062023**

(87) International publication number:
**WO 2011/079273 (30.06.2011 Gazette 2011/26)**

(54) **METHODS AND COMPOSITIONS FOR CARDIOVASCULAR DISEASES AND CONDITIONS**

VERFAHREN UND ZUSAMMENSETZUNGEN FÜR HERZ-KREISLAUF-ERKRANKUNGEN UND -LEIDEN

PROCÉDÉS ET COMPOSITIONS POUR MALADIES ET AFFECTIONS CARDIOVASCULAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.12.2009 US 289932 P**

(43) Date of publication of application:
**31.10.2012 Bulletin 2012/44**

(73) Proprietor: **ARCA biopharma, Inc.**
**Broomfield, CO 80021 (US)**

(72) Inventors:
- **BRISTOW, Michael, R.**
  **Broomfield, CO 80021 (US)**
- **PORT, J., David**
  **Broomfield, CO 80021 (US)**

(74) Representative: **Vossius & Partner**
**Patentanwälte Rechtsanwälte mbB**
**Siebertstrasse 3**
**81675 München (DE)**

(56) References cited:
**US-A1- 2003 225 134** **US-A1- 2003 225 134**
**US-A1- 2008 161 389** **US-A1- 2009 075 956**
**US-A1- 2009 075 956** **US-B1- 6 569 618**
**US-B1- 6 569 618**

- **I. PHILIP ET AL: "G894T Polymorphism in the Endothelial Nitric Oxide Synthase Gene Is Associated With an Enhanced Vascular Responsiveness to Phenylephrine", CIRCULATION, vol. 99, no. 24, 22 June 1999 (1999-06-22) , pages 3096-3098, XP055064416, ISSN: 0009-7322, DOI: 10.1161/01.CIR.99.24.3096**
- **ANDREAS EISENHARDT ET AL: "ACE gene I/D and NOS3 G894T polymorphisms and response to sildenafil in men with erectile dysfunction", UROLOGY, vol. 62, no. 1, 1 July 2003 (2003-07-01), pages 152-157, XP055064417, ISSN: 0090-4295, DOI: 10.1016/S0090-4295(03)00137-7**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 515 899 B1

**Description**

## I. FIELD OF THE INVENTION

Embodiments of this invention are directed generally to biology, molecular genetics, and medicine. Certain embodiments are directed to methods and compositions involving identifying and/or treating a patient that would benefit from therapy with a compound called with S-(6-Nitro-oxi-hexahydro-furo[3,2-b]furan-3-1-il)thioacetate, which is also known as LA-419.

## II. BACKGROUND

Heart failure, or end-stage CV disease, affects approximately five million Americans. Epidemiologic studies indicate a higher prevalence of risk factors for heart failure among African Americans in the United States (Burt *et al.,* 1995). Recent analyses of heart failure clinical trials show that the mortality rate and hospitalization rate for African Americans are significantly higher than for non-African Americans.

The thin layer of cells that lines the interior surface of blood vessels is called the endothelium. This layer modulates vascular tone through the release of nitric oxide (NO), a potent vasodilator that regulates regional blood flow (Ignarro *et al.,* 1987; Rees *et al.,* 1989). A reduction in NO bioavailability contributes to elevated vascular resistance and loss of sensitivity to stimuli of vasodilation, hallmark features of hypertension (Paniagua *et al.,* 2001; Panza *et al.,* 1990; Taddei *et al.,* 1993). In addition, NO has well characterized vascular benefits such as inhibition of smooth muscle cell proliferation and migration, blocking adhesion of leukocytes to the endothelium, and preventing platelet aggregation (Harrison, 1997). Agents that directly stimulate NO release have been investigated for the prevention and treatment of cardiovascular (CV) diseases and conditions.

In the United States, African Americans exhibit a higher prevalence of CV risk factors, especially hypertension (Burt *et al.,* 1995). One theory is that this may be attributed to differences in vascular physiology, including reduced NO bioavailability. Support for this was provided by a clinical evaluation of brachial artery activity that demonstrated reduced responsiveness of conductance vessels to both endogenous and exogenous NO in healthy African Americans, as compared with age-matched Whites (Campia *et al.,* 2002). As an explanation of this, it was reported that there is low bioavailability of NO from endothelium of African Americans, despite much higher levels of endothelial-dependent NO synthase (eNOS) (Kalinowski *et al.,* 2004). The cellular basis for this paradox was the finding that excessive $O_2^-$ generation by NAD(P)H oxidase and uncoupled eNOS resulted in the loss of functional NO due to its reactivity with $O_2^-$, resulting in peroxynitrite ($ONOO^-$) formation, a potent oxidant. It has recently been demonstrated that nebivolol, unlike another $\beta_1$-selective inhibitor (atenolol), is able to effectively reduce nitroxidative stress and restore NO bioavailability in African Americans (Mason *et al.,* 2005).

BiDil is a heart failure drug that has been recently approved for treatment in African Americans. It consists of a fixed dose combination of isosorbide dinitrate and hydralazine. Isosorbide dinitrate is a direct NO donor, and extended use causes tolerance. Hydralazine lowers blood pressure by relaxing vascular smooth muscle cells. This may be caused by reductions in inositol triphosphate levels, a second messenger that stimulates calcium release from the sarcoplasmic reticulum of smooth muscle.

The vasodilating effect of hydralazine leads to 1) decreased arterial blood pressure (diastolic more than systolic); 2) decreased peripheral vascular resistance; and, 3) increased heart rate, stroke volume, and cardiac output. Hydralazine may increase renin activity in plasma, presumably caused by increased secretion of renin in response to reflex sympathetic release. This increase in renin activity results in the production of angiotensin II, a stimulus for aldosterone with consequent sodium reabsorption. Hydalazine also has putative antioxidant activity that may contribute to reduced loss of NO.

**[0007]** Nitrates such as isosorbide dinitrate are direct NO donors that are linked to activation of guanylate cyclase, a mediator of smooth muscle cell vasodilation. They have been widely used for decades in the treatment of angina pectoris and hypertension, but work independently of the endothelium. In the formulation of sodium nitroprusside, NO is coordinated as a nitrosyl group liganded to iron in a square bipyramidal complex that is released spontaneously at physiological pH. A major disadvantage associated with extended use of nitrates is tolerance, leading to enhanced vascular $O_2^-$ production (Münzel *et al.,* 1995). Thus, there is a need for therapies that can enhance NO levels in the vessel wall without causing tolerance and enhancing oxidative stress.

Derivatives of isosorbide mononitrate and its use as vasodilating agents with reduced tolerance have previously been described and, in particular, the use of S-(6-Nitro-oxihexahydro-furo(3,2-b]furan-3-1-il)thioacetate (LA-419) in the treatment of cardiovascular disorders has previously been disclosed (US 2003/225134 A1).

## SUMMARY OF THE INVENTION

The present invention relates to the embodiments characterized in the claims. The present disclosure also,

relates to the following items.

1. The compound S-(6-Nitro-oxi-hexahydro-furo[3,2-b]furan-3-1-il)thioacetate for use in treating a cardiovascular disease or condition, wherein an effective amount of a pharmaceutical composition comprising said compound is administered to the patient after the patient has been tested and determined to be homozygous wildtype (G/G) at position 894 in the endothelial nitric oxide synthase (NOS3) gene.

2. The compound for use according to item 1, wherein the cardiovascular disease or condition is peripheral arterial disease, heart failure, coronary heart disease, coronary spasm, myocardial infarction, atherosclerosis, or pulmonary arterial hypertension.

3. The compound for use according to item 1 or 2, wherein at least one dose of the composition is to be administered to the patient, wherein the composition comprises about 1-40 mg of the compound, or wherein the composition comprises about 5-20 mg of the compound.

4. The compound for use according to any one of items 1 to 3, wherein multiple doses of the composition are to be administered to the patient.

5. The compound for use according to item 4, wherein multiple doses of the composition in a 24 hour period or wherein 1 to 3 doses of the composition, wherein the composition comprises about 1-40 mg of the compound are to be administered to the patient.

6. The compound for use according to item 1, wherein the patient has been tested to determine the patient's genotype at position 894 in the NOS3 gene after having been provided or furnished a biological sample from the patient.

7. The compound for use according to item 1, wherein the patient's genotype at position 894 in the NOS3 gene has been determined after a test has been ordered.

8. S-(6-Nitro-oxi-hexahydro-furo[3,2-b]furan-3-1-il)thioacetate for use in treating a patient that has been diagnosed with a disease or condition associated with NO production, wherein said disease is an ischemic cardiovascular disorder, glaucoma, or an intestinal disorder, wherein an effective amount of S-(6-Nitro-oxi-hexahydro-furo[3,2-b]furan-3-1-il)thioacetate is administered to the patient after the patient has been tested and determined to be homozygous wildtype (G/G) at position 894 in the endothelial nitric oxide synthase (NOS3) gene.

9. S-(6-Nitro-oxi-hexahydro-furo[3,2-b]furan-3-1-il)thioacetate for use in increasing nitric oxide (NO) levels in a patient that has been diagnosed with a disease or condition associated with NO production, wherein said disease is an ischemic cardiovascular disorder, glaucoma, or an intestinal disorder, wherein an effective amount of said S-(6-Nitro-oxi-hexahydro-furo[3,2-b]furan-3-1-il)thioacetate is administered to the patient after the patient has been tested and determined to be homozygous wildtype (G/G) at position 894 in the endothelial nitric oxide synthase (NOS3) gene.

10. An in vitro method for identifying a patient suitable for treatment with S-(6-Nitro-oxihexahydro-furo[3,2-b]furan-3-1-il)thioacetate comprising determining whether the patient is homozygous wildtype (G/G) at position 894 in the endothelial nitric oxide synthase (NOS3) gene, wherein the patient is suitable for treatment with S-(6-Nitrooxi-hexahydro-furo[3,2-b]furan-3-1-il)thioacetate if the patient is homozygous wildtype (G/G) at position 894 in the NOS3 gene.

11. A method for diagnosing a patient as a suitable candidate for treatment with with S-(6-Nitro-oxi-hexahydro-furo[3,2-b]furan-3-1-il)thioacetate comprising assaying a sample from the patient to determine whether the patient is homozygous wildtype (G/G) at position 894 in the endothelial nitric oxide synthase (NOS3) gene and reporting if the patient homozygous wildtype (G/G) at position 894 in the NOS3 gene.

12. Use of a kit for identifying a patient suitable for treatment with S-(6-Nitro-oxihexahydro-furo[3,2-b]furan-3-1-il)thioacetate or for diagnosing a patient as a suitable candidate for treatment with with S-(6-Nitro-oxi-hexahydro-furo[3,2-b]furan-3-1-il)thioacetate, wherein said kit comprises in a suitable container a probe or at least one set of primers for identifying the NOS3 polymorphism at position 894.

13. The kit of item 12, wherein the probe comprises at least one nucleic acid of between 15 and 100 nucleotides of SEQ ID NO:1 and/or SEQ ID NO:3, wherein the probe includes the nucleotide at position 894 of the NOS3 gene.

14. The kit of item 12, wherein the primers comprise at least one set of primers for amplifying a region of sequence that includes position 894 of the NOS3 gene.

The ability to identify which drug to treat a patient with is critical to the proper treatment of that patient. The present disclosure relates to the diagnosis and treatment of patients having a cardiovascular disease or condition. It is disclosed that the treatment involves an NO donor drug. It is disclosed that the treatment involves S-(6-Nitro-oxihexahydro-furo[3,2-b]furan-3-1-il)thioacetate (LA-419) or a salt, metabolite, or derivative thereof. It is contemplated that any embodiment involving LA-419, may also or alternatively involve a salt or prodrug thereof in other embodiments recited herein.

There are disclosed methods for treating a cardiovascular disease or condition in a patient comprising administering to the patient an effective amount of pharmaceutical composition comprising the compound S-(6-Nitro-oxi-hexahydro-furo[3,2-b]furan-3-1-il)thioacetate. The present disclosure concerns a patient that has been tested and determined to be homozygous wildtype (G/G) at position 894 in the endothelial nitric oxide synthase (NOS3) gene. It is disclosed that a patient may be administered LA-419 after it is known that the patient is homozygous wildtype (G/G) at position 894 of the NOS3 gene.

There are also disclosed methods for treating a patient with S-(6-Nitro-oxihexahydro-furo[3,2-b]furan-3-1-il)thioacetate comprising administering to the patient an effective amount of S-(6-Nitro-oxi-hexahydro-furo[3,2-b]furan-3-1-il)thioacetate after the patient is tested and determined to be homozygous wildtype (G/G) at position 894 in the endothelial nitric oxide synthase (NOS3) gene.

There are also disclosed methods for increasing nitric oxide (NO) levels in a patient comprising administering to the patient an effective amount of S-(6-Nitro-oxi-hexahydrofuro[3,2-b]furan-3-1-il)thioacetate after the patient is tested and determined to be homozygous wildtype (G/G) at position 894 in the endothelial nitric oxide synthase (NOS3) gene.

There are also disclosed methods for evaluating whether LA-419 is an appropriate therapy for a patient who has been diagnosed with or has symptoms of a cardiovascular disease or condition. Any embodiment may involve determining whether the patient is homozygous wildtype at position 894 (G/G) in the NOS3 gene. This determination may involve directly determining the sequence by genotyping the patient based on a test performed on a biological sample containing nucleic acids from the patient that determines the sequence at position 894 in both alleles. This genotyping involves analyzing the nucleic acids in a sample using chemical techniques well known to those of skill in the art. In some embodiments, the determination is indirect and involves, for instance, reading the results of a report or database that reveals the sequence at that position is at both alleles (either by reporting the patient is homozygous for a residue-for example, G/G or T/T-or that the patient is heterozygous at that residue).

There are disclosed methods for predicting efficacy of LA-419 in a patient with a cardiovascular disease or condition or with symptoms thereof. There are also disclosed methods for diagnosing a patient as a suitable candidate for treatment with with S-(6-Nitro-oxi-hexahydro-furo[3,2-b]furan-3-1-il)thioacetate comprising assaying a sample from the patient to determine whether the patient is homozygous wildtype (G/G) at position 894 in the endothelial nitric oxide synthase (NOS3) gene and reporting if the patient homozygous wildtype (G/G) at position 894 in the NOS3 gene.

The cardiovascular disease or condition may be a chronic ischemic cardiovascular disorder. The cardiovascular disease or condition may also be peripheral arterial disease, heart failure, coronary heart disease, coronary spasm, myocardial infarction, atherosclerosis, or pulmonary arterial hypertension.

It is disclosed that the administration of an effective amount of LA-419 to the patient may be such that the patient may achieve a therapeutic benefit from LA-419. It is disclosed that the amount of LA-419 administered to a patient is sufficient to provide a serum level of a LA-419 metabolite of at most, at least, or about 10, 50, 100, 150, 200, 250, 300, 350, 400, 450, 500, 550, 600, 650, 700, 750, 800, 850, 900, 950, 1000 ng/ml and/or mg/ml, or any range derivable therein.

It is disclosed that in some methods, the patient is administered at least one dose of the composition. The dose in the composition may include about 1-40 mg of LA-419. It is disclosed that the patient may be administered at least one dose of the composition, wherein the composition comprises about 5-20 mg of the compound. The patient may be administered multiple doses of the composition. It is contemplated that the patient may be administered multiple doses of the composition in a 24 hour period. Embodiments may involve a patient who is administered 1, 2, 3 or 4 doses of the composition, wherein the composition comprises about 1-40 mg of the compound. It is disclosed that these doses may be administered in a 24 hour period

or in a day. The NO donor, such as LA-419, can be administered at a dose of about, at least about, or at most about 0.01, 0.02, 0.03, 0.04, 0.05, 0.06, 0.07, 0.08, 009, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35,40, 50, 60, 70, 80, 90, 100, 110, 120, 130, 140, or 150 mg or mg/kg including all values and ranges there between. It is also disclosed that LA 419 or an NO donor may be administered 1. 2, 3, 4, 5, 6 or more times a day, a week, or a month, including all values and ranges there between. Alternatively, the drug may be administered on an as needed basis based on the patient's symptoms.

It is disclosed that an LA-419 metabolite may be administered to the patient. It is disclosed that the amount of LA-419 metabolite is the same as the dosage and regimen for LA-419. It is disclosed that the dosage may be 0.5x, 1.5x,

2x, 2.5x, 3x, 4x or more greater than the dosage of LA-419.

Obtaining, providing, or furnishing a biological sample from the patient for testing to determine the patient's genotype at position 894 in the NOS3 gene is disclosed. A biological sample can be a blood sample, a buccal smear, a tissue sample, or a primary culture of somatic cells from the patient. In certain aspects analyzing the sample comprises performing nucleic acid sequencing, restriction digestion, allele-specific nucleic acid amplification, single-stranded conformational polymorphism analysis, or allele specific hybridization analysis. The methods described herein can further comprising preparing a report containing information regarding the genotype of one or more NOS3 genes of the patient. In a further aspect the patient is determined to be homozygous wildtype at position 894 in the NOS3 gene and is subsequently treated with LA-419. The patient may be determined to be heterozygous or homozygous T/T at position 894 of the NOS3 gene. It is also disclosed that a patient is not treated with LA-419 and may be treated with an alternative NO donor.

In some methods, a step of ordering a test that determines the patient's genotype at position 894 in the NOS3 gene is disclosed. It is also disclosed that the test results may be obtained by or provided to a clinician, such as one who is considered LA-419 treatment for a patient.

The present disclosure also relates to patients who have been diagnosed with a disease or condition associated with NO production. It is also disclosed that la patient has been diagnosed with a cardiovascular disease or condition and/or the patient is exhibiting one or more symptoms of a cardiovascular disease or condition. The disease may be an ischemic cardiovascular disorder, glaucoma, or an intestinal disorder.

It is disclosed that the nucleic acid sequence can be determined using nucleic acid amplification, nucleic acid hybridization, restriction fragment length polymorphism (RFLP) analysis, single stranded conformational polymorphism (SSCP) analysis, nucleic acid sequencing, denaturing high performance liquid chromatography, comparative genome hybridization, and/or Southern blotting. In certain aspects nucleic acid amplification comprises polymerase chain reaction amplification or ligase chain reaction amplification. In a further aspect nucleic acid hybridization detection method comprises an allele specific oligonucleotide probe or a microarray of nucleic acid probes.

An isolated nucleic acid sequence is disclosed comprising 7, 8, 9, 10, 11, 12, 13, 14, 15, 16, 17, 18, 19, 20, 21, 22, 23, 24, 25, 26, 27, 28, 29, 30, 31, 32, 33, 34, 35,36,37, 38, 39, 40, 41, 42, 43, 44, 45, 46, 47, 48, 49, 50, 51, 52, 53, 54, 55, 56, 57, 58, 59, 60, 65,70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 600, 700, 800, 900, 1000 or more consecutive nucleotides of SEQ ID NO:1 or SEQ ID NO:3, including all values and ranges there between.

An amplification primer pair comprising two oligonucleotides that amplify a nucleic acid segment comprising nucleotide 894 of the human NOS3 gene, such as in (SEQ ID NO:1 or SEQ ID NO:3, or a nucleic acid that is 85, 90, 95, 98, or 100% identical to SEQ ID NO: 1 or 3 is disclosed. A primer may comprise all or part of any of SEQ ID NOs:1, 3, or any of 5-10. For example, a first primer can comprise the nucleic acid sequence of SEQ ID NO:5. A second primer can comprise the nucleic acid sequence of SEQ ID NO:6.

A nucleic acid probe that specifically hybridizes to a NOS3 nucleic acid comprising nucleotide 894 of SEQ ID NO:1 or SEQ ID NO:3 or a nucleic acid that is, is at least, or is at most, 85, 90, 95, 98, or 100% (or any range derivable therein) identical to one or both sequences is disclosed. The nucleic acid probe may be labeled. In a further aspect the nucleic acid probe is detectable upon binding or hybridization to a NOS3 nucleic acid comprising either the wildtype homozygous genotype or a genotype that is not homozygous wildtype at position 894 of the NOS3 gene.

There is also disclosed a kit for genotyping a NOS3 gene comprising oligonucleotides of at least 10 contiguous nucleotides of SEQ ID NO:1 or SEQ ID NO:3 that amplify a nucleic acid segment comprising the 894 polymorphism or wildtype sequence at position 894, or a nucleic acid probe that specifically hybridizes to a NOS3 gene comprising the non-wild-type sequence at position 894.

To achieve these methods, a doctor, medical practitioner, or their staff may obtain a biological sample for evaluation. The sample may be analyzed by the practitioner or their staff, or it may be sent to an outside or independent laboratory. The medical practitioner may be cognizant of whether the test is providing information regarding the patient's NOS3 genes, or the medical practitioner may be aware only that the test indicates directly or indirectly that the genotype of the patient reflects the homozygous wildtype sequence in the NOS3 genes (homozygous for wildtype sequence).

Similarly, the medical practitioner may be cognizant of whether the test is providing information regarding the patient's NOS3 genes or the medical practitioner may be aware only that the test indicates directly or indirectly that the genotype of the patient reflects the homozygous wildtype sequence (G/G), heterozygous alleles (G/T or T/G), or the homozygous nonwildtype alleles (T/T).

In any of these circumstances, the medical practitioner "knows" or identifies indirectly the relevant information that will allow him or her to determine whether a NO donor such as LA-419 is an appropriate medical treatment. It is contemplated that, for example, a laboratory conducts the test to determine that patient's genotype such its personnel also know the appropriate information. They may report back to the practitioner with the specific result of the test performed or the laboratory may simply report that LA-419 is an appropriate drug based on the laboratory results.

Moreover, through these different channels, the patient's genotype at position 894 of one or both NOS3 genes can be known.

There is also disclosed a tangible, computer-readable medium comprising a genotype of a subject, wherein the genotype exhibits the sequence at position 894 in one or both alleles of the NOS3 gene. It is disclosed that the medium comprising the genotype of the subject exhibits the presence of the wildtype sequence at position 894 of one or more NOS3 gene.

There are disclosed kits for determining the nucleotide sequence either directly or indirectly. Embodiments for directly determining the nucleotide sequence may involve a kit in a suitable container comprising a probe or at least one set of primers for identifying the NOS3 polymorphism at position 894. The probe may comprise at least one nucleic acid of between 15 and 100 nucleotides of SEQ ID NO:1 and/or SEQ ID NO:3, wherein the probe includes the nucleotide at position 894 of the NOS3 gene. Kits are
disclosed to involve primers comprising at least one set of primers for amplifying a region of sequence that includes position 894 of the NOS3 gene. Indirect methods may involve researching a database to determine the sequence or evaluating a protein sequence of the protein encoded by the patient's NOS3 alleles.

As used herein, the term "heart failure" is broadly used to mean any condition that reduces the ability of the heart to pump blood. As a result, congestion and edema develop in the tissues. Most frequently, heart failure is caused by decreased contractility of the myocardium, resulting from reduced coronary blood flow; however, many other factors may result in heart failure, including damage to the heart valves, vitamin deficiency, and primary cardiac muscle disease. Though the precise physiological mechanisms of heart failure are not entirely understood, heart failure is generally believed to involve disorders in several cardiac autonomic properties, including sympathetic, parasympathetic, and baroreceptor responses. The phrase "manifestations of heart failure" is used broadly to encompass all of the sequelae associated with heart failure, such as shortness of breath, pitting edema, an enlarged tender liver, engorged neck veins, pulmonary rates and the like including laboratory findings associated with heart failure.

The term "treatment" or equivalents encompasses the improvement and/or reversal of the symptoms of heart failure (*i,e.,* the ability of the heart to pump blood). "Improvement in the physiologic function" of the heart may be assessed using any of the measurements described herein (*e.g.,* measurement of ejection fraction, fractional shortening, left ventricular internal dimension, heart rate, etc.), as well as any effect upon subject's survival.

The term "dilated cardiomyopathy" refers to a type of heart failure characterized by the presence of a symmetrically dilated left ventricle with poor systolic contractile function and, in addition, frequently involves the right ventricle.

As used herein, the term "cardiac hypertrophy" refers to the process in which adult cardiac myocytes respond to stress through hypertrophic growth. Such growth is characterized by cell size increases without cell division, assembling of additional sarcomeres within the cell to attempt to increase force generation, and an activation of a fetal cardiac gene program that inherently reduces myocardial function. Cardiac hypertrophy is often associated with increased risk of morbidity and mortality, and thus studies aimed at understanding the molecular mechanisms of cardiac hypertrophy could have a significant impact on human health.

As used herein, the term "genotype" refers to the actual genetic make-up of an organism, while "phenotype" refers to physical traits displayed by an individual (responsiveness to LA-419 for the treatment of a chonic ischemic cardiovascular disease or condition).

Other embodiments are discussed throughout this application. Any embodiment discussed with respect to one aspect of the invention applies to other aspects of the invention as well and vice versa. The embodiments in the Example section are understood to be embodiments of the invention that are applicable to all aspects of the invention.

The terms "inhibiting," "reducing," or "prevention," or any variation of these terms, when used in the claims and/or the specification includes any measurable decrease or complete inhibition to achieve a desired result.

The use of the word "a" or "an" when used in conjunction with the term "comprising" in the claims and/or the specification may mean "one," but it is also consistent with the meaning of "one or more," "at least one," and "one or more than one."

It is contemplated that any embodiment discussed herein can be implemented with respect to any method or composition of the invention, and vice versa. Furthermore, compositions and kits of the invention can be used to achieve methods of the invention.

Throughout this application, the term "about" is used to indicate that a value includes the standard deviation of error for the device or method being employed to determine the value.

The use of the term "or" in the claims is used to mean "and/or" unless explicitly indicated to refer to alternatives only or the alternatives are mutually exclusive, although the disclosure supports a definition that refers to only alternatives and "and/or." It is also contemplated that anything listed using the term "or" may also be specifically excluded.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Other objects, features and advantages of the present invention will become apparent from the following detailed description.

## DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

**FIG. 1.** Schematic diagram of a NO nanosensor placed in close proximity to the surface of a single endothelial cell. The nanosensor measures the levels of NO, $O2^-$, and $ONOO^-$ from the intact endothelium in real time. The sensors are made by depositing a sensing material on the tip of carbon fiber with a diameter of approximately 0.5 μm. The fibers are sealed with nonconductive epoxy and electrically connected to wires (gold, copper) with conductive silver epoxy.

**FIG 2.** Comparative effects of LA-419 and ISDN on NO release from HUVECs isolated from non-Hispanic white donors. Cells were incubated with LA-419 or ISDN, each at 500 nM, for 12 hours prior to stimulation with CaI (1.0 μM). Values are reported as mean ± S.D. (N=5). **p*<0.05 vs. control (Dunnett multiple comparisons test; overall ANOVA: *p*=0.0015; F=10.326); †*p*=0.0129 vs. LA-419 treatment (Student t-test).

**FIG. 3.** Comparative effects of LA-419 and ISDN on ONOO- release from HUVECs isolated from non-Hispanic white donors. Cells were incubated with LA-419 or ISDN, each at 500 nM, for 12 hours prior to stimulation with CaI (1.0 μM). Values are reported as mean ± S.D. (N=5). **p*<0.01 vs. control (Dunnett multiple comparisons test; overall ANOVA: *p*<0.0001; F=34.909). Student t-test analysis of isosorbide dinitrate vs. LA-419 treatment: *p*=0.0995.

**FIG. 4.** Comparative effects of LA-419 and ISDN on the ratio of $NO/ONOO^-$ release from HUVECs isolated from non-Hispanic white donors. Cells were incubated with LA-419 or ISDN, each at 500 nM, for 12 hours prior to stimulation with CaI (1.0 μM). Values are reported as mean ± S.D. (N=5). **p*<0.01 vs. control (Dunnett multiple comparisons test; overall ANOVA: *p*<0.0001; F=20.154); †*p*=0.0024 vs. LA-419 treatment (Student t-test).

**FIG. 5.** Comparative effects of LA-419 and ISDN on NO release from HUVECs isolated from African American donors. Cells were incubated with LA-419 or ISDN, each at 500 nM, for 12 hours prior to stimulation with CaI (1.0 μM). Values are reported as mean ± S.D. (N=5). **p*<0.01 vs. control (Dunnett multiple comparisons test; overall ANOVA: *p*=0.0036; F=8.361); †*p*=0.0252 vs. LA-419 treatment (Student t-test).

**FIG. 6**. Comparative effects of LA-419 and ISDN on $ONOO^-$ release from HUVECs isolated from African American donors. Cells were incubated with LA-419 or ISDN, each at 500 nM, for 12 hours prior to stimulation with CaI (1.0 μM). Values are reported as mean ± S.D. (N=5). **p*<0.01 vs. control (Dunnett multiple comparisons test; overall ANOVA: *p*<0.0001; F=21.422). Student t-test analysis of isosorbide dinitrate vs. LA-419 treatment: *p*=0.4034.

**FIG. 7.** Comparative effects of LA-419 and ISDN on the ratio of $NO/ONOO^-$ release from HUVECs isolated from African American donors. Cells were incubated with LA-419 or ISDN, each at 500 nM, for 12 hours prior to stimulation with CaI (1.0 μM). Values are reported as mean ± S.D. (N=5). **p*<0.01 vs. control (Dunnett multiple comparisons test; overall ANOVA: *p*=0.0001; F=17.921); †*p*=0.0476 vs. LA-419 treatment (Student t-test).

## DETAILED DESCRIPTION OF THE INVENTION

Treatment with drugs that act as NO donors is widespread. However, their efficacy is limited because of the tolerance they induce in patients. Ideally, a drug that did not induce the tolerance currently observed would be used to treat patients in need of the effects of nitric oxide. As shown below, S-(6-Nitro-oxi-hexahydro-furo[3,2-b]furan-3-1-il)thioacetate (LA-419) is demonstrated to have these ideal properties. Compositions and methods described herein involve treatment of patients with LA-419, particularly, patients whose genotypye is homozygous wildtype (G/G) at position 894 in the endothelial nitric oxide-synthase gene, NOS3.

The present invention generally relates to determining a NOS3 polymorphism in an individual and determining responsiveness of the patient to treatment with LA-419. It is disclosed that efficacy of LA-419 can be predicted based on the patient's genotype. Specifically,
determining the genotype for an individual at the NOS3 gene (*e.g.,* determining if the subject is homozygous wildtype at position 894) is disclosed.

Pharmacogenomics allows a clinician or physician to target prophylactic or therapeutic treatments to individuals who will most benefit from the treatment and to avoid treatment of individuals who will experience symptomatic side effects. Thus, a physician or clinician may consider applying knowledge obtained in relevant pharmacogenomics analysis in determining whether to administer LA-419 as well as whether to modify the dosage, regimen, and/or therapeutically effective amounts to be administered so as to attain the effect desired by the treatment. It is disclosed that a physician or clinician may alter treatment of the subject by adding an additional therapy or using an alternative therapy to treatment with LA-419.

## I. TREATMENT OF CARDIOVASCULAR DISEASES AND CONDITIONS

Treatment of cardiovascular diseases and conditions, which is generally understood to refer to diseases, conditions, or disorders involving the heart or blood vessels, includes a variety of options, such as statins, diuretics, anticoagulants, beta blockers, vasodilators, ACE inhibitors, or calcium channel blockers. Vasodilators cause a relaxation of the smooth muscle that surrounds a blood vessel. It is achieved primarily by either lowering intracellular calcium concentration or dephosphorylating myosin. One way to achieve vasodilation is to induce nitric oxide.

### A. NO Donors

BiDil is a relatively new drug that has been recently approved for the treatment of heart failure in African Americans. BiDil consists of a fixed dose combination of isosorbide dinitrate (vasodilator) and hydralazine (antihypertensive). Heart failure, or end-stage cardiovascular disease, affects approximately five million. Americans. Epidemiologic studies indicate a higher prevalence of risk factors for heart failure among African Americans in the United States (Burt *et al.,* 1995). The complications associated with these diseases, such as stroke, heart, and renal failure, contribute to greater rates of mortality in this population. Recent analyses of heart failure clinical trials show that the mortality rate and the hospitalization rate for African Americans are significantly higher than for non-African Americans.

Isosorbide dinitrate is a direct NO donor and causes tolerance with extended use. Hydralazine is a diuretic with putative antioxidant activity that may contribute to reduce loss of NO through its reaction in superoxide levels. The manufacturer (Nitromed) has been allowed to claim in its approved label that BiDil works through an enhancement in the bioavailability of NO in the vessel. This mechanism may provide preferential advantages to African-American heart failure patients who suffer from a greater deficiency of NO than non-African Americans.

The clinical benefit of BiDil in African Americans with heart failure was tested in the A-HeFT trial, a double-blind, placebo controlled study with 1,050 patients at 169 sites in the United States. The trial was halted in July 2004 on the recommendation of the independent Data and Safety Monitoring Board and the steering committee for the trial due to significant survival benefit seen in patients taking the drug. The results of the BiDil trial were presented at the annual meeting of the American Heart Association on November 8, 2004. The trial enrolled a total of 1,050 African American patients who had New York Heart Association class III or IV heart failure with dilated ventricles, constituting moderately severe and severe levels of heart failure. The primary end point for the trial was death from any cause, hospitalization for heart failure, and change in the quality of life. The A-HeFT study was terminated early owing to a significantly higher mortality rate in the placebo group than in the treatment group. A 10.2 percent death rate was shown in the placebo group compared to a 6.2 percent death rate in the BiDil group, $p$=0.02. The mean composite score for the primary endpoint was significantly better in the group given BiDil than in the placebo group, $p$=0.01, as were its individual components: a 43 percent reduction in the rate of death from any cause, $p$=0.01; a 33 percent relative reduction in the rate of first hospitalization for heart failure, $p$=0.0001; and a statistically significant improvement in quality of life, $p$=0.02, measured by the Minnesota Living with Heart Failure questionnaire. Adverse events reported in the trial included symptoms of headache and dizziness, which were significantly more frequent in the group given BiDil, and exacerbations of congestive heart failure (both moderate and severe), which were significantly more frequent in the placebo group.

Other drugs that have been reported to have NO donor activity include:GEA 3162; 1,1-diethyl-2-hydroxy-2-nitrosohydrazine; diethylenetriamine; Molsidomine; isosorbide-5-mononitrate; S-Nitrosothiols; diethylamine dinitric oxide adduct; S-nitrosomercaptoethanol; 3-morpholino-sydnonimine; S-nitrosocysteine; spermine nitric oxide complex; NOC 18; 2,2'-(hydroxynitrosohydrazono)bis-ethanamine; S-Nitroso-N-Acetylpenicillamine; S-Nitrosoglutathione; S-nitro-N-acetylpenicillamine; PAPA NONOate; 3-(2-hydroxy-1-methyl-2-nitrosohydrazino)-N-methyl-1-propanamine; Nitroprusside; Isosorbide Dinitrate; FK 409.

### B. LA-419

The drug S-(6-Nitro-oxi-hexahydro-furo[3,2-b]furan-3-1-il)thioacetate, which is also known as LA-419, is a nitric oxide donor that is currently in clinical trials as a treatment for cardiovascular disorders. It has also been reported as a candidate therapy for the treatment of glaucoma and intestinal disorders (Megson *et al.,* 2009). LA-419 has the following

chemical structure:

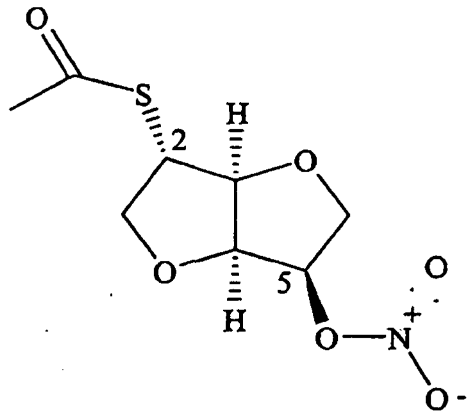

**II. DETECTION OF POLYMORPHISMS**

The polymorphism described herein is present at position 894 in the NOS3 gene. The presence of the polymorphism can be determined from the sequence of the gene or by using specific charactistics of the polymorphism, *e.g.,* restriction enzyme recognition site. As a result, a variety of different methodologies can be employed for the purpose of detecting polymorphisms in the NOS3 gene. Alternatively, the protein gene product can be evaluated to determine the patient's genotype.

**A. Nucleic Acids**

The present disclosure concerns various nucleic acids, including amplification primers, oligonucleotide probes, and other nucleic acid elements involved in the analysis of genomic DNA. In certain aspects, a nucleic acid comprises a wild-type, a mutant, or a polymorphic nucleic acid.

The terms "NOS3" polymorphism refer to a polymorphism in the NOS3 gene. A wildtype sequence of the NOS3 coding region is provided as SEQ ID NO:1; this sequence has a G at position 894. The sequence of the NOS3 with a polymorphism at position 894 (a T instead of a G) is shown as SEQ ID NO:3. The Genbank accession number NM_000603.4, which is hereby incorporated by reference, shows a T at position 894. Position 894 corresponds to nucleotide number 1187 in SEQ ID NO:1 and SEQ ID NO:3. It is contemplated by the inventors and understood by those of skill in the art that a patient's genotype may include other polymorphisms beside the polymorphism at position 894 (1187 from the first position of the cDNA sequence). These variations in sequence may be accommodated insofar as primers and probes are designed to detect the sequence at position 894. One of ordinary skill in the art would know how to identify these polymorphisms, which are readily known, for example, in the NCBI database under the NOS3 accession number on the World Wide Web at ncbi.nlm.nih.gov/SNP/snp_ref.cgi?showRare=on&chooseRs=coding&locusId=4846&mma=NM_000603.3&ctg=NT_007914.14&prot=NP_000594.2&orien=forward&refresh=refresh. It appears that 43 SNPs are currently identified in NOS3.

The location of a polymorphism can be designated based on the total number of nucleotides in the sequence starting as nucleotide 1 (which coincides with the first nucleotide encoding the first amino acid) and progressing in increments of one to the end of the sequence, i.e., nucleotide 894 of SEQ ID NO:1 and SEQ ID NO:3. The polypeptide sequence encoded by the wild-type 894 sequence is shown in SEQ ID NO:2; it has a Glu at amino acid 298. The polymorphic 894 sequence having a T encodes what is shown in SEQ ID NO:4; it has an Asp at amino acid 298.

It is disclosed that nucleic acids used herein may comprise or are complementary to 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1100, 1165, 1200, 1300, 1400, 1500, 1840, 1870 or more contiguous nucleotides, or any range derivable therein, of the human NOS3 sequence provided in SEQ ID NO:1 or SEQ ID NO:3, or any other sequence provided herein. One of skill in the art knows how to design and use primers and probes for hybridization and amplification of a sequence in the NOS3 gene. The sequence may be the

NOS3 coding sequence (or its complement) or it is based on the NOS3 transcript, such as a cDNA of this sequence.

These definitions generally refer to a single-stranded molecule, but in specific embodiments will also encompass an additional strand that is partially, substantially or fully complementary to the single-stranded molecule. Thus, a nucleic acid may encompass a doublestranded molecule or a triple-stranded molecule that comprises one or more complementary strand(s) or "complement(s)" of a particular sequence comprising a molecule. As used herein, a single stranded nucleic acid may be denoted by the prefix "ss", a double stranded nucleic acid by the prefix "ds", and a triple stranded nucleic acid by the prefix "ts."

### 1. Preparation of Nucleic Acids

A nucleic acid may be made by any technique known to one of ordinary skill in the art, such as for example, chemical synthesis, enzymatic production or biological production. Non-limiting examples of a synthetic nucleic acid (*e.g.,* a synthetic oligonucleotide), include a nucleic acid made by *in vitro* chemical synthesis using phosphotriester, phosphite or phosphoramidite chemistry and solid phase techniques such as described in European Patent 266,032, or via deoxynucleoside H-phosphonate intermediates as described by Froehler *et al.,* 1986 and U.S. Patent 5,705,629.

In the methods of the present invention, one or more oligonucleotide may be used. In certain aspects amplification oligonucleotides can be deisgned on either side or overlapping with the boundaries of the insertion site. In a further aspect an oligonucleotide specific for the sequence at 894, whether a G or a T, can be designed. These oligonucleotides can varying in length from 5, 10, 15, 16, 17, 18, 19, 20, 21, 22, 23, 24, 25, 26, 27, 28, 29, 30, 50, nucleotides or more, including all values and ranges there between. Various different mechanisms of oligonucleotide synthesis have been disclosed in for example, U.S. Patents 4,659,774,4,816,571, 5,141,813, 5,264,566, 4,959,463, 5,428,148, 5,554,744, 5,574,146, 5,602,244.

A non-limiting example of an enzymatically produced nucleic acid include one produced by enzymes in amplification reactions such as PCR™ (see for example, U.S. Patent 4,683,202 and U.S. Patent 4,682,195, or the synthesis of an oligonucleotide described in U.S. Patent 5,645,897, incorporated herein by reference. A non-limiting example of a biologically produced nucleic acid includes a recombinant nucleic acid produced (*i.e.,* replicated) in a living cell, such as a recombinant DNA vector replicated in bacteria (see for example, Sambrook *et al.* 2001).

### 2. Purification of Nucleic Acids

A nucleic acid may be purified on polyacrylamide gels, cesium chloride centrifugation gradients, chromatography columns or by any other means known to one of ordinary skill in the art (see for example, Sambrook *et al.,* 2001).

There is also disclosed a nucleic acid that is an isolated nucleic acid. As used herein, the term "isolated nucleic acid" refers to a nucleic acid molecule (*e.g.,* an RNA or DNA molecule) that has been isolated free of, or is otherwise free of, the bulk of the total genomic and transcribed nucleic acids of one or more cells. "Isolated nucleic acid" may refer to a nucleic acid that has been isolated free of, or is otherwise free of, bulk of cellular components or *in vitro* reaction components such as for example, macromolecules such as lipids or proteins, small biological molecules, and the like.

### 3. Nucleic Acid Segments

The nucleic acid may be a nucleic acid segment. As used herein, the term "nucleic acid segment," are fragments of a nucleic acid, such as, for a non-limiting example, those that encode only part of a NOS3 sequence, or part of the NOS3 gene locus or gene sequence. Thus, a "nucleic acid segment" may comprise any part of a gene sequence, including from about 2 nucleotides to the full length gene including promoter regions to the polyadenylation signal and any length that includes all the coding region.

Various nucleic acid segments may be designed based on a particular nucleic acid sequence, and may be of any length. By assigning numeric values to a sequence, for example, the first residue is 1, the second residue is 2, *etc.,* an algorithm defining all nucleic acid segments can be created:

$$n \text{ to } n + y$$

where n is an integer from 1 to the last number of the sequence and y is the length of the nucleic acid segment minus one, where n + y does not exceed the last number of the sequence. Thus, for a 10-mer, the nucleic acid segments correspond to bases 1 to 10, 2 to 11, 3 to 12 ... and so on. For a 15-mer, the nucleic acid segments correspond to bases 1 to 15, 2 to 16, 3 to 17 ... and so on. For a 20-mer, the nucleic segments correspond to bases 1 to 20, 2 to 21, 3 to 22 ... and so on. In certain embodiments, the nucleic acid segment may be a probe or primer. As used herein, a "probe"

generally refers to a nucleic acid used in a detection method or composition. As used herein, a "primer" generally refers to a nucleic acid used in an extension or amplification method or composition.

### 4. Nucleic Acid Complements

There is also disclosed a nucleic acid that is complementary to a nucleic acid. A nucleic acid is "complement(s)" or is "complementary" to another nucleic acid when it is capable of base-pairing with another nucleic acid according to the standard Watson-Crick, Hoogsteen or reverse Hoogsteen binding complementarity rules. As used herein "another nucleic acid" may refer to a separate molecule or a spatial separated sequence of the same molecule. In preferred embodiments, a complement is a hybridization probe or amplification primer for the detection of a nucleic acid polymorphism.

As used herein, the term "complementary" or "complement" also refers to a nucleic acid comprising a sequence of consecutive nucleobases or semiconsecutive nucleobases (*e.g.*, one or more nucleobase moieties are not present in the molecule) capable of hybridizing to another nucleic acid strand or duplex even if less than all the nucleobases do not base pair with a counterpart nucleobase. However, in some diagnostic or detection embodiments, completely complementary nucleic acids are used.

### 5. Nucleic Acid Detection and Evaluation

Genotyping can be performed using methods described in Small *et al.* (2002).

It will be understood by the skilled artisan that other standard techniques are available for genotyping and any technique may be used as described herein. General methods of nucleic acid detection methods are provided below.

It is disclosed that genotyping involves isolating from the patient a nucleic acid mixture comprising both copies of the NOS3 gene, or a fragment thereof, and determining the nucleotide sequence at position 894 of the NOS3 gene. This may involve determining the sequence based on the transcripts produced from both copies of the gene. Other polymorphisms, such as single nucleotide polymorphisms can be linked to and indicative of the polymorphism at position 894 described herein. Consequently, in some embodiments a polymorphism in linkage disequilibrium (LED or LD) with the polymorphism at position 894 may be used to determine the sequence at position 894.

Those in the art will readily recognize that nucleic acid molecules may be double-stranded molecules and that reference to a particular site on one strand refers, as well, to the corresponding site on a complementary strand. Thus, in defining a polymorphic site, reference to a sequence including an adenine, a thymine (uridine), a cytosine, or a guanine at a particular site on one strand of a nucleic acid molecule is also intended to include the thymine (uridine), adenine, guanine, or cytosine (respectively) at the corresponding site on a complementary strand of a nucleic acid molecule. Thus, reference may be made to either strand and still comprise the same polymorphic site and an oligonucleotide may be designed to hybridize to either strand. Throughout the text, in identifying a polymorphic site, reference is made to SEQ ID NO:1 or SEQ ID NO:3 for the purpose of convenience.

Typically, the nucleic acid mixture is isolated from a biological sample taken from the individual, such as a blood sample or tissue sample using standard techniques such as disclosed in Jones (1963) which is hereby incorporated by reference. Suitable tissue samples include whole blood, semen, saliva, tears, urine, fecal material, sweat, buccal, skin, and hair. The nucleic acid mixture may be comprised of genomic DNA.

The ability to predict a patient's response to LA-419 assists the treating physician in making decisions about how to treat a patient having a cardiovascular disease or condition. A patient whose genotype indicates a likelihood that the patient will respond well to LA-419 *(i.e.,* a patient homozygous wildtype at 894 in the NOS3 gene) would be a better candidate for therapy, and the physician may treat the patient with an alternate form of therapy.

In the genotyping methods used herein, the identity of a polymorphic site may be determined by amplifying a target region containing the polymorphic site directly from one or both copies of the NOS3 gene present in the individual and the sequence of the amplified region(s) determined by conventional methods or evaluated directly.

The target region(s) may be amplified using any oligonucleotide-directed amplification method, including but not limited to polymerase chain reaction (PCR) (U.S. Patent 4,965,188), ligase chain reaction (LCR) (Barany *et al.,* 1991; WO90/01069), and oligonucleotide ligation assay (OLA) (Landegren *et al.,* 1988). Oligonucleotides useful as primers or probes in such methods should specifically hybridize to a region of the nucleic acid that contains or is adjacent to the polymorphic site. Typically, the oligonucleotides are between 10 and 35 nucleotides in length and preferably, between 15 and 30 nucleotides in length. Most preferably, the oligonucleotides are 20 to 25 nucleotides long. The exact length of the oligonucleotide will depend on many factors that are routinely considered and practiced by the skilled artisan.

Other known nucleic acid amplification procedures may be used to amplify the target region including transcription-based amplification systems (U.S. Patent 5,130,238; EP 329,822; U.S. Patent 5,169,766, WO89/06700) and isothermal methods (Walker *et al.,* 1992).

A polymorphism in the target region may also be assayed before or after amplification using one of several

hybridization-based methods known in the art. Typically, allele-specific oligonucleotides are utilized in performing such methods. The allele-specific oligonucleotides may be used as differently labeled probe pairs, with one member of the pair showing a perfect match to one variant of a target sequence and the other member showing a perfect match to a different variant. More than one polymorphic site may be detected at once using a set of allele-specific oligonucleotides or oligonucleotide pairs.

Hybridization of an allele-specific oligonucleotide to a target polynucleotide may be performed with both entities in solution, or such hybridization may be performed when either the oligonucleotide or the target polynucleotide is covalently or noncovalently affixed to a solid support. Attachment may be mediated, for example, by antibody-antigen interactions, poly-L-Lys, streptavidin or avidin-biotin, salt bridges, hydrophobic interactions, chemical linkages, UV cross-linking baking, etc. Allele-specific oligonucleotides may be synthesized directly on the solid support or attached to the solid support subsequent to synthesis. Solid-supports suitable for use in detection methods of the invention include substrates made of silicon, glass, plastic, paper and the like, which may be formed, for example, into wells (as in 96-well plates), slides, sheets, membranes, fibers, chips, dishes, and beads. The solid support may be treated, coated or derivatized to facilitate the immobilization of the allele-specific oligonucleotide or target nucleic acid.

The genotype for one or more polymorphic sites in the NOS3 gene or other sites in LD with position 894 of an individual may also be determined by hybridization of one or both copies of the gene, or a fragment thereof, to nucleic acid arrays and subarrays such as described in WO 95/11995. The arrays would contain a battery of allele-specific oligonucleotides representing each of the polymorphic sites to be included in the genotype or haplotype.

The identity of polymorphisms may also be determined using a mismatch detection technique, including but not limited to the RNase protection method using riboprobes (Winter *et al.,* 1985; Meyers *et al.,* 1985) and proteins which recognize nucleotide mismatches, such as the *E. coli* mutS protein (Modrich, 1991). Alternatively, variant alleles can be identified by single strand conformation polymorphism (SSCP) analysis (Orita *et al.,* 1989; Humphries *et al.,* 1996) or denaturing gradient gel electrophoresis (DGGE) (Wartell *et al.,* 1990; Sheffield *et al.,* 1989).

A polymerase-mediated primer extension method may also be used to identify the polymorphism(s). Several such methods have been described in the patent and scientific literature. Extended primers containing a polymorphism may be detected by mass spectrometry as described in U.S. Pat. No. 5,605,798. An other primer extension method is allele-specific PCR (Ruano *et al.,* 1989; Ruano *et al.,* 1991; WO 93/22456; Turki *et al.,* 1995).

Polymorphic variation in the human NOS3 gene can also be detected using differential digestion of DNA by certain restriction enzymes (Small *et al.,* 2002) or by any other method that identifies the sequence of the polymorphic position in the NOS3 gene.

**a. Hybridization**

The use of a probe or primer of between 7, 8, 9, 10, 11, 12, 13, 14, 15, 16, 17, 18, 19, 20, 21, 22, 23, 24, 25, 50, 60, 70, 80, 90, or 100 consecutive nucleotides of SEQ ID NO:1 or SEQ ID NO:3, preferably between 17 and 100 nucleotides in length, or in some aspects of the invention up to 1-2 kilobases or more in length, allows the formation of a duplex molecule that is both stable and selective. Molecules having complementary sequences over contiguous stretches greater than 20 bases in length are generally preferred, to increase stability and/or selectivity of the hybrid molecules obtained. One will generally prefer to design nucleic acid molecules for hybridization having one or more complementary sequences of 20 to 30 nucleotides, or even longer where desired Such fragments may be readily prepared, for example, by directly synthesizing the fragment by chemical means or by introducing selected sequences into recombinant vectors for recombinant production.

Accordingly, the nucleotide sequences as disclosed herein may be used for their ability to selectively form duplex molecules with complementary stretches of DNAs and/or RNAs or to provide primers for amplification of DNA or RNA from samples. Depending on the application envisioned, one would desire to employ varying conditions of hybridization to achieve varying degrees of selectivity of the probe or primers for the target sequence.

For applications requiring high selectivity, one will typically desire to employ relatively high stringency conditions to form the hybrids. For example, relatively low salt and/or high temperature conditions, such as provided by about 0.02 M to about 0.10 M NaCl at temperatures of about 50°C to about 70°C. Such high stringency conditions tolerate little, if any, mismatch between the probe or primers and the template or target strand and would be particularly suitable for isolating specific genes or for detecting a specific polymorphism. It is generally appreciated that conditions can be rendered more stringent by the addition of increasing amounts of formamide. For example, under highly stringent conditions, hybridization to filter-bound DNA may be carried out in 0.5 M $NaBPO_4$, 7% sodium dodecyl sulfate (SDS), 1 mM EDTA at 65°C, and washing in 0.1 x SSC/0.1% SDS at 68°C (Ausubel *et al.,* 1989).

Conditions may be rendered less stringent by increasing salt concentration and/or decreasing temperature. For example, a medium stringency condition could be provided by about 0.1 to 0.25 M NaCl at temperatures of about 37°C to about 55°C, while a low stringency condition could be provided by about 0.15 M to about 0.9 M salt, at temperatures ranging from about 20°C to about 55°C. Under low stringent conditions, such as moderately stringent conditions the

washing maybe carried out for example in 0.2 x SSC/0.1% SDS at 42°C (Ausubel *et al.,* 1989). Hybridization conditions can be readily manipulated depending on the desired results.

In other embodiments, hybridization may be achieved under conditions of, for example, 50 mM Tris-HCl (pH 8.3), 75 mM KCl, 3 mM $MgCl_2$, 1.0 mM dithiothreitol, at temperatures between approximately 20°C to about 37°C. Other hybridization conditions utilized could include approximately 10 mM Tris-HCl (pH 8.3), 50 mM KCl, 1.5 mM $MgCl_2$, at temperatures ranging from approximately 40°C to about 72°C.

It is disclosed that it will be advantageous to employ nucleic acids of defined sequences of the present invention in combination with an appropriate means, such as a label, for determining hybridization. A wide variety of appropriate indicator means are known in the art, including fluorescent, radioactive, enzymatic or other ligands, such as avidin/biotin, which are capable of being detected. One may desire to employ a fluorescent label or an enzyme tag such as urease, alkaline phosphatase or peroxidase, instead of radioactive or other environmentally undesirable reagents. In the case of enzyme tags, colorimetric indicator substrates are known that can be employed to provide a detection means that is visibly or spectrophotometrically detectable, to identify specific hybridization with complementary nucleic acid containing samples. In other aspects, a particular nuclease cleavage site may be present and detection of a particular nucleotide sequence can be determined by the presence or absence of nucleic acid cleavage.

In general, it is envisioned that the probes or primers described herein will be useful as reagents in solution hybridization, as in PCR, for detection of expression or genotype of corresponding genes, as well as in embodiments employing a solid phase. In embodiments involving a solid phase, the test DNA (or RNA) is adsorbed or otherwise affixed to a selected matrix or surface. This fixed, single-stranded nucleic acid is then subjected to hybridization with selected probes under desired conditions. The conditions selected will depend on the particular circumstances (depending, for example, on the G+C content, type of target nucleic acid, source of nucleic acid, size of hybridization probe, *etc.).* Optimization of hybridization conditions for the particular application of interest is well known to those of skill in the art. After washing of the hybridized molecules to remove non-specifically bound probe molecules, hybridization is detected, and/or quantified, by determining the amount of bound label. Representative solid phase hybridization methods are disclosed in U.S. Patents 5,843,663, 5,900,481 and 5,919,626. Other methods of hybridization that may be used in the practice of the present invention are disclosed in U.S. Patents 5,849,481, 5,849,486 and 5,851,772.

**b. Amplification of Nucleic Acids**

Nucleic acids used as a template for amplification may be isolated from cells, tissues or other samples according to standard methodologies (Sambrook *et al.,* 2001). It is disclosed that analysis may be performed on whole cell or tissue homogenates or biological fluid samples with or without substantial purification of the template nucleic acid. The nucleic acid may be genomic DNA or fractionated or whole cell RNA. Where RNA is used, it may be desired to first convert the RNA to a complementary DNA.

The term "primer," as used herein, is meant to encompass any nucleic acid that is capable of priming the synthesis of a nascent nucleic acid in a template-dependent process. Typically, primers are oligonucleotides from ten to twenty and/or thirty base pairs in length, but longer sequences can be employed. Primers may be provided in double-stranded and/or single-stranded form, although the single-stranded form is preferred.

Pairs of primers designed to selectively hybridize to nucleic acids corresponding to the NOS3 gene locus, or variants thereof, and fragments thereof are contacted with the template nucleic acid under conditions that permit selective hybridization. Depending upon the desired application, high stringency hybridization conditions may be selected that will only allow hybridization to sequences that are completely complementary to the primers. It is disclosed that hybridization may occur under reduced stringency to allow for amplification of nucleic acids that contain one or more mismatches with the primer sequences. Once hybridized, the template-primer complex is contacted with one or more enzymes that facilitate template-dependent nucleic acid synthesis. Multiple rounds of amplification, also referred to as "cycles," are conducted until a sufficient amount of amplification product is produced.

The amplification product may be detected, analyzed or quantified. In certain applications, the detection may be performed by visual means. In certain applications, the detection may involve indirect identification of the product via chemiluminescence, radioactive scintigraphy of incorporated radiolabel or fluorescent label or even via a system using electrical and/or thermal impulse signals (Affymax technology; Bellus, 1994).

A number of template dependent processes are available to amplify the oligonucleotide sequences present in a given template sample. One of the best known amplification methods is the polymerase chain reaction (referred to as PCR™) which is described in detail in U.S. Patents 4,683,195, 4,683,202 and 4,800,159, and in Innis *et al*., 1988, each of which is incorporated herein by reference in their entirety.

Another method for amplification is ligase chain reaction ("LCR"), disclosed in European Application No. 320 308, incorporated herein by reference in its entirety. U.S. Patent 4,883,750 describes a method similar to LCR for binding probe pairs to a target sequence. A method based on PCR™ and oligonucleotide ligase assay (OLA) (described in further detail below), disclosed in U.S. Patent 5,912,148, may also be used.

Alternative methods for amplification of target nucleic acid sequences that may be used in the practice of the present invention are disclosed in U.S. Patents 5,843,650, 5,846,709, 5,846,783, 5,849,546, 5,849,497, 5,849,547, 5,858,652, 5,866,366, 5,916,776, 5,922,574, 5,928,905, 5,928,906, 5,932,451, 5,935,825, 5,939,291 and 5,942,391, Great Britain Application 2 202 328, and in PCT Application PCT/US89/01025.
Qbeta Replicase, described in PCT Application PCT/US87/00880, may also be used as an amplification method in the present invention.

An isothermal amplification method, in which restriction endonucleases and ligases are used to achieve the amplification of target molecules that contain nucleotide 5'-[alpha-thio]-triphosphates in one strand of a restriction site may also be useful in the amplification of nucleic acids in the present invention (Walker *et al.,* 1992). Strand Displacement Amplification (SDA), disclosed in U.S. Patent 5,916,779, is another method of carrying out isothermal amplification of nucleic acids which involves multiple rounds of strand displacement and synthesis, *i.e.*, nick translation.

Other nucleic acid amplification procedures include transcription-based amplification systems (TAS), including nucleic acid sequence based amplification (NASBA) and 3SR (Kwoh *et al.,* 1989; PCT Application WO 88/10315, incorporated herein by reference in their entirety). European Application 329 822 disclose a nucleic acid amplification process involving cyclically synthesizing single-stranded RNA ("ssRNA"), ssDNA, and double-stranded DNA (dsDNA), which may be used in accordance with the present invention.

PCT Application WO 89/06700 disclose a nucleic acid sequence amplification scheme based on the hybridization of a promoter region/primer sequence to a target single-stranded DNA ("ssDNA") followed by transcription of many RNA copies of the sequence. This scheme is not cyclic, *i.e.*, new templates are not produced from the resultant RNA transcripts. Other amplification methods include "RACE" and "one-sided PCR" (Frohman, 1990; *Ohara et al.,* 1989).

### c. Detection of Nucleic Acids

Following any amplification, it may be desirable to separate the amplification product from the template and/or the excess primer. In one embodiment, amplification products are separated by agarose, agarose-acrylamide or polyacrylamide gel electrophoresis using standard methods (Sambrook *et al.,* 2001). Separated amplification products may be cut out and eluted from the gel for further manipulation. Using low melting point agarose gels, the separated band may be removed by heating the gel, followed by extraction of the nucleic acid.

Separation of nucleic acids may also be effected by spin columns and/or chromatographic techniques known in art. There are many kinds of chromatography which may be used in the practice of the present invention, including adsorption, partition, ion-exchange, hydroxylapatite, molecular sieve, reverse-phase, column, paper, thin-layer, and gas chromatography as well as HPLC.

The amplification products may be visualized, with or without separation. A typical visualization method involves staining of a gel with ethidium bromide and visualization of bands under UV light. Alternatively, if the amplification products are integrally labeled with radio- or fluorometrically-labeled nucleotides, the separated amplification products can be exposed to x-ray film or visualized under the appropriate excitatory spectra.

Following separation of amplification products, a labeled nucleic acid probe may be brought into contact with the amplified marker sequence. The probe preferably is conjugated to a chromophore but may be radiolabeled. The probe may also be conjugated to a binding partner, such as an antibody or biotin, or another binding partner carrying a detectable moiety.

Detection may also be by Southern blotting and hybridization with a labeled probe. The techniques involved in Southern blotting are well known to those of skill in the art (see Sambrook *et al.,* 2001). One example of the foregoing is described in U.S. Patent 5,279,721, incorporated by reference herein, which discloses an apparatus and method for the automated electrophoresis and transfer of nucleic acids. The apparatus permits electrophoresis and blotting without external manipulation of the gel and is ideally suited to carrying out methods according to the present invention.

Other methods of nucleic acid detection that may be used in the practice of the instant invention are disclosed in U.S. Patents 5,840,873, 5,843,640, 5,843,651, 5,846,708, 5,846,717, 5,846,726, 5,846,729, 5,849,487, 5,853,990, 5,853,992, 5,853,993, 5,856,092, 5,861,244, 5,863,732, 5,863,753, 5,866,331, 5,905,024, 5,910,407, 5,912,124, 5,912,145, 5,919,630, 5,925,517, 5,928,862, 5,928,869, 5,929,227, 5,932,413 and 5,935,791.

### d. Other Assays

Other methods for genetic screening may be used within the scope of the present invention, for example, to detect mutations in genomic DNA, cDNA and/or RNA samples. Methods used to detect point mutations include denaturing gradient gel electrophoresis ("DGGE"), restriction fragment length polymorphism analysis ("RFLP"), chemical or enzymatic cleavage methods, direct sequencing of target regions amplified by PCR™ (see above), single-strand conformation polymorphism analysis ("SSCP") and other methods well known in the art.

One method of screening for point mutations is based on RNase cleavage of base pair mismatches in RNA/DNA

or. RNA/RNA heteroduplexes. As used herein, the term "mismatch" is defined as a region of one or more unpaired or mispaired nucleotides in a double-stranded RNA/RNA, RNA/DNA or DNA/DNA molecule. This definition thus includes mismatches due to insertion/deletion mutations, as well as single or multiple base point mutations.

U.S. Patent 4,946,773 describes an RNase A mismatch cleavage assay that involves annealing single-stranded DNA or RNA test samples to an RNA probe, and subsequent treatment of the nucleic acid duplexes with RNase A. For the detection of mismatches, the single-stranded products of the RNase A treatment, electrophoretically separated according to size, are compared to similarly treated control duplexes. Samples containing smaller fragments (cleavage products) not seen in the control duplex are scored as positive.

Other investigators have described the use of RNase I in mismatch assays. The use of RNase I for mismatch detection is described in literature from Promega Biotech. Promega markets a kit containing RNase I that is reported to cleave three out of four known mismatches. Others have described using the MutS protein or other DNA-repair enzymes for detection of single-base mismatches.

Alternative methods for detection of deletion, insertion or substitution mutations that may be used in the practice of the present invention are disclosed in U.S. Patents 5,849,483, 5,851,770, 5,866,337, 5,925,525 and 5,928,870.

**e. Specific Examples of Polymorphism Nucleic Acid Screening Methods**

Spontaneous mutations that arise during the course of evolution in the genomes of organisms are often not immediately transmitted throughout all of the members of the species, thereby creating polymorphic alleles that co-exist in the species populations. Often polymorphisms are the cause of genetic diseases. Several classes of polymorphisms have been identified. For example, variable nucleotide type polymorphisms (VNTRs), arise from spontaneous tandem duplications of di- or trinucleotide repeated motifs of nucleotides. If such variations alter the lengths of DNA fragments generated by restriction endonuclease cleavage, the variations are referred to as restriction fragment length polymorphisms (RFLPs). RFLPs are been widely used in human and animal genetic analyses.

Another class of polymorphisms are generated by the replacement of a single nucleotide. Such single nucleotide polymorphisms (SNPs) rarely result in changes in a restriction endonuclease site. Thus, SNPs are rarely detectable restriction fragment length analysis. SNPs are the most common genetic variations and occur once every 100 to 300 bases and several SNP mutations have been found that affect a single nucleotide in a protein-encoding gene in a manner sufficient to actually cause a genetic disease. SNP diseases are exemplified by hemophilia, sickle-cell anemia, hereditary hemochromatosis, late-onset alzheimer disease *etc.*

Several methods have been developed to screen polymorphisms and some examples are listed below. The reference of Kwok and Chen (2003) and Kwok (2001) provide overviews of some of these methods.

SNPs relating to ABCC2 can be characterized by the use of any of these methods or suitable modification thereof. Such methods include the direct or indirect sequencing of the site, the use of restriction enzymes where the respective alleles of the site create or destroy a restriction site, the use of allele-specific hybridization probes, the use of antibodies that are specific for the proteins encoded by the different alleles of the polymorphism, or any other biochemical interpretation.

(1) DNA Sequencing

The most commonly used method of characterizing a polymorphism is direct DNA sequencing of the genetic locus that flanks and includes the polymorphism. Such analysis can be accomplished using either the "dideoxy-mediated chain termination method," also known as the "Sanger Method" (Sanger *et al.,* 1975) or the "chemical degradation method," also known as the "Maxam-Gilbert method" (Maxam *et al.,* 1977). Sequencing in combination with genomic sequence-specific amplification technologies, such as the polymerase chain reaction may be utilized to facilitate the recovery of the desired genes (Mullis *et al.,* 1986; European Patent Application 50,424; European Patent Application. 84,796, European Patent Application 258,017, European Patent Application. 237,362; European Patent Application. 201,184; U.S. Patents 4,683,202; 4,582,788; and 4,683,194).

(2) Exonuclease Resistance

Other methods that can be employed to determine the identity of a nucleotide present at a polymorphic site utilize a specialized exonuclease-resistant nucleotide derivative (U.S. Patent 4,656,127). A primer complementary to an allelic sequence immediately 3'-to the polymorphic site is hybridized to the DNA under investigation. If the polymorphic site on the DNA contains a nucleotide that is complementary to the particular exonucleotide-resistant nucleotide derivative present, then that derivative will be incorporated by a polymerase onto the end of the hybridized primer. Such incorporation makes the primer resistant to exonuclease cleavage and thereby permits its detection. As the identity of the exonucleotide-resistant derivative is known one can determine the specific nucleotide present in the polymorphic site of the DNA.

(3) Microsequencing Methods

Several other primer-guided nucleotide incorporation procedures for assaying polymorphic sites in DNA have been described (Komher *et al.,* 1989; Sokolov, 1990; Syvanen 1990; Kuppuswamy *et al.,* 1991; Prezant *et al.,* 1992; Ugozzoll *et al.,* 1992; *Nyren et al.,* 1993). These methods rely on the incorporation of labeled deoxynucleotides to discriminate between bases at a polymorphic site. As the signal is proportional to the number of deoxynucleotides incorporated, polymorphisms that occur in runs of the same nucleotide result in a signal that is proportional to the length of the run (Syvanen *et al.,*1990).

(4) Extension in Solution

French Patent 2,650,840 and PCT Application WO91/02087 discuss a solution-based method for determining the identity of the nucleotide of a polymorphic site. According to these methods, a primer complementary to allelic sequences immediately 3'-to a polymorphic site is used. The identity of the nucleotide of that site is determined using labeled dideoxynucleotide derivatives which are incorporated at the end of the primer if complementary to the nucleotide of the polymorphic site.

(5) Genetic Bit Analysis or Solid-Phase Extension

PCT Application WO92/15712 describes a method that uses mixtures of labeled terminators and a primer that is complementary to the sequence 3' to a polymorphic site. The labeled terminator that is incorporated is complementary to the nucleotide present in the polymorphic site of the target molecule being evaluated and is thus identified. Here the primer or the target molecule is immobilized to a solid phase.

(6) Oligonucleotide Ligation Assay (OLA)

This is another solid phase method that uses different methodology (Landegren *et al.,* 1988). Two oligonucleotides, capable of hybridizing to abutting sequences of a single strand of a target DNA are used. One of these oligonucleotides is biotinylated while the other is detectably labeled. If the precise complementary sequence is found in a target molecule, the oligonucleotides will hybridize such that their termini abut, and create a ligation substrate. Ligation permits the recovery of the labeled oligonucleotide by using avidin. Other nucleic acid detection assays, based on this method, combined with PCR have also been described (Nickerson *et al.,* 1990). Here PCR is used to achieve the exponential amplification of target DNA, which is then detected using the OLA.

(7) Ligase/Polymerase-Mediated Genetic Bit Analysis

U.S. Patent 5,952,174 describes a method that also involves two primers capable of hybridizing to abutting sequences of a target molecule. The hybridized product is formed on a solid support to which the target is immobilized. Here the hybridization occurs such that the primers are separated from one another by a space of a single nucleotide. Incubating this hybridized product in the presence of a polymerase, a ligase, and a nucleoside triphosphate mixture containing at least one deoxynucleoside triphosphate allows the ligation of any pair of abutting hybridized oligonucleotides. Addition of a ligase results in two events required to generate a signal, extension and ligation. This provides a higher specificity and lower "noise" than methods using either extension or ligation alone and unlike the polymerase-based assays, this method enhances the specificity of the polymerase step by combining it with a second hybridization and a ligation step for a signal to be attached to the solid phase.

(8) Invasive Cleavage Reactions

Invasive cleavage reactions can be used to evaluate cellular DNA for a particular polymorphism. A technology called INVADER® employs such reactions *(e.g.,* de Arruda *et al.,* 2002; Stevens *et al.,* 2003). Generally, there are three nucleic acid molecules: 1) an oligonucleotide upstream of the target site ("upstream oligo"), 2) a probe oligonucleotide covering the target site ("probe"), and 3) a single-stranded DNA with the the target site ("target"). The upstream oligo and probe do not overlap but they contain contiguous sequences. The probe contains a donor fluorophore, such as fluoroscein, and an acceptor dye, such as Dabcyl. The nucleotide at the 3' terminal end of the upstream oligo overlaps ("invades") the first base pair of a probe-target duplex. Then the probe is cleaved by a structure-specific 5' nuclease causing separation of the fluorophore/quencher pair, which increases the amount of fluorescence that can be detected. *See* Lu *et al.,* 2004.

In some cases, the assay is conducted on a solid-surface or in an array format

(9) Other Methods To Detect SNPs

Several other specific methods for polymorphism detection and identification are presented below and may be used as such or with suitable modifications in conjunction with identifying polymorphisms of the NOS3 gene in the present invention. Several other methods are also described on the SNP web site of the NCBI on the World Wide Web at ncbi.nlm.nih.gov/SNP, incorporated herein by reference.

Extended haplotypes may be determined at any given locus in a population, which allows one to identify exactly which SNPs will be redundant and which will be essential in association studies. The latter is referred to as 'haplotype tag SNPs (htSNPs)', markers that capture the haplotypes of a gene or a region of linkage disequilibrium. See Johnson *et al.* (2001) and Ke and Cardon (2003), for exemplary methods.

The VDA-assay utilizes PCR amplification of genomic segments by long PCR methods using TaKaRa LA Taq reagents and other standard reaction conditions. The long amplification can amplify DNA sizes of about 2,000-12,000 bp. Hybridization of products to variant detector array (VDA) can be performed by a Affymetrix High Throughput Screening Center and analyzed with computerized software.

A method called Chip Assay uses PCR amplification of genomic segments by standard or long PCR protocols. Hybridization products are analyzed by VDA, Halushka *et al.* (1999). SNPs are generally classified as "Certain" or "Likely" based on computer analysis of hybridization patterns. By comparison to alternative detection methods such as nucleotide sequencing, "Certain" SNPs have been confirmed 100% of the time; and "Likely" SNPs have been confirmed 73% of the time by this method.

Other methods simply involve PCR amplification following digestion with the relevant restriction enzyme. Yet others involve sequencing of purified PCR products from known genomic regions.

In yet another method, individual exons or overlapping fragments of large exons are PCR-amplified. Primers are designed from published or database sequences and PCR-amplification of genomic DNA is performed using the following conditions: 200 ng DNA template, 0.5 μM each primer, 80μM each of dCTP, dATP, dTTP and dGTP, 5% formamide, 1.5mM $MgCl_2$, 0.5 U of Taq polymerase and 0.1 volume of the Taq buffer. Thermal cycling is performed and resulting PCR-products are analyzed by PCR-single strand conformation polymorphism (PCR-SSCP) analysis, under a variety of conditions, e.g, 5 or 10% polyacrylamide gel with 15% urea, with or without 5% glycerol. Electrophoresis is performed overnight. PCR-products that show mobility shifts are reamplified and sequenced to identify nucleotide variation.

In a method called CGAP-GAI (DEMIGLACE), sequence and alignment data (from a PHRAP.ace file), quality scores for the sequence base calls (from PHRED quality files), distance information (from PHYLIP dnadist and neighbour programs) and base-calling data (from PHRED '-d' switch) are loaded into memory. Sequences are aligned and examined for each vertical chunk ('slice') of the resulting assembly for disagreement. Any such slice is considered a candidate SNP (DEMIGLACE). A number of filters are used by DEMIGLACE to eliminate slices that are not likely to represent true polymorphisms. These include filters that: (i) exclude sequences in any given slice from SNP consideration where neighboring sequence quality scores drop 40% or more; (ii) exclude calls in which peak amplitude is below the fifteenth percentile of all base calls for that nucleotide type; (iii) disqualify regions of a sequence having a high number of disagreements with the consensus from participating in SNP calculations; (iv) removed from consideration any base call with an alternative call in which the peak takes up 25% or more of the area of the called peak; (v) exclude variations that occur in only one read direction. PHRED quality scores were converted into probability-of-error values for each nucleotide in the slice. Standard Baysian methods are used to calculate the posterior probability that there is evidence of nucleotide heterogeneity at a given location.

In a method called CU-RDF (RESEQ), PCR amplification is performed from DNA isolated from blood using specific primers for each SNP, and after typical cleanup protocols to remove unused primers and free nucleotides, direct sequencing using the same or nested primers.

In a method called DEBNICK (METHOD-B), a comparative analysis of clustered EST sequences is performed and confirmed by fluorescent-based DNA sequencing. In a related method, called DEBNICK (METHOD-C), comparative analysis of clustered EST sequences with phred quality > 20 at the site of the mismatch, average phred quality >= 20 over 5 bases 5'-FLANK and 3' to the SNP, no mismatches in 5 bases 5' and 3' to the SNP, at least two occurrences of each allele is performed and confirmed by examining traces.

In a method identified by ERO (RESEQ), new primers sets are designed for electronically published STSs and used to amplify DNA from 10 different mouse strains. The amplification product from each strain is then gel purified and sequenced using a standard dideoxy, cycle sequencing technique with $^{33}P$-labeled terminators. All the ddATP terminated reactions are then loaded in adjacent lanes of a sequencing gel followed by all of the ddGTP reactions and so on. SNPs are identified by visually scanning the radiographs.

In another method identified as ERO (RESEQ-HT), new primers sets are designed for electronically published murine DNA sequences and used to amplify DNA from 10 different mouse strains. The amplification product from each strain is prepared for sequencing by treating with Exonuclease I and Shrimp Alkaline Phosphatase. Sequencing is

performed using ABI Prism Big Dye Terminator Ready Reaction Kit (Perkin-Elmer) and sequence samples are run on the 3700 DNA Analyzer (96 Capillary Sequencer).

FGU-CBT (SCA2-SNP) identifies a method where the region containing the SNP were PCR amplified using the primers SCA2-FP3 and SCA2-RP3. Approximately 100 ng of genomic DNA is amplified in a 50 ml reaction volume containing a final concentration of 5 mM Tris, 25 mM KCl, 0.75 mM $MgCl_2$, 0.05% gelatin, 20 pmol of each primer and 0.5U of Taq DNA polymerase. Samples are denatured, annealed and extended and the PCR product is purified from a band cut out of the agarose gel using, for example, the QIAquick gel extraction kit (Qiagen) and is sequenced using dye terminator chemistry on an ABI Prism 377 automated DNA sequencer with the PCR primers.

In a method identified as JBLACK (SEQ/RESTRICT), two independent PCR reactions are performed with genomic DNA. Products from the first reaction are analyzed by sequencing, indicating a unique FspI restriction site. The mutation is confirmed in the product of the second PCR reaction by digesting with Fsp I.

In a method described as KWOK(1), SNPs are identified by comparing high quality genomic sequence data from four randomly chosen individuals by direct DNA sequencing of PCR products with dye-terminator chemistry (see Kwok *et al.,* 1996). In a related method identified as KWOK(2) SNPs are identified by comparing high quality genomic sequence data from overlapping large-insert clones such as bacterial artificial chromosomes (BACs) or P1-based artificial chromosomes (PACs). An STS containing this SNP is then developed and the existence of the SNP in various populations is confirmed by pooled DNA sequencing (see Taillon-Miller *et al.,* 1998). In another similar method called KWOK(3), SNPs are identified by comparing high quality genomic sequence data from overlapping large-insert clones BACs or PACs. The SNPs found by this approach represent DNA sequence variations between the two donor chromosomes but the allele frequencies in the general population have not yet been determined. In method KWOK(5), SNPs are identified by comparing high quality genomic sequence data from a homozygous DNA sample and one or more pooled DNA samples by direct DNA sequencing of PCR products with dye-terminator chemistry. The STSs used are developed from sequence data found in publicly available databases. Specifically, these STSs are amplified by PCR against a complete hydatidiform mole (CHM) that has been shown to be homozygous at all loci and a pool of DNA samples from 80 CEPH parents (see Kwok *et al.,* 1994).

In another such method, KWOK (OverlapSnpDetectionWithPolyBayes), SNPs are discovered by automated computer analysis of overlapping regions of large-insert human genomic clone sequences. For data acquisition, clone sequences are obtained directly from large-scale sequencing centers. This is necessary because base quality sequences are not present/available through GenBank. Raw data processing involves analyzed of clone sequences and accompanying base quality information for consistency. Finished ('base perfect', error rate lower than 1 in 10,000 bp) sequences with no associated base quality sequences are assigned a uniform base quality value of 40 (1 in 10,000 bp error rate). Draft sequences without base quality values are rejected. Processed sequences are entered into a local database. A version of each sequence with known human repeats masked is also stored. Repeat masking is performed with the program "MASKERAID." Overlap detection: Putative overlaps are detected with the program "WUBLAST." Several filtering steps followed in order to eliminate false overlap detection results, *i.e.* similarities between a pair of clone sequences that arise due to sequence duplication as opposed to true overlap. Total length of overlap, overall percent similarity, number of sequence differences between nucleotides with high base quality value "high-quality mismatches." Results are also compared to results of restriction fragment mapping of genomic clones at Washington University Genome Sequencing Center, finisher's reports on overlaps, and results of the sequence contig building effort at the NCBI. SNP detection: Overlapping pairs of clone sequence are analyzed for candidate SNP sites with the 'POLYBAYES' SNP detection software. Sequence differences between the pair of sequences are scored for the probability of representing true sequence variation as opposed to sequencing error. This process requires the presence of base quality values for both sequences. High-scoring candidates are extracted. The search is restricted to substitution-type single base pair variations. Confidence score of candidate SNP is computed by the POLYBAYES software.

In method identified by KWOK (TaqMan assay), the TaqMan assay is used to determine genotypes for 90 random individuals. In method identified by KYUGEN(Q1), DNA samples of indicated populations are pooled and analyzed by PLACE-SSCP. Peak heights of each allele in the pooled analysis are corrected by those in a heterozygote, and are subsequently used for calculation of allele frequencies. Allele frequencies higher than 10% are reliably quantified by this method. Allele frequency = 0 (zero) means that the allele was found among individuals, but the corresponding peak is not seen in the examination of pool. Allele frequency = 0-0.1 indicates that minor alleles are detected in the pool but the peaks are too low to reliably quantify.

In yet another method identified as KYUGEN (Method1), PCR products are post-labeled with fluorescent dyes and analyzed by an automated capillary electrophoresis system under SSCP conditions (PLACE-SSCP). Four or more individual DNAs are analyzed with or without two pooled DNA (Japanese pool and CEPH parents pool) in a series of experiments. Alleles are identified by visual inspection. Individual DNAs with different genotypes are sequenced and SNPs identified. Allele frequencies are estimated from peak heights in the pooled samples after correction of signal bias using peak heights in heterozygotes. For the PCR primers are tagged to have 5'-ATT or 5'-GTT at their ends for post-labeling of both strands. Samples of DNA (10 ng/ul) are amplified in reaction mixtures containing the buffer (10mM Tris-HCl, pH 8.3 or 9.3, 50mM KCl, 2.0mM $MgCl_2$), 0.25μM of each primer, 200μM of each dNTP, and 0.025 units/μl of Taq DNA polymerase premixed with anti-Taq antibody. The two strands of PCR products are differentially labeled with nucleotides modified with R110 and R6G by an exchange reaction of Klenow fragment of DNA polymerase I. The reaction is stopped by adding EDTA, and unincorporated nucleotides are dephosphorylated by adding calf intestinal alkaline phosphatase. For the SSCP: an aliquot of fluorescently labeled PCR products and TAMRA-labeled internal markers are added to deionized formamide, and denatured. Electrophoresis is performed in a capillary using an ABI Prism 310 Genetic Analyzer. Genescan softwares (P-E Biosystems) are used for data collection and data processing. DNA of individuals (two to eleven) including those who showed different genotypes on SSCP are subjected for direct sequencing using big-dye terminator chemistry, on ABI Prism 310 sequencers. Multiple sequence trace files obtained from ABI Prism 310 are processed and aligned by Phred/Phrap and viewed using Consed viewer. SNPs are identified by PolyPhred software and visual inspection.

In yet another method identified as KYUGEN (Method2), individuals with different genotypes are searched by denaturing HPLC (DHPLC) or PLACE-SSCP (Inazuka *et al.,* 1997) and their sequences are determined to identify SNPs. PCR is performed with primers tagged with 5'-ATT or 5'-GTT at their ends for post-labeling of both strands. DHPLC analysis is carried out using the WAVE DNA fragment analysis system (Transgenomic). PCR products are injected into DNASep column, and separated under the conditions determined using WAVEMaker program (Transgenomic). The two strands of PCR products that are differentially labeled with nucleotides modified with R110 and R6G by an exchange reaction of Klenow fragment of DNA polymerase I. The reaction is stopped by adding EDTA, and unincorporated nucleotides are dephosphorylated by adding calf intestinal alkaline phosphatase. SSCP followed by electrophoresis is performed in a capillary using an ABI Prism 310 Genetic Analyzer. Genescan softwares (P-E Biosystems). DNA of individuals including those who showed different genotypes on DHPLC or SSCP are subjected for direct sequencing using big-dye terminator chemistry, on ABI Prism 310 sequencer. Multiple sequence trace files obtained from ABI Prism 310 are processed and aligned by Phred/Phrap and viewed using Consed viewer. SNPs are identified by PolyPhred software and visual inspection. Trace chromatogram data of EST sequences in Unigene are processed with PHRED. To identify likely SNPs, single base mismatches are reported from multiple sequence alignments produced by the programs PHRAP, BRO and POA for each Unigene cluster. BRO corrected possible misreported EST orientations, while POA identified and analyzed non-linear alignment structures indicative of gene mixing/chimeras that might produce spurious SNPs. Bayesian inference is used to weigh evidence for true polymorphism versus sequencing error, misalignment or ambiguity, misclustering or chimeric EST sequences, assessing data such as raw chromatogram height, sharpness, overlap and spacing; sequencing error rates; context-sensitivity; cDNA library origin, etc.

In method identified as MARSHFIELD(Method-B), overlapping human DNA sequences which contained putative insertion/deletion polymorphisms are identified through searches of public databases. PCR primers which flanked each polymorphic site are selected from the consensus sequences. Primers are used to amplify individual or pooled human genomic DNA. Resulting PCR products are resolved on a denaturing polyacrylamide gel and a PhosphorImager is used to estimate allele frequencies from DNA pools.

### f. Linkage Disequilibrium

Polymorphisms in linkage disequilibrium with another polymorphism in which identification of one polymorphism is predictive of the identity of the linked polymorphism. "Linkage disequilibrium" ("LD" as used herein, though also referred to as "LED" in the art) refers to a situation where a particular combination of alleles (*i.e.*, a variant form of a given gene) or polymorphisms at two loci appears more frequently than would be expected by chance. "Significant" as used in respect to linkage disequilibrium, as determined by one of skill in the art, is contemplated to be a statistical p or $\alpha$ value that may be 0.25 or 0.1 and may be 0.1, 0.05. 0.001, 0.00001 or less. The polymorphism at position 874 in the NOS3 may be determined by evaluating the nucleic acid sequence of a polymorphism in linkage disequilibrium with the 874 polymorphism. The invention may be implemented in this manner with respect to one or more polymorphisms so as to allow haplotype analysis. "Haplotype" is used according to its plain and ordinary meaning to one skilled in the art. It refers to a collective genotype of two or more alleles or polymorphisms along one of the homologous chromosomes.

The term "polymorphism", as used herein, refers to a difference in the nucleotide or amino acid sequence of a given nucleotide or amino acid region as compared to a nucleotide or amino acid sequence in the corresponding region of another individual of the same species. Preferably, the species is human. A polymorphism is generally defined in relation to a "reference" sequence. In the subject application, "reference" sequence and "wild type" sequence are used interchangeably. Nucleotide polymorphisms include single nucleotide differences, differences in sequence of more than one nucleotide, and single or multiple nucleotide insertions, inversions, substitutions, and deletions. Amino acid polymorphisms include single amino acid differences, differences in sequence of more than one amino acid, and single or multiple amino acid insertions, substitutions, and deletions.

A "biological sample" encompasses a variety of sample types obtained from an individual and can be used in a diagnostic or monitoring assay. The definition encompasses blood and other liquid samples of biological origin, solid

tissue samples such as a biopsy specimen or tissue cultures or cells derived therefrom and the progeny thereof. The definition also includes samples that have been manipulated in any way after their procurement, such as by treatment with reagents, solubilization, or enrichment for certain components, such as polynucleotides. The term biological sample encompasses a clinical sample, and also includes cells in culture, cell supernatants, cell lysates, serum, plasma, biological fluid, and tissue samples. In one embodiment, the sample is collected by the individual. For example, an individual can collect a swap of tissue from the inside of the cheek for use as a nucleic acid sample. As known in the art, many types of samples can be used for the extraction of nucleic acids.

As used herein the term "treating" in reference to a disease or condition means a reduction in severity or elimination of one or more symptoms associated with a particular disease or condition. Therefore, treating a disorder does not necessarily mean a reduction in severity of all symptoms associated with a disorder and does not necessarily mean a complete reduction in the severity of one or more symptoms associated with a disorder. Treatment, as used in this context, covers any treatment of a symptomatic condition, such as an adverse reaction in a mammal, particularly in a human, and includes: (a) diagnosing and then preventing the adverse reaction from occurring in an individual which can be predisposed to the reaction but has not yet been diagnosed as having it; (b) inhibiting the adverse reaction, *i.e.,* arresting its development; and (c) relieving the adverse reaction, ***i**.e*., causing regression of the reaction.

The term "therapeutically effective amount" means an amount that is effective in treating a particular disorder; that is an amount that is effective for reducing the severity of one or more symptoms associated with the particular disease or condition for which treatment is sought. The term "ameliorate," as used for instance in the amelioration of a particular condition means to make one or more symptoms of the condition at least more tolerable, if not better. The term ameliorate does not necessarily mean an increase in toleration of all symptoms associated with a disorder and does not necessarily mean a complete reduction in the severity of one or more symptoms associated with a disorder.

A further step may be added wherein a portion of the NOS3 gene of SEQ ID NO:1 and/or SEQ ID NO:3 is amplified prior to the identifying step. In another embodiment, the identifying is performed by a method selected from the group consisting of a hybridization assay, a sequencing assay, a microsequencing assay, a MALDI-TOF assay, and an allele-specific amplification assay. In a further embodiment, the identifying is performed by an antibody-based assay.

Pharmacogenomics allows a clinician or physician to target prophylactic or therapeutic treatments to individuals who will most benefit from the treatment and to avoid treatment of individuals who will experience symptomatic side effects, in the case of LA-419 the adverse side effect can be tolerance and toxicity to the drug. Differences in metabolism of therapeutics can lead to severe toxicity or therapeutic failure by altering the relation between dose and blood concentration of the pharmacologically active drug. Thus, a physician or clinician may consider applying knowledge obtained in relevant pharmacogenomics studies in determining whether to administer LA-419 as well as tailoring the dosage, regimen, and/or therapeutically effective amounts to be administered so as to attain the effect desired by treatment with the modulator.

A determination of how a given NOS3 polymorphism is predictive of an individual's likelihood of responding to an NO donor can be accomplished by determining the genotype of the individual in the NOS3 gene, as described herein. Information generated from one or more of these approaches can be used to determine appropriate dosage and treatment regimens for prophylactic or therapeutic treatment of an individual. This knowledge, when applied to dosing or drug selection, can avoid adverse reactions or therapeutic failure and thus enhance therapeutic or prophylactic efficiency when treating an individual with a niacin receptor modulator, such as niacin or an analog thereof.

It is disclosed that the difference in protein sequence can provide a basis for determining the nucleotide sequence at position 894. The wildtype NOS3 sequence encodes a Glu at position 298, in contrast to the Asp at that position when there is a T ant position 894. One of skill in the art appreciates that reagents and assays are available to detect the different proteins. In some embodiments, a method or kit involves an antibody that recognizes both forms of the protein or only one form. The antibody may be a monoclonal antibody or a polyclonal antibody.

There are disclosed kits for use in the methods of the invention, for example, a kit for determining a level of probability for an individual for a condition responsiveness to LA-419 therapy, a kit for using a NOS3 zygosity of an individual for determining a suitability or an unsuitability of an individual for inclusion in a clinical trial, or a kit for determining a level of probability for a condition associated with heart failure. A kit can comprise reagents and instructions for performing the methods described herein. For example, a kit can include genotyping reagents such as reagents for isolating nucleic acid molecules and reagents for amplifying nucleic acid molecules such as primers. A kit can also include, for example, a NOS3 assay such as an ELISA. In addition, a kit can contain control samples, for example, to show that amplification reactions are not contaminated.

The contents of the kit are contained in packaging material, preferably to provide a sterile, contaminant-free environment. In addition, the packaging material contains instructions indicating how the materials within the kit can be employed. The instructions for use typically include a tangible expression describing the reagent concentration or at least one assay method parameter, such as the relative amounts of reagent and sample to be admixed, maintenance time periods for reagent/sample admixtures, temperature, buffer conditions, and the like.

## III. METHODS OF TREATING CARDIAC HYPERTROPHY

Once the NOS3 genotype of the individual is determined a therapeutic course of treatment may be individualized. In the method as disclosed herein, the trait of interest is a clinical response exhibited by a patient to some therapeutic treatment, for example, response to a NO donor drug such as, but not limited to, a LA-419. The term "clinical response" means a quantitative measure of the efficacy or potency of the therapy and adverse events (*i.e.,* side effects).

Thus, individuals homozygous for an insertional polymorphism in the NOS3 gene having or suspected of having or at risk of developing heart failure can be placed on a therapy that includes NO donors such as, but not limited to, LA-419. The NO donor may be administered alone or in combination with at least one other agent, such as a stabilizing compound.

### A. Routes of Administration

Administration of the NO donor may be by any number of routes including, but not limited to oral, intravenous, intramuscular, intra-arterial, intramedullary, intrathecal, intraventricular, intradermal, intratracheal, intravesicle, intraocular, transdermal, subcutaneous, intraperitoneal, intranasal, enteral, topical, sublingual, or rectal. Further details on techniques for formulation and administration may be found in the latest edition of Remington's Pharmaceutical Sciences (Maack Publishing Co., Easton, Pa.). In certain embodiments LA-419 or other NO donors are formulated for oral administration.

### B. Formulations

Where clinical applications are contemplated, pharmaceutical compositions will be prepared in a form appropriate for the intended application. Generally, this will entail preparing compositions that are essentially free of pyrogens, as well as other impurities that could be harmful to humans or animals.

One will generally desire to employ appropriate salts and buffers. Aqueous compositions comprise an effective amount of the drug, dissolved or dispersed in a pharmaceutically acceptable carrier or aqueous medium. The phrase "pharmaceutically or pharmacologically acceptable" refer to molecular entities and compositions that do not produce adverse, allergic, or other untoward reactions when administered to an animal or a human. As used herein, "pharmaceutically acceptable carrier" includes solvents, buffers, solutions, dispersion media, coatings, antibacterial and antifungal agents, isotonic and absorption delaying agents and the like acceptable for use in formulating pharmaceuticals, such as pharmaceuticals suitable for administration to humans. The use of such media and agents for pharmaceutically active substances is well known in the art. Except insofar as any conventional media or agent is incompatible with the active ingredients of the present invention, its use in therapeutic compositions is contemplated. Supplementary active ingredients also can be incorporated into the compositions, provided they do not inactivate the vectors or cells of the compositions.

The active compositions of the present invention may include classic pharmaceutical preparations. Administration of these compositions as disclosed herein may be via any common route so long as the target tissue is available via that route. This includes oral, nasal, or buccal. Alternatively, administration may be by intradermal, intraarterial, subcutaneous, intramuscular, intraperitoneal or intravenous injection, or by direct injection into cardiac tissue. Such compositions would normally be administered as pharmaceutically acceptable compositions, as described *supra.*

The active compounds may also be administered parenterally or intraperitoneally. By way of illustration, solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations generally contain a preservative to prevent the growth of microorganisms.

The pharmaceutical forms suitable for injectable use include, for example, sterile aqueous solutions or dispersions and sterile powders for the extemporaneous preparation of sterile injectable solutions or dispersions. Generally, these preparations are sterile and fluid to the extent that easy injectability exists. Preparations should be stable under the conditions of manufacture and storage and should be preserved against the contaminating action of microorganisms, such as bacteria and fungi. Appropriate solvents or dispersion media may contain, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial an antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

The compositions of the present invention generally may be formulated in a neutral or salt form. Pharmaceutically-acceptable salts include, for example, acid addition salts (formed with the free amino groups of the protein) derived from inorganic acids (e.g., hydrochloric or phosphoric acids, or from organic acids (e.g., acetic, oxalic, tartaric, mandelic, and the like. Salts formed with the free carboxyl groups of the protein can also be derived from inorganic bases *(e.g.,* sodium, potassium, ammonium, calcium, or ferric hydroxides) or from organic bases (*e.g.,* isopropylamine, trimethylamine, histidine, procaine and the like.

Upon formulation, solutions are preferably administered in a manner compatible with the dosage formulation and in such amount as is therapeutically effective. The formulations may easily be administered in a variety of dosage forms such as injectable solutions, drug release capsules and the like. For parenteral administration in an aqueous solution, for example, the solution generally is suitably buffered and the liquid diluent first rendered isotonic for example with sufficient saline or glucose. Such aqueous solutions may be used, for example, for intravenous, intramuscular, subcutaneous and intraperitoneal administration. Preferably, sterile aqueous media are employed as is known to those of skill in the art, particularly in light of the present disclosure. By way of illustration, a single dose may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologies standards.

### 1. Tablets and Capsules

Tablet or capsule formulations are well known to those of skill in the art. In some embodiments the drug as in disclosed in U.S. Patent 5,126,145.

In one embodiment, a tablet comprises, in admixture, about 5-30% high viscosity hydroxypropyl methyl cellulose, about 2-15% of a water-soluble pharmaceutical binder, about 2-20% of a hydrophobic component such as a waxy material, *e.g.,* a fatty acid, and about 30-90% active ingredient

### 2. Films

Methods for preventing or treating a patient having a homozygous wildtype polymorphism in the NOS3 gene following an invasive cardiac procedure comprising administering biodegradable, biocompatible polymeric film comprising a NO donor, such as LA-419, to a patient are disclosed. The polymeric films are thin compared to their length and breadth. The films typically have a uniform selected thickness between about 60 micrometers and about 5 mm. Films of between about 600 micrometers and 1 mm and between about 1 mm and about 5 mm thick, as well as films between about 60 micrometers and about 1000 micrometers, and between about 60 and about 300 micrometers are useful in the manufacture of therapeutic implants for insertion into a patient's body. The films can be administered to the patient in a manner similar to methods used in adhesion surgeries. For example, an NO donor film formulation can be sprayed or dropped onto a cardiac tissue site or artery during surgery, or a formed film can be placed over the selected tissue site. In an alternative embodiment, the film can be used as controlled release coating on a medical device such as a stent, as is discussed in further detail below.

Either biodegradable or nonbiodegradable polymers may be used to fabricate implants in which the NO donor is uniformly distributed throughout the polymer matrix. A number of suitable biodegradable polymers for use in making the biodegradable films of this invention are known to the art, including polyanhydrides and aliphatic polyesters, preferably polylactic acid (PLA), polyglycolic acid (PGA) and mixtures and copolymers thereof, more preferably 50:50 copolymers of PLA:PGA and most preferably 75:25 copolymers of PLA:PGA. Single enantiomers of PLA may also be used, preferably L-PLA, either alone or in combination with PGA. Polycarbonates, polyfumarates and caprolactones may also be used to make the implants of this invention.

The amount of the NO donor to be incorporated into the polymeric films of this invention is an amount effective to show a measurable effect in treating diseases having similar pathophysiological states, such as but not limited to heart failure. The composition of the present invention can be incorporated into the film by various techniques such as by solution methods, suspension methods, or melt pressing.

### 3. Transdermal Patch Device

Transdermal delivery involves delivery of a therapeutic agent through the skin for distribution within the body by circulation of the blood. Transdermal delivery can be compared to continuous, controlled intravenous delivery of a drug using the skin as a port of entry instead of an intravenous needle. The therapeutic agent passes through the outer

layers of the skin, diffuses into the capillaries or tiny blood vessels in the skin and then is transported into the main circulatory system.

Transdermal patch devices that provide a controlled, continuous administration of a therapeutic agent through the skin are well known in the art. Such devices, for example, are disclosed in U.S. Patents 4,627,429; 4,784,857; 5,662,925; 5,788,983; and 6,113,940.

Characteristically, these devices contain a drug impermeable backing layer which defines the outer surface of the device and a permeable skin attaching membrane, such as an adhesive layer, sealed to the barrier layer in such a way as to create a reservoir between them in which the therapeutic agent is placed. Some embodiments involve a formulation of the NO donor that is introduced into the reservoir of a transdermal patch and used by a patient who is homozygous wildtype at position 894 at the NOS3 gene.

#### 4. Medical Devices

The incorporation of an NO donor into a medical device is also disclosed that is then positioned to a desired target location within the body, whereupon the NO donor elutes from the medical device. As used herein, "medical device" refers to a device that is introduced temporarily or permanently into a mammal for the prophylaxis or therapy of a medical condition. These devices include any that are introduced subcutaneously, percutaneously or surgically to rest within an organ, tissue or lumen. Medical devices include, but are not limited to, stents, synthetic grafts, artificial heart valves, artificial hearts and fixtures to connect the prosthetic organ to the vascular circulation, venous valves, abdominal aortic aneurysm (AAA) grafts, inferior venal caval filters, catheters including permanent drug infusion catheters, embolic coils, embolic materials used in vascular embolization (*e.g.*, PVA foams), mesh repair materials, a Dracon vascular particle orthopedic metallic plates, rods and screws and vascular sutures.

The medical device such as a stent or graft may be coated with a matrix. The matrix used to coat the stent or graft according to this invention may be prepared from a variety of materials. A primary requirement for the matrix is that it be sufficiently elastic and flexible to remain unruptured on the exposed surfaces of the stent or synthetic graft.

#### 5. Controlled/Extended/Sustained/ProlongedRelease Administration

(1) There are also disclosed methods of treating heart failure patients by delivering a NO donor to a patient, having a homozygous wildtype polymorphism genotype, as a controlled release formulation. As used herein, the terms "controlled," "extended," "sustained," or "prolonged" release of the composition of the present invention will collectively be referred to herein as "controlled release," and includes continuous or discontinuous, and linear or non-linear release of the composition in various embodiments.

### C. Dosages

The amount of an NO donor (*e.g.,* LA-419) that is administered or prescribed to the patient can be about, at least about, or at most about 0.1, 0.5, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500 mg, or any range derivable therein. Alternatively, the amount administered or prescribed may be about, at least about, or at most about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4,0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7. 3.8, 3.9, 4.0, 4.1, 4.2, 4.3, 4.4, 4.5, 4.6, 4.7, 4.8, 4.9, 5.0 mg/kg, or any range derivable therein, with respect to the weight of the patient.

When provided in a discrete amount, each intake of the NO donor can be considered a "dose." A medical practitioner may prescribe or administer multiple doses of the NO donor over a particular time course (treatment regimen) or indefinitely.

The NO donor may be administered 1, 2, 3, 4, 5, 6, 7, 8, 9, 10, 11, 12, 13, 14, 15, 16, 17, 18, 19, 20, 30, 40, 50, 60, 70, 80, or more times or any range derivable therein. It is further contemplated that the drug may be taken for an indefinite period of time or for as long as the patient exhibits symptoms of the medical condition for which an NO donor was prescribed or administered. Also, the drug may be administered every 2, 3, 4, 5, 6, 7, 8, 9, 10, 15, 20, 25, 30, 35, 40, 45, 50, 55 minutes, 1, 2, 3, 4, 5, 6, 7, 8, 9, 10, 11, 12, 13, 14, 15, 16, 17, 18, 19, 20, 21, 22, 23, 24 hours, or 1, 2, 3, 4, 5, 6, 7 days, or 1, 2, 3, 4, 5 weeks, or 1, 2, 3, 4, 5, 6, 7, 8, 9, 10, 11, 12 months or more, or any range derivable therein. Alternatively, it may be administered systemically over any such period of time and be extended beyond more than a year. In certain embodiments, the drug is administered to or by the patient once, twice, three times, four times a

day, or as needed.

### D. Other Therapeutic Options

It is disclosed herein that methods may involve treating the patient with an NO donor that is not LA-419 or that is a diuretic, ACE-I, AII antagonist, beta-blocker, BNP, $Ca^{++}$-blocker, or an HDAC inhibitor. Alternatively, a different NO donor other than LA-419 may be used to treat the patient. These agents may be prescribed for or administered by or to the patient instead of or in addition to an NO donor after the NOS3 polymorphism(s) are evaluated.

As a second therapeutic regimen, the agent may be administered or taken at the same time as LA-419, or either before or after LA-419. The treatment may improve one or more symptoms of the cardiovascular disease or condition such as providing increased exercise capacity, increased cardiac ejection volume, decreased left ventricular end diastolic pressure, decreased pulmonary capillary wedge pressure, increased cardiac output or cardiac index, lowered pulmonary artery pressures, decreased left ventricular end systolic and diastolic dimensions, decreased left and right ventricular wall stress, decreased wall tension and wall thickness, increased quality of life, and decreased disease-related morbidity and mortality.

It is also disclosed to use an NO donor in combination with other therapeutic modalities. Thus, in addition to the therapies described above, one may also provide to the patient more "standard" pharmaceutical cardiac therapies. Examples of other therapies include, without limitation, beta blockers, anti-hypertensives, cardiotonics, anti-thrombotics, vasodilators, hormone antagonists, iontropes, diuretics, endothelin antagonists, calcium channel blockers, phosphodiesterase inhibitors, ACE inhibitors, angiotensin type 2 antagonists and cytokine blockers/inhibitors, and HDAC inhibitors.

Combinations may be achieved by contacting cardiac cells with a single composition or pharmacological formulation that includes both agents, or by contacting the cell with two distinct compositions or formulations, at the same time, wherein one composition includes the expression construct and the other includes the agent. Alternatively, the therapy using an NO donor such as LA-419 may precede or follow administration of the other agent(s) by intervals ranging from minutes to weeks. In embodiments where the other agent and expression construct are applied separately to the cell, one would generally ensure that a significant period of time did not expire between the time of each delivery, such that the agent and expression construct would still be able to exert an advantageously combined effect on the cell. In such instances, it is contemplated that one would typically contact the cell with both modalities within about 12-24 hours of each other and, more preferably, within about 6-12 hours of each other, with a delay time of only about 12 hours being most preferred. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations.

It also is conceivable that more than one administration of either LA-419, or the other agent will be desired. In this regard, various combinations may be employed. By way of illustration, where LA-419 is "A" and the other agent is "B", the following permutations based on 3 and 4 total administrations are exemplary:

A/B/A B/A/B B/B/A A/A/B B/A/A A/B/B B/B/B/A B/B/A/B

A/A/B/B A/B/A/B A/B/B/A B/B/A/A B/A/B/A B/A/A/B B/B/B/A

A/A/A/B B/A/A/A A/B/A/A A/A/B/A A/B/B/B B/A/B/B B/B/A/B

Other combinations are likewise contemplated.

### 1. Pharmacological Therapeutic Agents

Pharmacological therapeutic agents and methods of administration, dosages, *etc.,* are well known to those of skill in the art (see for example, the "Physicians Desk Reference", Klaassen's "The Pharmacological Basis of Therapeutics", "Remington's Pharmaceutical Sciences", and "The Merck Index, Eleventh Edition"

and may be combined with the invention in light of the disclosures herein. Some variation in dosage will necessarily occur depending on the condition of the subject being treated. The person responsible for administration will, in any event, determine the appropriate dose for the individual subject, and such invidual determinations are within the skill of those of ordinary skill in the art.

Non-limiting examples of a pharmacological therapeutic agent that may be used in the present invention include an antihyperlipoproteinemic agent, an antiarteriosclerotic agent, an antithrombotic/fibrinolytic agent, a blood coagulant, an antiarrhythmic agent, an antihypertensive agent, a vasopressor, a treatment agent for congestive heart failure, an antianginal agent, an antibacterial agent or a combination thereof.

**a. Antihyperlipoproteinemics**

In certain embodiments, administration of an agent that lowers the concentration of one of more blood lipids and/or lipoproteins, known herein as an "antihyperlipoproteinemic," may be combined with a cardiovascular therapy according to the present invention, particularly in treatment of athersclerosis and thickenings or blockages of vascular tissues. In certain aspects, an antihyperlipoproteinemic agent may comprise an aryloxyalkanoic/fibric acid derivative, a resin/bile acid sequesterant, a HMG CoA reductase inhibitor, a nicotinic acid derivative, a thyroid hormone or thyroid hormone analog, a miscellaneous agent or a combination thereof.

(1) Aryloxyalkanoic Acid/Fibric Acid Derivatives

Non-limiting examples of aryloxyalkanoic/fibric acid derivatives include beclobrate, enzafibrate, binifibrate, ciprofibrate, clinofibrate, clofibrate (atromide-S), clofibric acid, etofibrate, fenofibrate, gemfibrozil (lobid), nicofibrate, pirifibrate, ronifibrate, simfibrate and theofibrate.

(2) Resins/Bile Acid Sequesterants

Non-limiting examples of resins/bile acid sequesterants include cholestyramine (cholybar, questran), colestipol (colestid) and polidexide.

(3) HMG CoA Reductase Inhibitors

Non-limiting examples of HMG CoA reductase inhibitors include lovastatin (mevacor), pravastatin (pravochol) or simvastatin (zocor).

(4) Nicotinic Acid Derivatives

Non-limiting examples of nicotinic acid derivatives include nicotinate, acepimox, niceritrol, nicoclonate, nicomol and oxiniacic acid.

(5) Thryroid Hormones and Analogs

Non-limiting examples of thyroid hormones and analogs thereof include etoroxate, thyropropic acid and thyroxine.

(6) Miscellaneous Antihyperlipoproteinemics

Non-limiting examples of miscellaneous antihyperlipoproteinemics include acifran, azacosterol, benfluorex, β-benzalbutyramide, carnitine, chondroitin sulfate, clomestrone, detaxtran, dextran sulfate sodium, 5,8, 11, 14, 17-eicosapentaenoic acid, eritadenine, furazabol, meglutol, melinamide, mytatrienediol, ornithine, γ-oryzanol, pantethine, pentaerythritol tetraacetate, α-phenylbutyramide, pirozadil, probucol (lorelco), β-sitosterol, sultosilic acid-piperazine salt, tiadenol, triparanol and xenbucin.

**b. Antiarteriosclerotics**

Non-limiting examples of an antiarteriosclerotic include pyridinol carbamate.

**c. Antithrombotic/Fibrinolytic Agents**

It is also disclosed that administration of an agent that aids in the removal or prevention of blood clots may be combined with administration of a modulator, particularly in treatment of athersclerosis and vasculature (*e.g.,* arterial) blockages. Non-limiting examples of antithrombotic and/or fibrinolytic agents include anticoagulants, anticoagulant antagonists, antiplatelet agents, thrombolytic agents, thrombolytic agent antagonists or combinations thereof

In certain aspects, antithrombotic agents that can be administered orally, such as, for example, aspirin and wafarin (coumadin), are preferred.

(1) Anticoagulants

A non-limiting example of an anticoagulant include acenocoumarol, ancrod, anisindione, bromindione, clorindione, coumetarol, cyclocumarol, dextran sulfate sodium, dicumarol, diphenadione, ethyl biscoumacetate, ethylidene dicoumarol, fluindione, heparin, hirudin, lyapolate sodium, oxazidione, pentosan polysulfate, phenindione, phenprocoumon, phosvitin, picotamide, tioclomarol and warfarin.

(2) Antiplatelet Agents

Non-limiting examples of antiplatelet agents include aspirin, a dextran, dipyridamole (persantin), heparin, sulfinpyranone (anturane) and ticlopidine (ticlid).

(3) Thrombolytic Agents

Non-limiting examples of thrombolytic agents include tissue plaminogen activator (activase), plasmin, pro-urokinase, urokinase (abbokinase) streptokinase (streptase), anistreplase/APSAC (eminase).

**d. Blood Coagulants**

It is disclosed that if patient is suffering from a hemmorage or an increased likelyhood of hemmoraging, an agent that may enhance blood coagulation may be used. Non-limiting examples of a blood coagulation promoting agent include thrombolytic agent antagonists and anticoagulant antagonists.

(1) Anticoagulant Antagonists

Non-limiting examples of anticoagulant antagonists include protamine and vitamine Kl.

(2) Thrombolytic Agent Antagonists and Antithrombotics

Non-limiting examples of thrombolytic agent antagonists include amiocaproic acid (amicar) and tranexamic acid (amstat). Non-limiting examples of antithrombotics include anagrelide, argatroban, cilstazol, daltroban, defibrotide, enoxaparin, fraxiparine, indobufen, lamoparan, ozagrel, picotamide, plafibride, tedelparin, ticlopidine and triflusal.

**e. Antiarrhythmic Agents**

Non-limiting examples of antiarrhythmic agents include Class I antiarrythmic agents (sodium channel blockers), Class II antiarrythmic agents (beta-adrenergic blockers), Class II antiarrythmic agents (repolarization prolonging drugs), Class IV antiarrhythmic agents (calcium channel blockers) and miscellaneous antiarrythmic agents.

(1) Sodium Channel Blockers

Non-limiting examples of sodium channel blockers include Class IA, Class IB and Class IC antiarrhythmic agents. Non-limiting examples of Class IA antiarrhythmic agents include disppyramide (norpace), procainamide (pronestyl) and quinidine (quinidex). Non-limiting examples of Class IB antiarrhythmic agents include lidocaine (xylocaine), tocainide (tonocard) and mexiletine (mexitil). Non-limiting examples of Class IC antiarrhythmic agents include encainide (enkaid) and flecainide (tambocor).

(2) Beta Blockers

Non-limiting examples of a beta blocker, otherwise known as a β-adrenergic blocker, a β-adrenergic antagonist or a Class II antiarrhythmic agent, include acebutolol (sectral), alprenolol, amosulalol, arotinolol, atenolol, befunolol, betaxolol, bevantolol, bisoprolol, bopindolol, bucindolol, bucumolol, bufetolol, bufuralol, bunitrolol, bupranolol, butidrine hydrochloride, butofilolol, carazolol, carteolol, carvedilol, celiprolol, cetamolol, cloranolol, dilevalol, epanolol, esmolol (brevibloc), indenolol, labetalol, levobunolol, mepindolol, metipranolol, metoprolol, moprolol, nadolol, nadoxolol, nifenalol, nipradilol, oxprenolol, penbutolol, pindolol, practolol, pronethalol, propanolol (inderal), sotalol (betapace), sulfinalol, talinolol, tertatolol, timolol, toliprolol and xibinolol. In certain aspects, the beta blocker comprises an aryloxypropanolamine derivative. Non-limiting examples of aryloxypropanolamine derivatives include acebutolol, alprenolol, arotinolol, atenolol, betaxolol, bevantolol, bisoprolol, bopindolol, bunitrolol, butofilolol, carazolol, carteolol, carvedilol, celiprolol, cetamolol,

epanolol, indenolol, mepindolol, metipranolol, metoprolol, moprolol, nadolol, nipradilol, oxprenolol, penbutolol, pindolol, propanolol, talinolol, tertatolol, timolol and toliprolol.

(3) Repolarization Prolonging Agents

Non-limiting examples of an agent that prolong repolarization, also known as a Class III antiarrhythmic agent, include amiodarone (cordarone) and sotalol (betapace).

(4) Calcium Channel Blockers/Antagonist

Non-limiting examples of a calcium channel blocker, otherwise known as a Class IV antiarrythmic agent, include an arylalkylamine (e.g., bepridile, diltiazem, fendiline, gallopamil, prenylamine, terodiline, verapamil), a dihydropyridine derivative (felodipine, isradipine, nicardipine, nifedipine, nimodipine, nisoldipine, nitrendipine) a piperazinde derivative (*e.g.,* cinnarizine, flunarizine, lidoflazine) or a micellaneous calcium channel blocker such as bencyclane, etafenone, magnesium, mibefradil or perhexiline. In certain embodiments a calcium channel blocker comprises a long-acting dihydropyridine (nifedipine-type) calcium antagonist.

(5) Miscellaneous Antiarrhythmic Agents

Non-limiting examples of miscellaneous antiarrhymic agents include adenosine (adenocard), digoxin (lanoxin), acecainide, ajmaline, amoproxan, aprindine, bretylium tosylate, bunaftine, butobendine, capobenic acid, cifenline, disopyranide, hydroquinidine, indecainide, ipatropium bromide, lidocaine, lorajmine, lorcainide, meobentine, moricizine, pirmenol, prajmaline, propafenone, pyrinoline, quinidine polygalacturonate, quinidine sulfate and viquidil.

**f. Antihypertensive Agents**

Non-limiting examples of antihypertensive agents include sympatholytic, alpha/beta blockers, alpha blockers, anti-angiotensin II agents, beta blockers, calcium channel blockers, vasodilators and miscellaneous antihypertensives.

(1) Alpha Blockers

Non-limiting examples of an alpha blocker, also known as an $\alpha$-adrenergic blocker or an $\alpha$-adrenergic antagonist, include amosulalol, arotinolol, dapiprazole, doxazosin, ergoloid mesylates, fenspiride, indoramin, labetalol, nicergoline, prazosin, terazosin, tolazoline, trimazosin and yohimbine. In certain embodiments, an alpha blocker may comprise a quinazoline derivative. Non-limiting examples of quinazoline derivatives include alfuzosin, bunazosin, doxazosin, prazosin, terazosin and trimazosin.

(2) Alpha/Beta Blockers

It is disclosed that an antihypertensive agent is both an alpha and beta adrenergic antagonist. Non-limiting examples of an alpha/beta blocker comprise labetalol (normodyne, trandate).

(3) Anti-Angiotension II Agents

Non-limiting examples of anti-angiotension II agents include include angiotensin converting enzyme inhibitors and angiotension II receptor antagonists. Non-limiting examples of angiotension converting enzyme inhibitors (ACE inhibitors) include alacepril, enalapril (vasotec), captopril, cilazapril, delapril, enalaprilat, fosinopril, lisinopril, moveltopril, perindopril, quinapril and ramipril.. Non-limiting examples of an angiotensin II receptor blocker, also known as an angiotension II receptor antagonist, an ANG receptor blocker or an ANG-II type-1 receptor blocker (ARBS), include angiocandesartan, eprosartan, irbesartan, losartan and valsartan.

(4) Sympatholytics

Non-limiting examples of a sympatholytic include a centrally acting sympatholytic or a peripherially acting sympatholytic. Non-limiting examples of a centrally acting sympatholytic, also known as an central nervous system (CNS) sympatholytic, include clonidine (catapres), guanabenz (wytensin) guanfacine (tenex) and methyldopa (aldomet). Non-limiting examples of a peripherally acting sympatholytic include a ganglion blocking agent, an adrenergic neuron blocking agent, a $\beta$-adrenergic blocking agent or a alphal-adrenergic blocking agent. Non-limiting examples of a ganglion

blocking agent include mecamylamine (inversine) and trimethaphan (arfonad). Non-limiting of an adrenergic neuron blocking agent include guanethidine (ismelin) and reserpine (serpasil). Non-limiting examples of a β-adrenergic blocker include acenitolol (sectral), atenolol (tenormin), betaxolol (kerlone), carteolol (cartrol), labetalol (normodyne, trandate), metoprolol (lopressor), nadanol (corgard), penbutolol (levatol), pindolol (visken), propranolol (inderal) and timolol (blocadren). Non-limiting examples of alphal-adrenergic blocker include prazosin (minipress), doxazocin (cardura) and terazosin (hytrin).

(5) Vasodilators

It is disclosed that a cardiovasculator therapeutic agent may comprise a vasodilator (*e.g.,* a cerebral vasodilator, a coronary vasodilator or a peripheral vasodilator). In certain preferred embodiments, a vasodilator comprises a coronary vasodilator. Non-limiting examples of a coronary vasodilator include amotriphene, bendazol, benfurodil hemisuccinate, benziodarone, chloracizine, chromonar, clobenfurol, clonitrate, dilazep, dipyridamole, droprenilamine, efloxate, erythrityl tetranitrane, etafenone, fendiline, floredil, ganglefene, herestrol bis(β-diethylaminoethyl ether), hexobendine, itramin tosylate, khellin, LA-4195, lidoflanine, mannitol hexanitrane, medibazine, nicorglycerin, pentaerythritol tetranitrate, pentrinitrol, perhexiline, pimefylline, trapidil, tricromyl, trimetazidine, trolnitrate phosphate and visnadine.

In certain aspects, a vasodilator may comprise a chronic therapy vasodilator or a hypertensive emergency vasodilator. Non-limiting examples of a chronic therapy vasodilator include hydralazine (apresoline) and minoxidil (loniten). Non-limiting examples of a hypertensive emergency vasodilator include nitroprusside (nipride), diazoxide (hyperstat IV), hydralazine (apresoline), minoxidil (loniten) and verapamil.

(6) Miscellaneous Antihypertensives

Non-limiting examples of miscellaneous antihypertensives include ajmaline, γ-aminobutyric acid, bufeniode, cicletainine, ciclosidomine, a cryptenamine tannate, fenoldopam, flosequinan, ketanserin, mebutamate, mecamylamine, methyldopa, methyl 4-pyridyl ketone thiosemicarbazone, muzolimine, pargyline, pempidine, pinacidil, piperoxan, primaperone, a protoveratrine, raubasine, rescimetol, rilmenidene, saralasin, sodium nitrorusside, ticrynafen, trimethaphan camsylate, tyrosinase and urapidil.

In certain aspects, an antihypertensive may comprise an arylethanolamine derivative, a benzothiadiazine derivative, a *N*-carboxyalkyl(peptide/lactam) derivative, a dihydropyridine derivative, a guanidine derivative, a hydrazines/phthalazine, an imidazole derivative, a quanternary ammonium compound, a reserpine derivative or a suflonamide derivative.

**Arylethanolamine Derivatives.** Non-limiting examples of arylethanolamine derivatives include amosulalol, bufuralol, dilevalol, labetalol, pronethalol, sotalol and sulfinalol.

**Benzothiadiazine Derivatives.** Non-limiting examples of benzothiadiazine derivatives include althizide, bendroflumethiazide, benzthiazide, benzylhydrochlorothiazide, buthiazide, chlorothiazide, chlorthalidone, cyclopenthiazide, cyclothiazide, diazoxide, epithiazide, ethiazide, fenquizone, hydrochlorothizide, hydroflumethizide, methyclothiazide, meticrane, metolazone, paraflutizide, polythizide, tetrachlormethiazide and trichlormethiazide.

***N*-carboxyalkyl(peptide/lactam) Derivatives.** Non-limiting examples of *N*-carboxyalkyl(peptide/lactam) derivatives include alacepril, captopril, cilazapril, delapril, enalapril, enalaprilat, fosinopril, lisinopril, moveltipril, perindopril, quinapril and ramipril.

**Dihydropyridine Derivatives.** Non-limiting examples of dihydropyridine derivatives include amlodipine, felodipine, isradipine, nicardipine, nifedipine, nilvadipine, nisoldipine and nitrendipine.

**Guanidine Derivatives.** Non-limiting examples of guanidine derivatives include bethanidine, debrisoquin, guanabenz, guanacline, guanadrel, guanazodine, guanethidine, guanfacine, guanochlor, guanoxabenz and guanoxan.

**Hydrazines/Phthalazines.** Non-limiting examples of hydrazines/phthalazines include budralazine, cadralazine, dihydralazine, endralazine, hydracarbazine, hydralazine, pheniprazine, pildralazine and todralazine.

**Imidazole Derivatives.** Non-limiting examples of imidazole derivatives include clonidine, lofexidine, phentolamine, tiamenidine and tolonidine.

**Quanternary Ammonium Compounds.** Non-limiting examples of quanternary ammonium compounds include azamethonium bromide, chlorisondamine chloride, hexamethonium, pentacynium bis(methylsulfate), pentamethonium bromide, pentolinium tartrate, phenactropinium chloride and trimethidinium methosulfate.

**Reserpine Derivatives.** Non-limiting examples of reserpine derivatives include bietaserpine, deserpidine, rescinnamine, reserpine and syrosingopine.

**Suflonamide Derivatives.** Non-limiting examples of sulfonamide derivatives include ambuside, clopamide, furosemide, indapamide, quinethazone, tripamide and xipamide.

(7) Vasopressors

Vasopressors generally are used to increase blood pressure during shock, which may occur during a surgical procedure. Non-limiting examples of a vasopressor, also known as an antihypotensive, include amezinium methyl sulfate, angiotensin amide, dimetofrine, dopamine, etifelmin, etilefrin, gepefrine, metaraminol, midodrine, norepinephrine, pholedrine and synephrine.

**g. Treatment Agents for Congestive Heart Failure**

Non-limiting examples of agents for the treatment of congestive heart failure include anti-angiotension II agents, afterload-preload reduction treatment, diuretics and inotropic agents.

(1) Afterload-Preload Reduction

It is also disclosed that an animal patient that can not tolerate an angiotension antagonist may be treated with a combination therapy. Such therapy may combine adminstration of hydralazine (apresoline) and isosorbide dinitrate (isordil, sorbitrate).

(2) Diuretics

Non-limiting examples of a diuretic include a thiazide or benzothiadiazine derivative (*e.g.*, althiazide, bendroflumethazide, benzthiazide, benzylhydrochlorothiazide, buthiazide, chlorothiazide, chlorothiazide, chlorthalidone, cyclopenthiazide, epithiazide, ethiazide, ethiazide, fenquizone, hydrochlorothiazide, hydroflumethiazide, methyclothiazide, meticrane, metolazone, paraflutizide, polythizide, tetrachloromethiazide, trichlormethiazide), an organomercurial (*e.g.,* chlormerodrin, meralluride, mercamphamide, mercaptomerin sodium, mercumallylic acid, mercumatilin dodium, mercurous chloride, mersalyl), a pteridine (*e.g.*, furterene, triamterene), purines (*e.g.*, acefylline, 7-morpholinomethyltheophylline, pamobrom, protheobromine, theobromine), steroids including aldosterone antagonists (*e.g.*, canrenone, oleandrin, spironolactone), a sulfonamide derivative (*e.g.*, acetazolamide, ambuside, azosemide, bumetanide, butazolamide, chloraminophenamide, clofenamide, clopamide, clorexolone, diphenylmethane-4,4'-disulfonamide, disulfamide, ethoxzolamide, furosemide, indapamide, mefruside, methazolamide, piretanide, quinethazone, torasemide, tripamide, xipamide), a uracil *(e.g.,* aminometradine, amisometradine), a potassium sparing antagonist (*e.g.,* amiloride, triamterene) or a miscellaneous diuretic such as aminozine, arbutin, chlorazanil, ethacrynic acid, etozolin, hydracarbazine, isosorbide, mannitol, metochalcone, muzolimine, perhexiline, ticrnafen and urea.

(3) Inotropic Agents

Non-limiting examples of a positive inotropic agent, also known as a cardiotonic, include acefylline, an acetyldigitoxin, 2-amino-4-picoline, amrinone, benfurodil hemisuccinate, bucladesine, cerberosine, camphotamide, convallatoxin, cymarin, denopamine, deslanoside, digitalin, digitalis, digitoxin, digoxin, dobutamine, dopamine, dopexamine, enoximone, erythrophleine, fenalcomine, gitalin, gitoxin, glycocyamine, heptaminol, hydrastinine, ibopamine, a lanatoside, metamivam, milrinone, nerifolin, oleandrin, ouabain, oxyfedrine, prenalterol, proscillaridine, resibufogenin, scillaren, scillarenin, strphanthin, sulmazole, theobromine and xamoterol.

In particular aspects, an intropic agent is a cardiac glycoside, a beta-adrenergic agonist or a phosphodiesterase inhibitor. Non-limiting examples of a cardiac glycoside includes digoxin (lanoxin) and digitoxin (crystodigin). Non-limiting examples of a β-adrenergic agonist include albuterol, bambuterol, bitolterol, carbuterol, clenbuterol, clorprenaline, denopamine, dioxethedrine, dobutamine (dobutrex), dopamine (intropin), dopexamine, ephedrine, etafedrine, ethylnorepinephrine, fenoterol, formoterol, hexoprenatine, ibopamine, isoetharine, isoproterenol, mabuterol, metaproterenol, methoxyphenamine, oxyfedrine, pirbuterol, procaterol, protokylol, reproterol, rimiterol, ritodrine, soterenol, terbutaline, tretoquinol, tulobuterol and xamoterol. Non-limiting examples of a phosphodiesterase inhibitor include enoximone and amrinone (inocor).

(4) Antianginal Agents

Antianginal agents may comprise organonitrates, calcium channel blockers, beta blockers and combinations thereof.

Non-limiting examples of organonitrates, also known as nitrovasodilators, include nitroglycerin (nitro-bid, nitrostat), isosorbide dinitrate (isordil, sorbitrate) and amyl nitrate (aspirol, vaporole).

2. **Surgical Therapeutic Agents**

In certain aspects, the secondary therapeutic agent may comprise a surgery of some type, which includes, for example, preventative, diagnostic or staging, curative and palliative surgery. Surgery, and in particular a curative surgery, may be used in conjunction with other therapies, such as the present invention and one or more other agents.
Such surgical therapeutic agents for vascular and cardiovascular diseases and disorders are well known to those of skill in the art, and may comprise, but are not limited to, performing surgery on an organism, providing a cardiovascular mechanical prostheses, angioplasty, coronary artery reperfusion, catheter ablation, providing an implantable cardioverter defibrillator to the subject, mechanical circulatory support or a combination thereof. Non-limiting examples of a mechanical circulatory support that may be used in the present invention comprise an intra-aortic balloon counterpulsation, left ventricular assist device or combination thereof.

**IV. KITS**

There are kits disclosed for the detection of one or more NOS3 polymorphisms. It is disclosed that the polymorphism is at position 894. It is disclosed that the kits contain reagents specific for the detection or analysis of DNA (*e.g.,* oligonucleotide probes or primers). It is disclosed that the kits contain all of the components necessary to perform a detection assay, including all controls, directions for performing assays, and any necessary software for analysis and presentation of results. It is disclosed that individual probes and reagents for detection of NOS3 polymorphisms are provided as analyte specific reagents. It is disclosed that the kits are provided as in vitro diagnostics.

**V. EXAMPLES**

The following examples are given for the purpose of illustrating various embodiments of the invention and are not meant to limit the present invention in any fashion. One skilled in the art will appreciate readily that the present invention is well adapted to carry out the objects and obtain the ends and advantages mentioned, as well as those objects, ends and advantages inherent herein.

**EXAMPLE 1**

**COMPARISON OF LA-419 AND ISDN**

Donors and Cell Cultures

Human umbilical vein endothelial cells (HUVECs) were isolated into primary cultures. and purchased as proliferating cells from Clonetics (San Diego, California). All cell culture donors were healthy, with no pregnancy or prenatal complications. The cultured cells were incubated in 95% air / 5% $CO_2$ at 37°C and passage by an enzymatic (trypsin) procedure. The confluent cells (4 to 5 x $10^5$ cells per 35-mm dish) were placed with minimum essential medium containing 3 mM L-arginine and 0.1 mM $BH_4$ [(6R)-5,6,7,8-tetrahydrobiopterin]. Before the experiments, the cells (from second or third passage) were rinsed twice with Tyrode-HEPES buffer with 1.8 mM $CaCl_2$. ISDN was obtained from Sigma-Aldrich.

**Measurement of NO and $ONOO^-$ Levels**

Concurrent measurements of NO and $ONOO^-$ were carried out with tandem electrochemical nanosensors combined into one working unit with a total diameter of 200-500 nm (FIG. 1). Their design was based on previously developed and well-characterized chemically modified carbon-fiber technology (Malinski and Taha, 1992; Lvovich and Scheeline, 1997). Each of the nanosensors was made by depositing a sensing material on the tip of a carbon fiber (length 4-5 $\mu$m, diameter 0.2-0.5 $\mu$m). The fibers were sealed with nonconductive epoxy and electrically connected to copper wires with conductive silver epoxy. A conductive film of polymeric nickel (II) tetrakis (3-methoxy-4-hydroxyphenyl) porphyrin for the NO-sensor and polymeric film of Mn (III) [2.2] paracyclophahylporphyrin was used for the $ONOO^-$-sensor.
A module of NO/$ONOO^-$ nanosensors (diameter 1-2 $\mu$m) with a platinum wire (0.1 mm) counter electrode and saturated calomel reference electrode (SCE) were applied. Differential pulse voltammetry (DPV) and amperometry were performed with a computer-based Gamry VFP600 multichannel potentiostat. DPV was used to measure the basal NO and $ONOO^-$ concentrations, and amperometry was used to measure changes in NO and $ONOO^-$ concentrations from its basal level with time. The DPV current at the peak potential characteristic for NO oxidation (0.65 V) and $ONOO^-$ reduction (-0.45 V) was directly proportional to the local concentrations of these compounds in the immediate vicinity of the sensor. Linear calibration curves (current vs. concentration) were constructed for each sensor from 10 nM to 3 $\mu$M before and after measurements with aliquots of NO and $ONOO^-$ standard solutions, respectively. The detection limit of

the sensors was 1.0 nM.

The quantification of each analyte (concentration in nM) was performed using a maximum current from amperograms and standard calibration curves. A reproducibility of measurements with nanosensors is relatively high (between 5-12%). The NO/$ONOO^-$ nanosensor module was lowered with the help of a computer-controlled micromanipulator until it reached the surface of the cell membrane (a small piezoelectric signal, 0.1-0.2 pA, of 1-3 milliseconds duration was observed at this point). The sensors were slowly raised 5 $\pm$ 2 $\mu$m from the surface of a single endothelial cell. The eNOS agonist, calcium ionophore A23187 (CaI) as well as the different drugs were then added to the surrounding media at the cell surface with a nanoinjector that was also positioned by a computer controlled-micromanipulator.

The HUVEC preparation is stable over the course of these experiments with the cells remaining viable in culture for > 24 hours. Under non-stimulating conditions, basal levels of NO release are very low (< 30 nM). Measurement of NO release as a function of treatment was conducted in individual endothelial cells. Multiple measurements of NO release can be conducted on single cells following a brief refractory period. For robust statistical analysis, separate cells were used for each concentration and type of drug used in these analyses.

**Results**

A comparison was made about the effects of ISDN versus LA-419 on endothelial-dependent NO and ONOO- release in endothelium from white and African American donors. Exposure of human endothelial cells to ISDN for an extended period of time (12 h) had deleterious effects on their capacity to generate NO while causing a concomitant increase nitroxidative stress. These adverse effects of a conventional NO donor on endothelial function may contribute to mechanisms of tolerance and raise risk for atherothrombotic disease. Pretreatment of cells with ISDN resulted in a pronounced *decrease* in NO release by 11% (non-Hispanic whites) and 12% (African Americans) while increasing levels of ONOO- by 16% (whites) and 18% (African Americans) (FIGs. 2-3). These effects resulted in an overall *adverse* effect of ISDN on eNOS function as evidenced by a decrease in the NO/$ONOO^-$ ratio by 23% (non-Hispanic whites, $p<0.01$) and 25% (African Americans, $p<0.05$), as shown in FIG. 4. By contrast, treatment with LA-419 caused an opposite and small *increase* in NO release in HUVECs from both non-Hispanic whites and African Americans under identical conditions (FIGs. 2-3). As compared to ISDN, the NO/$ONOO^-$ ratio was not significantly different from control samples (FIG. 4), thus not disrupting eNOS function. These findings indicate that ISDN has a detrimental effect on endothelial function and eNOS coupling mechanisms in a manner that was not observed with LA-419, even in cells from African American donors as shown in the Table below:

| **Treatments** | **Non-Hispanic Whites** | | **African Americans** | |
|---|---|---|---|---|
| | **NO** | **$ONOO^-$** | **NO** | $ONOO^-$ |
| Control | 384 $\pm$ 24 | 279 $\pm$ 9 | 245 $\pm$ 13 | 468 $\pm$ 29 |
| LA-419 | 409 $\pm$ 30 | 307 $\pm$ 11 | 253 $\pm$ 15 | 532 $\pm$ 25 |
| ISDN | 343 $\pm$ 11 | 323 $\pm$ 9 | 218 $\pm$ 16 | 552 $\pm$ 11 |
| Values are mean $\pm$ S.D. (units = nM) | | | | |

These results of our analysis demonstrate an adverse effect of ISDN on endothelial function that may contribute to mechanisms of tolerance with use that was not observed with LA-419.

In these experiments LA-419 compared favorably against ISDN in its effect on endothelial-dependent NO bioavailability. In fact, these agents had opposite effects on eNOS function in cells from both white and African American donors who were well matched for CV risk. Thus, this is evidence that LA-419 produces less tolerance while maintaining vascular endothelial function compared to older nitrates such as ISDN.

## EXAMPLE 2

## EFFECT OF ENOS VARIANTS WITH LA-419

This example describes the genotype-specific response of the three major eNOS variants to 1$\mu$M and 5$\mu$M concentrations of LA-419. The three major genetic polymorphisms studied were the Glu298Asp variant (E298D or 894G$\rightarrow$T) in exon 7 of chromosome 17, the 786T$\rightarrow$C variant in the promoter region, and the intron 4 polymorphism (VNTR). All of these variants have possible effects on the cardiovascular system and endothelial dysfunction. For this study, human umbilical vein endothelial cells (HUVECs) from 50 donors between 18 and 32 years of age were used.

For the analysis several analysis techniques were employed. First, the effect of genotype on NO and ONOO-

release was explored within the different concentrations of LA-419 (control, 1μM, 5μM), calculated as the average peak release (nmols/L) of NO, $ONOO^-$, and $NO/ONOO^-$. The effect of genotype on the net increase in NO, $ONOO^-$, and $NO/ONOO^-$ for LA-419 (1μM and 5μM) as compared to control was also measured. In addition, an anlysis was done on the effect of genotype on the % change in NO, $ONOO^-$, and $NOIONOO^-$ production for LA-419 (1μM and 5μM), calculated as:

$$\% \, Change = [(avg.\ LA\text{-}419 - \text{avg. } control)/(avg.\ control)]*100$$

Finally, an evaluation was undertaken concerning the effect of genotype on the production of NO, $ONOO^-$, and $NO/ONOO^-$ as the fold difference of control for LA-419 (1μM and 5μM), calculated as:

*Fold Difference= (avg. LA-419*)/(*avg. control*)

Within each calculation technique the results were compared for statistical significance using a non-parametric ANOVA Kruskal-Wallace test or a non-parametric Mann-Whitney U test. Statistical significance was set at a p-value <0.05 in a two-tailed distribution. Because experiment-experiment variability is reduced by the normalization to each experiment's control, the % change or fold difference measurements are considered more statistically robust than the net increase or decrease data.

***Materials and Methods***

**eNOS Activity and Protein Abundance Measurements**

Same as Example 1.

**eNOS Genotyping**

The three major variants of the eNOS (NOS 3) gene that have possible effects on the cardiovascular system are Glu298Asp (E298D or 894G>T) in exon 7 of chromosome 17, - 786T>C in the promoter region and intron 4 (VNTR). One million HUVECs supplied by Elucida and shipped frozen were used for DNA extraction. GE Healthcare's Illustra Tissue and Cells GenomicPrep Mini Spin Kit was used to isolate DNA for genotyping.

**Glu298Asp (G→T) Polymorphism**

This exon 7 polymorphism is associated with acute myocardial infarction (AMI), increased risk for coronary heart disease (CHD), coronary spasm, early atherosclerosis, premature MI and abdominal aortic aneurism. Asp 298 (T894) is associated with AMI susceptibility, carotid atherosclerosis, CHD and recent MI's, severe CHD, early atherosclerosis, and coronary vasopastic angina (Napoli *et al.,* 2006). All these conditions have in common endothelial dysfunction, and the Asp 298 allele is associated with endothelial dysfunction even in normal volunteers (Imamura *et al.,* 2004) as well as in isolated human coronary arteries (Erbs *et al.,* 2006). The mechanism by which the Asp 298 allele exerts these effects has not been determined.

Exon 7 genotypes were identified by PCR-RFLP methodology. The primers used were: Forward, aaggcaggagacagtggatgga (SEQ ID NO:5); Reverse, cccagtcaatccctttggtgctca (SEQ ID NO:6). The cycling conditions were 94°C 4 minutes for 1 cycle; 94°C 30 seconds, 65°C 30 seconds, and 72°C 1 minute for 35 cycles; and 72°C for 5 minutes. PCR was followed by a restriction enzyme digest of the 258 bp product using Ban II to yield bands of 162 and 85 bp to indicate homozygous wild type, 258, 163, and 85 bp indicate a heterozygote and a homozygous mutant was uncut.

**-786 T→C Promoter Polymorphism**

This promoter polymorphism is associated with an imbalance of autonomic activity in CHD (Napoli *et al.,* 2006). T→C is associated with severe internal carotid artery disease but not with early atherosclerosis (Napoli *et al.,* 2006). CC carriers have increased endothelial dysfunction due to less generation of NO (Erbs *et al.,* 2006), which is likely to promote atherosclerosis and increase plaque formation.[2]

Promoter genotypes were also identified by PCR-RFLP methodology. The primers used were: Forward, tggagagtgctggtgtacccca (SEQ ID NO:7); Reverse, gcctccacccccaccctgtc (SEQ ID NO:8). The cycling conditions were 94°C 4 minutes for 1 cycle; 94°C 30 seconds, 65°C 30 seconds, and 72°C 1 minute for 35 cycles; and 72°C for 5 minutes.

PCR was followed by a restriction enzyme digest of the 180 bp product using MSP I to yield bands of 140 and 40 bp to indicate homozygous wild type, 140, 90, 50, and 40 bp indicate a heterozygote and a homozygous mutant gave bands of 90, 50, and 40 bp.

**Intron 4 polymorphism**

This variable tandem repeat polymorphism ("4a/4b") may also be associated with acute coronary syndromes and CHD (Napoli *et al.,* 2006) due to lower levels of NO production (Li *et al.,* 2004).

Intron 4 variants were identified by PCR and gel electrophoresis. The primers used were: Forward, aggccctatggtagtgccttt (SEQ ID NO:9); Reverse, tctcttagtgctgtggtcac (SEQ ID NO:10). The cycling conditions were 94°C 4 minutes for 1 cycle; 94°C 30 seconds, 63°C 30 seconds, and 72°C 1 minute for 35 cycles; and 72°C for 5 minutes. The wild type product, 4b/4b, yielded a 420 bp fragment. A heterozygote (4b/4a) gave bands of 420 and 393 bp. The mutant 4a/4a gave a 393 bp band; 4c/4c gave a 447 bp band and 4y/4y gave a 339 bp band.

*Results*

**Effect of Genotype within Group, Peak Release Measurements:** (Group is defined as control, LA-419 5 μM, and LA-419 1 μM)

The effect of genotype on NO, $ONOO^-$, and $NO/ONOO^-$ generation by LA-419 as measured by peak release was assessed. As seen in tables *al-a3,* in the control group (LA-419 vehicle alone), there were no significant differences in the levels of NO, $ONOO^-$ and $NO/ONOO^-$ between the gene variants of each polymorphism. Table *b1* demonstrates an increase in the peak release of NO in response to LA-419 (5μM), in all genotypes. A smaller increase in peak release of NO is produced in response to 1 μM LA-419 (Table *c1*). For average peak release, NO generation was not significantly different between genotypes in the three polymorphic sites, for either concentration of LA-419.

The levels of $ONOO^-$ production revealed no apparent decrease in response to LA-419 (1 μM) whereas a decrease in $ONOO^-$ production was detected in response to 5 μM LA-419 (Tables *b2* and *c2*). There did not appear to be a genotype dose dependent decrease in peak release of $ONOO^-$ for any of the polymorphisms.

The effects of genotype combinations on peak release of NO, $ONOO^-$, and $NO/ONOO^-$ within groups were also examined. For each polymorphism, major allele homozygotes were compared to minor allele carriers (Tables *d-f*). None of the measures were significantly different between major allele homozygotes and minor allele carriers, for either dose of LA-419.

Genotype effects on peak release of NO, $ONOO^-$, and $NO/ONOO^-$ were examined for the full allelic combinations of the exon 7 and promoter variants (Tables g-i). There was no apparent trend in a higher peak release of NO, decreased $ONOO^-$ generation, or higher $NO/ONOO^-$ in response to LA-419 (5μM and 1μM) for the exon 7 and promoter major allele combination (G/G+T/T) compared to other individual genotype combinations. Genotype effect on NO, $ONOO^-$, and $NO/ONOO^-$ levels was also examined for the major allele combination variant G/G+T/T compared to the combination of all other allelic variants, Tables *j-l*. There were no differences between the major allele combination variant and the other genotypes combined, for any peak release measure at either dose of LA-419.

**Effect of Genotype on Net Increase Over Control for 5 μM and 1 μM LA-419**

The effect of genotype on the net increase in NO, $ONOO^-$, and $NO/ONOO^-$ levels for LA-419 (1 μM and 5 μM) over control is shown in Tables m-n. For net NO generation in response to 5μM LA-419 (Table *m1*) there are nonsignificant trends for major allele-associated greater amounts of net NO release for both the exon 7 and promoter polymorphism. This possible genotype dependent response is not seen for 1 μM LA-419 (Table *nl*).

The effects of genotype combinations on the net increase in NO, $ONOO^-$, and $NO/ONOO^-$ levels for LA-419 (5 μM and 1 μM) over control were also examined (Tables *o* - *p*). The major allele gene variants for exon 7 and the promoter compared to their respective minor allele carriers showed a trend (p values of 0.10 and 0.12) for a greater (by 22% in each) net increase in NO in response to 5μM LA-419 (Table *ol*). In addition, full allelic combinations of the exon 7 and promoter polymorphisms were examined (Tables *q,* and r). In Table *ql* it can be seen that the major allele combination variant, G/G+T/T, produced a nonsignificant, numerically greater net increase in NO in response to 5μM LA-419 compared to the other individual allelic combinations. Furthermore, when the major allele variant G/G+T/T was compared to the combination of all other allelic variants the higher net increase in NO in response to 5μM LA-419 remained, with a trend towards significance (p=0.13, Table *sl*). There were no significant differences in the net increase in $ONOO^-$ for LA-419 (1μM and 5μM) over control for any of the genotype combinations.

**Effect of Genotype and Effect of Race on % Change for LA-419 (5 μM and 1 μM)**

The effects of LA-419 on % change as compared to control are given in Tables ul-3 (5μM LA-419) and vl-3 (1μM LA-419). For 5μM LA-419 the % increase in peak NO generation ranged from 11.6 to 17.4%, within genotype groups (Table ul). For the exon 7 G894T polymorphism, NO generation tended to be directly related to the presence of the G, major allele (p = 0.12). Similarly, for the promoter T-786C variant there was also a trend (p = 0.22) for NO generation to be related to the major allele. The intron 4 polymorphism pattern did not provide any evidence for an effect on NO generation. OONO' generation did not appear to be related to any allele of any of the three polymorphic loci (Table u2). There was a nonsignificant trend for the biologically important $NO/OONO^-$ ratio to be greater in the major allele homozygotes of both the exon 7 and promoter polymorphisms (Table u3), and in all genotypes the increase in ratio in response to 5μM LA-419 was substantial, ranging from 37-53%. For 1μM LA-419 there were no trends by genotype in degree of NO generation or $NO/OONO^-$enhancement (Table v1), which was more variable and tended to be less than for the 5μM concentration.

Tables *x1* and *x2* give the derived measure of "% change ratio" for the eNOS product measures, calculated by dividing the % change in NO by the % change in $OONO^-$. A positive value for this ratio means that either both the NO and OONO- values were positive or negative, and values <1.0 mean that the % decrease in $OONO^-$ was > the increase in NO. For 5 μM LA-419 (Table *x1*) all values are negative, meaning that for all genotype groups in all polymorphisms NO generation was > control and $OONO^-$ was < control. All ratios are <1.0, indicating a greater degree of $OONO^-$ lowering than NO generation increase in all groups. Results for 1 μM LA-419 are more variable, reflecting the variability in both NO and $OONO^-$ generation.

The effect of race on % change in NO, $ONOO^-$, and $NO/ONOO^-$ generation for the 5 μM LA-419 concentration (Tables *u1-u3*) demonstrated that Blacks tended to have lower levels of NO generation in comparison to other races. Among the self-identified races examined Blacks had the lowest % change in NO generation and the lowest % reduction in $ONOO^-$, resulting in the lowest % change in $NO/ONOO^-$ ratio (Tables *u1 - u3*). Although in Blacks the % change in NO was not significantly different (p=0.66), the % change in $ONOO^-$ and $NO/ONOO^-$ were both significant at p=0.01. These trends within race were consistent in response to the 1 μM LA-419 concentration (Tables *v1 - v3*), where Blacks were the only race that demonstrated an average *decrease* in NO generation and NO/OONO' ratio (Tables *v1 and v3*),

**Effect of Genotype Combinations on Fold Difference for LA-419 (5 μM and 1 μM**

The complete analysis of genotypes and genotype combinations is presented as "Fold Difference" values, which gives the same result as % change with values (>1.0-1.0) x 100 equaling % increase, and (1.0-<1.0) x 100 values equaling % decrease. Tables *y1-3* and *z1-3* give identical results (after transformation) and p values as the results in Tables *u1-3* and *v1-3.* The effects of genotype combinations on the fold difference of NO, $ONOO^-$, and $NO/ONOO^-$ generation for LA-419 are given in Tables *aa-rr.*

In Tables *aa1-aa3* the major allele homozygotes were compared to the minor allele carriers for each polymorphism for response to 5 μM LA-419. Within the exon 7 comparisons, the major allele homozygote had fold increase of NO production compared to minor allele carriers, 1.16±0.06 versus 1.13±0.06 (p=0.05). There was no significant difference in the percent of control for $ONOO^-$ generation in response to 5 μM LA-419 for the major allele homozygote compared to the minor allele carriers within exon 7 (Table *aa2*). Accordingly, the major allele homozygote for exon 7 had a higher % of control for the $NO/ONOO^-$ ratio in response to LA-419 (5μM) compared to the minor allele carriers, 1.48±0.18 versus 1.39±0.13 (p=0.042, Table aa3). Trends of the same type were exhibited for the promoter variant comparison (Tables *aa1-aa3),* but with nonsignificant p values.

Genotype effects for the full allelic combinations of the exon 7 and promoter variants are given in Tables cc and *dd.* There was not a significant trend for NO or $ONOO^-$ responses to LA-419 (either 5 μM and 1 μM) to be greater in the dominant allele combination (G/G+T/T) compared to all other genotypes. In fact, combining the major allele homozygotes weakened the statistical significance for the 5 μM LA-419 findings compared to the exon 7 dominant allele G/G genotype alone.

Genotype effect on fold difference was also examined for the major allele variant G/G+T/T compared to the combination of all other allelic variants, with the results given in Tables *ee* and *ff.* The dominant allelic combination (G/G+T/T) tended to have a higher fold increase for NO production (p = 0.09) and $NO/ONOO^-$ ratio (p = 0.13) in response to 5 μM LA-419 (Tables *ee1* and *ee3*), but showed no trends for such effects in response to 1 μM LA-419 (Tables *ff1* and *ff3*).

**Effect of Genotype on LA-419 Fold Difference Responses within Caucasians**

The effect of genotype on fold difference of NO, $ONOO^-$, and $NO/ONOO6^-$ responses within Caucasians was examined, with data presented in Tables *gg-nn.* Table *gg1* shows that both the exon 7 and promoter major allele variants

have a numerically higher fold difference for NO production in response to 5 μM LA-419, 1.19±0.07 (p = 0.02) and 1.17±0.08 (p = 0.15) respectively. Moreover, in Table *gg3* it can be seen that that NO/ONOO$^-$ ratios for the exon 7 and the promoter major alleles are numerically higher as compared to other genotypes, with respective p values of 0.06 and 0.07. There was no significant difference in the percent control for ONOO$^-$ generation in response to LA-419 (5μM) within Caucasians for any of the polymorphism. These trends were not identified at the LA-419 1 μM concentration (Tables *hh1 - hh3).*

Comparison of the effects of major allele homozygotes to minor allele carriers within Caucasians was also examined, with data presented in Tables *ii* and *jj.* Within the exon 7 comparison, the major allele homozygote had a fold difference for NO production in response to 5μM LA-419 compared to the minor allele carriers of 1.19±0.07 versus 1.11±0.05, which was significantly different at a p=0.01 (Table *ii1*). There was no significant difference in the percent of control for ONOO$^-$ generation in response to LA-419 (5μM) for the major alleles homozygote compared to the minor allele carriers within exon 7. Accordingly, the major allele homozygote for exon 7 had a fold difference for the NO/ONOO$^-$ ratio in response to 5μM LA-419 compared to the minor allele carriers, 1.47±0.12 versus 1.35±0.10 (p=0.027, Table *ii3).* Nonsignificant trends were present for the promoter variants, for both NO production (p = 0.09, Table *ii1*)) and the NO/ONOO$^-$ ratio (p = 0.17, Table 113). These trends were not seen at the LA-419 1μM concentration, *tables jj1 - jj3.*

Genotype effect on % of control within Caucasians was examined for the full allelic combinations of the exon 7 and promoter variants, as shown in Tables *kk* and *ll*. With the 5μM LA-419 concentration there was a numerically higher fold difference for NO production (p = 0.11, Table *kk1*) and the NO/ONOO$^-$ ratio (p =0.0496, Table *kk3*) for the dominant allele combination, G/G+T/T. Similar trends were not present for 1 μM LA-419 (Tables *ll1-ll3*).

The major allele variant (G/G+T/T) data compared to the combination of all other allelic variants within Caucasians are given in Tables *mm* and nn. The major allelic combination exhibited trends for a higher fold difference in NO production (p = 0.068, Table *mml*) and NO/ONOO$^-$ ratio (p = 0.13, Table *mm3*) in response to 5 μM LA-419. The G/G+T/T combination genotype also had a trend for a higher fold difference for NO production in response to 1 μM LA-419 (p=0.076, Table *nn1*) but no trends for a higher NO/OONO$^-$ ratio (Table *nn3*).

**Effect of Genotype on Fold Difference in Minor Allele Homozygotes vs. Major Allele Homozygotes and Heterozygotes**

In all races the effect of genotype on fold difference of NO, ONOO-, and NO/ONOO$^-$ was examined within each polymorphism for the minor allele homozygotes compared to the combination of the major allele homozygotes and heterozygotes. For both the exon 7 and promoter polymorphism, the minor allele homozygote had a numerically lower but statistically nonsignificant fold difference for NO production and NO/ONOO$^-$ ratio in response to 5μM LA-419 (Tables *oo1* and *oo3*). At the LA-419 1μM concentration this trend was present for the promoter polymorphism, but was not apparent in the exon 7 polymorphism (Tables *pp1* and *pp3).*

Genotype effects on fold difference for the minor allele homozygotes T/T+C/C compared to the combination of all other allelic variants (Tables *qq* and *rr*). There was no apparent trends in for differential fold differences for NO, ONOO$^-$, and NO/ONOO$^-$ in response to either dose of LA-419.

**eNOS Protein Abundance**

Using immunochemical methods, there was no change in any condition in relation to eNOS protein abundance, *Tables ss - L1.*

**Intron 4 Analysis**

There was no gene allele dose related response to LA-419 (1 μM and 5 μM) for the intron 4 polymorphism, as presented in Tables *M1-M4* and *N1-N3* as well as tables throughout this report.

**APPENDIX**

**eNOS Activity Analysis**

***Effect of Genotype within Group***

**Tables a1-a3:** Effect of eNOS genotypes within control group*

| **a1)** | | | | |
|---|---|---|---|---|
| **NO (control)** | | | | |
| **Variable** | **Level (n)** | **Peak release, nmols/L (mean ± sd)** | **Chi-sq statistic** | **p value** |
| **G894T variant** | | | | |
| | G/G (26) | 3443.87 (59.91) | 0.4138 | 0.81 |
| | G/T (18) | 354.76 (77.61) | | |
| | T/T (5) | 348.07 (100.76) | | |
| **T-786C variant** | | | | |
| | T/T (22) | 349.92 (51.75) | 0.0975 | 0.95 |
| | T/C (23) | 349.55 (84.47) | | |
| | C/C (4) | 332.17 (80.88) | | |
| **Intron 4 variant** | | | | |
| | b/b (31) | 353.53 (73.00) | 3.1640 | 0.367 |
| | b/a (10) | 320:67 (65.74) | | |
| | a/a (3) | 386.33 (72.90) | | |
| | c/c (1) | 368.67 (0) | | |
| **a2)** | | | | |
| **$ONOO^-$ (control)** | | | | |
| **Variable** | **Level (n)** | **Peak release, nmols/L (mean ± sd)** | **Chi-sq statistic** | **p value** |
| **G894T variant** | | | | |
| | G/G (26) | 363.01 (84.61) | 3.3296 | 0.19 |
| | G/T (18) | 36594 (87.41) | | |
| | T/T (5) | 449.27 (111.48) | | |
| **T-786C variant** | | | | |
| | T/T (22) | 362.95 (86.39) | 0.6190 | 0.73 |
| | T/C (23) | 376.83 (91.28) | | |
| | C/C (4) | 404.92 (121.75) | | |
| **Intron 4 variant** | | | | |
| | b/b (31) | 369.31 (86.00) | 0.9010 | 0.825 |
| | b/a (10) | 368.03 (100.38) | | |
| | a/a (3) | 395.00 (128.17) | | |
| | c/c (1) | 426.33 (0) | | |
| **a3)** | | | | |
| **$NO/ONOO^-$ (control)** | | | | |
| **Variable** | **Level (n)** | **Peak release, nmols/L (mean ± sd)** | **Chi-sq statistic** | **p value** |
| **G894T variant** | | | | |
| | G/G (26) | 1.02 (0.34) | 0.6330 | 0.73 |
| | G/T (18) | 1.05 (0.37) | | |

(continued)

| a3) | | | | |
|---|---|---|---|---|
| **NO/ONOO⁻ (control)** | | | | |
| **Variable** | **Level (n)** | **Peak release, nmols/L (mean ± sd)** | **Chi-sq statistic** | **p value** |
| **G894T variant** | | | | |
| | T/T(5) | 0.85 (0.44) | | |
| **T-786C variant** | | | | |
| | T/T (22) | 1.03 (0.33) | 0.6682 | 0.72 |
| | T/C (23) | 1.01 (0.39) | | |
| | C/C (4) | 0.92 (0.46) | | |
| **Intron 4 variant** | | | | |
| | b/b (31) | 1.03 (0.36) | 0.2703 | 0.966 |
| | b/a (10) | 0.97 (0.41) | | |
| | a/a (3) | 1.07 (0.45) | | |
| | c/c (1) | 0.86 (0) | | |
| Chi-sq statistic generated from the Non-parametric ANOVA Kruskal-Wallis test<br>*Control group is LA-419 vehicle. | | | | |

**Tables b1 - b3)** Effect of eNOS genotypes within group LA-419 5μM

| b1) | | | | |
|---|---|---|---|---|
| **NO 5μM)** | | | | |
| **Variable** | **Level (n)** | **Peak release, nmols/L (mean ± sd)** | **Chi-sq statistic** | **p value** |
| **G894T variant** | | | | |
| | G/G (26) | 398.92 (71.74) | 0.3530 | 0.84 |
| | G/T (18) | 400.63 (80.27) | | |
| | T/T(5) | 390.47 (124.39) | | |
| **T-786C variant** | | | | |
| | T/T (22) | 405.83 (58.51) | 0.5336 | 0.77 |
| | T/C (23) | 396.81 (95.72) | | |
| | C/C (4) | 370.17 (86.78) | | |
| **Intron 4 variant** | | | | |
| | b/b (31) | 400.83 (81.57) | 2.5597 | 0.465 |
| | b/a (10) | 375.20 (81.45) | | |
| | a/a (3) | 438.33 (79.68) | | |
| | c/c (1) | 432.67 (0) | | |
| **b2)** | | | | |
| **ONOO⁻ (LA-419 5μM)** | | | | |
| **Variable** | **Level (n)** | **Peak release, nmols/L (mean ± sd)** | **Chi-sq statistic** | **p value** |
| **G894T variant** | | | | |
| | G/G (26) | 288.82 (78.22) | 2.5405 | 0.28 |

(continued)

| | | | | |
|---|---|---|---|---|
| **b2)** | | | | |
| **$ONOO^-$ (LA-419 5μM)** | | | | |
| **Variable** | **Level (n)** | **Peak release, nmols/L (mean ± sd)** | **Chi-sq statistic** | **p value** |
| **G894T variant** | | | | |
| | G/T (18) | 300.15 (75.86) | | |
| | T/T (5) | 366.40 (103.55) | | |
| **T-786C variant** | | | | |
| | T/T (22) | 286.47 (76.65) | 1.5269 | 0.47 |
| | T/C (23) | 310.97 (82.59) | | |
| | C/C (4) | 322.33 (110.70) | | |
| **Intron 4 variant** | | | | |
| | b/b (31) | 298.99 (79.66) | 1.4513 | 0.694 |
| | b/a (10) | 295.73 (85.11) | | |
| | a/a (3) | 331.22 (117.30) | | |
| | c/c (1) | 328.33 (0) | | |
| **b3)** | | | | |
| **$NO/ONOO^-$ (LA-419 5μM)** | | | | |
| **Variable** | **Level (n)** | **Peak release, nmols/L (mean ± sd)** | **Chi-sq statistic** | **p value** |
| **G894T variant** | | | | |
| | G/G (26) | 1.50 (0.51) | 1.3707 | 0.50 |
| | G/T (18) | 1.44 (0.49) | | |
| | T/T (5) | 1.21 (0.68) | | |
| **T-786C variant** | | | | |
| | T/T (22) | 1.52 (0.45) | 0.8254 | 0.66 |
| | T/C (23) | 1.41 (0.56) | | |
| | C/C (4) | 1.32 (0.71) | | |
| **Intron 4 variant** | | | | |
| | b/b (31) | 1.46 (0.51) | 0.3979 | 0.941 |
| | b/a (10) | 1.42 (0.61) | | |
| | a/a (3) | 1.48 (0.64) | | |
| | c/c (1) | 1.32 (0) | | |
| Chi-sq statistic generated from the Non-parametric ANOVA Kruskal-Wallis test | | | | |

**Tables c1 - c3)** Effect of eNOS genotypes within group LA-419 1μM

| **c1)** | | | | |
|---|---|---|---|---|
| **NO (LA-419 1μM)** | | | | |
| **Variable** | **Level (n)** | **Peak release, nmols/L (mean ± sd)** | **Chi-sq statistic** | **p value** |
| **G894T variant** | | | | |
| | G/G (26) | 367.13 (80.46) | 4.6995 | 0.095 |
| | G/T (18) | 413.28 (58.46) | | |
| | T/T (5) | 368.80 (75.73) | | |
| **T-786C variant** | | | | |
| | T/T (22) | 380.73 (75.61) | 2.9185 | 0.23 |
| | T/C (23) | 396.32 (73.63) | | |
| | C/C (4) | 334.25 (68.06) | | |
| **Intron 4 variant** | | | | |
| | b/b (31) | 393.18 (77.21) | 2.7011 | 0.440 |
| | b/a (10) | 377.23 (69.05) | | |
| | a/a (3) | 360.56 (80.60) | | |
| | c/c (1) | 270.00 (0) | | |
| **c2** | | | | |
| **$ONOO^-$ (LA-4191μM)** | | | | |
| **Variable** | **Level (n)** | **Peak release, nmols/L (mean ± sd)** | **Chi-sq statistic** | **p value** |
| **G894T variant** | | | | |
| | G/G (26) | 344.53 (83.88) | 1.1225 | 0.57 |
| | G/T (18) | 319.43 (81.89) | | |
| | T/T (5) | 332.67 (129.30) | | |
| **T-786C variant** | | | | |
| | T/T (22) | 335.82 (80.98) | 3.5720 | 0.17 |
| | T/C (23) | 315.74 (78.13) | | |
| | C/C (4) | 430.17 (124.82) | | |
| **Intron 4 variant** | | | | |
| | b/b (31) | 317.60 (74.77) | 3.6974 | 0.296 |
| | b/a (10) | 330.47 (88.93) | | |
| | a/a (3) | 421.11 (124.25) | | |
| | c/c (1) | 423.67 (0) | | |
| **c3)** | | | | |
| **$NO/ONOO^-$ (LA-419 1μM)** | | | | |
| **Variable** | **Level (n)** | **Peak release, nmols/L (mean ± sd)** | **Chi-sq statistic** | **p value** |
| **G894T variant** | | | | |
| | G/G (26) | 1.16 (0.45) | 1.8020 | 0.41 |
| | G/T (18) | 1.38 (0.38) | | |

(continued)

| c3) | | | | |
|---|---|---|---|---|
| $NO/ONOO^-$ (LA-419 1μM) | | | | |
| **Variable** | **Level (n)** | **Peak release, nmols/L (mean ± sd)** | **Chi-sq statistic** | **p value** |
| **G894T variant** | | | | |
| | T/T (5) | 1.29 (0.63) | | |
| **T-786C variant** | | | | |
| | T/T (22) | 1.22 (0.43) | 3.3685 | 0.19 |
| | T/C (23) | 1.35 (0.44) | | |
| | C/C (4) | 0.88 (0.50) | | |
| **Intron 4 variant** | | | | |
| | b/b (31) | 1.33 (0.42) | 2.8821 | 0.410 |
| | b/a (10) | 1.25 (0.46) | | |
| | a/a (3) | 0.97 (0.57) | | |
| | c/c (1) | 0.64 (0) | | |
| Chi-sq statistic generated from the Non-parametric ANOVA Kruskal-Wallis test | | | | |

**Tables d1 - d3)** Effect of genotype within group control. Comparison between major allele homozygotes and minor allele carriers

| d1) | | | | |
|---|---|---|---|---|
| **NO (control)** | | | | |
| **Variable** | **Level (n)** | **Peak release, nmols/L (mean ± sd)** | **t statistic** | **p value** |
| **G894T variant** | | | | |
| | G/G (26) | 343.87 (59.91) | 0.5710 | 0.57 |
| | G/T & T/T (23) | 353.30 (80.67) | | |
| **T-786C variant** | | | | |
| | T/T (22) | 349.92 (51.75) | 0.0402 | 0.97 |
| | T/C & C/C (27) | 346.98 (82.66) | | |
| **Intron 4 variant** | | | | |
| | b/b (31) | 353.53 (73.00) | -0.7110 | 0.481 |
| | b/a, a/a, c/c (14) | 338.17 (68.21) | | |
| **d2)** | | | | |
| **$ONOO^-$ (control)** | | | | |
| **Variable** | **Level (n)** | **Peak release, nmols/L (mean ± sd)** | **t statistic** | **p value** |
| **G894T variant** | | | | |
| | G/G (26) | 363.01 (84.61) | 0.8014 | 0.43 |
| | G/T & T/T (23) | 384.06 (96.95) | | |

(continued)

| | | | | |
|---|---|---|---|---|
| **T-786C variant** | | | | |
| | T/T (22) | 362.95 (86.39) | -0.7035 | 0.49 |
| | T/C & C/C (27) | 380.99 (94.15) | | |
| **Intron 4 variant** | | | | |
| | b/b (31) | 369.31 (86.00) | 0.1103 | 0.913 |
| | b/a, a/a, c/c (14) | 377.98 (99.12) | | |
| **d3)** | | | | |
| **NO/ONOO$^-$ (control)** | | | | |
| **Variable** | **Level (n)** | **Peak release, nmols/L (mean ± sd)** | **t statistic** | **p value** |
| **G894T variant** | | | | |
| | G/G (26) | 1.02 (0.34) | -0.2104 | 0.83 |
| | G/T & T/T (23) | 1.01 (0.39) | | |
| **T-786C variant** | | | | |
| | T/T (22) | 1.03 (0.33) | 0.3518 | 0.73 |
| | T/C & C/C (27) | 1.00 (0.39) | | |
| **Intron 4 variant** | | | | |
| | b/b (31) | 1.03 (0.36) | -0.0123 | 0,990 |
| | b/a, a/a, c/c (14) | 0.99 (0.39) | | |
| t statistic generated from the Non-parametric Mann-Whitney U test | | | | |

**Tables e1 - e3)** Effect of genotype within group LA-419 (5μM). Comparison between major allele homozygotes and minor allele carriers

| | | | | |
|---|---|---|---|---|
| **e1)** | | | | |
| **NO (LA-419 5μM)** | | | | |
| **Variable** | **Level (n)** | **Peak release, nmols/L (mean ± sd)** | **t statistic** | **p value** |
| **G894T variant** | | | | |
| | G/G (26) | 398.92 (71.74) | -0.0100 | 0.99 |
| | G/T & T/T (23) | 398.42 (88.37) | | |
| **T-786C variant** | | | | |
| | T/T (22) | 405.83 (58.51) | 0.1709 | 0.85 |
| | T/C & C/C (27) | 392.86 (93.35) | | |
| **Intron 4 variant** | | | | |
| | b/b (31) | 400.83 (81.57) | -0.1103 | 0.913 |
| | b/a, a/a, c/c (14) | 392.83 (80.05) | | |

(continued)

| e2) | | | | |
|---|---|---|---|---|
| **$ONOO^-$ (LA-419 5μM)** | | | | |
| **Variable** | **Level (n)** | **Peak release, nmols/L (mean ± sd)** | **t statistic** | **p value** |
| **G894T variant** | | | | |
| | G/G (26) | 288.82 (78.22) | 1.1219 | 0.27 |
| | G/T & T/T (23) | 314.55 (84.72) | | |
| **T-786C variant** | | | | |
| | T/T (22) | 286.47 (76.65) | -1.2161 | 0.23 |
| | T/C & C/C (27) | 312.65 (84.87) | | |
| **Intron 4 variant** | | | | |
| | b/b (31) | 298.99 (79.66) | 0.2329 | 0.817 |
| | b/a, a/a, c/c (14) | 305.67 (86.01) | | |
| **e3)** | | | | |
| **$NO/ONOO^-$ (LA-419 5μM)** | | | | |
| **Variable** | **Level (n)** | **Peak release, nmols/L (mean ± sd)** | **t statistic** | **p value** |
| **G894T variant** | | | | |
| | G/G (26) | 1.50 (0.51) | -0.9917 | 0.33 |
| | G/T & T/T (23) | 1.39 (0.53) | | |
| **T-786C variant** | | | | |
| | T/T (22) | 1.52 (0.45) | 0.8744 | 0.39 |
| | T/C & C/C (27) | 1.39 (0.57) | | |
| **Intron 4 variant** | | | | |
| | b/b (31) | 1.46 (0.51) | 0.1103 | 0.913 |
| | b/a, a/a, c/c (14) | 1.43 (0.57) | | |
| t statistic generated from the Non-parametric Mann-Whitney U test | | | | |

**Tables f1 - f3)** Effect of genotype within group LA-419 (1μM). Comparison between major allele homozygotes and minor allele carriers

| f1) | | | | |
|---|---|---|---|---|
| **NO (LA-419 1μM)** | | | | |
| **Variable** | **Level (n)** | **Peak release, nmols/L (mean ± sd)** | **t statistic** | **p value** |
| **G894T variant** | | | | |
| | G/G (26) | 367.13 (80.46) | 1.7730 | 0.08 |
| | G/T & T/T (23) | 403.61 (63.52) | | |
| **T-786C variant** | | | | |
| | T/T (22) | 380.73 (75.61) | -0.6332 | 0.53 |
| | T/C & C/C (27) | 387.12 (75.01) | | |

(continued)

| | | | | |
|---|---|---|---|---|
| **Intron 4 variant** | | | | |
| | b/b (31) | 393.18 (77.21) | -1.0420 | 0.303 |
| | b/a, a/a, c/c (14) | 366.00 (71.50) | | |
| **f2)** | | | | |
| **$ONOO^-$ (LA-419 1μM)** | | | | |
| **Variable** | **Level (n)** | **Peak release, nmols/L (mean ± sd)** | **t statistic** | **p value** |
| **G894T variant** | | | | |
| | G/G (26) | 344.53 (83.88) | -1.0118 | 0.32 |
| | G/T & T/T (23) | 322.30 (90:85) | | |
| **T-786C variant** | | | | |
| | T/T (22) | 335.82 (80.98) | 0.3719 | 0.71 |
| | T/C & C/C (27) | 332.69 (93.16) | | |
| **Intron 4 variant** | | | | |
| | b/b (31) | 317.60 (74.77) | 1.1402 | 0.260 |
| | b/a, a/a, c/c (14) | 356.55 (98.40) | | |
| **f3)** | | | | |
| **$NO/ONOO^-$ (LA-419 1μM)** | | | | |
| **Variable** | **Level (n)** | **Peak release, nmols/L (mean ± sd)** | **t statistic** | **p value** |
| **G894T variant** | | | | |
| | G/G (26) | 1.16 (0.45) | 1.3322 | 0.19 |
| | G/T & T/T (23) | 1.36 (0.43) | | |
| **T-786C variant** | | | | |
| | T/T (22) | 1.22 (0.43) | -0.4121 | 0.68 |
| | T/C & C/C (27) | 1.28 (0.47) | | |
| **Intron 4 variant** | | | | |
| | b/b (31) | 1.33 (0.42) | -0.9439 | 0.350 |
| | b/a, a/a, c/c (14) | 1.15 (0.48) | | |
| t statistic generated from the Non-parametric Mann-Whitney U test | | | | |

**Tables g1 - g3)** Effect of genotype combinations within group control (Individual allelic combination). Reference group = G/G+T/T

| | | | | |
|---|---|---|---|---|
| **g1)** | | | | |
| **NO (control)** | | | | |
| **Variable** | **Level (n)** | **Peak release, nmols/L (mean ± sd)** | **Chi-sq statistic** | **p value** |
| **G894T + T-786C variant** | | | | |
| | G/G+T/T (18) | 343.07 (49.58) | 3.0055 | 0.81 |

(continued)

| **g1)** | | | | |
|---|---|---|---|---|
| **NO (control)** | | | | |
| **Variable** | **Level (n)** | **Peak release, nmols/L (mean ± sd)** | **Chi-sq statistic** | **p value** |
| **G894T + T-786C variant** | | | | |
| | G/G+T/C (8) | 345.67 (82.72) | | |
| | G/G/+C/C (0) | - | | |
| | G/T+T/T (4) | 380.75 (57.17) | | |
| | G/T+T/C (13) | 344.18 (85.29) | | |
| | G/T+C/C (1) | 388.33 (0) | | |
| | T/T+T/C (2) | 400.00 (127.28) | | |
| | T/T+C/C (3) | 313.44 (87.80) | | |
| **g2)** | | | | |
| **$ONOO^-$ (control)** | | | | |
| **Variable** | **Level (n)** | **Peak release, nmols/L (mean ± sd)** | **Chi-sq statistic** | **p value** |
| **G894T + T-786C variant** | | | | |
| | G/G+T/T (18) | 371.52 (91.89) | 5.5092 | 0.48 |
| | G/G+T/C (8) | 343.88 (66.73) | | |
| | G/G/+C/C (0) | - | | |
| | G/T+T/T (4) | 324.42 (44.40) | | |
| | G/T+T/C (13) | 383.92 (95.01) | | |
| | G/T+C/C (1) | 298.33 (0) | | |
| | T/T+T/C (2) | 462.50 (140.71) | | |
| | T/T+C/C (3) | 440.44 (121.09) | | |
| **g3)** | | | | |
| **$NO/ONOO^-$ (control)** | | | | |
| **Variable** | **Level (n)** | **Peak release, nmols/L (mean ± sd)** | **Chi-sq statistic** | **p value** |
| **G894T + T-786C variant** | | | | |
| | G/G+T/T (18) | 1.00 (0.34) | 2.4033 | 0.88 |
| | G/G+T/C (8) | 1.07 (0.37) | | |
| | G/G/+C/C (0) | - | | |
| | G/T+T/T (4) | 1.19 (0.25) | | |
| | G/T+T/C (13) | 0.98 (0.40) | | |
| | G/T+C/C (1) | 1.3016760 (0) | | |
| | T/T+T/C (2) | 0.95 (0.56) | | |

(continued)

| **g3)** | | | | |
|---|---|---|---|---|
| **NO/ONOO$^-$ (control)** | | | | |
| **Variable** | **Level (n)** | **Peak release, nmols/L (mean ± sd)** | **Chi-sq statistic** | **p value** |
| **G894T + T-786C variant** | | | | |
| | T/T+C/C (3) | 0.79 (0.46) | | |
| Chi-sq statistic generated from the Non-parametric ANOVA Kruskal-Wallis test | | | | |

**Tables h1 - h3)** Effect of genotype combinations within group LA-419 5μM (Individual allelic combination). Reference group = G/G+T/T

| **h1)** | | | | |
|---|---|---|---|---|
| **NO (LA-419 5μM)** | | | | |
| **Variable** | **Level (n)** | **Peak release, nmols/L (mean ± sd)** | **Chi-sq statistic** | **p value** |
| **G894T + T-786C variant** | | | | |
| | G/G+T/T (18) | 399.67 (56.96) | 2.7891 | 0.83 |
| | G/G+T/C (8) | 397.25 (102.46) | | |
| | G/G/+C/C (0) | - | | |
| | G/T+T/T (4) | 433.58 (65.75) | | |
| | G/T+T/C (13) | 387.90 (86.10) | | |
| | G/T+C/C (1) | 434.33 (0) | | |
| | T/T+T/C (2) | 453.00 (178.19) | | |
| | T/T+C/C (3) | 348.78 (92.48) | | |
| **h2)** | | | | |
| **ONOO$^-$ (LA-419 5μM)** | | | | |
| **Variable** | **Level (n)** | **Peak release, nmols/L (mean ± sd)** | **Chi-sq statistic** | **p value** |
| **G894T + T-786C variant** | | | | |
| | G/G+T/T (18) | 293.93 (83.07) | 5.1552 | 0.5241 |
| | G/G+T/C (8) | 277.33 (69.85) | | |
| | G/G/+C/C (0) | - | | |
| | G/T+T/T (4) | 252.92 (13.69) | | |
| | G/T+T/C (13) | 318.33 (82.55) | | |
| | G/T+C/C (1) | 252.67 (0) | | |
| | T/T+T/C (2) | 397.67 (96.64) | | |
| | T/T+C/C (3) | 345.56 (123.07) | | |

(continued)

| h3) | | | | |
|---|---|---|---|---|
| **NO/ONOO⁻ (LA-419 5μM)** | | | | |
| **Variable** | **Level (n)** | **Peak release, nmols/L (mean ± sd)** | **Chi-sq statistic** | **p value** |
| **G894T + T-786C variant** | | | | |
| | G/G+T/T (18) | 1.48 (0.48) | 3.1462 | 0.79 |
| | G/G+T/C (8) | 1.56 (0.60) | | |
| | G/G/+C/C (0) | - | | |
| | G/T+T/T (4) | 1.71 (0.21) | | |
| | G/T+T/C (13) | 1.34 (0.54) | | |
| | G/T+C/C (1) | 1.72 (0) | | |
| | T/T+T/C (2) | 1.23 (0.75) | | |
| | T/T+C/C (3) | 1.19 (0.80) | | |
| Chi-sq statistic generated from the Non-parametric ANOVA Kruskal-Wallis test | | | | |

**Tables i1 - i3)** Effect of genotype combinations within group LA-419 1μM (Individual allelic combination). Reference group = G/G+T/T

| i1) | | | | |
|---|---|---|---|---|
| **NO (LA-419 1μM)** | | | | |
| **Variable** | **Level (n)** | **Peak release, nmols/L (mean ± sd)** | **Chi-sq statistic** | **p value** |
| **G894T + T-786C variant** | | | | |
| | G/G+T/T (18) | 374.30 (78.06) | 7.3555 | 0.29 |
| | G/G+T/C (8) | 351.00 (88.83) | | |
| | G/G/+C/C (0) | - | | |
| | G/T+T/T (4) | 409.67 (64.25) | | |
| | G/T+T/C (13) | 420.44 (56.87) | | |
| | G/T+C/C (1) | 334.67 (0) | | |
| | T/T+T/C (2) | 420.83 (4.48) | | |
| | T/T+C/C (3) | 334.11 (83.36) | | |
| **i2)** | | | | |
| **ONOO⁻ (LA-419 1μM)** | | | | |
| **Variable** | **Level (n)** | **Peak release, nmols/L (mean ± sd)** | **Chi-sq statistic** | **p value** |
| **G894T + T-786C variant** | | | | |
| | G/G+T/T (18) | 338.98 (81.16) | 9.3929 | 0.15 |
| | G/G+T/C (8) | 357.00 (94.18) | | |
| | G/G/+C/C (0) | - | | |

(continued)

| i2) | | | | |
|---|---|---|---|---|
| **ONOO$^-$ (LA-419 1μM)** | | | | |
| **Variable** | **Level (n)** | **Peak release, nmols/L (mean ± sd)** | **Chi-sq statistic** | **p value** |
| **G894T + T-786C variant** | | | | |
| | G/T+T/T (4) | 321.58 (90.82) | | |
| | G/T+T/C (13) | 302.82 (59.66) | | |
| | G/T+C/C (1) | 526.67 (0) | | |
| | T/T+T/C (2) | 234.67 (23.10) | | |
| | T/T+C/C (3) | 398.00 (131.00) | | |
| **i3)** | | | | |
| **NO/ONOO$^-$ (LA-419 1μM)** | | | | |
| **Variable** | **Level (n)** | **Peak release, nmols/L (mean ± sd)** | **Chi-sq statistic** | **p value** |
| **G894T + T-786C variant** | | | | |
| | G/G+T/T (18) | 1.19 (0.43) | 9.1043 | 0.17 |
| | G/G+T/C (8) | 1.09 (0.51) | | |
| | G/G/+C/C (0) | - | | |
| | G/T+T/T (4) | 1.37 (0.44) | | |
| | G/T+T/C (13) | 1.44 (0.32) | | |
| | G/T+C/C (1) | 0.64 (0) | | |
| | T/T+T/C (2) | 1.80 (0.16) | | |
| | T/T+C/C (3) | 0.96 (0.58) | | |
| Chi-sq statistic generated from the Non-parametric ANOVA Kruskal-Wallis test | | | | |

**Tables j1-j3)** Effect of genotype combinations within group control (reference vs. all carriers combination).

| Reference group = G/G+T/T. | | | | |
|---|---|---|---|---|
| Combination = G/G+T/C, G/G/+C/C, G/T+T/T, G/T+T/C, G/T+C/C, T/T+T/C, T/T+C/C | | | | |
| **j1)** | | | | |
| **NO (control)** | | | | |
| **Variable** | **Level (n)** | **Peak release, nmols/L (mean ± sd)** | **t statistic** | **p value** |
| **Genotype** | | | | |
| | Reference (18) | 343.07 (49.58) | -0.5911 | 0.556 |
| | Combination (31) | 351.33 (79.88) | | |

(continued)

| j2) | | | | |
|---|---|---|---|---|
| **$ONOO^-$ (control)** | | | | |
| **Variable** | **Level (n)** | **Peak release, nmols/L (mean ± sd)** | **t statistic** | **p value** |
| **Genotype** | | | | |
| | Reference (18) | 371.52 (91.89) | -0.2489 | 0.80 |
| | Combination (31) | 373.69 (90.83) | | |
| **j3)** | | | | |
| **$NO/ONOO^-$ (control)** | | | | |
| **variable** | **Level (n)** | **Peak release, nmols/L (mean ± sd)** | **t statistic** | **p value** |
| **Genotype** | | | | |
| | Reference (18) | 1.00 (0.34) | -0.0933 | 0.93 |
| | Combination (31) | 1.02 (0.38) | | |
| t statistic generated from the Non-parametric Mann-Whitney U test | | | | |

**Tables k1 - k3)** Effect of genotype combinations within group LA-419 5μM (reference vs. all carriers combination).

| Reference group = G/G+T/T. | | | | |
|---|---|---|---|---|
| Combination = G/G+T/C, G/G/+C/C, G/T+T/T, G/T+T/C, G/T+C/C, T/T+T/C, T/T+C/C | | | | |
| **k1)** | | | | |
| **NO (LA-419 5μM)** | | | | |
| **Variable** | **Level (n)** | **Peak release, nmols/L (mean ± sd)** | **t statistic** | **p value** |
| **Genotype** | | | | |
| | Reference (18) | 399.67 (56.96) | -0.3629 | 0.72 |
| | Combination (31) | 398.12 (90.43) | | |
| **k2)** | | | | |
| **$ONOO^-$ (LA-419 5μM)** | | | | |
| **Variable** | **Level (n)** | **Peak release, nmols/L (mean ± sd)** | **t statistic** | **p value** |
| **Genotype** | | | | |
| | Reference (18) | 293.93 (83.07) | -0.7259 | 0.47 |
| | Combination (31) | 304.95 (81.70) | | |
| **k3)** | | | | |
| **$NO/ONOO^-$ (LA-419 5μM) I** | | | | |
| **Variable** | **Level (n)** | **Peak release, nmols/L (mean ± sd)** | **t statistic** | **p value** |
| **Genotype** | | | | |
| | Reference (18) | 1.48 (0.48) | 0.3837 | 0.70 |
| | Combination (31) | 1.43 (0.54) | | |
| t statistic generated from the Non-parametric Mann-Whitney U test | | | | |

**Tables l1 - l3)** Effect of genotype combinations within group LA-419 1μM (reference vs. all carriers combination).

| | | | | |
|---|---|---|---|---|
| Reference group = G/G+T/T. | | | | |
| Combination = G/G+T/C, G/G/+C/C, G/T+T/T, G/T+T/C, G/T+C/C, T/T+T/C, T/T+C/C | | | | |
| **l1)** | | | | |
| **NO (LA-419 1μM)** | | | | |
| **Variable** | **Level (n)** | **Peak release, nmols/L (mean ± sd)** | **t statistic** | **p value** |
| **Genotype** | | | | |
| | Reference (18) | 374.30 (78.06) | -1.1096 | 0.27 |
| | Combination (31) | 390.03 (73.13) | | |
| **l2)** | | | | |
| **$ONOO^-$ (LA-419 1μM)** | | | | |
| **Variable** | **Level (n)** | **Peak release, nmols/L (mean ± sd)** | **t statistic** | **p value** |
| **Genotype** | | | | |
| | Reference (18) | 338.98 (81.16) | 0.4667 | 0.64 |
| | Combination (31) | 331.26 (91.43) | | |
| **l3)** | | | | |
| **NO/$ONOO^-$ (LA-419 1μM)** | | | | |
| **Variable** | **Level (n)** | **Peak release, nmols/L (mean ± sd)** | **t statistic** | **p value** |
| **Genotype** | | | | |
| | Reference (18) | 1.19 (0.43) | -0.7155 | 0.48 |
| | Combination (31) | 1.29 (0.46) | | |
| t statistic generated from the Non-parametric Mann-Whitney U test | | | | |

***Net Increase Over Control***

**Tables m1 - m3)** Net increase in 5μM LA-419 over control - comparison between genotypes

| | | | | |
|---|---|---|---|---|
| **m1)** | | | | |
| **NO (5μM LA-419)** | | | | |
| **Variable** | **Level (n)** | **Net increase, nmols/L (mean ± sd)** | **Chi-sq statistic** | **p value** |
| **G894T variant** | | | | |
| | G/G (26) | 55.05 (21.03) | 2.9488 | 0.23 |
| | G/T (18) | 45.87 (19.24) | | |
| | T/T (5) | 42.40 (30.77) | | |
| ***T-786C variant*** | | | | |
| | T/T (22) | 55.91 (19.06) | 3.1740 | 0.20 |
| | T/C (23) | 47.26 (23.70) | | |
| | C/C (4) | 38.00 (17.39) | | |

(continued)

| | | | | |
|---|---|---|---|---|
| **Intron 4 variant** | | | | |
| | b/b (31) | 47.30 (22.42) | 1.2423 | 0.743 |
| | b/a (10) | 54.53 (23.50) | | |
| | a/a (3) | 52.00 (16.74) | | |
| | c/c (1) | 64.00 (0) | | |
| **m2)** | | | | |
| **$ONOO^-$ (5μM LA-419)** | | | | |
| **Variable** | **Level (n)** | **Net increase, nmols/L (mean ± sd)** | **Chi-sq statistic** | **p value** |
| **G894T variant** | | | | |
| | G/G (26) | -74.19 (29.80) | 1.7603 | 0.41 |
| | G/T (18) | -65.80 (26.77) | | |
| | T/T (5) | -82.87 (28.56) | | |
| **T-786C variant** | | | | |
| | T/T (22) | -76.48 (33.23) | 1.7456 | 0.42 |
| | T/C (23) | -65.86 (23.41) | | |
| | C/C (4) | -82.58 (26.16) | | |
| **Intron 4 variant** | | | | |
| | b/b (31) | -70.32 (30.56) | 1.4292 | 0.699 |
| | b/a (10) | -72.30 (25.81) | | |
| | a/a (3) | -63.78 (14.11) | | |
| | c/c (1) | -98.00 (0) | | |
| **m3)** | | | | |
| **$NO/ONOO^-$ (5μM LA-419)** | | | | |
| **Variable** | **Level (n)** | **Net increase, nmols/L (mean ± sd)** | **Chi-sq statistic** | **p value** |
| **G894T variant** | | | | |
| | G/G (26) | -0.85 (0.52) | 2.3998 | 0.30 |
| | G/T (18) | -0.94 (0.88) | | |
| | T/T (5) | -0.78 (1.05) | | |
| **T-786C variant** | | | | |
| | T/T (22) | -0.95 (0.67) | 2.0061 | 0.37 |
| | T/C (23) | -0.86 (0.80) | | |
| | C/C (4) | -0.52 (0.36) | | |
| **Intron 4 variant** | | | | |
| | b/b (31) | 0.43 (0.20) | 0.0468 | 0.997 |
| | b/a (10) | 0.45 (0.22) | | |
| | a/a (3) | 0.41 (0.19) | | |
| | c/c (1) | 0.46 (0) | | |
| Net increase = avg 5μM - avg Cntl. Chi-sq statistic generated from the Non-parametric ANOVA Kruskal-Wallis test | | | | |

**Tables n1 - n3)** Net increase in 1μM LA-419 over control - comparison between genotypes

| | | | | |
|---|---|---|---|---|
| **n1)** | | | | |
| **NO (1μM LA-419)** | | | | |
| **Variable** | **Level (n)** | **Net increase, nmols/L (mean ± sd)** | **Chi-sq statistic** | **p value** |
| **G894T variant** | | | | |
| | G/G (26) | 23.26 (70.68) | 1.1059 | 0.58 |
| | G/T (18) | 58.52 (72.43) | | |
| | T/T (5) | 20.73 (66.94) | | |
| **T-786C variant** | | | | |
| | T/T (22) | 30.80 (64.85) | 2.6735 | 0.26 |
| | T/C (23) | 46.77 (81.32) | | |
| | C/C (4) | 2.08 (39.61) | | |
| **Intron 4 variant** | | | | |
| | b/b (31) | 39.66 (70.27) | 3.6920 | 0.297 |
| | b/a (10) | 56.57 (67.97) | | |
| | a/a (3) | -25.78 (104.49) | | |
| | c/c (1) | -98.67 (0) | | |
| **n2)** | | | | |
| **$ONOO^-$ (1μM LA-419)** | | | | |
| **Variable** | **Level (n)** | **Net increase, nmols/L (mean ± sd)** | **Chi-sq statistic** | **p value** |
| **G894T variant** | | | | |
| | G/G (26) | -18.49 (93.20) | 3.2587 | 0.20 |
| | G/T (18) | -46.52 (105.03) | | |
| | T/T (5) | -116.60 (117.55) | | |
| **T-786C variant** | | | | |
| | T/T (22) | -27.14 (82.12) | 2.5212 | 0.28 |
| | T/C (23) | -61.09 (111.68) | | |
| | C/C (4) | 25.25 (135.63) | | |
| **Intron 4 variant** | | | | |
| | b/b (31) | -51.71 (108.03) | 2.0897 | 0.554 |
| | b/a (10) | -37.57 (99.15) | | |
| | a/a (3) | 26.11 (116.99) | | |
| | c/c (1) | -2.67 (0) | | |
| **n3)** | | | | |
| **$NO/ONOO^-$ (1μM LA-419)** | | | | |
| **Variable** | **Level (n)** | **Net increase, nmols/L (mean ± sd)** | **Chi-sq statistic** | **p value** |
| **G894T variant** | | | | |
| | G/G (26) | 0.53 (7.59) | 3.9613 | 0.14 |
| | G/T (18) | -1.12 (1.02) | | |

(continued)

| n3) | | | | |
|---|---|---|---|---|
| **NO/ONOO$^-$ (1μM LA-419)** | | | | |
| **Variable** | **Level (n)** | **Net increase, nmols/L (mean ± sd)** | **Chi-sq statistic** | **p value** |
| **G894T variant** | | | | |
| | T/T (5) | -0.31 (0.61) | | |
| **T-786C variant** | | | | |
| | T/T (22) | 0.77 (8.26) | 2.2432 | 0.33 |
| | T/C (23) | -0.99 (0.94) | | |
| | C/C (4) | -0.48 (0.48) | | |
| **Intron 4 variant** | | | | |
| | b/b (31) | 0.29 (0.47) | 3.0652 | 0.382 |
| | b/a (10) | 0.28 (0.43) | | |
| | a/a (3) | -0.11 (0.56) | | |
| | c/c (1) | -0.23 (0) | | |
| Net increase = avg 1μM - avg Cntl. Chi-sq statistic generated from the Non-parametric ANOVA Kruskal-Wallis test | | | | |

**Tables o1 - o3)** Net increase in 5μM LA-419 over control - comparison between major allele homozygotes and minor allele carriers

| o1) | | | | |
|---|---|---|---|---|
| **NO (5μM LA-419)** | | | | |
| **Variable** | **Level (n)** | **Net increase, nmols/L (mean ± sd)** | **t statistic** | **p value** |
| **G894T variant** | | | | |
| | G/G (26) | 55.05 (21.03) | -1.6829 | 0.10 |
| | G/T & T/T (23) | 45.12 (21.46) | | |
| **T-786C variant** | | | | |
| | T/T (22) | 55.91 (19.06) | 1.5780 | 0.12 |
| | T/C & C/C (27) | 45.89 (22.83) | | |
| **Intron 4 variant** | | | | |
| | b/b (31) | 47.30 (22.42) | 0.7723 | 0.444 |
| | b/a, a/a, c/c (14) | 47.30 (22.42) | | |
| **o2)** | | | | |
| **ONOO$^-$ (5μM LA-419)** | | | | |
| **Variable** | **Level (n)** | **Net increase, nmols/L (mean ± sd)** | **t statistic** | **p value** |
| **G894T variant** | | | | |
| | G/G (26) | -74.19 (29.80) | 0.4107 | 0.68 |
| | G/T & T/T (23) | -69.51 (27.46) | | |

(continued)

| | | | | |
|---|---|---|---|---|
| **T-786C variant** | | | | |
| | T/T (22) | -76.48 (33.23) | -0.7839 | 0.44 |
| | T/C & C/C (27) | -68.33 (24.07) | | |
| **Intron 4 variant** | | | | |
| | b/b (31) | -70.32 (30.56) | -0.4904 | 0.626 |
| | b/a, a/a, c/c (14) | -72.31 (23.65) | | |
| **o3)** | | | | |
| **NO/ONOO$^-$ (5μM LA-419)** | | | | |
| **Variable** | **Level (n)** | **Net increase, nmols/L (mean ± sd)** | **t statistic** | **p value** |
| **G894T variant** | | | | |
| | G/G (26) | -0.85 (0.52) | 1.0117 | 0.32 |
| | G/T & T/T (23) | -0.91 (0.90) | | |
| **T-786C variant** | | | | |
| | T/T (22) | -0.95 (0.67) | -1.0754 | 0.29 |
| | T/C & C/C (27) | -0.81 (0.76) | | |
| **Intron 4 variant** | | | | |
| | b/b (31) | 0.43 (0.20) | 0.1594 | 0.874 |
| | b/a, a/a, c/c (14) | 0.44 (0.20) | | |
| Net increase = avg 5μM - avg Cntl. t statistic generated from the Non-parametric Mann-Whitney U test | | | | |

**Tables p1 - p3)** Net increase in 1μM LA-419 over control - comparison between major allele homozygotes and minor allele carriers

| | | | | |
|---|---|---|---|---|
| **p1)** | | | | |
| **NO (1μM LA-419)** | | | | |
| **Variable** | **Level (n)** | **Net increase, nmols/L (mean ± sd)** | **t statistic** | **p value** |
| **G894T variant** | | | | |
| | G/G (26) | 23.26 (70.64) | 0.2204 | 0.83 |
| | G/T & T/T (23) | 50.30 (71.57) | | |
| **T-786C variant** | | | | |
| | T/T (22) | 30.80 (64.85) | 0.1307 | 0.90 |
| | T/C & C/C (27) | 40.15 (77.70) | | |
| **Intron 4 variant** | | | | |
| | b/b (31) | 39.66 (70.27) | -0.1103 | 0.913 |
| | b/a, a/a, c/c (14) | 27.83 (86.07) | | |

(continued)

| p2) | | | | |
|---|---|---|---|---|
| **$ONOO^-$ (1μM LA-419)** | | | | |
| **Variable** | **Level (n)** | **Net increase, nmols/L (mean ± sd)** | **t statistic** | **p value** |
| **G894T variant** | | | | |
| | G/G (26) | -18.49 (93.20) | -1.2321 | 0.22 |
| | G/T & T/T (23) | -61.75 (109.14) | | |
| **T-786C variant** | | | | |
| | T/T (22) | -27.14 (82.12) | 1.2664 | 0.21 |
| | T/C & C/C (27) | -48.30 (116.85) | | |
| **Intron 4 variant** | | | | |
| | b/b (31) | -51.71 (108.03) | 0.6620 | 0.511 |
| | b/a, a/a, c/c (14) | -21.43 (98.29) | | |
| **p3)** | | | | |
| **$NO/ONOO^-$ (1μM LA-419)** | | | | |
| **Variable** | **Level (n)** | **Net increase, nmols/L (mean ± sd)** | **t statistic** | **p value** |
| **G894T variant** | | | | |
| | G/G (26) | 0.53 (7.59) | 0.3105 | 0.76 |
| | G/T & T/T (23) | -0.93 (0.99) | | |
| **T-786C variant** | | | | |
| | T/T (22) | 0.77 (8.26) | -0.3518 | 0.73 |
| | T/C & C/C (27) | -0.91 (0.90) | | |
| **Intron 4 variant** | | | | |
| | b/b (31) | 0.29 (0.47) | -0.6252 | 0.535 |
| | b/a, a/a, c/c (14) | 0.16 (0.46) | | |
| Net increase = avg 1μM - avg Cntl. t statistic generated from the Non-parametric Mann-Whitney U test | | | | |

**Tables q1 - q3)** Net increase in 5μM LA-419 over control (Individual allelic combination). Reference group = G/G+T/T

| q1) | | | | |
|---|---|---|---|---|
| **NO (5 μM LA-419 increase over Control)** | | | | |
| **Variable** | **Level (n)** | **Net increase, nmols/L (mean ± sd)** | **Chi-sq statistic** | **p value** |
| **G894T + T-786C variant** | | | | |
| | G/G+T/T (18) | 56.59 (19.21) | 4.2869 | 0.64 |
| | G/G+T/C (8) | 51.58 (25.77) | | |
| | G/G/+C/C (0) | - | | |
| | G/T+T/T (4) | 52.83 (20.89) | | |
| | G/T+T/C (13) | 43.72 (19.86) | | |

(continued)

| q1) | | | | |
|---|---|---|---|---|
| **NO (5 μM LA-419 increase over Control)** | | | | |
| **Variable** | **Level (n)** | **Net increase, nmols/L (mean ± sd)** | **Chi-sq statistic** | **p value** |
| **G894T + T-786C variant** | | | | |
| | G/T+C/C (1) | 46.00 (0) | | |
| | T/T+T/C (2) | 53.00 (50.91) | | |
| | T/T+C/C (3) | 35.33 (20.27) | | |
| **q2)** | | | | |
| **$ONOO^-$ (5 μM LA-419 increase over Control)** | | | | |
| **Variable** | **Level (n)** | **Net increase, nmols/L (mean ± sd)** | **Chi-sq statistic** | **p value** |
| **G894T + T-786C variant** | | | | |
| | G/G+T/T (18) | -77.59 (31.16) | 5.1514 | 0.52 |
| | G/G+T/C (8) | -66.54 (26.77) | | |
| | G/G/+C/C (0) | - | | |
| | G/T+T/T (4) | -71.50 (46.74) | | |
| | G/T+T/C (13) | -65.59 (20.60) | | |
| | G/T+C/C (1) | -45.67 (0) | | |
| | T/T+T/C (2) | -64.83 (44.08) | | |
| | T/T+C/C (3) | -94.89 (10.87) | | |
| **q3)** | | | | |
| **NO/ONOO (5 μM LA-419 increase over Control)** | | | | |
| **Variable** | **Level (n)** | **Net increase, nmols/L (mean ± sd)** | **Chi-sq statistic** | **p value** |
| **G894T + T-786C variant** | | | | |
| | G/G+T/T (18) | -0.88 (0.54) | 4.8102 | 0.57 |
| | G/G+T/C (8) | -0.78 (0.51) | | |
| | G/G/+C/C (0) | - | | |
| | G/T+T/T (4) | -1.29 (1.15) | | |
| | G/T+T/C (13) | -0.83 (0.84) | | |
| | G/T+C/C (1) | -1.01(0) | | |
| | T/T+T/C (2) | -1.41 (1.74) | | |
| | T/T+C/C (3) | -0.36 (0.18) | | |
| Chi-sq statistic generated from the Non-parametric ANOVA Kruskal-Wallis test | | | | |

**Tables r1 - r3)** Net increase in 1μM LA-419 over control (Individual allelic combination). Reference group = G/G+T/T

| **r1)** | | | | |
|---|---|---|---|---|
| **NO (1 μM LA-419 increase over Control)** | | | | |
| **Variable** | **Level (n)** | **Net increase, nmols/L (mean ± sd)** | **Chi-sq statistic** | **p value** |
| **G894T + T-786C variant** | | | | |
| | G/G+T/T (18) | 31.22 (71.86) | 5.6060 | 0.47 |
| | G/G+T/C (8) | 5.33 (68.90) | | |
| | G/G/+C/C (0) | - | | |
| | G/T+T/T (4) | 28.92 (13.00) | | |
| | G/T+T/C (13) | 76.26 (75.55) | | |
| | G/T+C/C (1) | -53.67 (0) | | |
| | T/T+T/C (2) | 20.83 (131.76) | | |
| T/T+C/C | T/T+C/C (3) | 20.67 (16.77) | | |
| **r2)** | | | | |
| **ONOO$^-$ (1 μM LA-419 increase over Control)** | | | | |
| **Variable** | **Level (n)** | **Net increase, nmols/L (mean ± sd)** | **Chi-sq statistic** | **p value** |
| **G894T + T-786C variant** | | | | |
| | G/G+T/T (18) | -32.54 (86.18) | 10.6444 | 0.10 |
| | G/G+T/C (8) | 13.13 (106.46) | | |
| | G/G/+C/C (0) | - | | |
| | G/T+T/T (4) | -2.83 (64.48) | | |
| | G/T+T/C (13) | -81.10 (79.76) | | |
| | G/T+C/C (1) | 228.33 (0) | | |
| | T/T+T/C (2) | -227.83 (117.62) | | |
| | T/T+C/C (3) | -42.44 (9.91) | | |
| **r3)** | | | | |
| **NO/ONOO$^-$ (1 μM LA-419 increase over Control)** | | | | |
| **Variable** | **Level (n)** | **Net increase, nmols/L (mean ± sd)** | **Chi-sq statistic** | **p value** |
| **G894T + T-786C variant** | | | | |
| | G/G+T/T (18) | 1.16 (9.12) | 6.7479 | 0.34 |
| | G/G+T/C (8) | -0.88 (0.63) | | |
| | G/G/+C/C (0) | - | | |
| | G/T+T/T (4) | -0.99 (1.06) | | |
| | G/T+T/C (13) | -1.21 (1.06) | | |
| | G/T+C/C (1) | -0.24 (0) | | |

(continued)

| r3) | | | | |
|---|---|---|---|---|
| **NO/ONOO$^-$ (1 μM LA-419 increase over Control)** | | | | |
| **Variable** | **Level (n)** | **Net increase, nmols/L (mean ± sd)** | **Chi-sq statistic** | **p value** |
| **G894T + T-786C variant** | | | | |
| | T/T+T/C (2) | 0.07 (0.61) | | |
| | T/T+C/C (3) | -0.56 (0.56) | | |
| Chi-sq statistic generated from the Non-parametric ANOVA Kruskal-Wallis test | | | | |

**Tables s1 - s3)** Net increase in 5μM LA-419 over control. Comparison between major allele homozygote and minor allele carriers (reference vs. all carriers combination).

| Reference group = G/G+T/T. | | | | |
|---|---|---|---|---|
| Combination = G/G+T/C, G/G/+C/C, G/T+T/T, G/T+T/C, G/T+C/C, T/T+T/C, T/T+C/C | | | | |
| **s1)** | | | | |
| **NO (5 μM LA-419 increase over Control)** | | | | |
| **Variable** | **Level (n)** | **Net increase, nmols/L (mean ± sd)** | **t statistic** | **p value** |
| **Genotype** | | | | |
| | Reference (18) | 56.59 (19.21) | 1.55 | 0.13 |
| | Combination (31) | 46.78 (22.38) | | |
| **ONOO$^-$ (5 μM LA-419 increase over Control)** | | | | |
| **Variable** | **Level (n)** | **Net increase, nmols/L (mean ± sd)** | **t statistic** | **p value** |
| **Genotype** | | | | |
| | Reference (18) | -77.59 (31.16) | -0.8088 | 0.42 |
| | Combination (31) | -68.74 (26.87) | | |
| **s3)** | | | | |
| **NO/ONOO$^-$ (5 μM LA-419 increase over Control)** | | | | |
| **Variable** | **Level (n)** | **Net increase, nmols/L (mean ± sd)** | **t statistic** | **p value** |
| **Genotype** | | | | |
| | Reference (18) | -0.88 (0.54) | -0.9436 | 0.35 |
| | Combination (31) | -0.87 (0.81) | | |
| t statistic generated from the Non-parametric Mann-Whitney U test | | | | |

**Tables t1 - t3)** Net increase in 1μM LA-419 over control. Comparison between major allele homozygote and minor allele carriers (reference vs. all carriers combination).

| | | | | |
|---|---|---|---|---|
| Reference group = G/G+T/T. | | | | |
| Combinations = G/G+T/C, G/G/+C/C, G/T+T/T, G/T+T/C, G/T+C/C, T/T+T/C, T/T+C/C | | | | |
| **t1)** | | | | |
| **NO (1 μM LA-419 increase over Control)** | | | | |
| **Variable** | **Level (n)** | **Net increase, nmols/L (mean ± sd)** | **t statistic** | **p value** |
| **Genotype** | | | | |
| | Reference (18) | 31.22 (71.86) | 0.3837 | 0.70 |
| | Combination (31) | 38.70 (72.56) | | |
| **t2)** | | | | |
| **$ONOO^-$ (1 μM LA-419 increase over Control)** | | | | |
| **Variable** | **Level (n)** | **Net increase, nmols/L (mean ± sd)** | **t statistic** | **p value** |
| **Genotype** | | | | |
| | Reference (18) | -32.54 (86.18) | 0.7052 | 0.48 |
| | Combination (31) | -42.43 (111.75) | | |
| **t3)** | | | | |
| **$NO/ONOO^-$ (1 μM LA-419 increase over Control)** | | | | |
| **Variable** | **Level (n)** | **Net increase, nmols/L (mean ± sd)** | **t statistic** | **p value** |
| **Genotype** | | | | |
| | Reference (18) | 1.16 (9.12) | -0.3422 | 0.73 |
| | Combination (31) | -0.92 (0.90) | | |
| t statistic generated from the Non-parametric Mann-Whitney U test | | | | |

***Percent Change***

**Tables u1 - u3)** % Change - Mean response for all genotypes and races (5μM LA-419) **% Change = (avg. 5μM - avg. control)/(avg. control) *100**

| | | | | |
|---|---|---|---|---|
| **u1)** | | | | |
| **NO (5μM LA-419)** | | | | |
| **Variable** | **Level (n)** | **% Change (mean ± sd)** | **Chi-sq statistic** | **p value** |
| **G894T variant** | | | | |
| | G/G (26) | 15.93 (6.37) | 4.1721 | 0.12 |
| | G/T (18) | 13.43 (6.31) | | |
| | T/T (5) | 11.67 (6.86) | | |
| **T-786C variant** | | | | |
| | T/T (22) | 16.16 (5.97) | 3.0028 | 0.22 |

(continued)

| | | | | |
|---|---|---|---|---|
| **T-786C variant** | | | | |
| | T/C (23) | 13.58 (6.82) | | |
| | C/C (4) | 11.63 (5.99) | | |
| **Intron 4 variant** | | | | |
| | b/b (31) | 13.52 (6.80) | 2.1674 | 0.538 |
| | b/a (10) | 16.81 (6.88) | | |
| | a/a (3) | 13.60 (3.88) | | |
| | c/c (1) | 17.36 (0) | | |
| **Race** | | | | |
| | Asian (1) | 13.97 (0) | 3.2768 | 0.66 |
| | Black (12) | 12.82 (5.82) | | |
| | Caucasian (26) | 14.30 (6.78) | | |
| | Caucasian/Black (2) | 16.93 (4.34) | | |
| | Hispanic (6) | 17.23 (7.65) | | |
| | Not Reported (2) | 18.69 (5.53) | | |
| **u2)** | | | | |
| **$ONOO^-$ (5μM LA-419)** | | | | |
| **Variable** | **Level (n)** | **% Change (mean ± sd)** | **Chi-sq statistic** | **p value** |
| **G894T variant** | | | | |
| | G/G (26) | -20.65 (7.38) | 1.9795 | 0.37 |
| | G/T (18) | -17.85 (6.26) | | |
| | T/T (5) | -19.03 (8.40) | | |
| **T-786C variant** | | | | |
| | T/T (22) | -20.98 (8.19) | 0.9125 | 0.63 |
| | T/C (23) | -17.73 (5.53) | | |
| | C/C (4) | -21.03 (7.77) | | |
| **Intron 4 variant** | | | | |
| | b/b (31) | -19.19 (7.76) | 1.5576 | 0.669 |
| | b/a (10) | -19.70 (5.79) | | |
| | a/a (3) | -16.60 (3.31) | | |
| | c/c (1) | -22.99 (0). | | |
| **Race** | | | | |
| | Asian (1) | -27.79 (0) | 14.2033 | 0.01 † |
| | Black (12) | -16.43 (6.22) | | |
| | Caucasian (26) | -17.84 (5.90) | | |
| | Caucasian/Black (2) | -25.30 (9.90) | | |
| | Hispanic (6) | -25.17 (5.79) | | |
| | Not Reported (2) | -31.56 (5.34) | | |

(continued)

| u3) | | | | |
|---|---|---|---|---|
| **NO/ONOO$^-$ (5$\mu$M LA-419)** | | | | |
| **Variable** | **Level (n)** | **% Change (mean ± sd)** | **Chi-sq statistic** | **p value** |
| **G894T variant** | | | | |
| | G/G (26) | 47.91 (17.67) | 4.4466 | 0.11 |
| | G/T (18) | 38.86 (13.20) | | |
| | T/T (5) | 39.03 (13.43) | | |
| **T-786C variant** | | | | |
| | T/T (22) | 49.09 (17.76) | 2.9791 | 0.23 |
| | T/C (23) | 38.74 (13.67) | | |
| | C/C (4) | 42.33 (13.21) | | |
| **Intron 4 variant** | | | | |
| | b/b (31) | 42.24 (17.73) | 0.9604 | 0.811 |
| | b/a (10) | 45.81 (14.36) | | |
| | a/a (3) | 36.75 (3.58) | | |
| | c/c (1) | 52.87 (0) | | |
| **Race** | | | | |
| | Asian (1) | 57.83 (0) | 16.6884 | 0.01† |
| | Black (12) | 36.30 (14.59) | | |
| | Caucasian (26) | 39.65 (12.24) | | |
| | Caucasian/Black (2) | 58.92 (16.01) | | |
| | Hispanic (6) | 57.79 (12.24) | | |
| | Not Reported (2) | 75.55 (20.43) | | |
| Chi-sq statistic generated from the Non-parametric ANOVA Kruskal-Wallis test | | | | |

**Tables v1 - v3)** % Change - Mean response for all genotypes and races (1$\mu$M LA-419) **% Change = (avg. 1$\mu$M - avg. control)/(avg. control) *100**

| v1) | | | | |
|---|---|---|---|---|
| **NO (1$\mu$M LA-419)** | | | | |
| **Variable** | **Level (n)** | **% Change (mean ± sd)** | **Chi-sq statistic** | **p value** |
| **G894T variant** | | | | |
| | G/G (26) | 7.77 (18.82) | 0.5639 | 0.75 |
| | G/T (18) | 20.99 (29.98) | | |
| | T/T (5) | 8.69 (18.85) | | |
| **T-786C variant** | | | | |
| | T/T (22) | 9.48 (17.79) | 2.0563 | 0.36 |
| | T/C (23) | 17.70 (29.53) | | |

(continued)

| | | | | |
|---|---|---|---|---|
| **T-786C variant** | | | | |
| | C/C (4) | 1.91 (11.63) | | |
| **Intron 4 variant** | | | | |
| | b/b (31) | 13.46 (23.13) | 4.2767 | 0.233 |
| | b/a (10) | 20.51 (28.08) | | |
| | a/a (3) | -4.57 (24.67) | | |
| | c/c (1) | -26.76 (0) | | |
| **Race** | | | | |
| | Asian (1) | 8.95 (0) | 9.1530 | 0.10 |
| | Black (12) | -4.73 (18.81) | | |
| | Caucasian (26) | 21.25 (25.58) | | |
| | Caucasian/Black (2) | 9.57 (6.43) | | |
| | Hispanic (6) | 11.74 (17.99) | | |
| | Not Reported (2) | 14.50 (2.19) | | |
| **v2)** | | | | |
| **$ONOO^-$ (1μM LA-419)** | | | | |
| **Variable** | **Level (n)** | **% Change (mean ± sd)** | **Chi-sq statistic** | **p value** |
| **G894T variant** | | | | |
| | G/G (26) | -2.10 (27.33) | 2.8861 | 0.24 |
| | G/T (18) | -9.58 (26.95) | | |
| | T/T (5) | -25.44 (21.34) | | |
| **T-786C variant** | | | | |
| | T/T (22) | -5.17 (21.75) | 3.9697 | 0.14 |
| | T/C (23) | -12.40 (28.22) | | |
| | C/C (4) | 11.13 (43.85) | | |
| **Intron 4 variant** | | | | |
| | b/b (31) | -10.24 (27.17) | 2.5320 | 0.470 |
| | b/a (10) | -6.58 (30.92) | | |
| | a/a (3) | 9.62 (33.98) | | |
| | c/c (1) | -0.63 (0) | | |
| **Race** | | | | |
| | Asian (1) | -18.50 (0) | 15.2522 | 0.01† |
| | Black (12) | 16.25 (36.48) | | |
| | Caucasian (26) | -18.41 (17.30) | | |
| | Caucasian/Black (2) | 9.37 (21.79) | | |
| | Hispanic (6) | -6.29 (19.74) | | |
| | Not Reported (2) | -16.55 (2.66) | | |

(continued)

| v3) | | | | |
|---|---|---|---|---|
| **NO/ONOO$^-$ (1μM LA-419)** | | | | |
| **Variable** | **Level (n)** | **% Change (mean ± sd)** | **Chi-sq statistic** | **p value** |
| **G894T variant** | | | | |
| | G/G (26) | 21.83 (51.78) | 0.7130 | 0.70 |
| | G/T (18) | 50.45 (77.28) | | |
| | T/T (5) | 61.97 (81.82) | | |
| **T-786C variant** | | | | |
| | T/T (22) | 25.48 (51.97) | 2.6416 | 0.27 |
| | T/C (23) | 52.82 (77.96) | | |
| | C/C (4) | 2.52 (35.64) | | |
| **Intron 4 variant** | | | | |
| | b/b (31) | 43.27 (71.09) | 3.4675 | 0.325 |
| | b/a (10) | 42.92 (65.15) | | |
| | a/a (3) | -3.62 (44.56) | | |
| | c/c (1) | -26.15 (0) | | |
| **Race** | | | | |
| | Asian (1) | 33.68 (0) | 15.0458 | 0.01† |
| | Black (12) | -8.17 (36.37) | | |
| | Caucasian (26) | 61.80 (74.46) | | |
| | Caucasian/Black (2) | 3.77 (26.27) | | |
| | Hispanic (6) | 26.85 (45.85) | | |
| | Not Reported (2) | 37.24 (1.75) | | |
| Chi-sq statistic generated from the Non-parametric ANOVA Kruskal-Wallis test | | | | |

**Table x1)** Ratio of % Change NO/ % Change ONOO$^-$ (5μM LA-419):

| % Change NO / % Change ONOO$^-$ (5μM LA-419) | | | | |
|---|---|---|---|---|
| **Variable** | **Level (n)** | **% Change Ratio (mean ± sd)** | **Chi-sq statistic** | **p value** |
| **G894T variant** | | | | |
| | G/G (26) | -0.82 (0.40) | 0.5943 | 0.74 |
| | G/T (18) | -0.88 (0.60) | | |
| | T/T (5) | -0.80 (0.73) | | |
| **T-786C variant** | | | | |
| | T/T (22) | -0.90 (0.47) | 1.2795 | 0.53 |
| | T/C (23) | -0.81 (0.56) | | |
| | C/C (4) | -0.62 (0.39) | | |
| **Race** | | | | |
| | Asian (1) | -0.50 (0) | 1.9471 | 0.86 |

(continued)

| Race | | | | |
|---|---|---|---|---|
| | Black (12) | -0.79 (0.44) | | |
| | Caucasian (26) | -0.92 (0.59) | | |
| | Caucasian/Black (2) | -0.76 (0.47) | | |
| | Hispanic (6) | -0.73 (0.40) | | |
| | Not Reported (2) | -0.59 (0.08) | | |
| Chi-sq statistic generated from the Noa-parametric ANOVA Kruskal-Wallis test | | | | |

**Table x2)** Ratio of % Change NO/ % Change $ONOO^-$ (1μM LA-419):

| **% Change NO / % Change $ONOO^-$ (1μM LA-419)** | | | | |
|---|---|---|---|---|
| **Variable** | **Level (n)** | **% Change Ratio (mean ± sd)** | **Chi-sq statistic** | **p value** |
| **G894T variant** | | | | |
| | G/G (26) | 0.76 (8.64) | 1.8736 | 0.39 |
| | G/T (18) | -1.15 (0.91) | | |
| | T/T (5) | -0.66 (1.02) | | |
| **T-786C variant** | | | | |
| | T/T (22) | 1.06 (9.40) | 0.9272 | 0.63 |
| | T/C (23) | -1.06 (0.82) | | |
| | C/C (4) | -0.80 (1.04) | | |
| **Race** | | | | |
| | Asian (1) | -0.48 (0) | 2.2419 | 0.81 |
| | Black (12) | 2.81 (12.60) | | |
| | Caucasian (26) | -1.09 (1.30) | | |
| | Caucasian/Black (2) | -1.07 (1.80) | | |
| | Hispanic (6) | -0.90 (0.55) | | |
| | Not Reported (2) | -0.90 (0.28) | | |
| Chi-sq statistic generated from the Non-parametric ANOVA Kruskal-Wallis test | | | | |

***Fold Difference***

**Tables y1 - y3)** Fold difference LA-419 5μM - comparison between genotypes

| **Fold difference = (LA-419 5μM)/(control)** | | | | |
|---|---|---|---|---|
| **y1)** | | | | |
| **NO (LA-419 5μM)** | | | | |
| **Variable** | **Level (n)** | **Fold difference (mean ± sd)** | **Chi-sq statistic** | **p value** |
| **G894T variant** | | | | |
| | G/G (26) | 1.16 (0.06) | 4.1721 | 0.12 |
| | G/T (18) | 1.13 (0.06) | | |

(continued)

| Fold difference = (LA-419 5μM)/(control) | | | | |
|---|---|---|---|---|
| **y1)** | | | | |
| **NO (LA-419 5μM)** | | | | |
| **Variable** | **Level (n)** | **Fold difference (mean ± sd)** | **Chi-sq statistic** | **p value** |
| **G894T variant** | | | | |
| | T/T (5) | 1.12 (0.07) | | |
| **T-786C variant** | | | | |
| | T/T (22) | 1.16 (0.06) | 3.0028 | 0.22 |
| | T/C (23) | 1.14 (0.07) | | |
| | C/C (4) | 1.12 (0.06) | | |
| **Intron 4 variant** | | | | |
| | b/b (31) | 1.14 (0.07) | 2.1674 | 0.538 |
| | b/a (10) | 1.17 (0.07) | | |
| | a/a (3) | 1.14 (0.04) | | |
| | c/c (1) | 1.17 (0) | | |
| **y2)** | | | | |
| **$ONOO^-$ (LA-419 5μM)** | | | | |
| **Variable** | **Level (n)** | **Fold difference (mean ± sd)** | **Chi-sq statistic** | **p value** |
| **G894T variant** | | | | |
| | G/G (26) | 0.79 (0.07) | 1.9795 | 0.37 |
| | G/T (18) | 0.82 (0.06) | | |
| | T/T (5) | 0.81 (0.08) | | |
| **T-786C variant** | | | | |
| | T/T (22) | 0.79 (0.08) | 0.9125 | 0.63 |
| | T/C (23) | 0.82 (0.06) | | |
| | C/C (4) | 0.79 (0.08) | | |
| **Intron 4 variant** | | | | |
| | b/b (31) | 0.81 (0.08) | 1.5576 | 0.669 |
| | b/a (10) | 0.80 (0.06) | | |
| | a/a (3) | 0.83 (0.03) | | |
| | c/c (1) | 0.77 (0) | | |
| **y3)** | | | | |
| **$NO/ONOO^-$ (LA.419 5μM)** | | | | |
| **Variable** | **Level (n)** | **Fold difference (mean ± sd)** | **Chi-sq statistic** | **p value** |
| **G894T variant** | | | | |
| | G/G (26) | 1.48 (0.18) | 4.4466 | 0.108 |
| | G/T (18) | 1.39 (0.13) | | |
| | T/T (5) | 1.39 (0.13) | | |

(continued)

| T-786C variant | | | | |
|---|---|---|---|---|
| | T/T (22) | 1.49 (0.18) | 2.9791 | 0.226 |
| | T/C(23) | 1.39 (0.14) | | |
| | C/C (4) | 1.42 (0.13) | | |
| **Intron 4 variant** | | | | |
| | b/b (31) | 1.42 (0.18) | 0.9604 | 0.811 |
| | b/a (10) | 1.46 (0.14) | | |
| | a/a (3) | 1.37 (0.04) | | |
| | c/c (1) | 1.53 (0) | | |
| Fold difference = avg 5μM/avg Cntl. Chi-sq statistic generated from the Non-parametric ANOVA Kruskat-Wallis test | | | | |

**Tables z1 - z3) Fold difference LA-419 1μM - comparison between genotypes**

| Fold difference = (LA-419 1μM)/(control) | | | | |
|---|---|---|---|---|
| **z1)** | | | | |
| **NO (LA-419 1μM)** | | | | |
| **Variable** | **level (n)** | **Fold difference (mean ± sd)** | **Chi-sq statistic** | **p value** |
| **G894T variant** | | | | |
| | G/G (26) | 1.08 (0.19) | 0.5639 | 0.75 |
| | G/T (18) | 1.21 (0.30) | | |
| | T/T (5) | 1.09 (0.19) | | |
| **T-786C variant** | | | | |
| | T/T (22) | 1.09 (0.18) | 2.0563 | 0.36 |
| | T/C (23) | 1.18 (0.30) | | |
| | C/C (4) | 1.02 (0.12) | | |
| **Intron 4 variant** | | | | |
| | b/b (31) | 1.13 (0.23) | 4.276 | 0.233 |
| | b/a (10) | 1.21 (0.28) | | |
| | a/a (3) | 0.95 (0.25) | | |
| | c/c (1) | 0.73 (0) | | |
| **z2)** | | | | |
| **$ONOO^-$ (LA-419 1μM)** | | | | |
| **Variable** | **Level (n)** | **Fold difference (mean ± sd)** | **Chi-sq statistic** | **p value** |
| **G894T variant** | | | | |
| | G/G (26) | 0.98 (0.27) | 2.8861 | 0.24 |
| | G/T (18) | 0.90 (0.27) | | |
| | T/T(5) | 0.75 (0.21) | | |
| **T-786C variant** | | | | |
| | T/T (22) | 0.95 (0.22) | 3.9697 | 0.14 |

(continued)

| | | | | |
|---|---|---|---|---|
| **T-786C variant** | | | | |
| | T/C (23) | 0.88 (0.28) | | |
| | C/C (4) | 1.11 (0.44) | | |
| **Intron 4 variant** | | | | |
| | b/b (31) | 0.90 (0.27) | 2.5320 | 0.470 |
| | b/a (10) | 0.93 (0.31) | | |
| | a/a (3) | 1.10 (0.34) | | |
| | c/c (1) | 0.99 (0) | | |
| **z3)** | | | | |
| **NO/ONNOO$^-$ (LA-419 1μM)** | | | | |
| **Variable** | **Level (n)** | **Fold difference (mean ± sd)** | **Chi-sq statistic** | **p value** |
| **G894T variant** | | | | |
| | G/G (26) | 1.22 (0.52) | 0.7130 | 0.700 |
| | G/T (18) | 1.50 (0.77) | | |
| | T/T (5) | 1.62 (0.82) | | |
| **T-786C variant** | | | | |
| | T/T (22) | 1.25 (0.52) | 2.6416 | 0.267 |
| | T/C (23) | 1.53 (0.78) | | |
| | C/C (4) | 1.03 (0.36) | | |
| **Intron 4 variant** | | | | |
| | b/b (31) | 1.43 (0.71) | 3.4675 | 0.325 |
| | b/a (10) | 1.43 (0.65) | | |
| | a/a (3) | 0.96 (0.45) | | |
| | c/c (1) | 0.74 (0) | | |
| Fold difference = avg 1μM/avg Cntl. Chi-sq statistic generated from the Non-parametric ANOVA Kruskal-Wallis test | | | | |

**Tables aal - aa3)** Fold difference LA-419 5μM - comparison between major allele homozygotes and minor allele carriers

| | | | | |
|---|---|---|---|---|
| **Fold difference = (LA-419 5μM)/(control)** | | | | |
| **aa1)** | | | | |
| **NO (LA-419 5μM)** | | | | |
| **Variable** | **Level (n)** | **Fold difference (mean ± sd)** | **t statistic** | **p value** |
| **G894T variant** | | | | |
| | G/G (26) | 1.16 (0.06) | -1.9933 | 0.05t |
| | G/T & T/T (23) | 1.13 (0.06) | | |
| **T-786C variant** | | | | |
| | T/T (22) | 1.16 (0.06) | 1.5779 | 0.12 |
| | T/C & C/C (27) | 1.13 (0.07) | | |

(continued)

| | | | | |
|---|---|---|---|---|
| **Intron 4 variant** | | | | |
| | b/b (31) | 1.14 (0.07) | 1.2136 | 0.231 |
| | b/a, a/a, c/c (14) | 1.16 (0.06) | | |
| **aa2)** | | | | |
| **$ONOO^-$ (LA-419 5μM)** | | | | |
| **Variable** | **Level (n)** | **Fold difference (mean ± sd)** | **t statistic** | **p value** |
| **G894T variant** | | | | |
| | G/G (26) | 0.79 (0.07) | 1.3923 | 0.17 |
| | G/T & T/T (23) | 0.82 (0.07) | | |
| **T-786C variant** | | | | |
| | T/T (22) | 0.79 (0.08) | -0.8744 | 0.39 |
| | T/C & C/C (27) | 0.82 (0.06) | | |
| **Intron 4 variant** | | | | |
| | b/b (31) | 0.81 (0.08) | -0.3555 | 0.724 |
| | b/a, a/a, c/c (14) | 0.81 (0.05) | | |
| **aa3)** | | | | |
| **$NO/ONOO^-$ (LA-419 5μM)** | | | | |
| **Variable** | **Level (n)** | **Fold difference (mean ± sd)** | **t statistic** | **p value** |
| **G894T variant** | | | | |
| | G/G (26) | 1.48 (0.18) | -2.0935 | 0.042† |
| | G/T & T/T (23) | 1.39 (0.13) | | |
| **T-786C variant** | | | | |
| | T/T (22), | 1.49 (0.18) | 1.6784 | 0.100 |
| | T/C & C/C (27) | 1.39 (0.13) | | |
| **Intron 4 variant** | | | | |
| | b/b (31) | 1.42 (0.18) | 0.5516 | 0.584 |
| | b(a, a/a, c/c (14) | 1.44 (0.13) | | |
| Fold difference = avg 5μM / avg Cntl. t statistic generated from the Non-parametric Mann-Whitney U test | | | | |

**Tables bbl - bb3)** Fold difference LA-419 1μM - comparison between major allele homozygotes and minor allele carriers

| **Fold change = (LA-419 1μM)/(control)** | | | | |
|---|---|---|---|---|
| **bb1)** | | | | |
| **NO (LA-419 1μM)** | | | | |
| **Variable** | **Level (n)** | **Fold difference (mean ± sd)** | **t statistic** | **p value** |
| **G894T variant** | | | | |
| | G/G (26) | 1.08 (0.19) | 0.0100 | 0.99 |

(continued)

| Fold change = (LA-419 1μM)/(control) | | | | |
|---|---|---|---|---|
| **bb1)** | | | | |
| **NO (LA-419 1μM)** | | | | |
| **Variable** | **Level (n)** | **Fold difference (mean ± sd)** | **t statistic** | **p value** |
| **G894T variant** | | | | |
| | G/T & T/T (23) | 1.18 (0.28) | | |
| **T-786C variant** | | | | |
| | T/T (22) | 1.09 (0.18) | 0.2111 | 0.83 |
| | T/C & C/C (27) | 1.15 (0.28) | | |
| **Intron 4 variant** | | | | |
| | b/b (31) | 1.13 (0.23) | 0.0000 | 1.000 |
| | b/a, a/a, c/c (14) | 1.13 (0.23) | | |
| **bb2)** | | | | |
| **$ONOO^-$ (LA-419 1μM)** | | | | |
| **Variable** | **Level (n)** | **Fold difference (mean ± sd)** | **t statistic** | **p value** |
| **G894T variant** | | | | |
| | G/G (26) | 0.98 (0.27) | -1.3322 | 0.19 |
| | G/T & T/T (23) | 0.87 (0.26) | | |
| **T-786C variant** | | | | |
| | T/T (22) | 0.95 (0.22) | 1.4774 | 0.15 |
| | T/C & C/C (27) | 0.91 (0.31) | | |
| **Intron 4 variant** | | | | |
| | b/b (31) | 0.90 (0.27) | 0.7233 | 0.473 |
| | b/a, a/a, c/c (14) | 0.97 (0.30) | | |
| **bb3)** | | | | |
| **NO/$ONOO^-$ (LA-419 1μM)** | | | | |
| **Variable** | **Level (n)** | **Fold difference (mean ± sd)** | **t statistic** | **p value** |
| **G894T variant** | | | | |
| | G/G (26) | 1.22 (0.52) | 0.4508 | 0.654 |
| | G/T & T/T (23) | 1.53 (0.77) | | |
| **T-786C variant** | | | | |
| | T/T (22) | 1.25 (0.52) | -0.6131 | 0.543 |
| | T/C & C/C (27) | 1.45 (0.75) | | |
| **Intron 4 variant** | | | | |
| | b/b (31) | 1.43 (0.71) | -0.4781 | 0.635 |
| | b/a, a/a, c/c (14) | 1.28 (0.62) | | |
| Fold difference = avg 1μM / avg Cntl. t statistic generated from the Non-parametric Mann-Whitney U test | | | | |

**Tables cc1 - cc3)** Fold difference LA-419 5μM (Individual allelic combination). Reference group = G/G+T/T

| **Fold difference = (LA-419 5μM)/(control)** | | | | |
|---|---|---|---|---|
| **cc1)** | | | | |
| **NO (5 μM LA-419)** | | | | |
| **Variable** | **Level (n)** | **Fold difference (mean ± sd)** | **Chi-sq statistic** | **p value** |
| **G894T + T-786C variant** | | | | |
| | G/G+T/T (18) | 1.17 (0.06) | 4.6351 | 0.59 |
| | G/G+T/C (8) | 1.14 (0.07) | | |
| | G/G/+C/C (0) | - | | |
| | G/T+T/T (4) | 1.14 (0.05) | | |
| | G/T+T/C (13) | 1.13 (0.07) | | |
| | G/T+C/C (1) | 1.12 (0) | | |
| | T/T+T/C (2) | 1.12 (0.09) | | |
| | T/T+C/C (3) | 1.12 (0.07) | | |
| **cc2)** | | | | |
| **$ONOO^-$ (5 μM LA-419)** | | | | |
| **Variable** | **Level (n)** | **Fold difference (mean ± sd)** | **Chi-sq statistic** | **p value** |
| **G894T + T-786C variant** | | | | |
| | G/G+T/T (18) | 0.79 (0.08) | 4.4377 | 0.62 |
| | G/G+T/C (8) | 0.80 (0.07) | | |
| | G/G/+C/C (0) | - | | |
| | G/T+T/T (4) | 0.79 (0.12) | | |
| | G/T+T/C (13) | 0.83 (0.04) | | |
| | G/T+C/C (1) | 0.85 (0) | | |
| | T/T+T/C (2) | 0.87 (0.06) | | |
| | T/T+C/C (3) | 0.77 (0.08) | | |
| **cc3)** | | | | |
| **$NO/ONOO^-$ (5 μM LA-419)** | | | | |
| **Variable** | **Level (n)** | **Fold difference (mean ± sd)** | **Chi-sq statistic** | **p value** |
| **G894T + T-786C variant** | | | | |
| | G/G+T/T (18) | 1.50 (0.18) | 8.3116 | 0.216 |
| | G/G+T/C (8) | 1.44 (0.18) | | |
| | G/G/+C/C (0) | - | | |
| | G/T+T/T (4) | 1.47 (0.19) | | |
| | G/T+T/C (13) | 1.37 (0.11) | | |
| | G/T+C/C (1) | 1.32 (0) | | |
| | T/T+T/C (2) | 1.29 (0.01) | | |
| | T/T+C/C (3) | 1.46 (0.14) | | |
| Chi-sq statistic generated from the Non-parametric ANOVA Kruskal-Wallis test | | | | |

**Tables dd1 - dd3)** Fold difference LA-419 1μM (Individual allelic combination). Reference group = G/G+T/T

| **Fold difference = (LA-419 1μM)/(control)** | | | | |
|---|---|---|---|---|
| **dd1)** | | | | |
| **NO (1μM LA-419)** | | | | |
| **Variable** | **Level (n)** | **Fold difference (mean ± sd)** | **Chi-sq statistic** | **p value** |
| **G894T + T-786C variant** | | | | |
| | G/G+T/T (18) | 1.10 (0.20) | 5.0913 | 0.53 |
| | G/G+T/C (8) | 1.03 (0.17) | | |
| | G/G/+C/C (0) | - | | |
| | G/T+T/T (4) | 1.08 (0.03) | | |
| | G/T+T/C (13) | 1.28 (0.33) | | |
| | G/T+C/C (1) | 0.86 (0) | | |
| | T/T+T/C (2) | 1.11 (0.36) | | |
| | T/T+C/C (3) | 1.07 (0.06) | | |
| **dd2)** | | | | |
| **$ONOO^-$ (1 μM LA-419)** | | | | |
| **Variable** | **Level (n)** | **Fold difference (mean ± sd)** | **Chi-sq statistic** | **p value** |
| **G894T + T-786C variant** | T-786C | T-786C | T-786C | |
| | G/G+T/T (18) | 0.94 (0.23) | 11.1807 | 0.08 |
| | G/G+T/C (8) | 1.07 (0.36) | | |
| | G/G/+C/C (0) | - | | |
| | G/T+T/T (4) | 0.98 (0.18) | | |
| | G/T+T/C (13) | 0.81 (0.15) | | |
| | G/T+C/C (1) | 1.77 (0) | | |
| | T/T+T/C (2) | 0.52 (0.11) | | |
| | T/T+C/C (3) | 0.89 (0.06) | | |
| **dd3)** | | | | |
| **NO/$ONOO^-$ 1 μM LA-419)** | | | | |
| **Variable** | **Level (n)** | **Fold difference (mean ± sd)** | **Chi-sq statistic** | **p value** |
| **G894T + T-786C variant** | | | | |
| | G/G+T/T (18) | 1.28 (0.57) | 8.1332 | 0.229 |
| | G/G+T/C (8) | 1.07 (0.37) | | |
| | G/G/+C/C (0) | - | | |
| | G/T+T/T (4) | 1.12 (0.18) | | |
| | G/T+T/C (13) | 1.70 (0.81) | | |
| | G/T+C/C (1) | 0.49 (0) | | |
| | T/T+T/C (2) | 2.25 (1.17) | | |
| | T/T+C/C (3) | 1.20 (0.04) | | |
| Chi-sq statistic generated from the Non-parametric ANOVA Kruskal-Wallis test | | | | |

**Tables ee1 - ee3)** Fold difference LA-419 5μM. Comparison between major allele homozygote and minor allele carriers (reference vs. all carriers combination).

| Reference group = G/G+T/T. | | | | |
|---|---|---|---|---|
| Combination = G/G+T/C, G/G/+C/C, G/T+T/T, G/T+T/C, G/T+C/C, T/T+T/C, T/T+C/C | | | | |
| **Fold difference = (LA-419 5μM)/(control)** | | | | |
| **ee1** | | | | |
| **NO (5 μM LA-419)** | | | | |
| **Variable** | **Level (n)** | **Fold difference (mean ± sd)** | **t statistic** | **p value** |
| **Genotype** | | | | |
| | Reference (18) | 1.17 (0.06) | 1.7317 | 0.09 |
| | Combination (31) | 1.13 (0.06) | | |
| **ee2)** | | | | |
| **ONOO⁻ (5 μM LA-419)** | | | | |
| **Variable** | **Level (n)** | **Fold difference (mean ± sd)** | **t statistic** | **p value** |
| **Genotype** | | | | |
| | Reference (18) | 0.79 (0.06) | -0.8607 | 0.39 |
| | Combination (31) | 0.81 (0.07) | | |
| **ee3)** | | | | |
| **NO/ONOO⁻ (5 μM LA-419)** | | | | |
| **Variable** | **Level (n)** | **Fold difference (mean ± sd)** | **t statistic** | **p value** |
| **Genotype** | | | | |
| | Reference (18) | 1.50 (0.18) | 1.5451 | 0.130 |
| | Combination (31) | 1.40 (0.14) | | |
| t statistic generated from the Non-parametric Mann-Whitney U test | | | | |

**Tables ff1 - ff3)** Fold difference LA-419 1μM. Comparison between major allele homozygote and minor allele carriers (reference vs. all carriers combination).

| Reference group = G/G+T/T. | | | | |
|---|---|---|---|---|
| Combination = G/G+T/C, G/G/+C/C, G/T+T/T, G/T+T/C, G/T+C/C, T/T+T/C, T/T+C/C | | | | |
| **Fold difference = (LA-419 1μM)/(control)** | | | | |
| **ff1)** | | | | |
| **NO (1 μM LA-419)** | | | | |
| **Variable** | **Level (n)** | **Fold difference (mean ± sd)** | **t statistic** | **p value** |
| **Genotype** | | | | |
| | Reference (18) | 1.10 (0.20) | 0.5911 | 0.56 |
| | Combination (31) | 1.14 (0.26) | | |

(continued)

| ff2) | | | | |
|---|---|---|---|---|
| **ONOO⁻ (1 μM LA-419)** | | | | |
| **Variable** | **Level (n)** | **Fold difference (mean ± sd)** | **t statistic** | **p value** |
| **Genotype** | | | | |
| | Reference (18) | 0.94 (0.23) | 0.9851 | 0.33 |
| | Combination (31) | 0.92 (0.30) | | |
| **ff3)** | | | | |
| **NO/ONOO⁻ (1 μM LA-419)** | | | | |
| **Variable** | **Level (n)** | **Fold difference (mean ± sd)** | **t statistic** | **p value** |
| **Genotype** | | | | |
| | Reference (18) | 1.28 (0.57) | -0.0726 | 0.942 |
| | Combination (31) | 1.41 (0.71) | | |
| t statistic generated from the Non-parametric Mann-Whitney U test | | | | |

***Fold difference within Caucasian***

Tables gg1 - gg3) Caucasian Fold difference LA-419 5μM - comparison between genotypes

| **Fold difference = (LA-419 5μM)/(control)** | | | | |
|---|---|---|---|---|
| **gg1)** | | | | |
| **NO (LA-419 5μM) Caucasian** | | | | |
| **Variable** | **Level (n)** | **Fold difference (mean ± sd)** | **Chi-sq statistic** | **p value** |
| **G894T variant** | | | | |
| | G/G (10) | 1.19 (0.07) | 7.8918 | 0.02† |
| | G/T (13) | 1.12 (0.05) | | |
| | T/T (3) | 1.11 (0.07) | | |
| **T-786C variant** | | | | |
| | T/T (10) | 1.17 (0.08) | 3.7379 | 0.15 |
| | T/C (15) | 1.13 (0.06) | | |
| | C/C (1) | 1.08 (0) | | |
| **Intron 4 variant** | | | | |
| | b/b (18) | 1.13 (0.07) | 1.1544 | 0.56 |
| | b/a (5) | 1.16 (0.06) | | |
| | a/a (1) | 1.17 (0) | | |

(continued)

| gg2) | | | | |
|---|---|---|---|---|
| ONOO⁻ (LA-419 5μM) Caucasian | | | | |
| **Variable** | **Level (n)** | **Fold difference (mean ± sd)** | **Chi-sq statistic** | **p value** |
| **G894T variant** | | | | |
| | G/G (10) | 0.81 (0.0.4) | 1.6319 | 0.44 |
| | G/T (13) | 0.83 (0.06) | | |
| | T/T (3) | 0.80 (0.12) | | |
| **T-786C variant** | | | | |
| | T/T (10) | 0.82 (0.07) | 3.2593 | 0.20 |
| | T/C (15) | 0.83 (0.04) | | |
| | C/C (1) | 0.67 (0) | | |
| **Intron 4 variant** | | | | |
| | b/b (18) | 0.82 (0.06) | 0.4844 | 0.78 |
| | b/a (5) | 0.83 (0.05) | | |
| | a/a (1) | 0.85 (0) | | |
| | | | | |
| **gg3)** | | | | |
| **NO/ONOO⁻ (LA-419 5μM) Caucasian** | | | | |
| **Variable** | **Level (n)** | **Fold difference (mean ± sd)** | **Chi-sq statistic** | **p value** |
| **G894T variant** | | | | |
| | G/G (10) | 1.47 (0.12) | 5.6513 | 0.06 |
| | G/T (13) | 1.34 (0.09) | | |
| | T/T (3) | 1.39 (0.17) | | |
| **T-786C variant** | | | | |
| | T/T (10) | 1.49 (0.14) | 5.1960 | 0.07 |
| | T/C (15) | 1.35 (0.09) | | |
| | C/C (1) | 1.59 (0) | | |
| **Intron 4 variant** | | | | |
| | b/b (18) | 1.39 (0.13) | 0.1518 | 0.93 |
| | b/a (5) | 1.39 (0.12) | | |
| | a/a (1) | 1.39 (0) | | |
| Fold difference = avg 5μM / avg Cntl. Chi-sq statistic generated from the Non-parametric ANOVA Kruskal-Wallis test | | | | |

**Tables hh1 - hh3)** Caucasian Fold difference LA-419 1μM - comparison between genotypes

| **Fold difference = (LA-419 1μM)/(control)** | | | | |
|---|---|---|---|---|
| **hh1)** | | | | |
| **NO (LA-419 1μM) Caucasian** | | | | |
| **Variable** | **Level (n)** | **Fold difference (mean ± sd)** | **Chi-sq statistic** | **p value** |
| **G894T variant** | | | | |
| | G/G (10) | 1.21 (0.12) | 3.6332 | 0.16 |
| | G/T (13) | 1.24 (0.33) | | |
| | T/T (3) | 1.08 (0.26) | | |
| **T-786C variant** | | | | |
| | T/T (10) | 1.20 (0.13) | 3.1174 | 0.21 |
| | T/C (15) | 1.24 (0.32) | | |
| | C/C (1) | 1.02 (0) | | |
| **Intron 4 variant** | | | | |
| | b/b (18) | 1.21 (0.25) | 0.4540 | 0.80 |
| | b/a (5) | 1.28 (0.35) | | |
| | a/a (1) | 1.09 (0) | | |
| **hh2)** | | | | |
| **ONOO (LA-4191μM) Caucasian** | | | | |
| **Variable** | **Level (n)** | **Fold difference (mean ± sd)** | **Chi-sq statistic** | **p value** |
| **G894T variant** | | | | |
| | G/G (10) | 0.86 (0.18) | 5.1185 | 0.08 |
| | G/T (13) | 0.83 (0.15) | | |
| | T/T (3) | 0.63 (0.19) | | |
| **T-786C variant** | | | | |
| | T/T (10) | 0.86 (0.18) | 2.5840 | 0.27 |
| | T/C (15) | 0.79 (0.17) | | |
| | C/C (1) | 0.83 (0) | | |
| **Intron 4 variant** | | | | |
| | b/b (18) | 0.79 (0.20) | 0.5404 | 0.76 |
| | b/a (5) | 0.85 (0.12) | | |
| | a/a (1) | 0.89 (0) | | |
| **hh3)** | | | | |
| **NO/ONOO$^-$ (LA-419 1μm) Caucasians** | | | | |
| **Variable** | **Level (n)** | **Fold difference (mean ± sd)** | **Chi-sq statistic** | **p value** |
| **G894T variant** | | | | |
| | G/G (10) | 1.52 (0.61) | 2.7557 | 0.25 |
| | G/T (13) | 1.63 (0.82) | | |
| | T/T (3) | 1.90 (1.02) | | |

(continued)

| | | | | |
|---|---|---|---|---|
| **T-786C variant** | | | | |
| | T/T (10) | 1.50 (0.62) | 0.1225 | 0.94 |
| | T/C (15) | 1.72 (0.84) | | |
| | C/C (1) | 1.23 (0) | | |
| **Intron 4 variant** | | | | |
| | b/b (18) | 1.69 (0.80) | 0.0640 | 0.97 |
| | b/a (5) | 1.58 (0.77) | | |
| | a/a (1) | 1.23 (0) | | |
| Fold difference = avg 1μM / avg Cntl. Chi-sq statistic generated from the Non-parametric ANOVA Kruskal-Wallis test | | | | |

**Tables ii1 - ii3)** Caucasian Fold difference LA-419 5μM - comparison between major allele homozygotes and minor allele carriers

| **Fold difference = (LA-419 5μM)/(control)** | | | | |
|---|---|---|---|---|
| **ii1)** | | | | |
| **NO (5μM) Caucasian** | | | | |
| **Variable** | **Level (n)** | **Fold difference (mean ± sd)** | **t statistic** | **p value** |
| **G894T variant** | | | | |
| | G/G (10) | 1.19 (0.07) | 2.7670 | 0.001† |
| | G/T & T/T (16) | 1.11 (0.05) | | |
| **T-786C variant** | | | | |
| | T/T (10) | 1.17 (0.08) | 1.7656 | 0.09 |
| | T/C & C/C (16) | 1.12 (0.06) | | |
| **Intron 4 variant** | | | | |
| | b/b (18) | 1.13 (0.07) | 1.0333 | 0.31 |
| | b/a & a/a (6) | 1.16 (0.06) | | |
| **ii2)** | | | | |
| **$ONOO^-$ (5μM) Caucasian** | | | | |
| **Variable** | **Level (n)** | **Fold difference (mean ± sd)** | **t statistic** | **p value** |
| **G894T variant** | | | | |
| | G/G (10) | 0.81 (0.04) | -1.1859 | 0.25 |
| | G/T & T/T (16) | 0.83 (0.07) | | |
| **T-786C variant** | | | | |
| | T/T (10) | 0.82 (0.07) | -0.3953 | 0.67 |
| | T/C & C/C (16) | 0.82 (0.06) | | |
| **Intron 4 variant** | | | | |
| | b/b (18) | 0.82 (0.06) | 0.4333 | 0.67 |
| | b/a & a/a (6) | 0.83 (0.05) | | |

(continued)

| ii3) | | | | |
|---|---|---|---|---|
| **NO/ONOO$^-$ (5μM) Caucasian** | | | | |
| **Variable** | **Level (n)** | **Fold difference (mean ± sd)** | **t statistic** | **p value** |
| **G894T variant** | | | | |
| | G/G (10) | 1.47 (0.12) | 2.3454 | 0.027† |
| | G/T & T/T (16) | 1.35 (0.10) | | |
| **T-786C variant** | | | | |
| | T/T (10) | 1.45 (0.14) | 1.3967 | 0.17 |
| | T/C & C/C (16) | 1.36 (0.10) | | |
| **Intron 4 variant** | | | | |
| | b/b (18) | 1.39 (0.13) | 0.2333 | 0.82 |
| | b/a & a/a (6) | 1.39 (0.11) | | |
| Fold difference = avg 5μM / avg Cntl. t statistic generated from the Non-parametric Mann-Whitney U test | | | | |

**Tables jj1 - jj3)** Caucasian Fold difference LA-419 1μM - comparison between major allele homozygotes and minor allele carriers

| **Fold difference = (LA-419 1μM)/(control)** | | | | |
|---|---|---|---|---|
| **jj1)** | | | | |
| **NO (1μM) Caucasian** | | | | |
| **Variable** | **Level (n)** | **Fold difference (mean ± sd)** | **t statistic** | **p value** |
| **G894T variant** | | | | |
| | G/G (10) | 1.21 (0.12) | 1.7129 | 0.10 |
| | G/T & T/T (16) | 1.21 (0.32) | | |
| **T-786C variant** | | | | |
| | T/T (10) | 1.20 (0.13) | 1.0804 | 0.29 |
| | T/C & C/C (16) | 1.22 (0.31) | | |
| **Intron 4 variant** | | | | |
| | b/b (18) | 1.21 (0.25) | 0.3667 | 0.72 |
| | b/a & a/a (6) | 1.25 (0.33) | | |
| **jj2)** | | | | |
| **ONOO$^-$ (1μM) Caucasian** | | | | |
| **Variable** | **Level (n)** | **Fold difference (mean ± sd)** | **t statistic** | **p value** |
| **G894T variant** | | | | |
| | G/G (10) | 0.86 (0.18) | 1.0804 | 0.29 |
| | G/T & T/T (16) | 0.79 (0.17) | | |
| **T-786C variant** | | | | |
| | T/T (10) | 0.86 (0.18) | 1.5021 | 0.15 |

(continued)

| | | | | |
|---|---|---|---|---|
| **T-786C variant** | | | | |
| | T/C & C/C (16) | 0.79 (0.17) | | |
| **Intron 4 variant** | | | | |
| | b/b (18) | 0.79 (0.20) | 0.7000 | 0.49 |
| | b/a & a/a (6) | 0.86 (0.10) | | |
| **ii3)** | | | | |
| **NO/ONOO⁻ (1μM) Caucasian** | | | | |
| **Variable** | **Level (n)** | **Fold difference (mean ± sd)** | **t statistic** | **p value** |
| **G894T variant** | | | | |
| | G/G (10) | 1.52 (0.61) | 0.8696 | 0.39 |
| | G/T & T/T (16) | 1.68 (0.83) | | |
| **T-786C variant** | | | | |
| | T/T (10) | 1.50 (0.62) | -0.0264 | 0.98 |
| | T/C & C/C (16) | 1.69 (0.82) | | |
| **Intron 4 variant** | | | | |
| | b/b (18) | 1.69 (0.80) | -0.1667 | 0.87 |
| | b/a & a/a (6) | 1.52 (0.70) | | |
| Fold difference = avg 1μM / avg Cntl. t statistic generated from the Non-parametric Mann-Whitney U test | | | | |

**Tables kk1 - kk3)** Caucasian Fold difference LA-419 5μM (Individual allelic combination). Reference group = G/G+T/T

| **Fold difference = (LA-419 5μM)/(control)** | | | | |
|---|---|---|---|---|
| **kk1)** | | | | |
| **NO (5 μM LA-419) Caucasian** | | | | |
| **Variable** | **Level (n)** | **Fold difference (mean ± sd)** | **Chi-sq statistic** | **value** |
| **G894T + T-786C variant** | | | | |
| | G/G+T/T (7) | 1.19 (0.08) | 8.9129 | 0.11 |
| | G/G+T/C (3) | 1.19 (0.02) | | |
| | G/G/+C/C (0) | - | | |
| | G/T+T/T (3) | 1.14 (0.06) | | |
| | G/T+T/C (10) | 1.11 (0.05) | | |
| | G/T+C/C (0) | - | | |
| | T/T+T/C (2) | 1.12 (0.09) | | |
| | T/T+C/C (1) | 1.08 (0) | | |

(continued)

| | | | | |
|---|---|---|---|---|
| **kk2)** | | | | |
| **$ONOO^-$ (5 μM LA-419) Caucasian** | | | | |
| **Variable** | **Level (n)** | **Fold difference (mean ± sd)** | **Chi-sq statistic** | **p value** |
| **G894T + T-786C variant** | | | | |
| | G/G+T/T (7) | 0.81 (0.04) | 5.6036 | 0.35 |
| | G/G+T/C (3) | 0.81 (0.03) | | |
| | G/G/+C/C (0) | - | | |
| | G/T+T/T (3) | 0.83 (0.11) | | |
| | G/T+T/C (10) | 0.84 (0.04) | | |
| | G/T+C/C (0) | - | | |
| | T/T+T/C (2) | 0.87 (0.06) | | |
| | T/T+C/C (1) | 0.67 (0) | | |
| **kk3)** | | | | |
| **NO/$ONOO^-$ (5 μM LA-419) Caucasian** | | | | |
| **Variable** | **Level (n)** | **Fold difference (mean ± sd)** | **Chi-sq statistic** | **p value** |
| **G894T + T-786C variant** | | | | |
| | G/G+T/T (7) | 1.47 (0.14) | 11.0915 | 0.0496† |
| | G/G+T/C (3) | 1.47 (0.08) | | |
| | G/G/+C/C (0) | - | | |
| | G/T+T/T (3) | 1.39 (0.14) | | |
| | G/T+T/C (10) | 1.33 (0.06) | | |
| | G/T+C/C (0) | - | | |
| | T/T+T/C (2) | 1.29 (0.01) | | |
| | T/T+C/C (1) | 1.59 (0) | | |
| Chi-sq statistic generated from the Non-parametric ANOVA Kruskal-Wallis test | | | | |

**Tables ll1 - ll3)** Caucasian Fold difference LA-419 1μM (Individual allelic combination). Reference group = G/G+T/T

| | | | | |
|---|---|---|---|---|
| **Fold difference = (LA-419 1μM)/(control)** | | | | |
| **ll1)** | | | | |
| **NO (1μM LA-419) Caucasian** | | | | |
| **Variable** | **Level (n)** | **Fold difference (mean ± sd)** | **Chi-sq statistic** | **p value** |
| **G894T + T-786C variant** | | | | |
| | G/G+T/T (7) | 1.25 (0.12) | 5.7933 | 0.33 |
| | G/G+T/C (3) | 1.13 (0.03) | | |

(continued)

| Fold difference = (LA-419 1μM)/(control) | | | | |
|---|---|---|---|---|
| **II1)** | | | | |
| **NO (1μM LA-419) Caucasian** | | | | |
| **Variable** | **Level (n)** | **Fold difference (mean ± sd)** | **Chi-sq statistic** | **p value** |
| **G894T + T-786C variant** | | | | |
| | G/G/+C/C (0) | - | | |
| | G/T+T/T (3) | 1.08 (0.03) | | |
| | G/T+T/C (10) | 1.29 (0.37) | | |
| | G/T+C/C (0) | - | | |
| | T/T+T/C (2) | 1.11 (0.36) | | |
| | T/T+C/C (1) | 1.02 (0) | | |
| **II2)** | | | | |
| **ONOO$^-$ (1 μM LA-419) Caucasian** | | | | |
| **Variable** | **Level (n)** | **Fold difference (mean ± sd)** | **Chi-sq statistic** | **p value** |
| **G894T + T-786C variant** | | | | |
| | G/G+T/T (7) | 0.84 (0.22) | 6.2678 | 0.28 |
| | G/G+T/C (3) | 0.89 (0.02) | | |
| | G/G/+C/C (0) | - | | |
| | G/T+T/T (3) | 0.90 (0.06) | | |
| | G/T+T/C (10) | 0.81 (0.16) | | |
| | G/T+C/C (0) | - | | |
| | T/T+T/C (2) | 0.52 (0.11) | | |
| | T/T+C/C (1) | 0.83 (0) | | |
| **II3)** | | | | |
| **NO/ONOO$^-$** (1 **μM LA-419) Caucasian** | | | | |
| **Variable** | **Level (n)** | **Fold difference (mean ± sd)** | **Chi-sq statistic** | **p value** |
| **G894T + T-786C variant** | | | | |
| | G/G+T/T (7) | 1.63 (0.72) | 4.3243 | 0.50 |
| | G/G+T/C (3) | 1.27 (0.04) | | |
| | G/G/+C/C (0) | - | | |
| | G/T+T/T (3) | 1.21 (0.04) | | |
| | G/T+T/C (10) | 1.75 (0.91) | | |
| | G/T+C/C (0) | - | | |
| | T/T+T/C (2) | 2.25 (1.17) | | |

(continued)

| II3) | | | | |
|---|---|---|---|---|
| NO/ONOO$^-$ (1 μM LA-419) Caucasian | | | | |
| Variable | Level (n) | Fold difference (mean ± sd) | Chi-sq statistic | p value |
| G894T + T-786C variant | | | | |
| | T/T+C/C (1) | 1.23 (0) | | |
| Chi-sq statistic generated from the Non-parametric ANOVA Kruskal-Wallis test | | | | |

**Tables mm1 - mm3)** Caucasian Fold difference LA-419 5μM. Comparison between major allele homozygote and minor allele carriers (reference vs. all carriers combination).

| Reference group = G/G+T/T. | | | | |
|---|---|---|---|---|
| Combination = G/G+T/C, G/G/+C/C, G/T+T/T, G/T+T/C, G/T+C/C, T/T+T/C, T/T+C/C | | | | |
| **Fold difference = (LA-419 5μM)/(control)** | | | | |
| **mm1)** | | | | |
| **NO (5 μM LA-419) Caucasian** | | | | |
| **Variable** | **Level (n)** | **Fold difference (mean ± sd)** | **t statistic** | **p value** |
| **Genotype** | | | | |
| | Reference (7) | 1.19 (0.08) | 1.9076 | 0.068 |
| | Combination (19) | 1.13 (0.06) | | |
| **mm2)** | | | | |
| **ONOO$^-$ (5 μM LA-419) Caucasian** | | | | |
| **Variable** | **Level (n)** | **Fold difference (mean ± sd)** | **t statistic** | **p value** |
| **Genotype** | | | | |
| | Reference (7) | 0.81 (0.04) | -0.9249 | 0.36 |
| | Combination (19) | 0.83 (0.06) | | |
| **mm3)** | | | | |
| **NO/ONOO$^-$ (5 μM LA-419) Caucasian** | | | | |
| **Variable** | **Level (n)** | **Fold difference (mean ± sd)** | **t statistic** | **p value** |
| **Genotype** | | | | |
| | Reference (7) | 1.47 (0.14) | 1.5608 | 0.13 |
| | Combination (19) | 1.37 (0.10) | | |
| t statistic generated from the Non-parametric Mann-Whitney U test | | | | |

Tables **nn1 - nn3)** Caucasian Fold difference LA-419 1μM. Comparison between major allele homozygote and minor allele carriers (reference vs. all carriers combination).

| Reference group = G/G+T/T. | | | | |
|---|---|---|---|---|
| Combination = G/G+T/C, G/G/+C/C, G/T+T/T, G/T+T/C, G/T+C/C, T/T+T/C, T/T+C/C | | | | |
| **Fold difference = (LA-419 1μM)/(control)** | | | | |
| **nn1)** | | | | |
| **NO (1 μM LA-419) Caucasian** | | | | |
| **Variable** | **Level (n)** | **Fold difference (mean ± sd)** | **t statistic** | **p value** |
| **Genotype** | | | | |
| | Reference (7) | 1.25 (0.12) | 1.8498 | 0.076 |
| | Combination (19) | 1.20 (0.29) | | |
| **nn2)** | | | | |
| **$ONOO^-$ (1 μM LA-419) Caucasian** | | | | |
| **Variable** | **Level (n)** | **Fold difference (mean ± sd)** | **t statistic** | **p value** |
| **Genotype** | | | | |
| | Reference (7) | 0.84 (0.22) | 0.9827 | 0.34 |
| | Combination (19) | 0.81 (0.16) | | |
| **nn3)** | | | | |
| **NO/$ONOO^-$ (1 μM LA-419) Caucasian** | | | | |
| **Variable** | **Level (n)** | **Fold difference (mean ± sd)** | **t statistic** | **p value** |
| **Genotype** | | | | |
| | Reference (7) | 1.63 (0.72) | 0.6937 | 0.49 |
| | Combination (19) | 1.61 (0.77) | | |
| t statistic generated from the Non-parametric Mann-Whitney U test | | | | |

***Fold Difference Minor Allele vs. Major Allele and Heterozygotes***

**Tables oo1 - oo3)** Minor Allele vs. Major and Heterozygotes Fold difference LA-419 5μM

| **Fold difference = (LA-419 5μM)/(control)** | | | | |
|---|---|---|---|---|
| **oo1)** | | | | |
| **NO (5μM) Minor Allele vs. Major and Heterozygotes** | | | | |
| **Variable** | **Level (n)** | **Fold difference (mean ± sd)** | **t statistic** | **p value** |
| **G894T variant** | | | | |
| | T/T (5) | 1.12 (0.07) | -1.0734 | 0.288 |
| | G/G & G/T (44) | 1.15 (0.06) | | |
| **T-786C variant** | | | | |
| | C/C (4) | 1.12 (0.06) | -1.0772 | 0.287 |

(continued)

| | | | | |
|---|---|---|---|---|
| **T-786C variant** | | | | |
| | T/T & T/C (45) | 1.15 (0.06) | | |
| **Intron 4 variant** | | | | |
| | a/a (3) | 1.14 (0.04) | -0.2048 | 0.839 |
| | b/b, b/a, c/c (42) | 1.14 (0.07) | | |
| **oo2)** | | | | |
| **$ONOO^-$ (5μM) Minor Allele vs. Major and Heterozygotes** | | | | |
| **Variable** | **Level (n)** | **Fold difference (mean ± sd)** | **t statistic** | **p value** |
| **G894T variant** | | | | |
| | T/T (5) | 0.81 (0.08) | 0.3798 | 0.706 |
| | G/G & G/T (44) | 0.80 (0.07) | | |
| **T-786C variant** | | | | |
| | C/C (4) | 0.79 (0.08) | -0.0913 | 0.928 |
| | T/T & T/C (45) | 0.81 (0.07) | | |
| **Intron 4 variant** | | | | |
| | a/a (3) | 0.83 (0.03) | 0.7508 | 0.457 |
| | b/b, b/a, c/c (42) | 0.81 (0.07) | | |
| **oo3)** | | | | |
| **$NO/ONOO^-$ (5μM) Minor Allele vs. Major and Heterozygotes** | | | | |
| **Variable** | **Level (n)** | **Fold difference (mean ± sd)** | **t statistic** | **p value** |
| **G894T variant** | | | | |
| | T/T (5) | 1.39 (0.13) | -0.8753 | 0.386 |
| | G/G & G/T (44) | 1.44 (0.16) | | |
| **T-786C variant** | | | | |
| | G/G (4) | 1.42 (0.13) | -0.0913 | 0.928 |
| | T/T & T/C (45) | 1.44 (0.16) | | |
| **Intron 4 variant** | | | | |
| | a/a (3) | 1.37 (0.04) | -0.3868 | 0.701 |
| | b/b, b/a, c/c (42) | 1.43 (0.17) | | |
| Fold difference = avg 5μM / avg Cntl. t statistic generated from the Non-parametric Mann-Whitney U test | | | | |

**Tables pp1 - pp3)** Minor Allele vs. Major and Heterozygotes Fold difference LA-419 1μM

| | | | | |
|---|---|---|---|---|
| **Fold difference = (LA-419 1μM)/(control)** | | | | |
| **pp1)** | | | | |
| **NO (1μM) Minor Allele vs. Major and Heterozygotes** | | | | |
| **Variable** | **Level (n)** | **Fold difference (mean ± sd)** | **t statistic** | **p value** |
| **G894T variant** | | | | |
| | T/T (5) | 1.07 (0.19) | -0.6771 | 0.502 |

(continued)

| Fold difference = (LA-419 1μM)/(control) | | | | |
|---|---|---|---|---|
| **pp1)** | | | | |
| **NO (1μM) Minor Allele vs. Major and Heterozygotes** | | | | |
| **Variable** | **Level (n)** | **Fold difference (mean ± sd)** | **t statistic** | **p value** |
| **G894T variant** | | | | |
| | G/G & G/T (44) | 1.13 (0.25) | | |
| **T-786C variant** | | | | |
| | C/C (4) | 1.02 (0.12) | -1.4058 | 0.166 |
| | T/T & T/C (45) | 1.14 (0.25) | | |
| **Intron 4 variant** | | | | |
| | a/a (3) | 0.95 (0.25) | -0.9783 | 0.333 |
| | b/b, b/a, c/c (42) | 0.95 (0.25) | | |
| **pp2)** | | | | |
| **$ONOO^-$ 1μM) Minor Allele vs. and Heterozygotes** | | | | |
| **Variable** | **Level (n)** | **Fold difference (mean ± sd)** | **t statistic** | **p value** |
| **G894T variant** | | | | |
| | T/T (5) | 0.75 (0.21) | -1.4368 | 0.157 |
| | G/G & G/T (44) | 0.95 (0.27) | | |
| **T-786C variant** | | | | |
| | C/C (4) | 1.11 (0.44) | 0.8581 | 0.395 |
| | T/T & T/C (45) | 0.91 (0.25) | | |
| **Intron 4 variant** | | | | |
| | a/a (3) | 1.10 (0.34) | 1.0238 | 0.312 |
| | b/b, b/a, c/c (42) | 0.91 (0.27) | | |
| **pp3)** | | | | |
| **NO/$ONOO^-$ (1μM) Minor Allele vs. Major and Heterozygotes** | | | | |
| **Variable** | **Level (n)** | **Fold difference (mean ± sd)** | **t statistic** | **p value** |
| **G894T variant** | | | | |
| | T/T (5) | 1.62 (0.82) | 0.8092 | 0.422 |
| | G/G & G/T (44) | 1.34 (0.64) | | |
| **T-786C variant** | | | | |
| | C/C (4) | 1.03 (0.36) | -1.2598 | 0.214 |
| | T/T & T/C (45) | 1.39 (0.67) | | |
| **Intron 4 variant** | | | | |
| | a/a (3) | 0.96 (0.45) | -1.1148 | 0.271 |
| | b/b, b/a, c/c (42) | 1.42 (0.69) | | |
| Fold difference = avg 1μM / avg Cntl. t statistic generated from the Non-parametric Mann-Whitney U test | | | | |

**Tables qq1 - qq3)** Fold difference LA-419 5μM. Comparison between minor allele homozygote and major allele and heterozygotes (reference vs. combination).

| Reference group = T/T+C/C | | | | |
|---|---|---|---|---|
| Combination = G/G+T/T, G/G+T/C, G/G/+C/C, G/T+T/T, G/T+T/C, G/T+C/C, T/T+T/C | | | | |
| **Fold difference = (LA-419 5μM)/(control)** | | | | |
| **qq1)** | | | | |
| **NO (5 μM LA-419) Minor Allele vs. Major and Heterozygotes** | | | | |
| **Variable** | **Level (n)** | **Fold difference (mean ± sd)** | **t statistic** | **p value** |
| **Genotype** | | | | |
| | Reference (3) | 1.12 (0.07) | -0.8549 | 0.397 |
| | Combination (46) | 1.14 (0.06) | | |
| **qq2)** | | | | |
| **$ONOO^-$ (5 μM LA-419) Minor Allele vs. Major and Heterozygotes** | | | | |
| **Variable** | **Level (n)** | **Fold difference (mean ± sd)** | **t statistic** | **p value** |
| **Genotype** | | | | |
| | Reference (3) | 0.77 (0.08) | -0.5630 | 0.576 |
| | Combination (46) | 0.81 (0.07) | | |
| **qq3)** | | | | |
| **NO/$ONOO^-$ (5 μM LA-419) Minor Allele vs. Major and Heterozygotes)** | | | | |
| **Variable** | **Level (n)** | **Fold difference (mean ± sd)** | **t statistic** | **p value** |
| **Genotype** | | | | |
| | Reference (3) | 1.46 (0.14) | 0.3545 | 0.725 |
| | Combination (46) | 1.44 (0.16) | | |
| t statistic generated from the Non-parametric Mann-Whitney U test | | | | |

**Tables rr1 - rr3**) Fold difference LA-419 1μM. Comparison between minor allele homozygote and major allele and heterozygotes (reference vs. combination).

| Reference group = T/T+C/C | | | | |
|---|---|---|---|---|
| Combination = G/G+T/T, G/G+T/C, G/G/+C/C, G/T+T/T, G/T+T/C, G/T+C/C, T/T+T/C | | | | |
| **Fold difference = (LA-419 1μM)/(control)** | | | | |
| **rr1)** | | | | |
| **NO (1 μM LA-419) Minor Allele vs. Major and Heterozygotes** | | | | |
| **Variable** | **Level (n)** | **Fold difference (mean ± sd)** | **t statistic** | **p value** |
| **Genotype** | | | | |
| | Reference (3) - | 1.07 (0.06) | -0.7715 | 0.444 |
| | Combination (46) | 1.13 (0.25) | | |

(continued)

| rr2) | | | | |
|---|---|---|---|---|
| **$ONOO^-$ (1 μM LA-419) Minor Allele vs. Major and Heterozygotes** | | | | |
| **Variable** | **Level (n)** | **Fold difference (mean±sd)** | **t statistic** | **p value** |
| **Genotype** | | | | |
| | Reference (3) | 0.89 (0.06) | 0.0209 | 0.984 . |
| | Combination (46) | 0.93 (0.28) | | |
| | | | | |
| **$NO/ONOO^-$ (1 μM LA-419) Minor Allele vs. Major and Heterozygotes** | | | | |
| **Variable** | **Level (n)** | **Fold difference (mean ± sd)** | **t statistic** | **p value** |
| **Genotype** | | | | |
| | Reference (3) | 1.20 (0.04) | -0.5213 | 0.605 |
| | Combination (46) | 1.37 (0.68) | | |
| t statistic generated from the Non-parametric Mann-Whitney U test | | | | |

**eNOS Protein Abundance**

**Table ss)** eNOS % change, mean response for all genotypes and races (5μM LA-419)

| **% Change = (avg. 5μM - avg. control)/(avg. control) *100** | | | | |
|---|---|---|---|---|
| **ss)** | | | | |
| **eNOS protein abundance (5 μM LA-419)** | | | | |
| **Variable** | **Level (n)** | **% Change (mean ± sd)** | **Chi-sq statistic** | **p value** |
| **G894T variant** | | | | |
| | G/G (26) | -1.07 (8.07) | 0.0050 | 0.998 |
| | G/T (18) | -0.79 (7.54) | | |
| | T/T (5) | -1.54 (6.56) | | |
| **T-786C variant** | | | | |
| | T/T (22) | -0.64 (5.88) | 3.0416 | 0.219 |
| | T/C (23) | -0.37 (9.0) | | |
| | C/C (4) | -6.82 (6.09) | | |
| **Intron 4 variant** | | | | |
| | b/b (31) | -1.78 (6.88) | 1.5653 | 0.667 |
| | b/a (10) | 0.68 (9.65) | | |
| | a/a (3) | 1.11 (10.89) | | |
| | c/c (1) | -5.66 (0) | | |
| **Race** | | | | |
| | Asian (1) | -17.56 (0) | 15.1070 | 0.01 † |
| | Black (12) | -5.95 (6.82) | | |

(continued)

| Race | | | | |
|---|---|---|---|---|
| | Caucasian (26) | 2.50 (6.89) | | |
| | Caucasian/Black (2) | -4.89 (0.49) | | |
| | Hispanic (6) | -1.16 (4.99) | | |
| | Not Reported (2) | -4.62 (0.81) | | |
| Chi-sq statistic generated from the Non-parametric ANOVA Kruskal-Wallis test | | | | |

**Tables tt1 - tt2**) Effect of genotype on eNOS within group control and 5μM LA-419; protein abundance, pg/ug total protein

| tt1) | | | | |
|---|---|---|---|---|
| **eNOS protein abundance (control)** | | | | |
| **Variable** | **Level (n)** | **pg/ug (mean ± sd)** | **Chi-sq statistic** | **p value** |
| **G894T variant** | | | | |
| | G/G (26) | 6.32 (1.45) | 1.4382 | 0.487 |
| | G/T (18) | 5.82 (1.33) | | |
| | T/T (5) | 5.87 (1.83) | | |
| **T-786C variant** | | | | |
| | T/T (22) | 6.19 (1.43) | 2.9222 | 0.232 |
| | T/C (23) | 5.79 (1.38) | | |
| | C/C (4) | 7.22 (1.54) | | |
| **Intron 4 variant** | | | | |
| | b/b (31) | 5.89 (1.29) | 3.2139 | 0.360 |
| | b/a (10) | 5.97 (1.67) | | |
| | a/a (3) | 7.22 (1.85) | | |
| | c/c (1) | 7.83 (0) | | |
| **tt2)** | | | | |
| **eNOS protein abundance (5 μMLA-419)** | | | | |
| **Variable** | **Level (n)** | **pg/ug (mean ± sd)** | **Chi-sq statistic** | **p value** |
| **G894T variant** | | | | |
| | G/G (26) | 6.20 (1.31) | 1.7547 | 0.416 |
| | G/T (18) | 5.73 (1.17) | | |
| | T/T (5) | 5.69 (1.44) | | |
| **T-786C variant** | | | | |
| | T/T (22) | 6.11 (1.26) | 2.7507 | 0.253 |
| | T/C (23) | 5.73 (1.29) | | |
| | C/C (4) | 6.67 (1.16) | | |
| **Intron 4 variant** | | | | |
| | b/b (31) | 5.75 (1.13) | 4.6691 | 0.198 |
| | b/a (10) | 5.95 (1.56) | | |

(continued)

| | | | | |
|---|---|---|---|---|
| **Intron 4 variant** | | | | |
| | a/a (3) | 7.17 (1.19) | | |
| | c/c (1) | 7.39 (0) | | |
| Chi-sq statistic generated from the Non-parametric ANOVA Kruskal-Wallis test | | | | |

**Tables uu1 - uu2**) Effect of genotype on eNOS protein abundance (pg/ug total protein) within group control and 5μM LA-419 - comparison between major allele homozygotes and minor allele carriers

| | | | | |
|---|---|---|---|---|
| **uu1)** | | | | |
| **eNOS protein abundance (control) Major Allele vs. Minor Allele Carrier** | | | | |
| **Variable** | **Level (n)** | **pg/ug (mean ± sd)** | **t statistic** | **p value** |
| **G894T variant** | | | | |
| | G/G (26) | 6.32 (1.45) | -1.1519 | 0.255 |
| | G/T & T/T (23) | 5.83 (1.41) | | |
| **T-786C variant** | | | | |
| | T/T (22) | 6.19 (1.43) | 0.4523 | 0.653 |
| | T/C & C/C (27) | 6.01 (1.47) | | |
| **Intron 4 variant** | | | | |
| | b/b (31) | 5.89 (1.29) | 0.8949 | 0.376 |
| | b/a, a/a, c/c (14) | 6.37 (1.71) | | |
| **uu2)** | | | | |
| **eNOS protein abundance (5μM) Major Allele vs. Minor Allele Carrier** | | | | |
| **Variable** | **Level (n)** | **pg/ug (mean ± sd)** | **t statistic** | **p value** |
| **G894T variant** | | | | |
| | G/G (26) | 6.20 (1.31) | -1.2922 | 0.203 |
| | G/T & T/T (23) | 5.72 (1.20) | | |
| **T-786C variant** | | | | |
| | T/T (22) | 6.11 (1.26) | 0.8945 | 0.376 |
| | T/C & C/C (27) | 5.87 (1.30) | | |
| **Intron 4 variant** | | | | |
| | b/b (31) | 5.75 (1.13) | 1.0910 | 0.281 |
| | b/a, a/a, c/c (14) | 6.31 (1.50) | | |
| t statistic generated from the Non-parametric Mann-Whitney U test | | | | |

**Tables vv1 - vv2**) Effect of genotype on eNOS protein abundance (pg/ug total protein) within group control and 5μM LA-419 (Individual allelic combination).

| Reference group = G/G+T/T | | | | |
|---|---|---|---|---|
| **vv1)** | | | | |
| **eNOS protein abundance (control)** | | | | |
| **Variable** | **Level (n)** | **pg/ug (mean ± sd)** | **Chi-sq statistic** | **p value** |
| **G894T + T786C variant** | | | | |
| | G/G+T/T (18) | 6.26 (1.42) | 8.4968 | 0.204 |
| | G/G+T/C (8) | 6.44 (1.61) | | |
| | G/G/+C/C (0) | - | | |
| | G/T+T/T (4) | 5.85 (1.64) | | |
| | G/T+T/C (13) | 5.62 (1.15) | | |
| | G/T+C/C (1) | 8.24 (0) | | |
| | T/T+T/C (2) | 4.36 (0.32) | | |
| | T/T+C/C (3) | 6.88 (1.69) | | |
| **vv2)** | | | | |
| **eNOS protein abundance (5 μM LA-419)** | | | | |
| **Variable** | **Level (n)** | **pg/ug (mean ± sd)** | **Chi-sq statistic** | **p value** |
| **G894T + T786C variant** | | | | |
| | G/G+T/T (18) | 6.17 (1.24) | 6.7528 | 0.344 |
| | G/G+T/C (8) | 6.27 (1.56) | | |
| | G/G/+C/C (0) | - | | |
| | G/T+T/T (4) | 5.82 (1.49) | | |
| | G/T+T/C (13) | 5.59 (1.08) | | |
| | G/T+C/C (1) | 7.25 (0) | | |
| | T/T+T/C (2) | 4.52 (0.30) | | |
| | T/T+C/C (3) | 6.47 (1.34) | | |
| Chi-sq statistic generated from the Non-parametric ANOVA Kruskal-Wallis test | | | | |

**Tables xx1 - xx2**) Effect of genotype on eNOS protein abundance (pg/ug total protein) within group control and 5μM LA-419. Comparison between minor allele homozygote and major allele and heterozygotes (reference vs. combination).

| Reference group = T/T+C/C | | | | |
|---|---|---|---|---|
| Combination = G/G+T/T, G/G+T/C, G/G/+C/C, G/T+T/T, G/T+T/C, G/T+C/C, T/T+T/C | | | | |
| **xx1)** | | | | |
| **eNOS protein abundance (control)** | | | | |
| **Variable** | **Level (n)** | **pg/ug (mean ± sd)** | **t statistic** | **p value** |
| **Genotype** | | | | |
| | Reference (18) | 6.26 (1.42) | 0.5911 | 0.557 |

(continued)

| | | | | |
|---|---|---|---|---|
| Reference group = T/T+C/C | | | | |
| Combination = G/G+T/T, G/G+T/C, G/G/+C/C, G/T+T/T, G/T+T/C, G/T+C/C, T/T+T/C | | | | |
| **xx1)** | | | | |
| **eNOS protein abundance (control)** | | | | |
| **Variable** | **Level (n)** | **pg/ug (mean ± sd)** | **t statistic** | **p value** |
| **Genotype** | | | | |
| | Combination (31) | 5.99 (1.46) | | |
| **xx2)** | | | | |
| **eNOS protein abundance (5 μM LA-419)** | | | | |
| **Variable** | **Level (n)** | **pg/ug (mean ± sd)** | **t statistic** | **p value** |
| **Genotype** | | | | |
| | Reference (18) | 6.17 (1.24) | 0.9436 | 0.350 |
| | Combination (31) | 5.86 (1.30) | | |
| t statistic generated from the Non-parametric Mann-Whitney U test | | | | |

**Table yy)** eNOS protein abundance (pg/ug total protein), net increase in 5μM LA-419 over control - comparison between genotypes

| | | | | |
|---|---|---|---|---|
| **yy)** | | | | |
| **eNOS protein abundance (5μM LA-419)** | | | | |
| **Variable** | **Level (n)** | **Net increase pg/ug (mean ± sd)** | **Chi-sq statistic** | **p value** |
| **G894T variant** | | | | |
| | G/G (26) | -0.12 (0.50) | 0.1444 | 0.930 |
| | G/T (18) | -0.08 (0.46) | | |
| | T/T (5) | -0.18 (0.45) | | |
| **T786C variant** | | | | |
| | T/T (22) | -0.08 (0.36) | 3.7371 | 0.154 |
| | T/C (23) | -0.06 (0.54) | | |
| | C/C (4) | -0.55 (0.47) | | |
| **Intron 4 variant** | | | | |
| | b/b (31) | -0.14 (0.45) | 1.8758 | 0.599 |
| | b/a (10) | -0.02 (0.56) | | |
| | a/a (3) | -0.05 (0.69) | | |
| | c/c (1) | -0.44 (0) | | |
| Net increase = avg 5 μM - avg Cntl. Chi-sq statistic generated from the Non-parametric ANOVA Kruskal-Wallis test | | | | |

**Table zz)** eNOS protein abundance (pg/ug total protein), net increase in 5μM LA-419 over control - comparison between major allele homozygotes and minor allele carriers

| zz) | | | | |
|---|---|---|---|---|
| **eNOS protein abundance 5μM LA-419)** | | | | |
| **Variable** | **Level (n)** | **Net increase pg/ug (mean ± sd)** | **t statistic** | **p value** |
| **G894T variant** | | | | |
| | G/G (26) | -0.12 (0.50) | 0.1503 | 0.881 |
| | G/T & T/T (23) | -0.10 (0.45) | | |
| **T786C variant** | | | | |
| | T/T (22) | -0.08 (0.36) | -0.1106 | 0.912 |
| | T/C & C/C (27) | -0.13 (0.55) | | |
| **Intron 4 variant** | | | | |
| | b/b (31) | -0.14 (0.45) | 0.4536 | 0.652 |
| | b/a, a/a, c/c (14) | -0.05 (0.55) | | |
| Net increase = avg 5μM - avg Cntl. t statistic generated from the Non-parametric Mann-Whitney U test | | | | |

**Table A)** eNOS protein abundance (pg/ug total protein), net increase in 5μM LA-419 over control (Individual allelic combination). Reference group = G/G+T/T

| A) | | | | |
|---|---|---|---|---|
| **eNOS protein abundance (5 μM LA-419 increase over Control)** | | | | |
| **Variable** | **Level (n)** | **Net increase pg/ug (mean ± sd)** | **Chi-sq statistic** | **p value** |
| **G894T + T786C variant** | | | | |
| | G/G+T/T (18) | -0.09 (0.35) | 5.1150 | 0.5292 |
| | G/G+T/C (8) | -0.17 (0.77) | | |
| | G/G/+C/C (0) | - | | |
| | G/T+T/T (4) | -0.03 (0.46) | | |
| | G/T+T/C (13) | -0.03 (0.42) | | |
| G/T+C/C | G/T+C/C (1) | -0.99 (0) | | |
| | T/T+T/C (2) | 0.17 (0.02) | | |
| | T/T+C/C (3) | -0.41 (0.46) | | |
| Chi-sq statistic generated from the Non-parametric ANOVA Kruskal-Wallis test | | | | |

**Table B)** eNOS protein abundance (pg/ug total protein), net increase in 5μM LA-419 over control. Comparison between major allele homozygote and minor allele carriers (reference vs. all carriers combination).

| Reference group = G/G+T/T. | | | | |
|---|---|---|---|---|
| Combination = G/G+T/C, G/G/+C/C, G/T+T/T, G/T+T/C, G/T+C/C, T/T+T/C, T/T+C/C | | | | |
| **B)** | | | | |
| **eNOS protein abundance (5 μM LA-419 increase over Control)** | | | | |
| **Variable** | **Level (n)** | **Net increase pg/ug (mean ± sd)** | **t statistic** | **p value** |
| **Genotype** | | | | |
| | Reference (18) | -0.09 (0.35) | -0.1970 | 0.845 |
| | Combination (31) | -0.12 (0.53) | | |
| t statistic generated from the Non-parametric Mann-Whitney U test | | | | |

**Table C)** Fold difference eNOS protein abundance, LA-419 5μM - comparison between genotypes

| **C)** | | | | |
|---|---|---|---|---|
| **Fold difference = (LA-419 5μM)/(control)** | | | | |
| **eNOS protein abundance (LA-419 5μM)** | | | | |
| **Variable** | **Level (n)** | **Fold difference (mean ± sd)** | **Chi-sq statistic** | **p value** |
| **G894T variant** | | | | |
| | G/G (26) | 1.00 (0.08) | 0.0050 | 0.998 |
| | G/T (18) | 0.99 (0.08) | | |
| | T/T (5) | 0.98 (0.07) | | |
| **T-786C variant** | | | | |
| | T/T (22) | 0.99 (0.06) | 3.0416 | 0.218 |
| | T/C (23) | 1.00 (0.09) | | |
| | C/C (4) | 0.93 (0.06) | | |
| **Intron 4 variant** | | | | |
| | b/b (31) | 0.98 (0.07) | 1.5653 | 0.667 |
| | b/a (10) | 1.01 (0.10) | | |
| | a/a (3) | 1.01 (0.11) | | |
| | c/c (1) | 0.94 (0) | | |
| Fold difference = avg 5μM / avg Cntl. Chi-sq statistic generated from the Non-parametric ANOVA Kruskal-Wallis test | | | | |

**Table D)** Fold difference eNOS protein abundance, LA-419 5μM - comparison between major allele homozygotes and minor allele carriers

| Fold difference = (LA-419 5μM)/(control) | | | | |
|---|---|---|---|---|
| **D)** | | | | |
| **eNOS protein abundance (5μM)** | | | | |
| **Variable** | **Level (n)** | **Fold difference (mean ± sd)** | **t statistic** | **p value** |
| **G894T variant** | | | | |
| | G/G (26) | 0.99 (0.08) | 0.0000 | 1.000 |
| | G/T & T/T (23) | 0.99 (0.07) | | |
| **T-786C variant** | | | | |
| | T/T (22) | 0.99 (0.06) | 0.0000 | 1.000 |
| | T/C & C/C (27) | 0.99 (0.09) | | |
| **Intron 4 variant** | | | | |
| | b/b (31) | 0.98 (0.07) | 0.6497 | 0.519 |
| | b/a, a/a, c/c (14) | 1.00 (0.09) | | |
| Fold difference = avg 5μM / avg Cntl. t statistic generated from the Non-parametric Mann-Whitney U test | | | | |

**Table E)** Fold difference eNOS protein abundance, LA-419 5μM (Individual allelic combination).

| E) | | | | |
|---|---|---|---|---|
| Reference group = G/G+T/T | | | | |
| **Fold difference = (LA-419 5μM/(control)** | | | | |
| **eNOS protein abundance (5μM LA-419)** | | | | |
| **Variable** | **Level (n)** | **Fold difference (mean ± sd)** | **Chi-sq statistic** | **p value** |
| **G894T + T-786C variant** | | | | |
| | G/G+T/T (18) | 0.99 (0.06) | 4.6815 | 0.585 |
| | G/G+T/C (8) | 0.98 (0.12) | | |
| | G/G/+C/C (0) | - | | |
| | G/T+T/T (4) | 1.00 (0.08) | | |
| | G/T+T/C (13) | 1.00 (0.07) | | |
| | G/T+C/C (1) | 0.88 (0) | | |
| | T/T+T/C (2) | 1.04 (0.01) | | |
| | T/T+C/C (3) | 0.95 (0.06) | | |
| Chi-sq statistic generated from the Non-parametric ANOVA Kruskal-Wallis test | | | | |

**Table F)** Fold difference eNOS protein abundance, LA-419 5μM. Comparison between major allele homozygote and minor allele carriers (reference vs. all carriers combination).

| Reference group = G/G+T/T. | | | | |
|---|---|---|---|---|
| Combination = G/G+T/C, G/G/+C/C, G/T+T/T, G/T+T/C, G/T+C/C, T/T+T/C, T/T+C/C | | | | |
| **Fold difference = (LA-419 5μM)/(control)** | | | | |
| **F)** | | | | |
| **eNOS protein abundance (5μM LA-419)** | | | | |
| **Variable** | **Level (n)** | **Fold difference (mean ± sd)** | **t statistic** | **p value** |
| **Genotype** | | | | |
| | Reference (18) | 0.99 (0.06) | -0.0518 | 0.959 |
| | Combination (31) | 0.99 (0.09) | | |
| t statistic generated from the Non-parametric Mann-Whitney U test | | | | |

**Table G)** Fold difference eNOS protein abundance, LA-419 5μM. Minor Allele vs. Major Allele and Heterozygotes

| G) | | | | |
|---|---|---|---|---|
| **Fold difference = (LA-419 5μM)/(control)** | | | | |
| **eNOS protein abundance (5μM LA-419) Minor Allele Homozygotes vs. Major Allele Homozygotes plus Heterozygotes** | | | | |
| **Variable** | **Level (n)** | **Fold difference (mean ± sd)** | **t statistic** | **p value** |
| **G894T variant** | | | | |
| | T/T (5) | 0.98 (0.07) | -0.0495 | 0.961 |
| | G/G & G/T (44) | 0.99 (0.08) | | |
| **T786C variant** | | | | |
| | C/C (4) | 0.93 (0.06) | -1.6614 | 0.103 |
| | T/T & T/C (45) | 0.99 (0.08) | | |
| **Intron 4 variant** | | | | |
| | a/a (3) | 1.01 (0.11) | 0.1138 | 0.910 |
| | b/b, b/a, c/c (42) | 0.99 (0.08) | | |
| Fold difference = avg 5μM / avg Cntl. t statistic generated from the Non-parametric Mann-Whitney U test | | | | |

**Table H)** Fold difference in eNOS protein abundance, LA-419 5μM. Comparison between minor allele homozygotes and major allele homozygotes plus heterozygotes (reference vs. combination).

| **H** | | | | |
|---|---|---|---|---|
| Reference group = T/T+C/C | | | | |
| Combination = G/G+T/T, G/G+T/C, G/G/+C/C, G/T+T/T, G/T+T/C, G/T+C/C, T/T+T/C | | | | |
| **Fold difference = (LA-419 5μM)/(control)** | | | | |
| **eNOS protein abundance (5μM LA-419) Minor Allele Homozygotes vs. Major Allele Homozygotes plus Heterozygotes** | | | | |
| **Variable** | **Level (n)** | **Fold difference (mean ± sd)** | **t statistics** | **p value** |
| **Genotype** | | | | |
| | Reference (3) | 0.95 (0.06) | -0.9800 | 0.332 |
| | Combination (46) | 0.99 (0.08) | | |
| t statistic generated from the Non-parametric Mann-Whitney U test | | | | |

**Table I)** Caucasian fold difference for eNOS protein abundance, LA-419 5μM. Comparison between genotypes:

| **I)** | | | | |
|---|---|---|---|---|
| **Fold difference = (LA-419 5μM/(control)** | | | | |
| **eNOS protein abundance (LA-419 5μM) Caucasian** | | | | |
| **Variable** | **Level (n)** | **Fold difference (mean ± sd)** | **Chi-sq statistic** | **p value** |
| **G894T variant** | | | | |
| | G/G (10) | 1.06 (0.05) | 3.8950 | 0.14 |
| | G/T (13) | 1.00 (0.08) | | |
| | T/T (3) | 1.03 (0.02) | | |
| **T-786C variant** | | | | |
| | T/T (10) | 1.03 (0.06) | 0.0564 | 0.97 |
| | T/C (15) | 1.02 (0.08) | | |
| | C/C (1) | 1.01 (0) | | |
| **Intron 4 variant** | | | | |
| | b/b (18) | 1.00 (0.06) | 6.5351 | 0.038 † |
| | b/a (5) | 1.07 (0.06) | | |
| | a/a (1) | 1.14 (0) | | |
| Fold difference = avg 5μM / avg Cntl. Chi-sq statistic generated from the Non-parametric ANOVA Kruskal-Wallis test | | | | |

**Table J)** Caucasian Fold difference in eNOS protein abundance, LA-419 5μM. Comparison between major allele homozygotes and minor allele carriers

| J) | | | | |
|---|---|---|---|---|
| **Fold difference = (LA-419 5μM)/(control)** | | | | |
| **eNOS protein abundance (5μM) Caucasian** | | | | |
| **Variable** | **Level (n)** | **Fold difference (mean ± sd)** | **t statistic** | **p value** |
| **G894T variant** | | | | |
| | G/G (10) | 1.06 (0.05) | 1.7656 | 0.09 |
| | G/T & T/T (16) | 1.01 (0.07) | | |
| **T-786C variant** | | | | |
| | T/T (10) | 1.03 (0.06) | 0.1318 | 0.90 |
| | T/C & C/C (16) | 1.02 (0.08) | | |
| **Intron 4 variant** | | | | |
| | b/b (18) | 1.00 (0.06) | 2.4333 | 0.02 † |
| | b/a & a/a (6) | 1.08 (0.06) | | |
| Fold change = avg 5μM / avg Cntl. t statistic generated from the Non-parametric Mann-Whitney U test | | | | |

**Table K)** Caucasian Fold difference in eNOS protein abundance LA-419 5μM (Individual allelic combination). Reference group = G/G+T/T

| K) | | | | |
|---|---|---|---|---|
| **Fold difference = (LA-419 5μM)/(control)** | | | | |
| **eNOS protein abundance (5 μM LA-419) Caucasian** | | | | |
| **Variable** | **Level (n)** | **Fold difference (mean ± sd)** | **Chi-sq statistic** | **p value** |
| **G894T + T-786C variant** | | | | |
| | G/G+T/T (7) | 1.04 (0.04) | 6.0565 | 0.30 |
| | G/G+T/C (3) | 1.10 (0.05) | | |
| | G/G/+C/C (0) | - | | |
| | G/T+T/T (3) | 1.02 (0.09) | | |
| | G/T+T/C (10) | 1.00 (0.08) | | |
| | G/T+C/C (0) | - | | |
| | T/T+T/C (2) | 1.04 (0.01) | | |
| | T/T+C/C (1) | 1.01 (0) | | |
| Chi-sq statistic generated from the Non-parametric ANOVA Kruskal-Wallis test | | | | |

**Table L)** Caucasian Fold difference in eNOS protein abundance, LA-419 5μM. Comparison between major allele homozygote and minor allele carriers (reference vs. all carriers combination).

| L) | | | | |
|---|---|---|---|---|
| Reference group = G/G+T/T. | | | | |
| Combination = G/G+T/C, G/G/+C/C, G/T+T/T, G/T+T/C, G/T+C/C, T/T+T/C, T/T+C/C | | | | |
| **Fold difference = (LA-419 5μM)/(control)** | | | | |
| **eNOS protein abundance (5 μM LA-419) Caucasian** | | | | |
| **Variable** | **Level (n)** | **Fold difference (mean ± sd)** | **t statistic** | **p value** |
| **Genotype** | | | | |
| | Reference (7) | 1.04 (0.04) | 0.4625 | 0.65 |
| | Combination (19) | 1.02 (0.08) | | |
| t statistic generated from the Non-parametric Mann-Whitney U test | | | | |

**INTRON 4 ANALYSIS: Extra Tables**

**Table M1- M4) %** Change in Intron 4, 5 μM LA-419. Comparison between major allele homozygotes and minor allele carriers

| M1) | | | | |
|---|---|---|---|---|
| **NO(5μM LA-419)** | | | | |
| **Variable** | **Level (n)** | **% Change (mean ± sd)** | **t statistic** | **p value** |
| **Intron 4 variant** | | | | |
| | b/b (31) | 13.52 (6.80) | 1.2136 | 0.231 |
| | b/a, a/a, c/c (14) | 16.16 (6.09) | | |
| **M2)** | | | | |
| **ONOO (5 μM LA-419)** | | | | |
| **Variable** | **Level (n)** | **% Change (mean ± sd)** | **t statistic** | **p value** |
| **Intron 4 variant** | | | | |
| | b/b (31) | -19.19 (7.76) | -0,3555 | 0.724 |
| | b/a, a/a, c/c (14) | -19.27 (5.27) | | |
| **M3)** | | | | |
| **$NO/ONOO^-$ (5μM LA-419)** | | | | |
| **Variable** | **Level (n)** | **% Change (mean ± sd)** | **t statistic** | **p value** |
| **Intron 4 variant** | | | | |
| | b/b(31) | 42.24 (17.73) | 0.5516 | 0.584 |
| | b/a, a/a, c/c (14) | 44.37 (12.85) | | |

(continued)

| M4) | | | | |
|---|---|---|---|---|
| **eNOS protein abundance (5μM LA-419)** | | | | |
| **Variable** | **Level (n)** | **% Change (meat ± sd)** | **t statistic** | **p value** |
| **Intron 4 variant** | | | | |
| | b/b (31) | -1.78 (6.88) | 0.6497 | 0.519 |
| | b/a, a/a, c/c (14) | 0.32 (9.26) | | |
| t statistic generated from the Non-parametric Mann-Whitney U test | | | | |

**Tables N1 - N3)** % Change Intron 4, 1 μM LA-419. Comparison between major allele homozygotes and minor allele carriers

| N1) | | | | |
|---|---|---|---|---|
| **NO (1 μM LA-419)** | | | | |
| **Variable** | **Level (n)** | **% Change (mean ± sd)** | **t statistic** | **p value** |
| **Intron 4 variant** | | | | |
| | b/b (31) | 13.46 (23.13) | 0.0000 | 1.00 |
| | b/a, a/a, c/c (14) | 11.76 (29.57) | | |
| **N2)** | | | | |
| **ONOO (1 μM LA-419)** | | | | |
| **Variable** | **Level (n)** | **% Change (mean ± sd)** | **t statistic** | **p value** |
| **Intron 4 variant** | | | | |
| | b/b (31) | -10.24 (27.17) | 0.7233 | 0.473 |
| | b/a, a/a, c/c (14) | -2.68 (29.78) | | |
| **N3)** | | | | |
| **NO/ONOO$^-$ (1 μM LA-419)** | | | | |
| **Variable** | **Level (n)** | **% Change (mean ± sd)** | **t statistic** | **p value** |
| **Intron 4 variant** | | | | |
| | b/b (31) | 43.27 (71.09) | -0.4781 | 0.635 |
| | b/a, a/a, c/c (14) | 28.01 (62.22) | | |
| t statistic generated from the Non-parametric Mann-Whitney U test | | | | |

**References**

U.S. Patent 4,582,788
U.S. Patent 4,627,429
U.S. Patent 4,659,774
U.S. Patent 4,683,194
U.S. Patent 4,683,195
U.S. Patent 4,683,202
U.S. Patent 4,683,202
U.S. Patent 4,683,202
U.S. Patent 4,784,857

U.S. Patent 4,800,159
U.S. Patent 4,816,571
U.S. Patent 4,883,750
U.S. Patent 4,946,773
U.S. Patent 4,959,463
U.S. Patent 4,965,188
U.S. Patent 5,126,145
U.S. Patent 5,130,238
U.S. Patent 5,141,813
U.S. Patent 5,169,766
U.S. Patent 5,264,566
U.S. Patent 5,279,721
U.S. Patent 5,428,148
U.S. Patent 5,554,744
U.S. Patent 5,574,146
U.S. Patent 5,602,244
U.S. Patent 5,605,798
U.S. Patent 5,645,897
U.S. Patent 5,662,925
U.S. Patent 5,705,629
U.S. Patent 5,788,983
U.S. Patent 5,840,873
U.S. Patent 5,843,640
U.S. Patent 5,843,650
U.S. Patent 5,843,651
U.S. Patent 5,843,663
U.S. Patent 5,846,708
U.S. Patent 5,846,709
U.S. Patent 5,846,717
U.S. Patent 5,846,726
U.S. Patent 5,846,729
U.S. Patent 5,846,783
U.S. Patent 5,849,481
U.S. Patent 5,849,483
U.S. Patent 5,849;486
U.S. Patent 5,849,487
U.S. Patent 5,849,497
U.S. Patent 5,849,546
U.S. Patent 5,849,547
U.S. Patent 5,851,770
U.S. Patent 5,851,772
U.S. Patent 5,853,990
U.S. Patent 5,853,992
U.S. Patent 5,853,993
U.S. Patent 5,856,092
U.S. Patent 5,858,652
U.S. Patent 5,861,244
U.S. Patent 5,863,732
U.S. Patent 5,863,753
U.S. Patent 5,866,331
U.S. Patent 5,866,337
U.S. Patent 5,866,366
U.S. Patent 5,900,481
U.S. Patent 5,905,024
U.S. Patent 5,910,407
U.S. Patent 5,912,124
U.S. Patent 5,912,145
U.S. Patent 5,912,148

U.S. Patent 5,916,776
U.S. Patent 5,916,779
U.S. Patent 5,919,626
U.S. Patent 5,919,630
U.S. Patent 5,922,574
U.S. Patent 5,925,517
U.S. Patent 5,925,525
U.S. Patent 5,928,862
U.S. Patent 5,928,869
U.S. Patent 5,928,870
U.S. Patent 5,928,905
U.S. Patent 5,928,906
U.S. Patent 5,929,227
U.S. Patent 5,932,413
U.S. Patent 5,932,451
U.S. Patent 5,935,791
U.S. Patent 5,935,825
U.S. Patent 5,939,291
U.S. Patent 5,942,391
U.S. Patent 5,952,174
U.S. Patent 6,113,940
U.S. Patent 4,656,127
U.S. Patent 4,682,195

Ausubel et al., Current Protocols in Molecular Biology, John Wiley & Sons, NY, 1989.
Barany et al., Proc. Natl. Acad. Sci. USA, 88:189-193,1991.
Bellus,J. Macromol. Sci. Pure Appl. Chem., A31(1): 1355-1376, 1994.
Burt et al., Hypertension, 25:305-313, 1995.
Campia et al., J. Am. Coll. Cardiol., 40:754-760,2002.
de Arruda et al., Expert Rev. Mol. Diagn., 2(5):487-496, 2002.
Erbs et al., Eur. J. Cardiovasc. Prev. Rehabil., 13:826-831,2006.
European Patent 201,184
European Patent 237,362
European Patent 258,017
European Patent 266,032
European Patent 320 308
European Patent 329,822
European Patent Appln. 50,424
European Patent Appln. 84,796
French Patent 2,650,840
Froehler et al., Nucleic Acids Res., 14:5399-5407, 1986.
Frohman, In: PCR Protocols: A Guide To Methods And Applications, Academic Press, N.Y., 1990.
Great Britain Appln. 2 202 328
Halushka et al., Nat. Genet., 22(3):239-247,1999.
Harrison, J. Clin. Invest., 100:2153-2157, 1997.
Humphries et al., In: Molecular Diagnosis of Genetic Diseases, Elles (Ed.), 321-340, 1996.
Ignarro et al., Proc. Natl. Acad. Sci. USA, 84:9265-9269, 1987.
Imamura et al., Can. J. Cardiol., 20:1229-1234, 2004.
Inazuka et al., Genome Res, 7(11):1094-1103, 1997.
Innis et al., Proc. Natl. Acad. Sci. USA, 85(24):9436-9440, 1988.
Johnson et al., Nat. Genet., 29(2):233-237, 2001.
Jones, Nature, 199:280-282, 1963.
Kalinowski et al., Circulation, 109:2511-2517, 2004.
Ke and Cardon Bioinformatics, 19(2):287-288, 2003.
Klaassen's The Pharmacological Basis of Therapeutics
Komher, et al., Nucl. Acids. Res. 17:7779-7784, 1989.
Kuppuswamy et al., Proc. Natl. Acad. Sci. USA, 88:1143-1147,1991.
Kwoh et al., Proc. Natl. Acad. Sci. USA, 86:1173, 1989.

Kwok and Chen, Curr Issues Mol. Biol., Apr;5(2):43-60, 2003.
Kwok et al., Genomics, 23(1):138-144, 1994.
Kwok et al., Genomics, 31(1):123-6,1996.
Kwok, Annu. Rev. Genomics Hum. Genet., 2:235-258, 2001.
Landegren et al., Science 241:1077-1080, 1988.
Li et al., Am. J. Hypertens., 17:560-567, 2004.
Lu et al., Biopolymers, 73:606-613,2004.
Lvovich and Scheeline, Anal. Chem., 69:454-462, 1997.
Malinski and Taha, Nature, 358:676-678, 1992.
Mason et al., Circulation, 112:3795-3801,2005.
Maxam et al., Proc. Natl. Acad. Sci. USA, 74:560, 1977.
Megson et al., Curr. Opin. Investig. Drugs, 10(3):276-85, 2009.
Meyers, et al., Science, 230:1242, 1985.
Modrich, Ann. Rev. Genet., 25:229-253, 1991.
Mullis et al., Cold Spring Harbor Symp. Quant. Biol. 51:263-273, 1986.
Münzel et al., J. Clin. Invest., 95:187-194, 1995.
Napoli et al., Nitric Oxide, 15 265-279, 2006.
Nickerson et al., Proc. Natl. Acad Sci. USA, 87:8923-8927,1990.
Nyren et al., Anal. Biochem. 208:171-175, 1993.
Ohara et a/., Proc. Natl. Acad. Sci. USA, 86:5673-5677, 1989.
Orita et al., Genomics, 5:874-879, 1989.
Paniagua et al., Circulation, 103:1752-1758, 2001.
Panza et al., N. Engl. J. Med., 323:22-27, 1990.
PCT Appln. PCT/US87/00880
PCT Appln. PCT/US89/01025
PCT Appln. WO 88/10315
PCT Appln. WO 89/06700
PCT Appln. WO 93/22456
PCT Appln. WO 95/11995
PCT Appln. WO89/06700
PCT Appln. WO90/01069
PCT Appln. WO91/02087
PCT Appln. WO92/15712
Physicians Desk Reference
Prezant et al., Hum. Mutat., 1:159-164, 1992.
Rees et al., Proc. Natl. Acad. Sci. USA, 86:3375-3378, 1989.
Remington’s Pharmaceutical Sciences" 15th Edition, pages 1035-1038 and 1570-1580, 1990.
Ruano et al., Nucl. Acids Res., 19:6877-6882, 1991.
Ruano et al., Nucl. Acids Res., 17:8392, 1989.
Sambrook et al., In: Molecular cloning, Cold Spring Harbor Laboratory Press, Cold Spring Harbor, NY, 2001.
Sanger et al., J. Molec. Biol., 94:441, 1975.
Sheffield et al., Proc. Natl. Acad. Sci. USA, 86:232-236, 1989.
Small et al, N. Engl. J. Med., 347:1135-1142, 2002.
Sokolov, Nucl. Acids Res. 18:3671, 1990.
Stevens et al., Biotechniques, 34:198-203, 2003.
Syvanen et al., Genomics 8:684-692, 1990.
Syvanen et al., Genomics 8:684-692, 1990.
Taddei et al., Hypertension, 21:929-933, 1993.
Taillon-Miller et al., Genome Res, 8(7):748-754, 1998.
The Merck Index, Eleventh Edition
Turki et al., J. Clin. Invest., 95:1635-1641, 1995.
Ugozzoll et al., GATA 9:107-112, 1992.
Walker et al., Proc. Natl. Acad. Sci. USA, 89:392-396, 1992.
Wartell et al., Nucl. Acids Res., 18:2699-2706, 1990.
Winter et al., Proc. Natl. Acad. Sci. USA, 82:7575, 1985.

## Claims

1. The compound S-(6-Nitro-oxi-hexahydro-furo[3,2-b]furan-3-1-il)thioacetate for use in treating a cardiovascular disease or condition, wherein an effective amount of a pharmaceutical composition comprising said compound is administered to the patient after the patient has been tested and determined to be homozygous wildtype (G/G) at position 894 in the endothelial nitric oxide synthase (NOS3) gene.

2. Use of the compound S-(6-Nitro-oxi-hexahydro-furo[3,2-b]furan-3-1-il)thioacetate for the preparation of a pharmaceutical composition for treating a cardiovascular disease or condition, wherein an effective amount of a pharmaceutical composition comprising said compound is administered to the patient after the patient has been tested and determined to be homozygous wildtype (G/G) at position 894 in the endothelial nitric oxide synthase (NOS3) gene.

3. The compound for use according to claim 1 or the use according to claim 2, wherein the cardiovascular disease or condition is peripheral arterial disease, heart failure, coronary heart disease, coronary spasm, myocardial infarction, atherosclerosis, or pulmonary arterial hypertension.

4. The compound for use according to claim 1 or 3 or the use according to claim 2 or 3, wherein at least one dose of the composition is to be administered to the patient, wherein the composition comprises about 1-40 mg of the compound, or wherein the composition comprises about 5-20 mg of the compound.

5. The compound for use according to any one of claims 1, 3 and 4 or the use according to any one of claims 2 to 4, wherein multiple doses of the composition are to be administered to the patient.

6. The compound for use or the use according to claim 5, wherein multiple doses of the composition in a 24 hour period

or wherein 1 to 3 doses of the composition, wherein the composition comprises about 1-40 mg of the compound are to be administered to the patient.

**7.** The compound for use according to claim 1 or the use according to claim 2, wherein the patient has been tested to determine the patient's genotype at position 894 in the NOS3 gene after having been provided or furnished a biological sample from the patient.

**8.** The compound for use according to claim 1 or the use according to claim 2, wherein the patient's genotype at position 894 in the NOS3 gene has been determined after a test has been ordered.

**9.** S-(6-Nitro-oxi-hexahydro-furo[3,2-b]furan-3-1-il)thioacetate for use in treating a patient that has been diagnosed with a disease or condition associated with NO production, wherein said disease is an ischemic cardiovascular disorder, glaucoma, or an intestinal disorder, wherein an effective amount of S-(6-Nitro-oxi-hexahydro-furo[3,2-b]furan-3-1-il)thioacetate is administered to the patient after the patient has been tested and determined to be homozygous wildtype (G/G) at position 894 in the endothelial nitric oxide synthase (NOS3) gene.

**10.** Use of S-(6-Nitro-oxi-hexahydro-furo[3,2-b]furan-3-1-il)thioacetate for the preparation of a pharmaceutical composition for treating a patient that has been diagnosed with a disease or condition associated with NO production, wherein said disease is an ischemic cardiovascular disorder, glaucoma, or an intestinal disorder, wherein an effective amount of S-(6-Nitro-oxi-hexahydro-furo[3,2-b]ftuan-3-1-il)thioacetate is administered to the patient after the patient has been tested and determined to be homozygous wildtype (G/G) at position 894 in the endothelial nitric oxide synthase (NOS3) gene.

**11.** S-(6-Nitro-oxi-hexahydro-furo[3,2-b]furan-3-1-il)thioacetate for use in increasing nitric oxide (NO) levels in a patient that has been diagnosed with a disease or condition associated with NO production, wherein said disease is an ischemic cardiovascular disorder, glaucoma, or an intestinal disorder, wherein an effective amount of said S-(6-Nitro-oxi-hexahydro-furo[3,2-b]furan-3-1-il)thioacetate is administered to the patient after the patient has been tested and determined to be homozygous wildtype (G/G) at position 894 in the endothelial nitric oxide synthase (NOS3) gene.

**12.** Use of S-(6-Nitro-oxi-hexahydro-furo[3,2-b]furan-3-1-il)thioacetate for the preparation of a pharmaceutical composition for increasing nitric oxide (NO) levels in a patient that has been diagnosed with a disease or condition associated with NO production, wherein said disease is an ischemic cardiovascular disorder, glaucoma, or an intestinal disorder, wherein an effective amount of said S-(6-Nitro-oxi-hexahydro-furo[3,2-b]furan-3-1-il)thioacetate is administered to the patient after the patient has been tested and determined to be homozygous wildtype (G/G) at position 894 in the endothelial nitric oxide synthase (NOS3) gene.

**13.** An in vitro method for identifying a patient suitable for treatment with S-(6-Nitro-oxi-hexahydro-furo[3,2-b]furan-3-1-il)thioacetate comprising determining whether the patient is homozygous wildtype (G/G) at position 894 in the endothelial nitric oxide synthase (NOS3) gene, wherein the patient is suitable for treatment with S-(6-Nitro-oxi-hexahydro-furo[3,2-b]furan-3-1-il)thioacetate if the patient is homozygous wildtype (G/G) at position 894 in the NOS3 gene.

**14.** A method for diagnosing a patient as a suitable candidate for treatment with with S-(6-Nitro-oxi-hexahydro-furo[3,2-b]furan-3-1-il)thioacetate comprising assaying a sample from the patient to determine whether the patient is homozygous wildtype (G/G) at position 894 in the endothelial nitric oxide synthase (NOS3) gene and reporting if the patient is homozygous wildtype (G/G) at position 894 in the NOS3 gene.

**15.** Use of a kit for identifying a patient suitable for treatment with S-(6-Nitro-oxi-hexahydro-furo[3,2-b]furan-3-1-il)thioacetate or for diagnosing a patient as a suitable candidate for treatment with with S-(6-Nitro-oxi-hexahydro-furo[3,2-b]furan-3-1-il)thioacetate, wherein said kit comprises in a suitable container a probe or at least one set of primers for identifying the NOS3 polymorphism at position 894.

**16.** The use of claim 15, wherein the probe comprises at least one nucleic acid of between 15 and 100 nucleotides of SEQ ID NO:1 and/or SEQ ID NO:3, wherein the probe includes the nucleotide at position 894 of the NOS3 gene.

**17.** The use of claim 15, wherein the primers comprise at least one set of primers for amplifying a region of sequence that includes position 894 of the NOS3 gene.

**Patentansprüche**

1. Verbindung S-(6-Nitro-oxi-hexahydro-furo[3,2-b]furan-3-1-il)thioacetat zur Verwendung bei der Behandlung einer kardiovaskulären Erkrankung oder eines kardiovaskulären Krankheitsbildes, wobei eine wirksame Menge eines Arzneimittels, das diese Verbindung umfasst, an den Patienten verabreicht wird, nachdem der Patient getestet und der homozygote Wildtyp (G/G) an Position 894 in dem Gen der endothelialen Stickstoffmonoxidsynthase (endothelial nitric oxide synthase gene; NOS3) bestimmt wurde.

2. Verwendung der Verbindung S-(6-Nitro-oxi-hexahydro-furo[3,2-b]furan-3-1-il)thioacetat zur Herstellung eines Arzneimittels zur Behandlung einer kardiovaskulären Erkrankung oder eines kardiovaskulären Krankheitsbildes, wobei eine wirksame Menge eines Arzneimittels, das diese Verbindung umfasst, an den Patienten verabreicht wird, nachdem der Patient getestet und der homozygote Wildtyp (G/G) an Position 894 in dem Gen der endothelialen Stickstoffmonoxidsynthase bestimmt wurde.

3. Verbindung zur Verwendung nach Anspruch 1 oder Verwendung nach Anspruch 2, wobei die kardiovaskuläre Erkrankung oder das kardiovaskuläre Krankheitsbild periphere arterielle Verschlusskrankheit, Herzversagen, koronare Herzerkrankung, koronarer Spasmus, myokardialer Infarkt, Atherosklerose oder pulmonale arterielle Hypertonie ist.

4. Verbindung zur Verwendung nach Anspruch 1 oder 3 oder Verwendung nach Anspruch 2 oder 3, wobei mindestens eine Dosis der Zusammensetzung an den Patienten zu verabreichen ist, wobei die Zusammensetzung etwa 1 bis 40 mg der Verbindung umfasst oder wobei die Zusammensetzung etwa 5 bis 20 mg der Verbindung umfasst.

5. Verbindung zur Verwendung nach einem der Ansprüche 1, 3 und 4 oder Verwendung nach einem der Ansprüche 2 bis 4, wobei mehrere Dosen der Zusammensetzung an den Patienten zu verabreichen sind.

6. Verbindung zur Verwendung oder Verwendung nach Anspruch 5, wobei dem Patienten mehrere Dosen der Zusammensetzung in einem Zeitraum von 24 Stunden oder 1 bis 3 Dosen der Zusammensetzung, wobei die Zusammensetzung etwa 1 bis 40 mg der Verbindung umfasst, zu verabreichen sind.

7. Verbindung zur Verwendung nach Anspruch 1 oder Verwendung nach Anspruch 2, wobei der Patient getestet wurde, um den Genotyp des Patienten an Position 894 in dem NOS3-Gen zu bestimmen, nachdem von dem Patienten eine biologische Probe bereitgestellt oder gegeben wurde.

8. Verbindung zur Verwendung nach Anspruch 1 oder Verwendung nach Anspruch 2, wobei der Genotyp des Patienten an Position 894 in dem NOS3-Gen nach Anordnung eines Tests bestimmt wurde.

9. S-(6-Nitro-oxi-hexahydro-furo[3,2-b]furan-3-1-il)thioacetat zur Verwendung bei der Behandlung eines Patienten, bei dem eine Erkrankung oder ein Krankheitsbild diagnostiziert wurde, die/das mit der Herstellung von NO in Verbindung steht, wobei die Erkrankung eine ischämische kardiovaskuläre Störung, Glaukom oder eine intestinale Störung ist, wobei eine wirksame Menge an S-(6-Nitro-oxi-hexahydro-furo[3,2-b]furan-3-1-il)thioacetat an den Patienten verabreicht wird, nachdem der Patient getestet und der homozygote Wildtyp (G/G) an Position 894 in dem Gen der endothelialen Stickstoffmonoxidsynthase bestimmt wurde.

10. Verwendung von S-(6-Nitro-oxi-hexahydro-furo[3,2-b]furan-3-1-il)thioacetat zur Herstellung eines Arzneimittels zur Behandlung eines Patienten, bei dem eine Erkrankung oder ein Krankheitsbild diagnostiziert wurde, die/das mit der Herstellung von NO in Verbindung steht, wobei die Erkrankung eine ischämische kardiovaskuläre Störung, Glaukom oder eine intestinale Störung ist, wobei eine wirksame Menge an S-(6-Nitro-oxi-hexahydro-furo[3,2-b]furan-3-1-il)thioacetat an den Patienten verabreicht wird, nachdem der Patient getestet und der homozygote Wildtyp (G/G) an Position 894 in dem Gen der endothelialen Stickstoffmonoxidsynthase bestimmt wurde.

11. S-(6-Nitro-oxi-hexahydro-furo[3,2-b]furan-3-1-il)thioacetat zur Verwendung zum Erhöhen von Stickstoffoxid (NO)-Spiegeln in einem Patienten, bei dem eine Erkrankung oder ein Krankheitsbild diagnostiziert wurde, die/das mit der Herstellung von NO in Verbindung steht, wobei die Erkrankung eine ischämische kardiovaskuläre Störung, Glaukom oder eine intestinale Störung ist, wobei eine wirksame Menge an S-(6-Nitro-oxi-hexahydro-furo[3,2-b]furan-3-1-il)thioacetat an den Patienten verabreicht wird, nachdem der Patient getestet und der homozygote Wildtyp (G/G) an Position 894 in dem Gen der endothelialen Stickstoffmonoxidsynthase bestimmt wurde.

12. Verwendung von S-(6-Nitro-oxi-hexahydro-furo[3,2-b]furan-3-1-il)thioacetat zur Herstellung eines Arzneimittels zum Erhöhen von Stickstoffoxid (NO)-Spiegeln in einem Patienten, bei dem eine Erkrankung oder ein Krankheitsbild diagnostiziert wurde, die/das mit der Herstellung von NO in Verbindung steht, wobei die Erkrankung eine ischämische kardiovaskuläre Störung, Glaukom oder eine intestinale Störung ist, wobei eine wirksame Menge an S-(6-Nitro-oxi-hexahydro-furo[3,2-b]furan-3-1-il)thioacetat an den Patienten verabreicht wird, nachdem der Patient getestet und der homozygote Wildtyp (G/G) an Position 894 in dem Gen der endothelialen Stickstoffmonoxidsynthase bestimmt wurde.

13. *In* vitro-Verfahren zum Identifizieren eines Patienten, der für eine Behandlung mit S-(6-Nitro-oxi-hexahydro-furo[3,2-b]furan-3-1-il)thioacetat geeignet ist, umfassend das Bestimmen, ob der Patient homozygot Wildtyp (G/G) an Position 894 in dem Gen der endothelialen Stickstoffmonoxidsynthase ist, wobei der Patient für die Behandlung mit S-(6-Nitro-oxi-hexahydro-furo[3,2-b]furan-3-1-il)thioacetat geeignet ist, wenn der Patient homozygot Wildtyp (G/G) an Position 894 in dem NOS3-Gen ist.

14. Verfahren zum Diagnostizieren eines Patienten als geeigneten Kandidaten für eine Behandlung mit S-(6-Nitro-oxi-hexahydro-furo[3,2-b]furan-3-1-il)thioacetat, umfassend das Testen einer Probe des Patienten, um zu bestimmen, ob der Patient homozygot Wildtyp (G/G) an Position 894 in dem Gen der endothelialen Stickstoffmonoxidsynthase ist und Berichten, ob der Patient homozygot Wildtyp (G/G) an Position 894 in dem NOS3-Gen ist.

15. Verwendung eines Kits zum Identifizieren eines Patienten, der für eine Behandlung mit S-(6-Nitro-oxi-hexahydro-furo[3,2-b]furan-3-1-il)thioacetat geeignet ist, oder zum Diagnostizieren eines Patienten als geeigneten Kandidaten für eine Behandlung mit S-(6-Nitro-oxi-hexahydro-furo[3,2-b]furan-3-1-il)thioacetat, wobei das Kit in einem geeigneten Behälter eine Sonde oder mindestens ein Primer-Set zum Identifizieren des NOS3-Polymorphismus an Position 894 umfasst.

16. Verwendung nach Anspruch 15, wobei die Sonde mindestens eine Nucleinsäure von zwischen 15 und 100 Nucleotiden der SEQ ID NO:1 und/oder SEQ ID NO:3 umfasst, wobei die Sonde das Nucleotid an Position 894 des NOS3-Gens einschließt.

17. Verwendung nach Anspruch 15, wobei die Primer mindestens ein Primer-Set zum Amplifizieren einer Sequenzregion, die die Position 894 des NOS3-Gens einschließt, umfassen.

**Revendications**

1. Composé S-(6-nitro-oxy-hexahydro-furo[3,2-b]furan-3-1-yl)thioacétate pour utilisation dans le traitement d'une maladie ou d'une affection cardiovasculaire, dans lequel une quantité efficace d'une composition pharmaceutique comprenant ledit composé est administrée au patient après que le patient a été testé et déterminé comme étant de type homozygote sauvage (G/G) à la position 894 du gène de l'oxyde nitrique synthase endothéliale (NOS3).

2. Utilisation du composé S-(6-nitro-oxy-hexahydro-furo[3,2-b]furan-3-1-yl)thioacétate pour la préparation d'une composition pharmaceutique destinée au traitement d'une maladie ou d'une affection cardiovasculaire, dans laquelle une quantité efficace d'une composition pharmaceutique comprenant ledit composé est administrée au patient après que le patient a été testé et déterminé comme étant de type homozygote sauvage (G/G) à la position 894 du gène de l'oxyde nitrique synthase endothéliale (NOS3).

3. Composé pour utilisation selon la revendication 1 ou utilisation selon la revendication 2, dans lequel (laquelle) la maladie ou l'affection cardiovasculaire est une maladie artérielle périphérique, une insuffisance cardiaque, une coronaropathie, un spasme coronaire, un infarctus du myocarde, une athérosclérose ou une hypertension artérielle pulmonaire.

4. Composé pour utilisation selon la revendication 1 ou 3 ou utilisation selon la revendication 2 ou 3, dans lequel (laquelle) au moins une dose de la composition doit être administrée au patient, dans lequel (laquelle) la composition comprend environ 1 à 40 mg du composé, ou dans lequel (laquelle) la composition comprend environ 5 à 20 mg du composé.

5. Composé pour utilisation selon l'une quelconque des revendications 1, 3 et 4 ou utilisation selon l'une quelconque des revendications 2 à 4, dans lequel (laquelle) de multiples doses de la composition doivent être administrées au

patient.

6. Composé pour utilisation ou utilisation selon la revendication 5, dans lequel (laquelle) de multiples doses de la composition sur une période de 24 heures ou dans lequel (laquelle) 1 à 3 doses de la composition, dans lequel (laquelle) la composition comprend environ 1 à 40 mg du composé, doivent être administrées au patient.

7. Composé pour utilisation selon la revendication 1 ou utilisation selon la revendication 2, dans lequel (laquelle) le patient a été testé afin de déterminer le génotype du patient à la position 894 du gène NOS3 après que l'on a reçu ou obtenu un échantillon biologique auprès du patient.

8. Composé pour utilisation selon la revendication 1 ou utilisation selon la revendication 2, dans lequel (laquelle) le génotype du patient à la position 894 du gène NOS3 a été déterminé après qu'un test a été demandé.

9. S-(6-Nitro-oxy-hexahydro-furo[3,2-b]furan-3-1-yl)thioacétate pour utilisation dans le traitement d'un patient chez lequel on a diagnostiqué une maladie ou une affection associée à la production de NO, dans lequel ladite maladie est un trouble cardiovasculaire ischémique, un glaucome ou un trouble intestinal, dans lequel une quantité efficace de S-(6-nitro-oxy-hexahydro-furo[3,2-b]furan-3-1-yl)thioacétate est administrée au patient après que le patient a été testé et déterminé comme étant de type homozygote sauvage (G/G) à la position 894 du gène de l'oxyde nitrique synthase endothéliale (NOS3).

10. Utilisation de S-(6-nitro-oxy-hexahydro-furo[3,2-b]furan-3-1-yl)thioacétate pour la préparation d'une composition pharmaceutique destinée au traitement d'un patient chez lequel on a diagnostiqué une maladie ou une affection associée à la production de NO, dans laquelle ladite maladie est un trouble cardiovasculaire ischémique, un glaucome ou un trouble intestinal, dans laquelle une quantité efficace de S-(6-nitro-oxy-hexahydro-furo[3,2-b]furan-3-1-yl)thioacétate est administrée au patient après que le patient a été testé et déterminé comme étant de type homozygote sauvage (G/G) à la position 894 du gène de l'oxyde nitrique synthase endothéliale (NOS3).

11. S-(6-Nitro-oxy-hexahydro-furo[3,2-b]furan-3-1-yl)thioacétate pour utilisation dans l'augmentation des taux d'oxyde nitrique (NO) chez un patient chez lequel on a diagnostiqué une maladie ou une affection associée à la production de NO, dans lequel ladite maladie est un trouble cardiovasculaire ischémique, un glaucome ou un trouble intestinal, dans lequel une quantité efficace dudit S-(6-nitro-oxy-hexahydro-furo[3,2-b]furan-3-1-yl)thioacétate est administrée au patient après que le patient a été testé et déterminé comme étant de type homozygote sauvage (G/G) à la position 894 du gène de l'oxyde nitrique synthase endothéliale (NOS3).

12. Utilisation de S-(6-nitro-oxy-hexahydro-furo[3,2-b]furan-3-1-yl)thioacétate pour la préparation d'une composition pharmaceutique destinée à augmenter les taux d'oxyde nitrique (NO) chez un patient chez lequel on a diagnostiqué une maladie ou une affection associée à la production de NO, dans laquelle ladite maladie est un trouble cardiovasculaire ischémique, un glaucome ou un trouble intestinal, dans laquelle une quantité efficace dudit S-(6-nitro-oxy-hexahydro-furo[3,2-b]furan-3-1-yl)thioacétate est administrée au patient après que le patient a été testé et déterminé comme étant de type homozygote sauvage (G/G) à la position 894 du gène de l'oxyde nitrique synthase endothéliale (NOS3).

13. Procédé *in vitro* d'identification d'un patient convenant au traitement par le S-(6-nitro-oxy-hexahydro-furo[3,2-b]furan-3-1-yl)thioacétate comprenant le fait de déterminer si le patient est de type homozygote sauvage (G/G) à la position 894 du gène de l'oxyde nitrique synthase endothéliale (NOS3), dans lequel le patient convient au traitement par le S-(6-nitro-oxy-hexahydro-furo[3,2-b]furan-3-1-yl)thioacétate si le patient est de type homozygote sauvage (G/G) à la position 894 du gène NOS3.

14. Procédé de diagnostic d'un patient en tant que candidat convenant au traitement par le S-(6-nitro-oxy-hexahydro-furo[3,2-b]furan-3-1-yl)thioacétate comprenant l'analyse d'un échantillon provenant du patient pour déterminer si le patient est de type homozygote sauvage (G/G) à la position 894 du gène de l'oxyde nitrique synthase endothéliale (NOS3) et le fait d'indiquer si le patient est de type homozygote sauvage (G/G) à la position 894 du gène NOS3.

15. Utilisation d'un kit d'identification d'un patient convenant au traitement par le S-(6-nitro-oxyhexahydro-furo[3,2-b]furan-3-1-yl)thioacétate ou de diagnostic d'un patient en tant que candidat convenant au traitement par le S-(6-nitro-oxy-hexahydro-furo[3,2-b]furan-3-1-yl)thioacétate, dans laquelle ledit kit comprend dans un récipient approprié une sonde ou au moins un ensemble d'amorces pour identifier le polymorphisme de NOS3 à la position 894.

**16.** Utilisation selon la revendication 15, dans lequel la sonde comprend au moins un acide nucléique comprenant entre 15 et 100 nucléotides de SEQ ID NO : 1 et/ou SEQ ID NO : 3, dans lequel la sonde inclut le nucléotide à la position 894 du gène NOS3.

**17.** Utilisation selon la revendication 15, dans lequel les amorces comprennent au moins un ensemble d'amorces permettant d'amplifier une région de séquence qui inclut la position 894 du gène NOS3.

FIG. 1

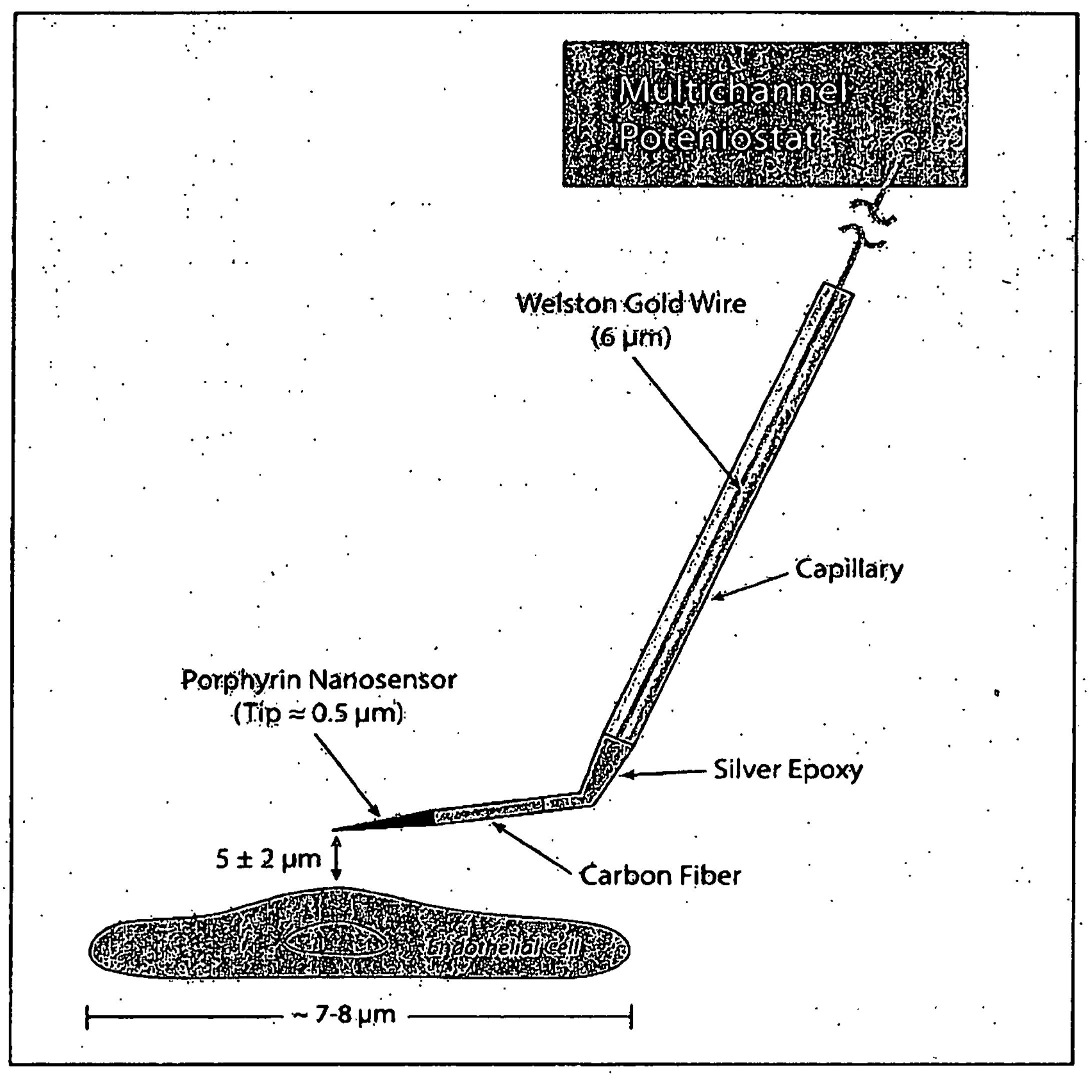
Multichannel
Poteniostat
Welston Gold Wire
(6 μm)
Capillary
Porphyrin Nanosensor
(Tip ≈ 0.5 μm)
Silver Epoxy
5 ± 2 μm
Carbon Fiber
Endothelial cell
~ 7-8 μm

FIG. 2

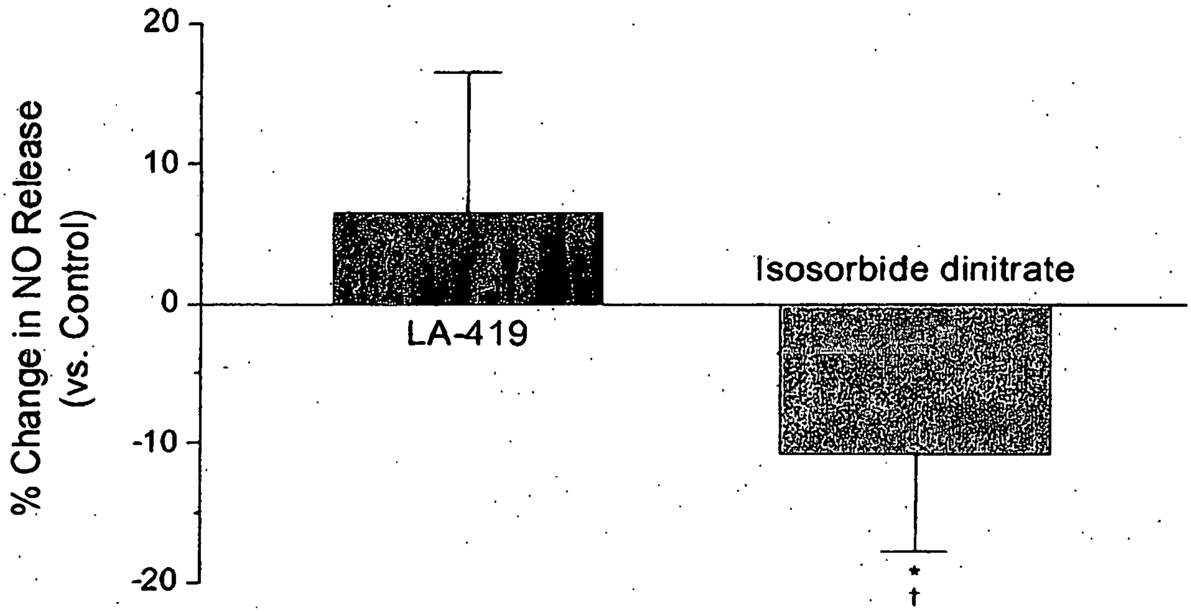
% Change in NO Release
(vs. Control)
20
10
0
-10
-20
LA-419
Isosorbide dinitrate
*
†

**FIG. 3**

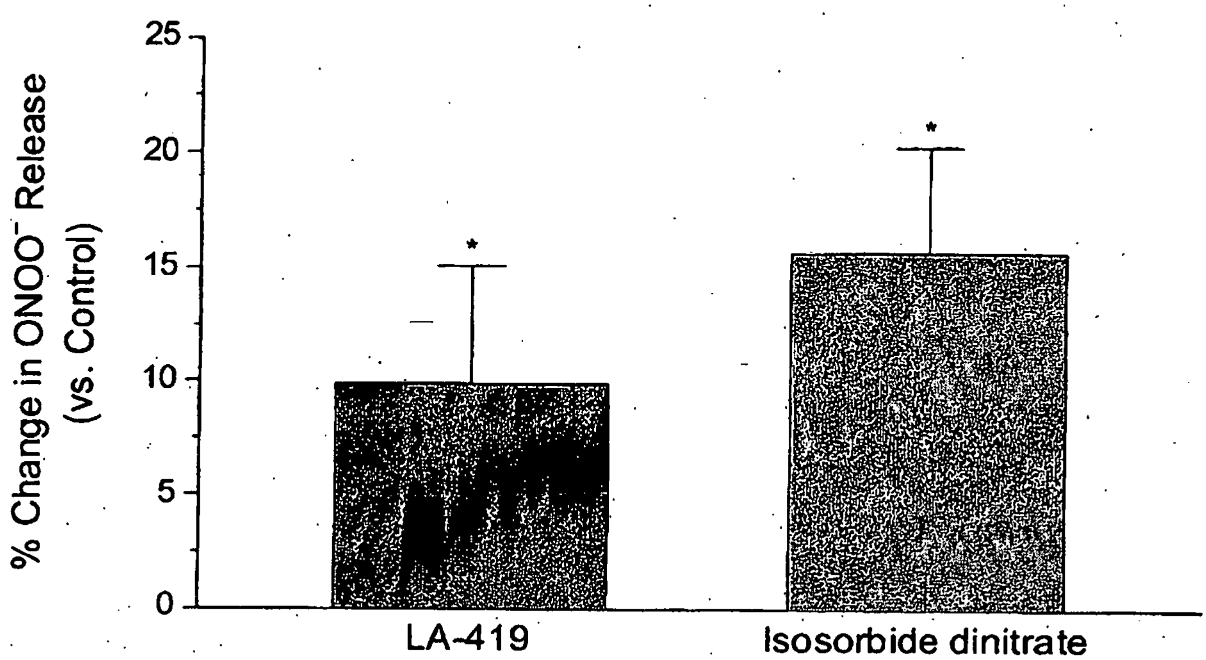
25
20
15
10
5
0
% Change in ONOO⁻ Release
(vs. Control)
*
*
LA-419
Isosorbide dinitrate

FIG. 4

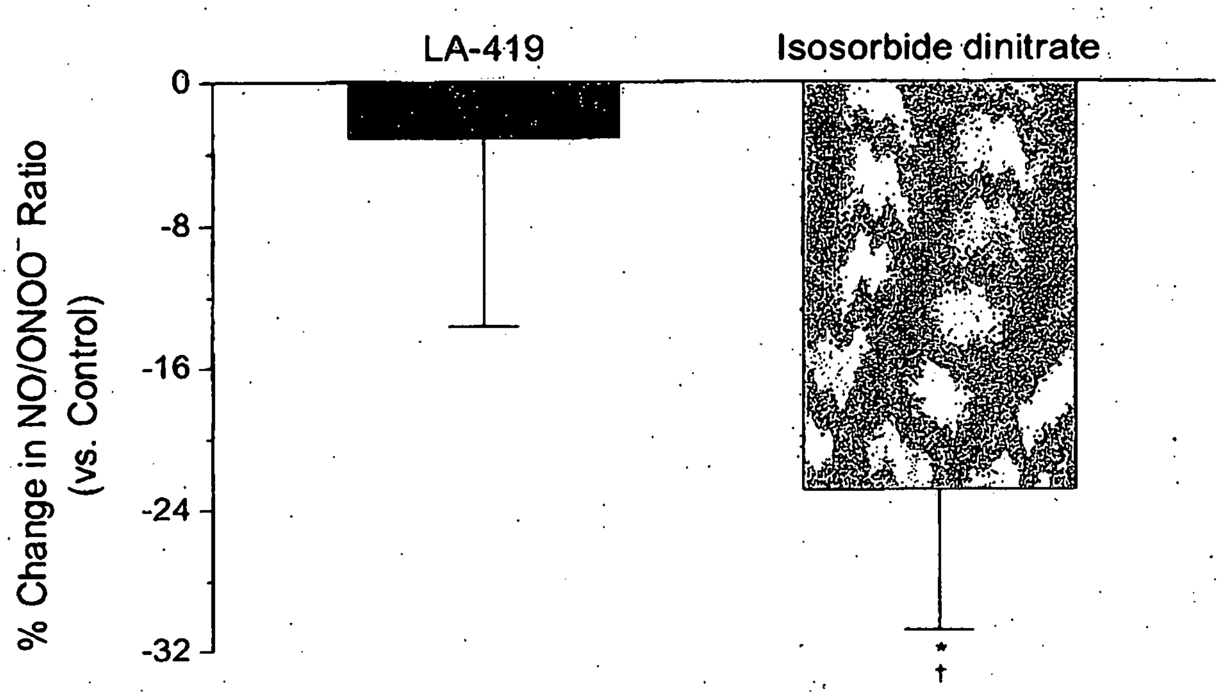
LA-419
Isosorbide dinitrate
0
-8
-16
-24
-32
% Change in NO/ONOO⁻ Ratio
(vs. Control)
*
†

FIG. 5

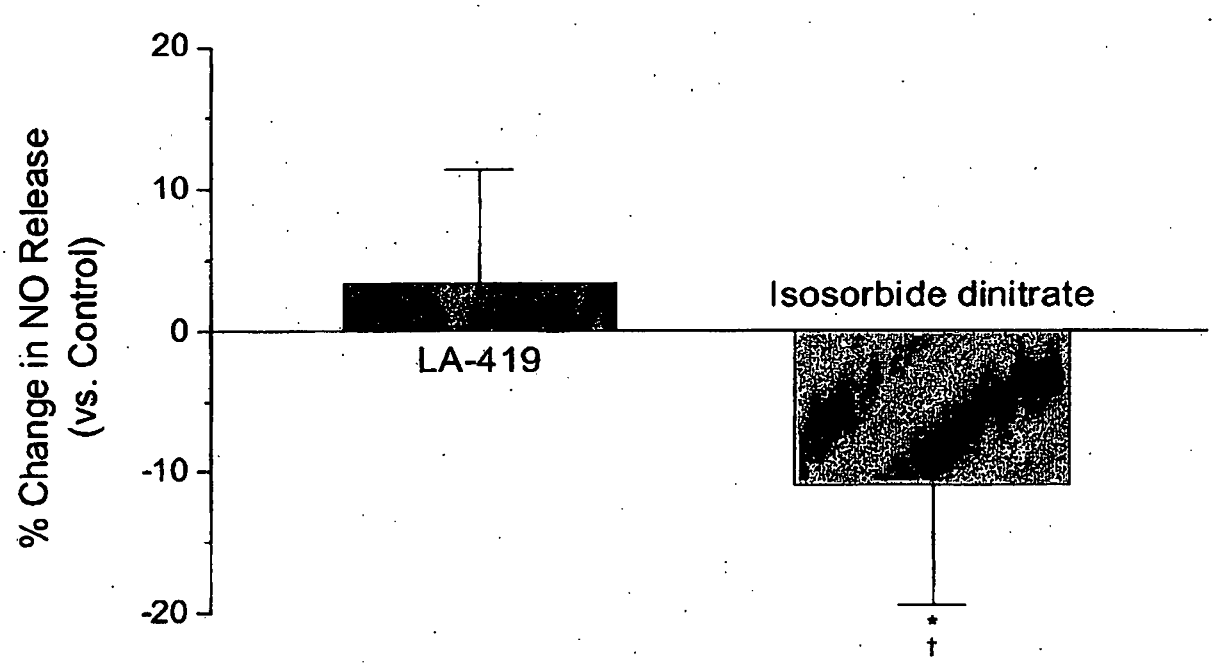
20
10
0
-10
-20
% Change in NO Release
(vs. Control)
LA-419
Isosorbide dinitrate
*
†

FIG. 6

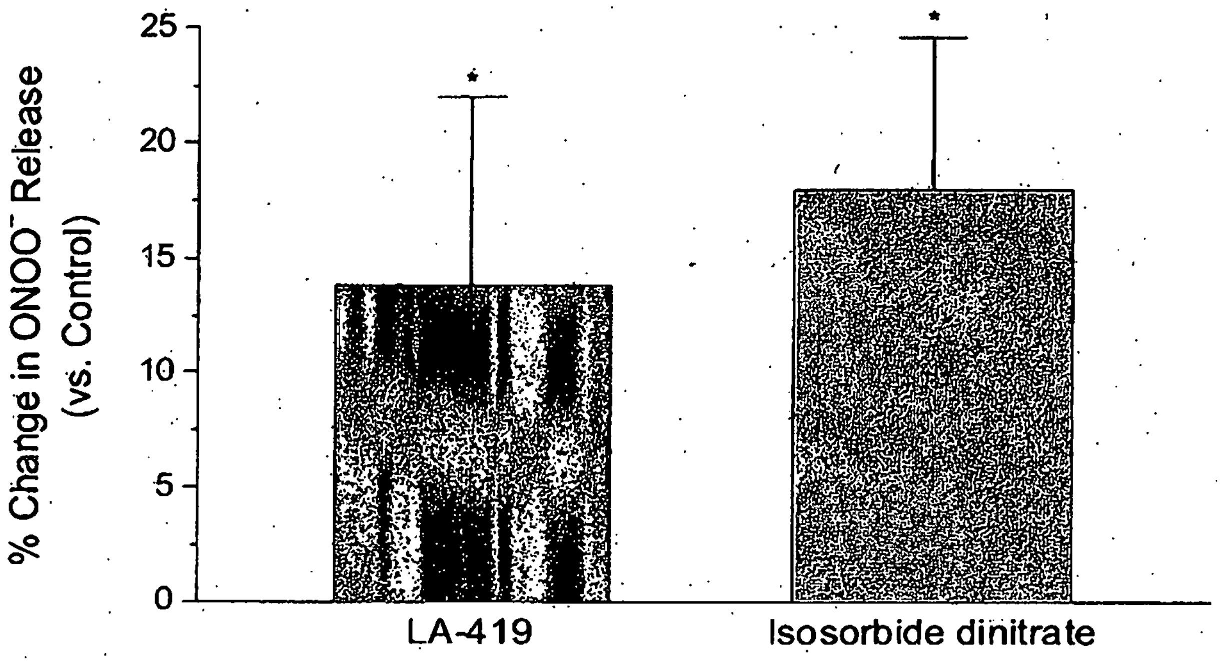
% Change in ONOO⁻ Release
(vs. Control)
25
20
15
10
5
0
*
*
LA-419
Isosorbide dinitrate

FIG. 7

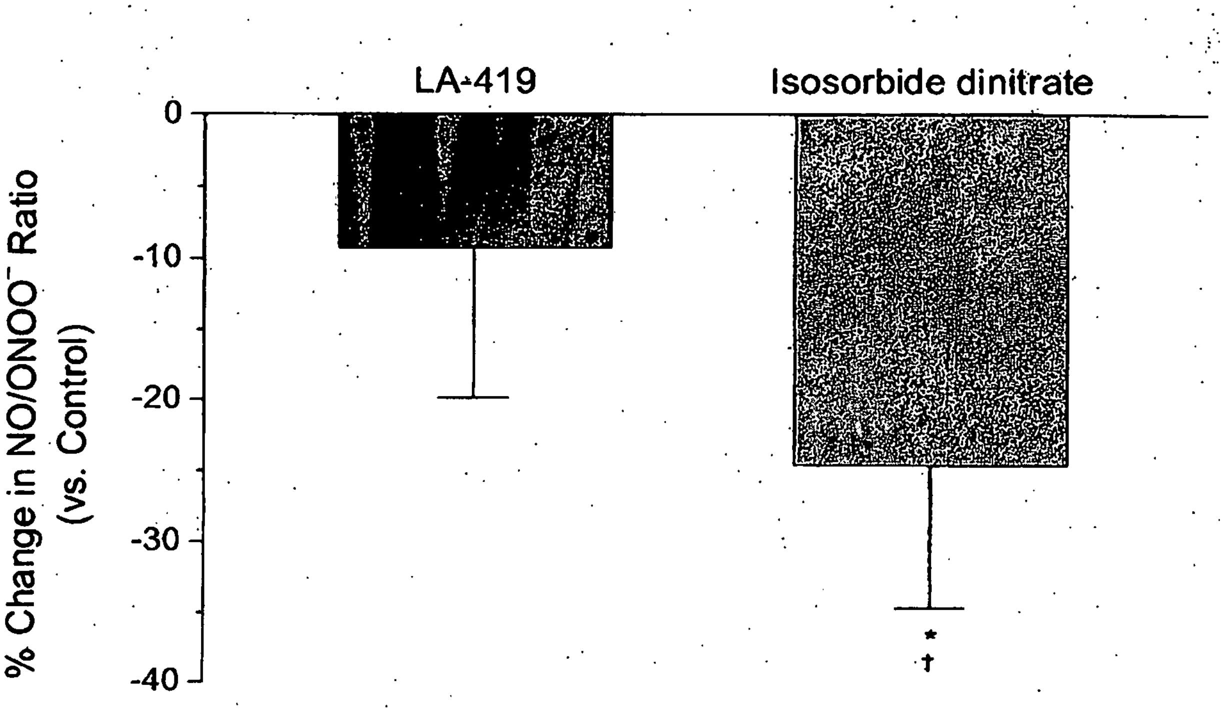
LA-419
Isosorbide dinitrate
0
-10
-20
-30
-40
% Change in NO/ONOO⁻ Ratio
(vs. Control)
*
†

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2003225134 A1
- EP 266032 A
- US 5705629 A
- US 4659774 A
- US 4816571 A
- US 5141813 A
- US 5264566 A
- US 4959463 A
- US 5428148 A
- US 5554744 A
- US 5574146 A
- US 5602244 A
- US 4683202 A
- US 4682195 A
- US 5645897 A
- US 4965188 A
- WO 9001069 A
- US 5130238 A
- EP 329822 A
- US 5169766 A
- WO 8906700 A
- WO 9511995 A
- US 5605798 A
- WO 9322456 A
- US 5843663 A
- US 5900481 A
- US 5919626 A
- US 5849481 A
- US 5849486 A
- US 5851772 A
- US 4683195 A
- US 4800159 A
- EP 320308 A
- US 4883750 A
- US 5912148 A
- US 5843650 A
- US 5846709 A
- US 5846783 A
- US 5849546 A
- US 5849497 A
- US 5849547 A
- US 5858652 A
- US 5866366 A
- US 5916776 A
- US 5922574 A
- US 5928905 A
- US 5928906 A
- US 5932451 A
- US 5935825 A
- US 5939291 A
- US 5942391 A
- GB 2202328 A
- US 8901025 W
- US 8700880 W
- US 5916779 A
- WO 8810315 A
- US 5279721 A
- US 5840873 A
- US 5843640 A
- US 5843651 A
- US 5846708 A
- US 5846717 A
- US 5846726 A
- US 5846729 A
- US 5849487 A
- US 5853990 A
- US 5853992 A
- US 5853993 A
- US 5856092 A
- US 5861244 A
- US 5863732 A
- US 5863753 A
- US 5866331 A
- US 5905024 A
- US 5910407 A
- US 5912124 A
- US 5912145 A
- US 5919630 A
- US 5925517 A
- US 5928862 A
- US 5928869 A
- US 5929227 A
- US 5932413 A
- US 5935791 A
- US 4946773 A
- US 5849483 A
- US 5851770 A
- US 5866337 A
- US 5925525 A
- US 5928870 A
- EP 50424 A
- EP 84796 A
- EP 258017 A
- EP 237362 A
- EP 201184 A
- EP 4582788 A
- EP 4683194 A
- US 4656127 A

- FR 2650840
- WO 9102087 A
- WO 9215712 A
- US 5952174 A
- US 5126145 A
- US 4627429 A
- US 4784857 A
- US 5662925 A
- US 5788983 A
- US 6113940 A
- US 4582788 A
- US 4683194 A
- WO 61289932 A


**Non-patent literature cited in the description**


- Remington's Pharmaceutical Sciences. 1035-1038, 1570-1580
- **AUSUBEL et al.** Current Protocols in Molecular Biology. John Wiley & Sons, 1989
- **BARANY et al.** *Proc. Natl. Acad. Sci. USA,* 1991, vol. 88, 189-193
- **BELLUS.** *J. Macromol. Sci. Pure Appl. Chem.,* 1994, vol. A31 (1), 1355-1376
- **BURT et al.** *Hypertension,* 1995, vol. 25, 305-313
- **CAMPIA et al.** *J. Am. Coll. Cardiol.,* 2002, vol. 40, 754-760
- **DE ARRUDA et al.** *Expert Rev. Mol. Diagn.,* 2002, vol. 2 (5), 487-496
- **ERBS et al.** *Eur. J. Cardiovasc. Prev. Rehabil.,* 2006, vol. 13, 826-831
- **FROEHLER et al.** *Nucleic Acids Res.,* 1986, vol. 14, 5399-5407
- **FROHMAN.** PCR Protocols: A Guide To Methods And Applications. Academic Press, 1990
- **HALUSHKA et al.** *Nat. Genet.,* 1999, vol. 22 (3), 239-247
- **HARRISON.** *J. Clin. Invest.,* 1997, vol. 100, 2153-2157
- **HUMPHRIES et al.** Molecular Diagnosis of Genetic Diseases. 1996, 321-340
- **IGNARRO et al.** *Proc. Natl. Acad. Sci. USA,* 1987, vol. 84, 9265-9269
- **IMAMURA et al.** *Can. J. Cardiol.,* 2004, vol. 20, 1229-1234
- **INAZUKA et al.** *Genome Res,* 1997, vol. 7 (11), 1094-1103
- **INNIS et al.** *Proc. Natl. Acad. Sci. USA,* 1988, vol. 85 (24), 9436-9440
- **JOHNSON et al.** *Nat. Genet.,* 2001, vol. 29 (2), 233-237
- **JONES.** *Nature,* 1963, vol. 199, 280-282
- **KALINOWSKI et al.** *Circulation,* 2004, vol. 109, 2511-2517
- *Ke and Cardon Bioinformatics,* 2003, vol. 19 (2), 287-288
- **KOMHER et al.** *Nucl. Acids. Res.,* 1989, vol. 17, 7779-7784
- **KUPPUSWAMY et al.** *Proc. Natl. Acad. Sci. USA,* 1991, vol. 88, 1143-1147
- **KWOH et al.** *Proc. Natl. Acad. Sci. USA,* 1989, vol. 86, 1173
- **KWOK ; CHEN.** *Curr Issues Mol. Biol.,* 2003, vol. 5 (2), 43-60
- **KWOK et al.** *Genomics,* 1994, vol. 23 (1), 138-144
- **KWOK et al.** *Genomics,* 1996, vol. 31 (1), 123-6
- **KWOK.** *Annu. Rev. Genomics Hum. Genet.,* 2001, vol. 2, 235-258
- **LANDEGREN et al.** *Science,* 1988, vol. 241, 1077-1080
- **LI et al.** *Am. J. Hypertens.,* 2004, vol. 17, 560-567
- **LU et al.** *Biopolymers,* 2004, vol. 73, 606-613
- **LVOVICH ; SCHEELINE.** *Anal. Chem.,* 1997, vol. 69, 454-462
- **MALINSKI ; TAHA.** *Nature,* 1992, vol. 358, 676-678
- **MASON et al.** *Circulation,* 2005, vol. 112, 3795-3801
- **MAXAM et al.** *Proc. Natl. Acad. Sci. USA,* 1977, vol. 74, 560
- **MEGSON et al.** *Curr. Opin. Investig. Drugs,* 2009, vol. 10 (3), 276-85
- **MEYERS et al.** *Science,* 1985, vol. 230, 1242
- **MODRICH.** *Ann. Rev. Genet.,* 1991, vol. 25, 229-253
- **MULLIS et al.** *Cold Spring Harbor Symp. Quant. Biol.,* 1986, vol. 51, 263-273
- **MÜNZEL et al.** *J. Clin. Invest.,* 1995, vol. 95, 187-194
- **NAPOLI et al.** *Nitric Oxide,* 2006, vol. 15, 265-279
- **NICKERSON et al.** *Proc. Natl. Acad Sci. USA,* 1990, vol. 87, 8923-8927
- **NYREN et al.** *Anal. Biochem.,* 1993, vol. 208, 171-175
- **OHARA.** *Proc. Natl. Acad. Sci. USA,* 1989, vol. 86, 5673-5677
- **ORITA et al.** *Genomics,* 1989, vol. 5, 874-879
- **PANIAGUA et al.** *Circulation,* 2001, vol. 103, 1752-1758
- **PANZA et al.** *N. Engl. J. Med.,* 1990, vol. 323, 22-27
- **PREZANT et al.** *Hum. Mutat.,* 1992, vol. 1, 159-164
- **REES et al.** *Proc. Natl. Acad. Sci. USA,* 1989, vol. 86, 3375-3378

- Remington's Pharmaceutical Sciences. 1990, 1035-1038, 1570-1580
- **RUANO et al.** *Nucl. Acids Res.,* 1991, vol. 19, 6877-6882
- **RUANO et al.** *Nucl. Acids Res.,* 1989, vol. 17, 8392
- **SAMBROOK et al.** Molecular cloning. Cold Spring Harbor Laboratory Press, 2001
- **SANGER et al.** *J. Molec. Biol.,* 1975, vol. 94, 441
- **SHEFFIELD et al.** *Proc. Natl. Acad. Sci. USA,* 1989, vol. 86, 232-236
- **SMALL et al.** *N. Engl. J. Med.,* 2002, vol. 347, 1135-1142
- **SOKOLOV.** *Nucl. Acids Res.,* 1990, vol. 18, 3671
- **STEVENS et al.** *Biotechniques,* 2003, vol. 34, 198-203
- **SYVANEN et al.** *Genomics,* 1990, vol. 8, 684-692
- **TADDEI et al.** *Hypertension,* 1993, vol. 21, 929-933
- **TAILLON-MILLER et al.** *Genome Res,* 1998, vol. 8 (7), 748-754
- **TURKI et al.** *J. Clin. Invest.,* 1995, vol. 95, 1635-1641
- **UGOZZOLL et al.** *GATA,* 1992, vol. 9, 107-112
- **WALKER et al.** *Proc. Natl. Acad. Sci. USA,* 1992, vol. 89, 392-396
- **WARTELL et al.** *Nucl. Acids Res.,* 1990, vol. 18, 2699-2706
- **WINTER et al.** *Proc. Natl. Acad. Sci. USA,* 1985, vol. 82, 7575

SEQUENCE LISTING

<110> BRISTOW, MICHAEL R.
PORT, J. DAVID

<120> METHODS AND COMPOSITIONS FOR CARDIOVASCULAR DISEASES AND CONDITIONS

<130> ARCA.P0024WO

<140> UNKNOWN
<141> 2010-12-23

<150> 61/289,932
<151> 2009-12-23

<160> 10

<170> Patent In version 3.5

<210> 1
<211> 4345
<212> DNA
<213> Homo sapiens

<220>
<221> CDS
<222> (297)..(3908)

<400> 1

```
actgaaacta ggggcaagga gacgaagaga acatgaaagt taaactttaa gatgaagaac      60

aaagctgaac atactgatgc attggatctt tggagaggat ctcagaactc attgtactta     120

atttacaggc taaaacctta gaagaggaat ttattatatc ctacacaaga ctccagggaa     180

gcacatggcc ttggactgaa ggctggcatc tggaagctgt cagccaccag caccttctgc     240

agcaggaaaa ggccagggct ctgctggagc aggcagcaga gtggacgcac agtaac atg     299
                                                              Met
                                                              1

ggc aac ttg aag agc gtg gcc cag gag cct ggg cca ccc tgc ggc ctg       347
Gly Asn Leu Lys Ser Val Ala Gln Glu Pro Gly Pro Pro Cys Gly Leu
            5                   10                  15

ggg ctg ggg ctg ggc ctt ggg ctg tgc ggc aag cag ggc cca gcc acc       395
Gly Leu Gly Leu Gly Leu Gly Leu Cys Gly Lys Gln Gly Pro Ala Thr
        20                  25                  30

ccg gcc cct gag ccc agc cgg gcc cca gca tcc cta ctc cca cca gcg       443
Pro Ala Pro Glu Pro Ser Arg Ala Pro Ala Ser Leu Leu Pro Pro Ala
    35                  40                  45

cca gaa cac agc ccc ccg agc tcc ccg cta acc cag ccc cca gag ggg       491
Pro Glu His Ser Pro Pro Ser Ser Pro Leu Thr Gln Pro Pro Glu Gly
50                  55                  60                  65

ccc aag ttc cct cgt gtg aag aac tgg gag gtg ggg agc atc acc tat       539
Pro Lys Phe Pro Arg Val Lys Asn Trp Glu Val Gly Ser Ile Thr Tyr
                70                  75                  80

gac acc ctc agc gcc cag gcg cag cag gat ggg ccc tgc acc cca aga       587
Asp Thr Leu Ser Ala Gln Ala Gln Gln Asp Gly Pro Cys Thr Pro Arg
```

```
                85                  90                  95

cgc tgc ctg ggc tcc ctg gta ttt cca cgg aaa cta cag ggc cgg ccc       635
Arg Cys Leu Gly Ser Leu Val Phe Pro Arg Lys Leu Gln Gly Arg Pro
        100                 105                 110

tcc ccc ggc ccc ccg gcc cct gag cag ctg ctg agt cag gcc cgg gac       683
Ser Pro Gly Pro Pro Ala Pro Glu Gln Leu Leu Ser Gln Ala Arg Asp
    115                 120                 125

ttc atc aac cag tac tac agc tcc att aag agg agc ggc tcc cag gcc       731
Phe Ile Asn Gln Tyr Tyr Ser Ser Ile Lys Arg Ser Gly Ser Gln Ala
130                 135                 140                 145

cac gaa cag cgg ctt caa gag gtg gaa gcc gag gtg gca gcc aca ggc       779
His Glu Gln Arg Leu Gln Glu Val Glu Ala Glu Val Ala Ala Thr Gly
                150                 155                 160

acc tac cag ctt agg gag agc gag ctg gtg ttc ggg gct aag cag gcc       827
Thr Tyr Gln Leu Arg Glu Ser Glu Leu Val Phe Gly Ala Lys Gln Ala
            165                 170                 175

tgg cgc aac gct ccc cgc tgc gtg ggc cgg atc cag tgg ggg aag ctg       875
Trp Arg Asn Ala Pro Arg Cys Val Gly Arg Ile Gln Trp Gly Lys Leu
        180                 185                 190

cag gtg ttc gat gcc cgg gac tgc agg tct gca cag gaa atg ttc acc       923
Gln Val Phe Asp Ala Arg Asp Cys Arg Ser Ala Gln Glu Met Phe Thr
    195                 200                 205

tac atc tgc aac cac atc aag tat gcc acc aac cgg ggc aac ctt cgc       971
Tyr Ile Cys Asn His Ile Lys Tyr Ala Thr Asn Arg Gly Asn Leu Arg
210                 215                 220                 225

tcg gcc atc aca gtg ttc ccg cag cgc tgc cct ggc cga gga gac ttc      1019
Ser Ala Ile Thr Val Phe Pro Gln Arg Cys Pro Gly Arg Gly Asp Phe
                230                 235                 240

cga atc tgg aac agc cag ctg gtg cgc tac gcg ggc tac cgg cag cag      1067
Arg Ile Trp Asn Ser Gln Leu Val Arg Tyr Ala Gly Tyr Arg Gln Gln
            245                 250                 255

gat ggc tct gtg cgg ggg gac cca gcc aac gtg gag atc acc gag ctc      1115
Asp Gly Ser Val Arg Gly Asp Pro Ala Asn Val Glu Ile Thr Glu Leu
        260                 265                 270

tgc att cag cac ggc tgg acc cca gga aac ggt cgc ttc gac gtg ctg      1163
Cys Ile Gln His Gly Trp Thr Pro Gly Asn Gly Arg Phe Asp Val Leu
    275                 280                 285

ccc ctg ctg ctg cag gcc cca gat gag ccc cca gaa ctc ttc ctt ctg      1211
Pro Leu Leu Leu Gln Ala Pro Asp Glu Pro Pro Glu Leu Phe Leu Leu
290                 295                 300                 305

ccc ccc gag ctg gtc ctt gag gtg ccc ctg gag cac ccc acg ctg gag      1259
Pro Pro Glu Leu Val Leu Glu Val Pro Leu Glu His Pro Thr Leu Glu
                310                 315                 320

tgg ttt gca gcc ctg ggc ctg cgc tgg tac gcc ctc ccg gca gtg tcc      1307
Trp Phe Ala Ala Leu Gly Leu Arg Trp Tyr Ala Leu Pro Ala Val Ser
            325                 330                 335

aac atg ctg ctg gaa att ggg ggc ctg gag ttc ccc gca gcc ccc ttc      1355
Asn Met Leu Leu Glu Ile Gly Gly Leu Glu Phe Pro Ala Ala Pro Phe
        340                 345                 350
```

```
agt ggc tgg tac atg agc act gag atc ggc acg agg aac ctg tgt gac      1403
Ser Gly Trp Tyr Met Ser Thr Glu Ile Gly Thr Arg Asn Leu Cys Asp
    355                 360                 365

cct cac cgc tac aac atc ctg gag gat gtg gct gtc tgc atg gac ctg      1451
Pro His Arg Tyr Asn Ile Leu Glu Asp Val Ala Val Cys Met Asp Leu
370                 375                 380                 385

gat acc cgg acc acc tcg tcc ctg tgg aaa gac aag gca gca gtg gaa      1499
Asp Thr Arg Thr Thr Ser Ser Leu Trp Lys Asp Lys Ala Ala Val Glu
                390                 395                 400

atc aac gtg gcc gtg ctg cac agt tac cag cta gcc aaa gtc acc atc      1547
Ile Asn Val Ala Val Leu His Ser Tyr Gln Leu Ala Lys Val Thr Ile
            405                 410                 415

gtg gac cac cac gcc gcc acg gcc tct ttc atg aag cac ctg gag aat      1595
Val Asp His His Ala Ala Thr Ala Ser Phe Met Lys His Leu Glu Asn
        420                 425                 430

gag cag aag gcc agg ggg ggc tgc cct gca gac tgg gcc tgg atc gtg      1643
Glu Gln Lys Ala Arg Gly Gly Cys Pro Ala Asp Trp Ala Trp Ile Val
    435                 440                 445

ccc ccc atc tcg ggc agc ctc act cct gtt ttc cat cag gag atg gtc      1691
Pro Pro Ile Ser Gly Ser Leu Thr Pro Val Phe His Gln Glu Met Val
450                 455                 460                 465

aac tat ttc ctg tcc ccg gcc ttc cgc tac cag cca gac ccc tgg aag      1739
Asn Tyr Phe Leu Ser Pro Ala Phe Arg Tyr Gln Pro Asp Pro Trp Lys
                470                 475                 480

ggg agt gcc gcc aag ggc acc ggc atc acc agg aag aag acc ttt aaa      1787
Gly Ser Ala Ala Lys Gly Thr Gly Ile Thr Arg Lys Lys Thr Phe Lys
            485                 490                 495

gaa gtg gcc aac gcc gtg aag atc tcc gcc tcg ctc atg ggc acg gtg      1835
Glu Val Ala Asn Ala Val Lys Ile Ser Ala Ser Leu Met Gly Thr Val
        500                 505                 510

atg gcg aag cga gtg aag gcg aca atc ctg tat ggc tcc gag acc ggc      1883
Met Ala Lys Arg Val Lys Ala Thr Ile Leu Tyr Gly Ser Glu Thr Gly
    515                 520                 525

cgg gcc cag agc tac gca cag cag ctg ggg aga ctc ttc cgg aag gct      1931
Arg Ala Gln Ser Tyr Ala Gln Gln Leu Gly Arg Leu Phe Arg Lys Ala
530                 535                 540                 545

ttt gat ccc cgg gtc ctg tgt atg gat gag tat gac gtg gtg tcc ctc      1979
Phe Asp Pro Arg Val Leu Cys Met Asp Glu Tyr Asp Val Val Ser Leu
                550                 555                 560

gaa cac gag acg ctg gtg ctg gtg gta acc agc aca ttt ggg aat ggg      2027
Glu His Glu Thr Leu Val Leu Val Val Thr Ser Thr Phe Gly Asn Gly
            565                 570                 575

gat ccc ccg gag aat gga gag agc ttt gca gct gcc ctg atg gag atg      2075
Asp Pro Pro Glu Asn Gly Glu Ser Phe Ala Ala Ala Leu Met Glu Met
        580                 585                 590

tcc ggc ccc tac aac agc tcc cct cgg ccg gaa cag cac aag agt tat      2123
Ser Gly Pro Tyr Asn Ser Ser Pro Arg Pro Glu Gln His Lys Ser Tyr
    595                 600                 605
```

```
aag atc cgc ttc aac agc atc tcc tgc tca gac cca ctg gtg tcc tct        2171
Lys Ile Arg Phe Asn Ser Ile Ser Cys Ser Asp Pro Leu Val Ser Ser
610                 615                 620                 625

tgg cgg cgg aag agg aag gag tcc agt aac aca gac agt gca ggg gcc        2219
Trp Arg Arg Lys Arg Lys Glu Ser Ser Asn Thr Asp Ser Ala Gly Ala
                630                 635                 640

ctg ggc acc ctc agg ttc tgt gtg ttc ggg ctc ggc tcc cgg gca tac        2267
Leu Gly Thr Leu Arg Phe Cys Val Phe Gly Leu Gly Ser Arg Ala Tyr
            645                 650                 655

ccc cac ttc tgc gcc ttt gct cgt gcc gtg gac aca cgg ctg gag gaa        2315
Pro His Phe Cys Ala Phe Ala Arg Ala Val Asp Thr Arg Leu Glu Glu
        660                 665                 670

ctg ggc ggg gag cgg ctg ctg cag ctg ggc cag ggc gac gag ctg tgc        2363
Leu Gly Gly Glu Arg Leu Leu Gln Leu Gly Gln Gly Asp Glu Leu Cys
    675                 680                 685

ggc cag gag gag gcc ttc cga ggc tgg gcc cag gct gcc ttc cag gcc        2411
Gly Gln Glu Glu Ala Phe Arg Gly Trp Ala Gln Ala Ala Phe Gln Ala
690                 695                 700                 705

gcc tgt gag acc ttc tgt gtg gga gag gat gcc aag gcc gcc gcc cga        2459
Ala Cys Glu Thr Phe Cys Val Gly Glu Asp Ala Lys Ala Ala Ala Arg
                710                 715                 720

gac atc ttc agc ccc aaa cgg agc tgg aag cgc cag agg tac cgg ctg        2507
Asp Ile Phe Ser Pro Lys Arg Ser Trp Lys Arg Gln Arg Tyr Arg Leu
            725                 730                 735

agc gcc cag gcc gag ggc ctg cag ttg ctg cca ggt ctg atc cac gtg        2555
Ser Ala Gln Ala Glu Gly Leu Gln Leu Leu Pro Gly Leu Ile His Val
        740                 745                 750

cac agg cgg aag atg ttc cag gct aca atc cgc tca gtg gaa aac ctg        2603
His Arg Arg Lys Met Phe Gln Ala Thr Ile Arg Ser Val Glu Asn Leu
    755                 760                 765

caa agc agc aag tcc acg agg gcc acc atc ctg gtg cgc ctg gac acc        2651
Gln Ser Ser Lys Ser Thr Arg Ala Thr Ile Leu Val Arg Leu Asp Thr
770                 775                 780                 785

gga ggc cag gag ggg ctg cag tac cag ccg ggg gac cac ata ggt gtc        2699
Gly Gly Gln Glu Gly Leu Gln Tyr Gln Pro Gly Asp His Ile Gly Val
                790                 795                 800

tgc ccg ccc aac cgg ccc ggc ctt gtg gag gcg ctg ctg agc cgc gtg        2747
Cys Pro Pro Asn Arg Pro Gly Leu Val Glu Ala Leu Leu Ser Arg Val
            805                 810                 815

gag gac ccg ccg gcg ccc act gag ccc gtg gca gta gag cag ctg gag        2795
Glu Asp Pro Pro Ala Pro Thr Glu Pro Val Ala Val Glu Gln Leu Glu
        820                 825                 830

aag ggc agc cct ggt ggc cct ccc ccc ggc tgg gtg cgg gac ccc cgg        2843
Lys Gly Ser Pro Gly Gly Pro Pro Pro Gly Trp Val Arg Asp Pro Arg
    835                 840                 845

ctg ccc ccg tgc acg ctg cgc cag gct ctc acc ttc ttc ctg gac atc        2891
Leu Pro Pro Cys Thr Leu Arg Gln Ala Leu Thr Phe Phe Leu Asp Ile
850                 855                 860                 865

acc tcc cca ccc agc cct cag ctc ttg cgg ctg ctc agc acc ttg gca        2939
```

```
Thr Ser Pro Pro Ser Pro Gln Leu Leu Arg Leu Leu Ser Thr Leu Ala
                870                 875                 880

gaa gag ccc agg gaa cag cag gag ctg gag gcc ctc agc cag gat ccc      2987
Glu Glu Pro Arg Glu Gln Gln Glu Leu Glu Ala Leu Ser Gln Asp Pro
            885                 890                 895

cga cgc tac gag gag tgg aag tgg ttc cgc tgc ccc acg ctg ctg gag      3035
Arg Arg Tyr Glu Glu Trp Lys Trp Phe Arg Cys Pro Thr Leu Leu Glu
        900                 905                 910

gtg ctg gag cag ttc ccg tcg gtg gcg ctg cct gcc cca ctg ctc ctc      3083
Val Leu Glu Gln Phe Pro Ser Val Ala Leu Pro Ala Pro Leu Leu Leu
    915                 920                 925

acc cag ctg cct ctg ctc cag ccc cgg tac tac tca gtc agc tcg gca      3131
Thr Gln Leu Pro Leu Leu Gln Pro Arg Tyr Tyr Ser Val Ser Ser Ala
930                 935                 940                 945

ccc agc acc cac cca gga gag atc cac ctc act gta gct gtg ctg gca      3179
Pro Ser Thr His Pro Gly Glu Ile His Leu Thr Val Ala Val Leu Ala
                950                 955                 960

tac agg act cag gat ggg ctg ggc ccc ctg cac tat gga gtc tgc tcc      3227
Tyr Arg Thr Gln Asp Gly Leu Gly Pro Leu His Tyr Gly Val Cys Ser
            965                 970                 975

acg tgg cta agc cag ctc aag ccc gga gac cct gtg ccc tgc ttc atc      3275
Thr Trp Leu Ser Gln Leu Lys Pro Gly Asp Pro Val Pro Cys Phe Ile
        980                 985                 990

cgg ggg gct ccc tcc ttc cgg  ctg cca ccc gat ccc  agc ttg ccc tgc      3323
Arg Gly Ala Pro Ser Phe Arg  Leu Pro Pro Asp Pro  Ser Leu Pro Cys
    995                 1000                 1005

atc  ctg gtg ggt cca ggc  act ggc att gcc ccc  ttc cgg gga ttc      3368
Ile  Leu Val Gly Pro Gly  Thr Gly Ile Ala Pro  Phe Arg Gly Phe
1010                 1015                 1020

tgg  cag gag cgg ctg cat  gac att gag agc aaa  ggg ctg cag ccc      3413
Trp  Gln Glu Arg Leu His  Asp Ile Glu Ser Lys  Gly Leu Gln Pro
1025                 1030                 1035

act  ccc atg act ttg gtg  ttc ggc tgc cga tgc  tcc caa ctt gac      3458
Thr  Pro Met Thr Leu Val  Phe Gly Cys Arg Cys  Ser Gln Leu Asp
1040                 1045                 1050

cat  ctc tac cgc gac gag  gtg cag aac gcc cag  cag cgc ggg gtg      3503
His  Leu Tyr Arg Asp Glu  Val Gln Asn Ala Gln  Gln Arg Gly Val
1055                 1060                 1065

ttt  ggc cga gtc ctc acc  gcc ttc tcc cgg gaa  cct gac aac ccc      3548
Phe  Gly Arg Val Leu Thr  Ala Phe Ser Arg Glu  Pro Asp Asn Pro
1070                 1075                 1080

aag  acc tac gtg cag gac  atc ctg agg acg gag  ctg gct gcg gag      3593
Lys  Thr Tyr Val Gln Asp  Ile Leu Arg Thr Glu  Leu Ala Ala Glu
1085                 1090                 1095

gtg  cac cgc gtg ctg tgc  ctc gag cgg ggc cac  atg ttt gtc tgc      3638
Val  His Arg Val Leu Cys  Leu Glu Arg Gly His  Met Phe Val Cys
1100                 1105                 1110

ggc  gat gtt acc atg gca  acc aac gtc ctg cag  acc gtg cag cgc      3683
Gly  Asp Val Thr Met Ala  Thr Asn Val Leu Gln  Thr Val Gln Arg
```

1115 1120 1125

atc ctg gcg acg gag ggc gac atg gag ctg gac gag gcc ggc gac 3728
Ile Leu Ala Thr Glu Gly Asp Met Glu Leu Asp Glu Ala Gly Asp
1130 1135 1140

gtc atc ggc gtg ctg cgg gat cag caa cgc tac cac gaa gac att 3773
Val Ile Gly Val Leu Arg Asp Gln Gln Arg Tyr His Glu Asp Ile
1145 1150 1155

ttc ggg ctc acg ctg cgc acc cag gag gtg aca agc cgc ata cgc 3818
Phe Gly Leu Thr Leu Arg Thr Gln Glu Val Thr Ser Arg Ile Arg
1160 1165 1170

acc cag agc ttt tcc ttg cag gag cgt cag ttg cgg ggc gca gtg 3863
Thr Gln Ser Phe Ser Leu Gln Glu Arg Gln Leu Arg Gly Ala Val
1175 1180 1185

ccc tgg gcg ttc gac cct ccc ggc tca gac acc aac agc ccc tga 3908
Pro Trp Ala Phe Asp Pro Pro Gly Ser Asp Thr Asn Ser Pro
1190 1195 1200

gagccgcctg gctttccctt ccagttccgg gagagcggct gcccgactca ggtccgcccg 3968

accaggatca gccccgctcc tcccctcttg aggtggtgcc ttctcacatc tgtccagagg 4028

ctgcaaggat tcagcattat tcctccagga aggagcaaaa cgcctctttt ccctctctag 4088

gcctgttgcc tcgggcctgg gtccgcctta atctggaagg cccctcccag cagcggtacc 4148

ccagggccta ctgccacccg cttcctgttt cttagtcgaa tgttagattc ctcttgcctc 4208

tctcaggagt atcttacctg taaagtctaa tctctaaatc aagtatttat tattgaagat 4268

ttaccataag ggactgtgcc agatgttagg agaactacta aagtgcctac cccagctcat 4328

gtggattaca aaaaaa 4345

<210> 2
<211> 1203
<212> PRT
<213> Homo sapiens

<400> 2

```
Met Gly Asn Leu Lys Ser Val Ala Gln Glu Pro Gly Pro Pro Cys Gly
1               5                   10                  15

Leu Gly Leu Gly Leu Gly Leu Gly Leu Cys Gly Lys Gln Gly Pro Ala
            20                  25                  30

Thr Pro Ala Pro Glu Pro Ser Arg Ala Pro Ala Ser Leu Leu Pro Pro
        35                  40                  45

Ala Pro Glu His Ser Pro Pro Ser Ser Pro Leu Thr Gln Pro Pro Glu
    50                  55                  60

Gly Pro Lys Phe Pro Arg Val Lys Asn Trp Glu Val Gly Ser Ile Thr
65                  70                  75                  80
```

Tyr Asp Thr Leu Ser Ala Gln Ala Gln Gln Asp Gly Pro Cys Thr Pro
85 90 95

Arg Arg Cys Leu Gly Ser Leu Val Phe Pro Arg Lys Leu Gln Gly Arg
100 105 110

Pro Ser Pro Gly Pro Pro Ala Pro Glu Gln Leu Leu Ser Gln Ala Arg
115 120 125

Asp Phe Ile Asn Gln Tyr Tyr Ser Ser Ile Lys Arg Ser Gly Ser Gln
130 135 140

Ala His Glu Gln Arg Leu Gln Glu Val Glu Ala Glu Val Ala Ala Thr
145 150 155 160

Gly Thr Tyr Gln Leu Arg Glu Ser Glu Leu Val Phe Gly Ala Lys Gln
165 170 175

Ala Trp Arg Asn Ala Pro Arg Cys Val Gly Arg Ile Gln Trp Gly Lys
180 185 190

Leu Gln Val Phe Asp Ala Arg Asp Cys Arg Ser Ala Gln Glu Met Phe
195 200 205

Thr Tyr Ile Cys Asn His Ile Lys Tyr Ala Thr Asn Arg Gly Asn Leu
210 215 220

Arg Ser Ala Ile Thr Val Phe Pro Gln Arg Cys Pro Gly Arg Gly Asp
225 230 235 240

Phe Arg Ile Trp Asn Ser Gln Leu Val Arg Tyr Ala Gly Tyr Arg Gln
245 250 255

Gln Asp Gly Ser Val Arg Gly Asp Pro Ala Asn Val Glu Ile Thr Glu
260 265 270

Leu Cys Ile Gln His Gly Trp Thr Pro Gly Asn Gly Arg Phe Asp Val
275 280 285

Leu Pro Leu Leu Leu Gln Ala Pro Asp Glu Pro Pro Glu Leu Phe Leu
290 295 300

Leu Pro Pro Glu Leu Val Leu Glu Val Pro Leu Glu His Pro Thr Leu
305 310 315 320

Glu Trp Phe Ala Ala Leu Gly Leu Arg Trp Tyr Ala Leu Pro Ala Val
325 330 335

Ser Asn Met Leu Leu Glu Ile Gly Gly Leu Glu Phe Pro Ala Ala Pro
340 345 350

Phe Ser Gly Trp Tyr Met Ser Thr Glu Ile Gly Thr Arg Asn Leu Cys
355 360 365

Asp Pro His Arg Tyr Asn Ile Leu Glu Asp Val Ala Val Cys Met Asp
370 375 380

Leu Asp Thr Arg Thr Thr Ser Ser Leu Trp Lys Asp Lys Ala Ala Val
385 390 395 400

Glu Ile Asn Val Ala Val Leu His Ser Tyr Gln Leu Ala Lys Val Thr
405 410 415

Ile Val Asp His His Ala Ala Thr Ala Ser Phe Met Lys His Leu Glu
420 425 430

Asn Glu Gln Lys Ala Arg Gly Gly Cys Pro Ala Asp Trp Ala Trp Ile
435 440 445

Val Pro Pro Ile Ser Gly Ser Leu Thr Pro Val Phe His Gln Glu Met
450 455 460

Val Asn Tyr Phe Leu Ser Pro Ala Phe Arg Tyr Gln Pro Asp Pro Trp
465 470 475 480

Lys Gly Ser Ala Ala Lys Gly Thr Gly Ile Thr Arg Lys Lys Thr Phe
485 490 495

Lys Glu Val Ala Asn Ala Val Lys Ile Ser Ala Ser Leu Met Gly Thr
500 505 510

Val Met Ala Lys Arg Val Lys Ala Thr Ile Leu Tyr Gly Ser Glu Thr
515 520 525

Gly Arg Ala Gln Ser Tyr Ala Gln Gln Leu Gly Arg Leu Phe Arg Lys
530 535 540

Ala Phe Asp Pro Arg Val Leu Cys Met Asp Glu Tyr Asp Val Val Ser
545 550 555 560

Leu Glu His Glu Thr Leu Val Leu Val Val Thr Ser Thr Phe Gly Asn
565 570 575

Gly Asp Pro Pro Glu Asn Gly Glu Ser Phe Ala Ala Ala Leu Met Glu
580 585 590

Met Ser Gly Pro Tyr Asn Ser Ser Pro Arg Pro Glu Gln His Lys Ser

595 600 605

Tyr Lys Ile Arg Phe Asn Ser Ile Ser Cys Ser Asp Pro Leu Val Ser
610 615 620

Ser Trp Arg Arg Lys Arg Lys Glu Ser Ser Asn Thr Asp Ser Ala Gly
625 630 635 640

Ala Leu Gly Thr Leu Arg Phe Cys Val Phe Gly Leu Gly Ser Arg Ala
645 650 655

Tyr Pro His Phe Cys Ala Phe Ala Arg Ala Val Asp Thr Arg Leu Glu
660 665 670

Glu Leu Gly Gly Glu Arg Leu Leu Gln Leu Gly Gln Gly Asp Glu Leu
675 680 685

Cys Gly Gln Glu Glu Ala Phe Arg Gly Trp Ala Gln Ala Ala Phe Gln
690 695 700

Ala Ala Cys Glu Thr Phe Cys Val Gly Glu Asp Ala Lys Ala Ala Ala
705 710 715 720

Arg Asp Ile Phe Ser Pro Lys Arg Ser Trp Lys Arg Gln Arg Tyr Arg
725 730 735

Leu Ser Ala Gln Ala Glu Gly Leu Gln Leu Leu Pro Gly Leu Ile His
740 745 750

Val His Arg Arg Lys Met Phe Gln Ala Thr Ile Arg Ser Val Glu Asn
755 760 765

Leu Gln Ser Ser Lys Ser Thr Arg Ala Thr Ile Leu Val Arg Leu Asp
770 775 780

Thr Gly Gly Gln Glu Gly Leu Gln Tyr Gln Pro Gly Asp His Ile Gly
785 790 795 800

Val Cys Pro Pro Asn Arg Pro Gly Leu Val Glu Ala Leu Leu Ser Arg
805 810 815

Val Glu Asp Pro Pro Ala Pro Thr Glu Pro Val Ala Val Glu Gln Leu
820 825 830

Glu Lys Gly Ser Pro Gly Gly Pro Pro Pro Gly Trp Val Arg Asp Pro
835 840 845

Arg Leu Pro Pro Cys Thr Leu Arg Gln Ala Leu Thr Phe Phe Leu Asp
850 855 860

Ile Thr Ser Pro Pro Ser Pro Gln Leu Leu Arg Leu Leu Ser Thr Leu
865 870 875 880

Ala Glu Glu Pro Arg Glu Gln Gln Glu Leu Glu Ala Leu Ser Gln Asp
885 890 895

Pro Arg Arg Tyr Glu Glu Trp Lys Trp Phe Arg Cys Pro Thr Leu Leu
900 905 910

Glu Val Leu Glu Gln Phe Pro Ser Val Ala Leu Pro Ala Pro Leu Leu
915 920 925

Leu Thr Gln Leu Pro Leu Leu Gln Pro Arg Tyr Tyr Ser Val Ser Ser
930 935 940

Ala Pro Ser Thr His Pro Gly Glu Ile His Leu Thr Val Ala Val Leu
945 950 955 960

Ala Tyr Arg Thr Gln Asp Gly Leu Gly Pro Leu His Tyr Gly Val Cys
965 970 975

Ser Thr Trp Leu Ser Gln Leu Lys Pro Gly Asp Pro Val Pro Cys Phe
980 985 990

Ile Arg Gly Ala Pro Ser Phe Arg Leu Pro Pro Asp Pro Ser Leu Pro
995 1000 1005

Cys Ile Leu Val Gly Pro Gly Thr Gly Ile Ala Pro Phe Arg Gly
1010 1015 1020

Phe Trp Gln Glu Arg Leu His Asp Ile Glu Ser Lys Gly Leu Gln
1025 1030 1035

Pro Thr Pro Met Thr Leu Val Phe Gly Cys Arg Cys Ser Gln Leu
1040 1045 1050

Asp His Leu Tyr Arg Asp Glu Val Gln Asn Ala Gln Gln Arg Gly
1055 1060 1065

Val Phe Gly Arg Val Leu Thr Ala Phe Ser Arg Glu Pro Asp Asn
1070 1075 1080

Pro Lys Thr Tyr Val Gln Asp Ile Leu Arg Thr Glu Leu Ala Ala
1085 1090 1095

Glu Val His Arg Val Leu Cys Leu Glu Arg Gly His Met Phe Val
1100 1105 1110

Cys Gly Asp Val Thr Met Ala Thr Asn Val Leu Gln Thr Val Gln
1115 1120 1125

Arg Ile Leu Ala Thr Glu Gly Asp Met Glu Leu Asp Glu Ala Gly
1130 1135 1140

Asp Val Ile Gly Val Leu Arg Asp Gln Gln Arg Tyr His Glu Asp
1145 1150 1155

Ile Phe Gly Leu Thr Leu Arg Thr Gln Glu Val Thr Ser Arg Ile
1160 1165 1170

Arg Thr Gln Ser Phe Ser Leu Gln Glu Arg Gln Leu Arg Gly Ala
1175 1180 1185

Val Pro Trp Ala Phe Asp Pro Pro Gly Ser Asp Thr Asn Ser Pro
1190 1195 1200

<210> 3
<211> 4345
<212> DNA
<213> Homo sapiens

<220>
<221> CDS
<222> (297)..(3908)

<400> 3

actgaaacta ggggcaagga gacgaagaga acatgaaagt taaactttaa gatgaagaac 60

aaagctgaac atactgatgc attggatctt tggagaggat ctcagaactc attgtactta 120

atttacaggc taaaacctta gaagaggaat ttattatatc ctacacaaga ctccagggaa 180

gcacatggcc ttggactgaa ggctggcatc tggaagctgt cagccaccag caccttctgc 240

agcaggaaaa ggccagggct ctgctggagc aggcagcaga gtggacgcac agtaac atg 299
Met
1

ggc aac ttg aag agc gtg gcc cag gag cct ggg cca ccc tgc ggc ctg 347
Gly Asn Leu Lys Ser Val Ala Gln Glu Pro Gly Pro Pro Cys Gly Leu
5 10 15

ggg ctg ggg ctg ggc ctt ggg ctg tgc ggc aag cag ggc cca gcc acc 395
Gly Leu Gly Leu Gly Leu Gly Leu Cys Gly Lys Gln Gly Pro Ala Thr
20 25 30

ccg gcc cct gag ccc agc cgg gcc cca gca tcc cta ctc cca cca gcg 443
Pro Ala Pro Glu Pro Ser Arg Ala Pro Ala Ser Leu Leu Pro Pro Ala
35 40 45

cca gaa cac agc ccc ccg agc tcc ccg cta acc cag ccc cca gag ggg 491
Pro Glu His Ser Pro Pro Ser Ser Pro Leu Thr Gln Pro Pro Glu Gly
50 55 60 65

ccc aag ttc cct cgt gtg aag aac tgg gag gtg ggg agc atc acc tat 539

```
Pro Lys Phe Pro Arg Val Lys Asn Trp Glu Val Gly Ser Ile Thr Tyr
                70                  75                  80

gac acc ctc agc gcc cag gcg cag cag gat ggg ccc tgc acc cca aga      587
Asp Thr Leu Ser Ala Gln Ala Gln Gln Asp Gly Pro Cys Thr Pro Arg
            85                  90                  95

cgc tgc ctg ggc tcc ctg gta ttt cca cgg aaa cta cag ggc cgg ccc      635
Arg Cys Leu Gly Ser Leu Val Phe Pro Arg Lys Leu Gln Gly Arg Pro
        100                 105                 110

tcc ccc ggc ccc ccg gcc cct gag cag ctg ctg agt cag gcc cgg gac      683
Ser Pro Gly Pro Pro Ala Pro Glu Gln Leu Leu Ser Gln Ala Arg Asp
    115                 120                 125

ttc atc aac cag tac tac agc tcc att aag agg agc ggc tcc cag gcc      731
Phe Ile Asn Gln Tyr Tyr Ser Ser Ile Lys Arg Ser Gly Ser Gln Ala
130                 135                 140                 145

cac gaa cag cgg ctt caa gag gtg gaa gcc gag gtg gca gcc aca ggc      779
His Glu Gln Arg Leu Gln Glu Val Glu Ala Glu Val Ala Ala Thr Gly
                150                 155                 160

acc tac cag ctt agg gag agc gag ctg gtg ttc ggg gct aag cag gcc      827
Thr Tyr Gln Leu Arg Glu Ser Glu Leu Val Phe Gly Ala Lys Gln Ala
            165                 170                 175

tgg cgc aac gct ccc cgc tgc gtg ggc cgg atc cag tgg ggg aag ctg      875
Trp Arg Asn Ala Pro Arg Cys Val Gly Arg Ile Gln Trp Gly Lys Leu
        180                 185                 190

cag gtg ttc gat gcc cgg gac tgc agg tct gca cag gaa atg ttc acc      923
Gln Val Phe Asp Ala Arg Asp Cys Arg Ser Ala Gln Glu Met Phe Thr
    195                 200                 205

tac atc tgc aac cac atc aag tat gcc acc aac cgg ggc aac ctt cgc      971
Tyr Ile Cys Asn His Ile Lys Tyr Ala Thr Asn Arg Gly Asn Leu Arg
210                 215                 220                 225

tcg gcc atc aca gtg ttc ccg cag cgc tgc cct ggc cga gga gac ttc     1019
Ser Ala Ile Thr Val Phe Pro Gln Arg Cys Pro Gly Arg Gly Asp Phe
                230                 235                 240

cga atc tgg aac agc cag ctg gtg cgc tac gcg ggc tac cgg cag cag     1067
Arg Ile Trp Asn Ser Gln Leu Val Arg Tyr Ala Gly Tyr Arg Gln Gln
            245                 250                 255

gat ggc tct gtg cgg ggg gac cca gcc aac gtg gag atc acc gag ctc     1115
Asp Gly Ser Val Arg Gly Asp Pro Ala Asn Val Glu Ile Thr Glu Leu
        260                 265                 270

tgc att cag cac ggc tgg acc cca gga aac ggt cgc ttc gac gtg ctg     1163
Cys Ile Gln His Gly Trp Thr Pro Gly Asn Gly Arg Phe Asp Val Leu
    275                 280                 285

ccc ctg ctg ctg cag gcc cca gat gat ccc cca gaa ctc ttc ctt ctg     1211
Pro Leu Leu Leu Gln Ala Pro Asp Asp Pro Pro Glu Leu Phe Leu Leu
290                 295                 300                 305

ccc ccc gag ctg gtc ctt gag gtg ccc ctg gag cac ccc acg ctg gag     1259
Pro Pro Glu Leu Val Leu Glu Val Pro Leu Glu His Pro Thr Leu Glu
                310                 315                 320

tgg ttt gca gcc ctg ggc ctg cgc tgg tac gcc ctc ccg gca gtg tcc     1307
Trp Phe Ala Ala Leu Gly Leu Arg Trp Tyr Ala Leu Pro Ala Val Ser
```

325 330 335

aac atg ctg ctg gaa att ggg ggc ctg gag ttc ccc gca gcc ccc ttc 1355
Asn Met Leu Leu Glu Ile Gly Gly Leu Glu Phe Pro Ala Ala Pro Phe
340 345 350

agt ggc tgg tac atg agc act gag atc ggc acg agg aac ctg tgt gac 1403
Ser Gly Trp Tyr Met Ser Thr Glu Ile Gly Thr Arg Asn Leu Cys Asp
355 360 365

cct cac cgc tac aac atc ctg gag gat gtg gct gtc tgc atg gac ctg 1451
Pro His Arg Tyr Asn Ile Leu Glu Asp Val Ala Val Cys Met Asp Leu
370 375 380 385

gat acc cgg acc acc tcg tcc ctg tgg aaa gac aag gca gca gtg gaa 1499
Asp Thr Arg Thr Thr Ser Ser Leu Trp Lys Asp Lys Ala Ala Val Glu
390 395 400

atc aac gtg gcc gtg ctg cac agt tac cag cta gcc aaa gtc acc atc 1547
Ile Asn Val Ala Val Leu His Ser Tyr Gln Leu Ala Lys Val Thr Ile
405 410 415

gtg gac cac cac gcc gcc acg gcc tct ttc atg aag cac ctg gag aat 1595
Val Asp His His Ala Ala Thr Ala Ser Phe Met Lys His Leu Glu Asn
420 425 430

gag cag aag gcc agg ggg ggc tgc cct gca gac tgg gcc tgg atc gtg 1643
Glu Gln Lys Ala Arg Gly Gly Cys Pro Ala Asp Trp Ala Trp Ile Val
435 440 445

ccc ccc atc tcg ggc agc ctc act cct gtt ttc cat cag gag atg gtc 1691
Pro Pro Ile Ser Gly Ser Leu Thr Pro Val Phe His Gln Glu Met Val
450 455 460 465

aac tat ttc ctg tcc ccg gcc ttc cgc tac cag cca gac ccc tgg aag 1739
Asn Tyr Phe Leu Ser Pro Ala Phe Arg Tyr Gln Pro Asp Pro Trp Lys
470 475 480

ggg agt gcc gcc aag ggc acc ggc atc acc agg aag aag acc ttt aaa 1787
Gly Ser Ala Ala Lys Gly Thr Gly Ile Thr Arg Lys Lys Thr Phe Lys
485 490 495

gaa gtg gcc aac gcc gtg aag atc tcc gcc tcg ctc atg ggc acg gtg 1835
Glu Val Ala Asn Ala Val Lys Ile Ser Ala Ser Leu Met Gly Thr Val
500 505 510

atg gcg aag cga gtg aag gcg aca atc ctg tat ggc tcc gag acc ggc 1883
Met Ala Lys Arg Val Lys Ala Thr Ile Leu Tyr Gly Ser Glu Thr Gly
515 520 525

cgg gcc cag agc tac gca cag cag ctg ggg aga ctc ttc cgg aag gct 1931
Arg Ala Gln Ser Tyr Ala Gln Gln Leu Gly Arg Leu Phe Arg Lys Ala
530 535 540 545

ttt gat ccc cgg gtc ctg tgt atg gat gag tat gac gtg gtg tcc ctc 1979
Phe Asp Pro Arg Val Leu Cys Met Asp Glu Tyr Asp Val Val Ser Leu
550 555 560

gaa cac gag acg ctg gtg ctg gtg gta acc agc aca ttt ggg aat ggg 2027
Glu His Glu Thr Leu Val Leu Val Val Thr Ser Thr Phe Gly Asn Gly
565 570 575

gat ccc ccg gag aat gga gag agc ttt gca gct gcc ctg atg gag atg 2075
Asp Pro Pro Glu Asn Gly Glu Ser Phe Ala Ala Ala Leu Met Glu Met
580 585 590

tcc ggc ccc tac aac agc tcc cct cgg ccg gaa cag cac aag agt tat 2123
Ser Gly Pro Tyr Asn Ser Ser Pro Arg Pro Glu Gln His Lys Ser Tyr
595 600 605

aag atc cgc ttc aac agc atc tcc tgc tca gac cca ctg gtg tcc tct 2171
Lys Ile Arg Phe Asn Ser Ile Ser Cys Ser Asp Pro Leu Val Ser Ser
610 615 620 625

tgg cgg cgg aag agg aag gag tcc agt aac aca gac agt gca ggg gcc 2219
Trp Arg Arg Lys Arg Lys Glu Ser Ser Asn Thr Asp Ser Ala Gly Ala
630 635 640

ctg ggc acc ctc agg ttc tgt gtg ttc ggg ctc ggc tcc cgg gca tac 2267
Leu Gly Thr Leu Arg Phe Cys Val Phe Gly Leu Gly Ser Arg Ala Tyr
645 650 655

ccc cac ttc tgc gcc ttt gct cgt gcc gtg gac aca cgg ctg gag gaa 2315
Pro His Phe Cys Ala Phe Ala Arg Ala Val Asp Thr Arg Leu Glu Glu
660 665 670

ctg ggc ggg gag cgg ctg ctg cag ctg ggc cag ggc gac gag ctg tgc 2363
Leu Gly Gly Glu Arg Leu Leu Gln Leu Gly Gln Gly Asp Glu Leu Cys
675 680 685

ggc cag gag gag gcc ttc cga ggc tgg gcc cag gct gcc ttc cag gcc 2411
Gly Gln Glu Glu Ala Phe Arg Gly Trp Ala Gln Ala Ala Phe Gln Ala
690 695 700 705

gcc tgt gag acc ttc tgt gtg gga gag gat gcc aag gcc gcc gcc cga 2459
Ala Cys Glu Thr Phe Cys Val Gly Glu Asp Ala Lys Ala Ala Ala Arg
710 715 720

gac atc ttc agc ccc aaa cgg agc tgg aag cgc cag agg tac cgg ctg 2507
Asp Ile Phe Ser Pro Lys Arg Ser Trp Lys Arg Gln Arg Tyr Arg Leu
725 730 735

agc gcc cag gcc gag ggc ctg cag ttg ctg cca ggt ctg atc cac gtg 2555
Ser Ala Gln Ala Glu Gly Leu Gln Leu Leu Pro Gly Leu Ile His Val
740 745 750

cac agg cgg aag atg ttc cag gct aca atc cgc tca gtg gaa aac ctg 2603
His Arg Arg Lys Met Phe Gln Ala Thr Ile Arg Ser Val Glu Asn Leu
755 760 765

caa agc agc aag tcc acg agg gcc acc atc ctg gtg cgc ctg gac acc 2651
Gln Ser Ser Lys Ser Thr Arg Ala Thr Ile Leu Val Arg Leu Asp Thr
770 775 780 785

gga ggc cag gag ggg ctg cag tac cag ccg ggg gac cac ata ggt gtc 2699
Gly Gly Gln Glu Gly Leu Gln Tyr Gln Pro Gly Asp His Ile Gly Val
790 795 800

tgc ccg ccc aac cgg ccc ggc ctt gtg gag gcg ctg ctg agc cgc gtg 2747
Cys Pro Pro Asn Arg Pro Gly Leu Val Glu Ala Leu Leu Ser Arg Val
805 810 815

gag gac ccg ccg gcg ccc act gag ccc gtg gca gta gag cag ctg gag 2795
Glu Asp Pro Pro Ala Pro Thr Glu Pro Val Ala Val Glu Gln Leu Glu
820 825 830

aag ggc agc cct ggt ggc cct ccc ccc ggc tgg gtg cgg gac ccc cgg 2843
Lys Gly Ser Pro Gly Gly Pro Pro Pro Gly Trp Val Arg Asp Pro Arg
835 840 845

```
ctg ccc ccg tgc acg ctg cgc cag gct ctc acc ttc ttc ctg gac atc       2891
Leu Pro Pro Cys Thr Leu Arg Gln Ala Leu Thr Phe Phe Leu Asp Ile
850                 855                 860                 865

acc tcc cca ccc agc cct cag ctc ttg cgg ctg ctc agc acc ttg gca       2939
Thr Ser Pro Pro Ser Pro Gln Leu Leu Arg Leu Leu Ser Thr Leu Ala
                870                 875                 880

gaa gag ccc agg gaa cag cag gag ctg gag gcc ctc agc cag gat ccc       2987
Glu Glu Pro Arg Glu Gln Gln Glu Leu Glu Ala Leu Ser Gln Asp Pro
            885                 890                 895

cga cgc tac gag gag tgg aag tgg ttc cgc tgc ccc acg ctg ctg gag       3035
Arg Arg Tyr Glu Glu Trp Lys Trp Phe Arg Cys Pro Thr Leu Leu Glu
        900                 905                 910

gtg ctg gag cag ttc ccg tcg gtg gcg ctg cct gcc cca ctg ctc ctc       3083
Val Leu Glu Gln Phe Pro Ser Val Ala Leu Pro Ala Pro Leu Leu Leu
    915                 920                 925

acc cag ctg cct ctg ctc cag ccc cgg tac tac tca gtc agc tcg gca       3131
Thr Gln Leu Pro Leu Leu Gln Pro Arg Tyr Tyr Ser Val Ser Ser Ala
930                 935                 940                 945

ccc agc acc cac cca gga gag atc cac ctc act gta gct gtg ctg gca       3179
Pro Ser Thr His Pro Gly Glu Ile His Leu Thr Val Ala Val Leu Ala
                950                 955                 960

tac agg act cag gat ggg ctg ggc ccc ctg cac tat gga gtc tgc tcc       3227
Tyr Arg Thr Gln Asp Gly Leu Gly Pro Leu His Tyr Gly Val Cys Ser
            965                 970                 975

acg tgg cta agc cag ctc aag ccc gga gac cct gtg ccc tgc ttc atc       3275
Thr Trp Leu Ser Gln Leu Lys Pro Gly Asp Pro Val Pro Cys Phe Ile
        980                 985                 990

cgg ggg gct ccc tcc ttc cgg  ctg cca ccc gat ccc  agc ttg ccc tgc     3323
Arg Gly Ala Pro Ser Phe Arg  Leu Pro Pro Asp Pro  Ser Leu Pro Cys
    995                 1000                1005

atc  ctg gtg ggt cca ggc  act ggc att gcc ccc  ttc cgg gga ttc        3368
Ile  Leu Val Gly Pro Gly  Thr Gly Ile Ala Pro  Phe Arg Gly Phe
1010                 1015                 1020

tgg  cag gag cgg ctg cat  gac att gag agc aaa  ggg ctg cag ccc        3413
Trp  Gln Glu Arg Leu His  Asp Ile Glu Ser Lys  Gly Leu Gln Pro
1025                 1030                 1035

act  ccc atg act ttg gtg  ttc ggc tgc cga tgc  tcc caa ctt gac        3458
Thr  Pro Met Thr Leu Val  Phe Gly Cys Arg Cys  Ser Gln Leu Asp
1040                 1045                 1050

cat  ctc tac cgc gac gag  gtg cag aac gcc cag  cag cgc ggg gtg        3503
His  Leu Tyr Arg Asp Glu  Val Gln Asn Ala Gln  Gln Arg Gly Val
1055                 1060                 1065

ttt  ggc cga gtc ctc acc  gcc ttc tcc cgg gaa  cct gac aac ccc        3548
Phe  Gly Arg Val Leu Thr  Ala Phe Ser Arg Glu  Pro Asp Asn Pro
1070                 1075                 1080

aag  acc tac gtg cag gac  atc ctg agg acg gag  ctg gct gcg gag        3593
Lys  Thr Tyr Val Gln Asp  Ile Leu Arg Thr Glu  Leu Ala Ala Glu
1085                 1090                 1095

gtg  cac cgc gtg ctg tgc  ctc gag cgg ggc cac  atg ttt gtc tgc        3638
```

Val His Arg Val Leu Cys Leu Glu Arg Gly His Met Phe Val Cys
1100 1105 1110

ggc gat gtt acc atg gca acc aac gtc ctg cag acc gtg cag cgc 3683
Gly Asp Val Thr Met Ala Thr Asn Val Leu Gln Thr Val Gln Arg
1115 1120 1125

atc ctg gcg acg gag ggc gac atg gag ctg gac gag gcc ggc gac 3728
Ile Leu Ala Thr Glu Gly Asp Met Glu Leu Asp Glu Ala Gly Asp
1130 1135 1140

gtc atc ggc gtg ctg cgg gat cag caa cgc tac cac gaa gac att 3773
Val Ile Gly Val Leu Arg Asp Gln Gln Arg Tyr His Glu Asp Ile
1145 1150 1155

ttc ggg ctc acg ctg cgc acc cag gag gtg aca agc cgc ata cgc 3818
Phe Gly Leu Thr Leu Arg Thr Gln Glu Val Thr Ser Arg Ile Arg
1160 1165 1170

acc cag agc ttt tcc ttg cag gag cgt cag ttg cgg ggc gca gtg 3863
Thr Gln Ser Phe Ser Leu Gln Glu Arg Gln Leu Arg Gly Ala Val
1175 1180 1185

ccc tgg gcg ttc gac cct ccc ggc tca gac acc aac agc ccc tga 3908
Pro Trp Ala Phe Asp Pro Pro Gly Ser Asp Thr Asn Ser Pro
1190 1195 1200

gagccgcctg gctttccctt ccagttccgg gagagcggct gcccgactca ggtccgcccg 3968

accaggatca gccccgctcc tcccctcttg aggtggtgcc ttctcacatc tgtccagagg 4028

ctgcaaggat tcagcattat tcctccagga aggagcaaaa cgcctctttt ccctctctag 4088

gcctgttgcc tcgggcctgg gtccgcctta atctggaagg cccctcccag cagcggtacc 4148

ccagggccta ctgccacccg cttcctgttt cttagtcgaa tgttagattc ctcttgcctc 4208

tctcaggagt atcttacctg taaagtctaa tctctaaatc aagtatttat tattgaagat 4268

ttaccataag ggactgtgcc agatgttagg agaactacta aagtgcctac cccagctcat 4328

gtggattaca aaaaaaa 4345

<210> 4
<211> 1203
<212> PRT
<213> Homo sapiens

<400> 4

Met Gly Asn Leu Lys Ser Val Ala Gln Glu Pro Gly Pro Pro Cys Gly
1               5                   10                  15

Leu Gly Leu Gly Leu Gly Leu Gly Leu Cys Gly Lys Gln Gly Pro Ala
            20                  25                  30

Thr Pro Ala Pro Glu Pro Ser Arg Ala Pro Ala Ser Leu Leu Pro Pro
        35                  40                  45

Ala Pro Glu His Ser Pro Pro Ser Ser Pro Leu Thr Gln Pro Pro Glu
    50                  55                  60

Gly Pro Lys Phe Pro Arg Val Lys Asn Trp Glu Val Gly Ser Ile Thr
65 70 75 80

Tyr Asp Thr Leu Ser Ala Gln Ala Gln Gln Asp Gly Pro Cys Thr Pro
85 90 95

Arg Arg Cys Leu Gly Ser Leu Val Phe Pro Arg Lys Leu Gln Gly Arg
100 105 110

Pro Ser Pro Gly Pro Pro Ala Pro Glu Gln Leu Leu Ser Gln Ala Arg
115 120 125

Asp Phe Ile Asn Gln Tyr Tyr Ser Ser Ile Lys Arg Ser Gly Ser Gln
130 135 140

Ala His Glu Gln Arg Leu Gln Glu Val Glu Ala Glu Val Ala Ala Thr
145 150 155 160

Gly Thr Tyr Gln Leu Arg Glu Ser Glu Leu Val Phe Gly Ala Lys Gln
165 170 175

Ala Trp Arg Asn Ala Pro Arg Cys Val Gly Arg Ile Gln Trp Gly Lys
180 185 190

Leu Gln Val Phe Asp Ala Arg Asp Cys Arg Ser Ala Gln Glu Met Phe
195 200 205

Thr Tyr Ile Cys Asn His Ile Lys Tyr Ala Thr Asn Arg Gly Asn Leu
210 215 220

Arg Ser Ala Ile Thr Val Phe Pro Gln Arg Cys Pro Gly Arg Gly Asp
225 230 235 240

Phe Arg Ile Trp Asn Ser Gln Leu Val Arg Tyr Ala Gly Tyr Arg Gln
245 250 255

Gln Asp Gly Ser Val Arg Gly Asp Pro Ala Asn Val Glu Ile Thr Glu
260 265 270

Leu Cys Ile Gln His Gly Trp Thr Pro Gly Asn Gly Arg Phe Asp Val
275 280 285

Leu Pro Leu Leu Leu Gln Ala Pro Asp Asp Pro Pro Glu Leu Phe Leu
290 295 300

Leu Pro Pro Glu Leu Val Leu Glu Val Pro Leu Glu His Pro Thr Leu
305 310 315 320

Glu Trp Phe Ala Ala Leu Gly Leu Arg Trp Tyr Ala Leu Pro Ala Val
325 330 335

Ser Asn Met Leu Leu Glu Ile Gly Gly Leu Glu Phe Pro Ala Ala Pro
340 345 350

Phe Ser Gly Trp Tyr Met Ser Thr Glu Ile Gly Thr Arg Asn Leu Cys
355 360 365

Asp Pro His Arg Tyr Asn Ile Leu Glu Asp Val Ala Val Cys Met Asp
370 375 380

Leu Asp Thr Arg Thr Thr Ser Ser Leu Trp Lys Asp Lys Ala Ala Val
385 390 395 400

Glu Ile Asn Val Ala Val Leu His Ser Tyr Gln Leu Ala Lys Val Thr
405 410 415

Ile Val Asp His His Ala Ala Thr Ala Ser Phe Met Lys His Leu Glu
420 425 430

Asn Glu Gln Lys Ala Arg Gly Gly Cys Pro Ala Asp Trp Ala Trp Ile
435 440 445

Val Pro Pro Ile Ser Gly Ser Leu Thr Pro Val Phe His Gln Glu Met
450 455 460

Val Asn Tyr Phe Leu Ser Pro Ala Phe Arg Tyr Gln Pro Asp Pro Trp
465 470 475 480

Lys Gly Ser Ala Ala Lys Gly Thr Gly Ile Thr Arg Lys Lys Thr Phe
485 490 495

Lys Glu Val Ala Asn Ala Val Lys Ile Ser Ala Ser Leu Met Gly Thr
500 505 510

Val Met Ala Lys Arg Val Lys Ala Thr Ile Leu Tyr Gly Ser Glu Thr
515 520 525

Gly Arg Ala Gln Ser Tyr Ala Gln Gln Leu Gly Arg Leu Phe Arg Lys
530 535 540

Ala Phe Asp Pro Arg Val Leu Cys Met Asp Glu Tyr Asp Val Val Ser
545 550 555 560

Leu Glu His Glu Thr Leu Val Leu Val Val Thr Ser Thr Phe Gly Asn
565 570 575

Gly Asp Pro Pro Glu Asn Gly Glu Ser Phe Ala Ala Ala Leu Met Glu
580 585 590

Met Ser Gly Pro Tyr Asn Ser Ser Pro Arg Pro Glu Gln His Lys Ser
595 600 605

Tyr Lys Ile Arg Phe Asn Ser Ile Ser Cys Ser Asp Pro Leu Val Ser
610 615 620

Ser Trp Arg Arg Lys Arg Lys Glu Ser Ser Asn Thr Asp Ser Ala Gly
625 630 635 640

Ala Leu Gly Thr Leu Arg Phe Cys Val Phe Gly Leu Gly Ser Arg Ala
645 650 655

Tyr Pro His Phe Cys Ala Phe Ala Arg Ala Val Asp Thr Arg Leu Glu
660 665 670

Glu Leu Gly Gly Glu Arg Leu Leu Gln Leu Gly Gln Gly Asp Glu Leu
675 680 685

Cys Gly Gln Glu Glu Ala Phe Arg Gly Trp Ala Gln Ala Ala Phe Gln
690 695 700

Ala Ala Cys Glu Thr Phe Cys Val Gly Glu Asp Ala Lys Ala Ala Ala
705 710 715 720

Arg Asp Ile Phe Ser Pro Lys Arg Ser Trp Lys Arg Gln Arg Tyr Arg
725 730 735

Leu Ser Ala Gln Ala Glu Gly Leu Gln Leu Leu Pro Gly Leu Ile His
740 745 750

Val His Arg Arg Lys Met Phe Gln Ala Thr Ile Arg Ser Val Glu Asn
755 760 765

Leu Gln Ser Ser Lys Ser Thr Arg Ala Thr Ile Leu Val Arg Leu Asp
770 775 780

Thr Gly Gly Gln Glu Gly Leu Gln Tyr Gln Pro Gly Asp His Ile Gly
785 790 795 800

Val Cys Pro Pro Asn Arg Pro Gly Leu Val Glu Ala Leu Leu Ser Arg
805 810 815

Val Glu Asp Pro Pro Ala Pro Thr Glu Pro Val Ala Val Glu Gln Leu
820 825 830

Glu Lys Gly Ser Pro Gly Gly Pro Pro Pro Gly Trp Val Arg Asp Pro

835 840 845

Arg Leu Pro Pro Cys Thr Leu Arg Gln Ala Leu Thr Phe Phe Leu Asp
850 855 860

Ile Thr Ser Pro Pro Ser Pro Gln Leu Leu Arg Leu Leu Ser Thr Leu
865 870 875 880

Ala Glu Glu Pro Arg Glu Gln Gln Glu Leu Glu Ala Leu Ser Gln Asp
885 890 895

Pro Arg Arg Tyr Glu Glu Trp Lys Trp Phe Arg Cys Pro Thr Leu Leu
900 905 910

Glu Val Leu Glu Gln Phe Pro Ser Val Ala Leu Pro Ala Pro Leu Leu
915 920 925

Leu Thr Gln Leu Pro Leu Leu Gln Pro Arg Tyr Tyr Ser Val Ser Ser
930 935 940

Ala Pro Ser Thr His Pro Gly Glu Ile His Leu Thr Val Ala Val Leu
945 950 955 960

Ala Tyr Arg Thr Gln Asp Gly Leu Gly Pro Leu His Tyr Gly Val Cys
965 970 975

Ser Thr Trp Leu Ser Gln Leu Lys Pro Gly Asp Pro Val Pro Cys Phe
980 985 990

Ile Arg Gly Ala Pro Ser Phe Arg Leu Pro Pro Asp Pro Ser Leu Pro
995 1000 1005

Cys Ile Leu Val Gly Pro Gly Thr Gly Ile Ala Pro Phe Arg Gly
1010 1015 1020

Phe Trp Gln Glu Arg Leu His Asp Ile Glu Ser Lys Gly Leu Gln
1025 1030 1035

Pro Thr Pro Met Thr Leu Val Phe Gly Cys Arg Cys Ser Gln Leu
1040 1045 1050

Asp His Leu Tyr Arg Asp Glu Val Gln Asn Ala Gln Gln Arg Gly
1055 1060 1065

Val Phe Gly Arg Val Leu Thr Ala Phe Ser Arg Glu Pro Asp Asn
1070 1075 1080

Pro Lys Thr Tyr Val Gln Asp Ile Leu Arg Thr Glu Leu Ala Ala
1085 1090 1095

Glu Val His Arg Val Leu Cys Leu Glu Arg Gly His Met Phe Val
1100 1105 1110

Cys Gly Asp Val Thr Met Ala Thr Asn Val Leu Gln Thr Val Gln
1115 1120 1125

Arg Ile Leu Ala Thr Glu Gly Asp Met Glu Leu Asp Glu Ala Gly
1130 1135 1140

Asp Val Ile Gly Val Leu Arg Asp Gln Gln Arg Tyr His Glu Asp
1145 1150 1155

Ile Phe Gly Leu Thr Leu Arg Thr Gln Glu Val Thr Ser Arg Ile
1160 1165 1170

Arg Thr Gln Ser Phe Ser Leu Gln Glu Arg Gln Leu Arg Gly Ala
1175 1180 1185

Val Pro Trp Ala Phe Asp Pro Pro Gly Ser Asp Thr Asn Ser Pro
1190 1195 1200

<210> 5
<211> 22
<212> DNA
<213> Artificial Sequence

<220>
<223> Synthetic primer

<400> 5
aaggcaggag acagtggatg ga 22

<210> 6
<211> 24
<212> DNA
<213> Artificial Sequence

<220>
<223> Synthetic primer

<400> 6
cccagtcaat ccctttggtg ctca 24

<210> 7
<211> 22
<212> DNA
<213> Artificial Sequence

<220>
<223> Synthetic primer

<400> 7
tggagagtgc tggtgtaccc ca 22

<210> 8
<211> 20
<212> DNA
<213> Artificial Sequence

<220>
<223> Synthetic primer

<400> 8
gcctccaccc ccaccctgtc 20

<210> 9
<211> 21
<212> DNA
<213> Artificial Sequence

<220>
<223> Synthetic primer

<400> 9
aggccctatg gtagtgcctt t 21

<210> 10
<211> 20
<212> DNA
<213> Artificial Sequence

<220>
<223> Synthetic primer

<400> 10
tctcttagtg ctgtggtcac 20